(19) Europäisches Patentamt
European Patent Office
Office européen des brevets

(11) EP 3 054 982 B1

(12) **EUROPEAN PATENT SPECIFICATION**

(45) Date of publication and mention of the grant of the patent:
**01.05.2019 Bulletin 2019/18**

(21) Application number: **14784022.7**

(22) Date of filing: **07.10.2014**

(51) Int Cl.:
*A61K 47/60* (2017.01) *A61K 47/69* (2017.01)
*A61P 19/02* (2006.01)

(86) International application number:
**PCT/EP2014/071385**

(87) International publication number:
**WO 2015/052154 (16.04.2015 Gazette 2015/15)**

(54) **HYDROGEL-LINKED IL-1RA PRODRUG**

HYDROGEL-KONJUGIERTES IL-1RA PRODRUG

PROMÉDICAMENT DE IL-1RA LIÉ À UN HYDROGEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2013 EP 13187766**

(43) Date of publication of application:
**17.08.2016 Bulletin 2016/33**

(73) Proprietor: **Ascendis Pharma A/S**
**2900 Hellerup (DK)**

(72) Inventors:
- **RAU, Harald**
  **69221 Dossenheim (DE)**
- **ZETTLER, Joachim**
  **69126 Heidelberg (DE)**
- **HERSEL, Ulrich**
  **69121 Heidelberg (DE)**
- **KALUZA, Nora**
  **80698 Munich (DE)**
- **KRUSCH, Mathias**
  **69434 Hirschhorn (DE)**
- **VOIGT, Tobias**
  **64625 Bensheim (DE)**

(74) Representative: **Büchel, Edwin**
**Patentanwälte**
**Isenbruck Bösl Hörschler PartG mbB**
**Eastsite One**
**Seckenheimer Landstraße 4**
**68163 Mannheim (DE)**

(56) References cited:
**WO-A1-97/28828 WO-A1-2011/012715**
**WO-A1-2011/012718 WO-A1-2013/053856**
**WO-A1-2014/056915 WO-A2-2009/095479**

- **AKASH MUHAMMAD SAJID HAMID ET AL: "IL-1Ra and its Delivery Strategies: Inserting the Association in Perspective", PHARMACEUTICAL RESEARCH (DORDRECHT), vol. 30, no. 11, 22 June 2013 (2013-06-22) , pages 2951-2966, XP002734194, ISSN: 0724-8741**
- **JOHANNE MARTEL-PELLETIER ET AL: "OsteoarthrItIs A single injection of anakinra for treating knee OA?", NATURE REVIEWS RHEUMATOLOGY, vol. 5, no. 7, 1 July 2009 (2009-07-01), pages 363-364, XP055393825, GB ISSN: 1759-4790, DOI: 10.1038/nrrheum.2009.120**
- **WHITMIRE RACHEL E ET AL: "Self-assembling nanoparticles for intra-articular delivery of anti-inflammatory proteins", BIOMATERIALS, vol. 33, no. 30, October 2012 (2012-10), pages 7665-7675, ISSN: 0142-9612**
- **KIMMERLING K A ET AL: "SUSTAINED INTRA-ARTICULAR DELIVERY OF IL-1RA FROM A THERMALLY-RESPONSIVE ELASTIN-LIKE POLYPEPTIDE AS A THERAPY FOR POST-TRAUMATIC ARTHRITIS", EUROPEAN CELLS & MATERIALS, vol. 29, January 2015 (2015-01), pages 124-140, ISSN: 1473-2262**
- **ELSAID K. A. ET AL: "Intra-articular interleukin-1 receptor antagonist (IL1-ra) microspheres for posttraumatic osteoarthritis: in vitro biological activity and in vivo disease modifying effect", J. EXPER. ORTHOPAEDICS, vol. 3, no. 18, 2016, pages 1-10,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 054 982 B1

- BENDELE ALISON ET AL: "Effects of interleukin-1 receptor antagonist in a slow-release hylan vehicle on rat type II collagen arthritis", PHARMACEUTICAL RESEARCH (NEW YORK), vol. 15, no. 10, October 1998 (1998-10), pages 1557-1561, ISSN: 0724-8741
- LAVI GALIA ET AL: "Sustained delivery of IL-1Ra from biodegradable microspheres reduces the number of murine B16 melanoma lung metastases", JOURNAL OF CONTROLLED RELEASE, vol. 123, no. 2, November 2007 (2007-11), pages 123-130, ISSN: 0168-3659(print)

## Description

The present invention relates to a hydrogel-linked IL-1ra prodrug for use in a method of treating an inflammatory condition of the joint via intra-articular administration.

The interleukin-1 receptor antagonist (IL-1ra) is a protein that in humans is encoded by the IL1RN gene. IL-1ra is a member of the interleukin 1 cytokine family and is an agent that binds to the cell surface interleukin-1 receptor (IL-1R). IL-1R is the same receptor that binds interleukin 1 alpha (IL-1 alpha) and interleukin 1 beta (IL-1 beta). IL-1ra inhibits the binding of IL-1 alpha and IL-1 beta to IL-1R, and modulates a variety of interleukin 1-related immune and inflammatory responses. An interleukin 1 receptor antagonist (Anakinra) is used in the treatment of rheumatoid arthritis, an autoimmune disease in which IL-1 plays a key role. It is commercially produced as Kineret®, which is a human recombinant form of IL-1ra.

The anakinra molecule is a recombinant, non-glycosylated version of human IL-1ra prepared from cultures of genetically modified *Escherichia coli* using recombinant DNA technology. It consists of 153 amino acids and has a molecular weight of 17.3 kDa and differs from native human IL-1ra sequence in that it has the addition of a single methionine residue on its amino terminus.

Anakinra had an absolute bioavailability of 95% after subcutaneous (s.c.) bolus injection. Peak plasma concentrations of anakinra generally occurred 3 to 7 hours after s.c. administration of clinically relevant doses (1 to 2 mg/kg). The terminal half-life ranged from 4 to 6 hours. After daily s.c. dosing for up to 24 weeks, no unexpected accumulations of anakinra were observed in the plasma samples of rheumatoid arthritis patients.

Anakinra is sold under the trade name Kineret® and is produced by the pharmaceutical company Amgen. It is delivered as an injection concentrate containing 100 mg for each single dose.

Due to its comparatively low molecular weight of 17.3 kDa, IL-1ra is rapidly removed from blood circulation by renal clearance. Therefore, in therapeutic applications, IL-1ra has to be administered by subcutaneous injection on a daily basis in relatively high dose (100 mg). But even at a daily frequency of injection, plasma levels of IL-1ra exhibit maximal peak to trough fluctuation, as no accumulation in plasma is observed. Furthermore, daily injections of high dose IL-1ra diminish patient compliance and cause side effects like local tolerability issues or increased risk of infections. Therefore, there is a need for IL-1ra delivery technologies to provide for extended therapeutic levels of IL-1ra.

Furthermore, it is of interest to apply long-acting IL-1ra in a localized fashion to certain body compartments, organs or tissues. This has been described in WO-A 98/22130. The need for long-acting IL-1ra was exemplified by the failure of IL-1ra to produce sustained relief for osteoarthritis patients after intra-articular injection. Lack of efficacy was mainly related to the short half-life of IL-1ra in the synovial fluid ("Intraarticular Injection of Anakinra in Osteoarthritis of the Knee: A Multicenter, Randomized, Double-Blind, Placebo-Controlled Study"; Chevalier et al, Arthritis & Rheumatism Vol. 61, No. 3 (2009), 344-352).

Various approaches have been taken to provide for long-acting IL-1ra.

For instance, IL-1ra was formulated with hyaluronic acid (HA) and the corresponding formulation exhibited slower release of IL-1ra into the bloodstream and maintained therapeutic blood levels of IL-1ra for a longer time compared to a formulation not containing HA polymers ("Effects of interleukin-1 receptor antagonist in a slow-release hylan vehicle on rat type II collagen arthritis"; Bendele A., McAbee T., Woodward M., Scherrer J., Collins D., Frazier J., Chlipala E. and McCabe D.; Pharm Res 15(10):1557-61 (1998)). In the rats studied, IL-1ra levels were below 0.1 $\mu$g/mL 12 hours post dosing. When IL-1ra was given in the 2% HA formulation, blood levels were above 0.2 $\mu$g/mL for the last 12 hours of the 24 hours dosing period. Still, this effect results in only a modest extension of the short half-life of IL-1ra and is not suitable for a twice or once weekly dosage form.

Very similar data were disclosed in WO-A 97/28828 and US-A 2002/009454. These documents detail compositions and methods for treating inflammatory diseases using hyaluronan-based controlled release polymer formulations comprising IL-1ra.

Sustained-release delayed gels are described in US-A 2001/0007673, where slow release formulations are based on thixotropic alginate gels, protein drug and at least one bound polyvalent metal ion. The rate of gelation is controlled by the free calcium level. These mixtures can be placed in the body where they can gel after injection. Extended release profiles for IL1-ra formulations were claimed but not exemplified.

IL-1ra was delivered from microencapsulated genetically engineered cells, which overexpress and secrete IL-1ra ("A continuous delivery system of IL-1 receptor antagonist reduces angiogenesis and inhibits tumor development"; Bar D., Apte R. N., Voronov E., Dinarello C. A. and Cohen S.; FASEB J 18(1):161-3 (2004)). No pharmacokinetic data for IL-1ra release are available from this publication.

EP-A 0 975 334 claims a method for preparing polymeric microparticles containing IL-1ra through unique utilization of direct lyophilization of an emulsion or suspension. IL-1ra particles were prepared by spray-drying but not studied for their release kinetics.

IL-1ra was encapsulated with stabilizers in biodegradable poly-(lactic/glycolic acid) (PLGA) microspheres. In vitro cytokine release and bioactivity studies in cultured melanoma B16 cells revealed the microspheres to be capable

of sustained IL-1ra release on a daily level (Lavi G., Voronov E., Dinarello C. A., Apte R. N. and Cohen S.; J Control Release 123(2):123-30 (2007)). In vivo, the sustained release of IL-1ra from PLGA microspheres was proven by following IL-1ra levels in plasma over two weeks. Still, plasma levels dropped to 1/100 of the maximal concentration during the second day post dosing, indicative of the burst release typical for PLGA formulations.

Biodegradable polyacetal derivatives and corresponding IL-1ra conjugates are described in US-A 2008/019940. Here, the protein drug was coupled to a water soluble polyacetal and thereby permanently modified. Furthermore, both coupling and in vivo hydrolysis of the polyacetal give rise to an ill-defined mixture of IL-1ra-containing degradation products, making it difficult to assess safety and pharmacological effects in a reproducible fashion.

A problem frequently encountered in the development of slow-release formulations of IL-1ra is the tendency of the molecule to form aggregates (WO-A 2005/097195). Such aggregates may cause undesired immunogenic responses upon administration and may result in lower bioavailability and ill-defined release kinetics.

Therefore, an object of the present invention is to develop long-acting IL-1ra which at least partially overcomes the before mentioned shortcomings.

This object is achieved with a hydrogel-linked IL-1ra prodrug or pharmaceutically acceptable salt thereof for use in a method of treating an inflammatory condition of the joint via intra-articular administration, of the formula L - D, wherein

(i) - D is an IL-1ra moiety;
and

(ii) -L comprises a reversible prodrug linker moiety $-L^1$ represented by formula (I),

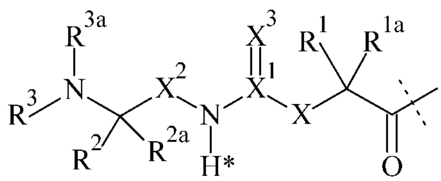 (I)

,

wherein the dashed line indicates the attachment to a nitrogen of D by forming an amide bond;

X is $C(R^4R^{4a})$; $N(R^4)$; O; $C(R^4R^{4a})-C(R^5R^{5a})$; $C(R^5R^{5a})-C(R^4R^{4a})$; $C(R^4R^{4a})-N(R^6)$; $N(R^6)-C(R^4R^{4a})$; $C(R^4R^{4a})-O$; $O-C(R^4R^{4a})$; or $C(R^7R^{7a})$;

$X^1$ is C; or S(O);

$X^2$ is $C(R^8R^{8a})$; or $C(R^8R^{8a})-C(R^9R^{9a})$;

$X^3$ is O; S; or N-CN;

$R^1$, $R^{1a}$, $R^2$, $R^{2a}$, $R^3$, $R^{3a}$, $R^4$, $R^{4a}$, $R^5$, $R^{5a}$, $R^6$, $R^8$, $R^{8a}$, $R^9$, $R^{9a}$ are independently selected from the group consisting of H; and $C_{1-6}$ alkyl;

$R^7$ is $N(R^{10}R^{10a})$; or $NR^{10}-(C=O)-R^{11}$;

$R^{7a}$, $R^{10}$, $R^{10a}$, $R^{11}$ are independently of each other H; or $C_{1-6}$ alkyl;

Optionally, one or more of the pairs $R^{1a}/R^{4a}$, $R^{1a}/R^{5a}$, $R^{1a}/R^{7a}$, $R^{4a}/R^{5a}$, $R^{8a}/R^{9a}$ form a chemical bond;

Optionally, one or more of the pairs $R^1/R^{1a}$, $R^2/R^{2a}$, $R^4/R^{4a}$, $R^5/R^{5a}$, $R^8/R^{8a}$, $R^9/R^{9a}$ are joined together with the atom to which they are attached to form a $C_{3-7}$ cycloalkyl; or 4- to 7-membered heterocyclyl;

Optionally, one or more of the pairs $R^1/R^4$, $R^1/R^5$, $R^1/R^6$, $R^1/R^{7a}$, $R^4/R^5$, $R^4/R^6$, $R^8/R^9$, $R^2/R^3$ are joined together with the atoms to which they are attached to form a ring A;

Optionally, $R^3/R^{3a}$ are joined together with the nitrogen atom to which they are attached to form a 4 to 7 membered heterocycle;

A is selected from the group consisting of phenyl; naphthyl; indenyl; indanyl; tetralinyl; $C_{3-10}$ cycloalkyl; 4- to 7-membered heterocyclyl; and 9- to 11-membered heterobicyclyl; and

wherein $L^1$ is substituted with one group $L^2$-Z and wherein $L^1$ is optionally further substituted, provided that the hydrogen marked with the asterisk in formula (I) is not replaced by $L^2$-Z or a substituent and that $R^3$ and $R^{3a}$ are independently of each other H or are connected to N through an $SP^3$-hybridized carbon atom;
wherein

$L^2$ is a single chemical bond or a spacer; and
Z is a hydrogel.

It was now surprisingly discovered that a hydrogel-linked IL-1ra prodrug or pharmaceutically acceptable salt thereof of the present invention provide sustained IL-1ra release from a subcutaneous or locally applied depot and can thus overcome at least some of the above-mentioned shortcomings.

Within the present invention the terms are used having the meaning as follows.

As used herein, the term "hydrogel" means a hydrophilic or amphiphilic polymeric network composed of homopolymers or copolymers, which is insoluble due to the presence of covalent chemical crosslinks. The crosslinks provide the network structure and physical integrity.

As used herein, the term "reagent" means a chemical compound which comprises at least one functional group for reaction with the functional group of another reagent or moiety.

As used herein, the term "backbone reagent" means a reagent, which is suitable as a starting material for forming hydrogels. As used herein, a backbone reagent preferably does not comprise biodegradable linkages. A backbone reagent may comprise a "branching core" which refers to an atom or moiety to which more than one other moiety is attached.

As used herein, the term "crosslinker reagent" means a linear or branched reagent, which is suitable as a starting material for crosslinking backbone reagents. Preferably, the crosslinker reagent is a linear chemical compound. Preferably, a crosslinker reagent comprises at least two biodegradable linkages.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-", whereas each "-" indicates attachment to another moiety. Accordingly, a biologically active moiety is released from a prodrug as a drug, i.e. an IL-1ra moiety is released from the hydrogel-linked IL-1ra prodrug of the present invention as IL-1ra.

Accordingly, the phrase "in bound form" is used to refer to the corresponding moiety of a reagent, i.e. "lysine in bound form" refers to a lysine moiety which lacks one or more atom(s) of the lysine reagent and is part of a molecule.

As used herein, the term "functional group" means a group of atoms which can react with other functional groups. Functional groups include the following groups: carboxylic acid (-(C=O)OH), primary or secondary amine ($-NH_2$, -NH-), maleimide, thiol (-SH), sulfonic acid (-(O=S=O)OH), carbonate, carbamate (-O(C=O)N<), hydroxy (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, vinyl sulfone, vinyl ketone, diazoalkane, oxirane, and aziridine.

As used herein, the term "activated functional group" means a functional group, which is connected to an activating group, i.e. a functional group was reacted with an activating reagent. Preferred activated functional groups include activated ester groups, activated carbamate groups, activated carbonate groups and activated thiocarbonate groups. Preferred activating groups are selected from the group consisting of formulas ((f-i) to (f-vi):

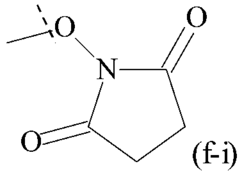 (f-i) , 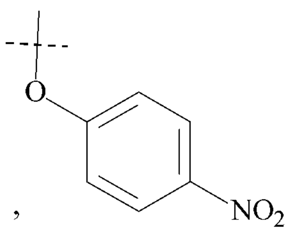 (f-ii) , 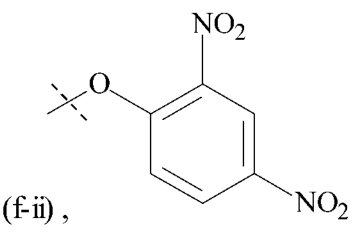 (f-iii),

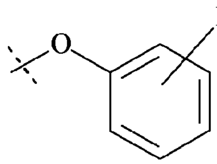 (f-iv), 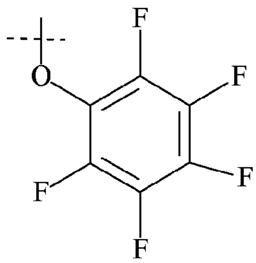 (f-v) and 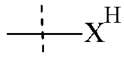 (f-vi)

wherein

the dashed lines indicate attachment to the rest of the molecule;
b is 1, 2, 3 or 4; and
$X^H$ is Cl, Br, I, or F.

Accordingly, a preferred activated ester has the formula

$$-(C=O)-Y^1,$$

wherein
$Y^1$ is selected from the group consisting of formulas (f-i), (f-ii), (f-iii), (f-iv), (f-v) and (f-vi).

Accordingly, a preferred activated carbamate has the formula

$$-N-(C=O)-Y^1,$$

wherein
$Y^1$ is selected from the group consisting of formulas (f-i), (f-ii), (f-iii), (f-iv), (f-v) and (f-vi).

Accordingly, a preferred activated carbonate has the formula

$$-O-(C=O)-Y^1,$$

wherein
$Y^1$ is selected from the group consisting of formulas (f-i), (f-ii), (f-iii), (f-iv), (f-v) and (f-vi).

Accordingly, a preferred activated thiocarbonate has the formula

$$-S-(C=O)-Y^1,$$

wherein
$Y^1$ is selected from the group consisting of formulas (f-i), (f-ii), (f-iii), (f-iv), (f-v) and (f-vi).

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. It is understood that a polymer may for example also comprise functional groups or capping moieties. Preferably, a polymer has a molecular weight of at least 0.5 kDa, e.g. a molecular weight of at least 1 kDa, a molecular weight of at least 2 kDa, a molecular weight of at least 3 kDa or a molecular weight of at least 5 kDa.

As used herein, the term "polymeric" means a reagent or a moiety comprising one or more polymer(s).

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers. As used herein, the term "number average molecular weight" means the ordinary arithmetic means of the molecular weights of the individual polymers.

As used herein, the term "polymerization" or "polymerizing" means the process of reacting monomer or macromonomer reagents in a chemical reaction to form polymer chains or networks, including hydrogels.

As used herein, the term "macromonomer" means a molecule that was obtained from the polymerization of monomer reagents.

As used herein, the term "condensation polymerization" or "condensation reaction" means a chemical reaction,

in which the functional groups of two reagents react to form one single molecule, i.e. the reaction product, and a low molecular weight molecule, for example water, is released.

As used herein, the term "suspension polymerization" means a heterogeneous and/or biphasic polymerization reaction, wherein the monomer reagents are dissolved in a first solvent, forming the disperse phase which is emulsified in a second solvent, forming the continuous phase. In the present invention, the monomer reagents are the at least one backbone reagent and the at least one crosslinker reagent. Both the first solvent and the monomer reagents are not soluble in the second solvent. Such emulsion is formed by stirring, shaking, exposure to ultrasound or Microsieve™ emulsification, more preferably by stirring or Microsieve™ emulsification and more preferably by stirring. This emulsion is stabilized by an appropriate emulsifier. The polymerization is initiated by addition of a base as initiator which is soluble in the first solvent. A suitable commonly known base suitable as initiator may be a tertiary base, such as tetramethylethylenediamine (TMEDA).

As used herein, the term "immiscible" means the property where two substances are not capable of combining to form a homogeneous mixture.

As used herein, the term "polyamine" means a reagent or moiety comprising more than one amine (-NH- and/or $-NH_2$), e.g. from 2 to 64 amines, from 4 to 48 amines, from 6 to 32 amines, from 8 to 24 amines, or from 10 to 16 amines. Particularly preferred polyamines comprise from 2 to 32 amines.

As used herein, the term "PEG-based comprising at least X% PEG" in relation to a moiety or reagent means that said moiety or reagent comprises at least X% (w/w) ethylene glycol units ($-CH_2CH_2O-$), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties especially selected from the following substituents and linkages:

- $C_{1-50}$ alkyl, $C_{2-50}$ alkenyl, $C_{2-50}$ alkynyl, $C_{3-10}$ cycloalkyl, 4- to 7-membered heterocyclyl, 8- to 11-membered heterobicyclyl, phenyl; naphthyl; indenyl; indanyl; and tetralinyl; and
- linkages selected from the group comprising

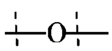, 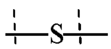, 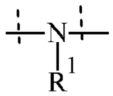, 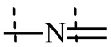, 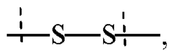, 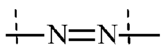,

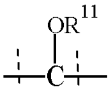, 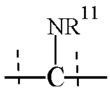, 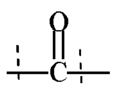, 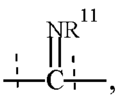, 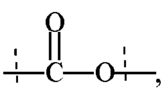, 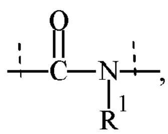,

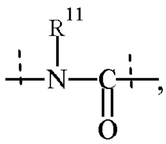, 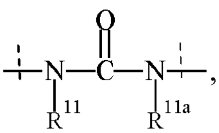, 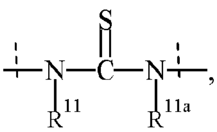, 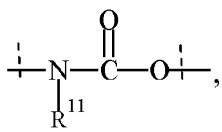,

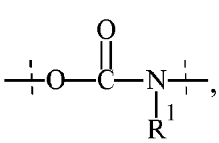, 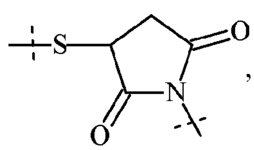, and 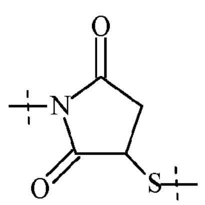,

wherein
dashed lines indicate attachment to the remainder of the moiety or reagent, and $R^{11}$ and $R^{11a}$ are independently of each other selected from H and $C_{1-6}$ alkyl.

As used herein, the term "$C_{1-4}$ alkyl" alone or in combination means a straight-chain or branched alkyl group having 1 to 4 carbon atoms. If present at the end of a molecule, examples of straight-chain and branched $C_{1-4}$ alkyl groups are methyl, ethyl, n-propyl, isopropyl, n-butyl, isobutyl, sec-butyl and tert-butyl. When two moieties of a molecule

are linked by the $C_{1-4}$ alkyl group, then examples for such $C_{1-4}$ alkyl groups are $-CH_2-$, $-CH_2-CH_2-$, $-CH(CH_3)-$, $-CH_2-CH_2-CH_2-$, $-CH(C_2H_5)-$, $-C(CH_3)_2-$, $-CH_2-CH_2-CH_2-CH_2-$, and $-CH_2-CH_2-CH_2(CH_3)-$. Each hydrogen atom of a $C_{1-4}$ alkyl group may be replaced by a substituent as defined below.

As used herein, the term "$C_{1-6}$ alkyl" alone or in combination means a straight-chain or branched alkyl group having 1 to 6 carbon atoms. If present at the end of a molecule, examples of straight-chain and branched $C_{1-6}$ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the $C_{1-6}$ alkyl group, then examples for such $C_{1-6}$ alkyl groups are $-CH_2-$, $-CH_2-CH_2-$, $-CH(CH_3)-$, $-CH_2-CH_2-CH_2-$, $-CH(C_2H_5)-$ and $-C(CH_3)_2-$. Each hydrogen atom of a $C_{1-6}$ alkyl group may be replaced by a substituent as defined below.

Accordingly, as used herein, the term "$C_{1-20}$ alkyl" alone or in combination means a straight-chain or branched alkyl group having 1 to 20 carbon atoms. The term "$C_{8-18}$ alkyl" alone or in combination means a straight-chain or branched alkyl group having 8 to 18 carbon atoms. Accordingly, as used herein, the term "$C_{1-50}$ alkyl" alone or in combination means a straight-chain or branched alkyl group having 1 to 50 carbon atoms. Each hydrogen atom of a $C_{1-20}$ alkyl group, a $C_{8-18}$ alkyl group and $C_{1-50}$ alkyl group may be replaced by a substituent. In each case the alkyl group may be present at the end of a molecule or two moieties of a molecule may be linked by the alkyl group.

As used herein, the term "$C_{2-6}$ alkenyl" alone or in combination means a straight-chain or branched hydrocarbon moiety comprising at least one carbon-carbon double bond having 2 to 6 carbon atoms. If present at the end of a molecule, examples are $-CH=CH_2$, $-CH=CH-CH_3$, $-CH_2-CH=CH_2$, $-CH=CHCH_2-CH_3$ and $-CH=CH-CH=CH_2$. When two moieties of a molecule are linked by the $C_{2-6}$ alkenyl group, then an example for such $C_{2-6}$ alkenyl is $-CH=CH-$. Each hydrogen atom of a $C_{2-6}$ alkenyl group may be replaced by a substituent as defined below.

Accordingly, as used herein, the term "$C_{2-20}$ alkenyl" alone or in combination means a straight-chain or branched hydrocarbon residue comprising at least one carbon-carbon double bond having 2 to 20 carbon atoms. The term "$C_{2-50}$ alkenyl" alone or in combination means a straight-chain or branched hydrocarbon residue comprising at least one carbon-carbon double bond having 2 to 50 carbon atoms. If present at the end of a molecule, examples are $-CH=CH_2$, $-CH=CH-CH_3$, $-CH_2-CH=CH_2$, $-CH=CHCH_2-CH_3$ and $-CH=CH-CH=CH_2$. When two moieties of a molecule are linked by the alkenyl group, then an example is e.g. $-CH=CH-$. Each hydrogen atom of a $C_{2-20}$ alkenyl or $C_{2-50}$ alkenyl group may be replaced by a substituent as defined below.

As used herein, the term "$C_{2-6}$ alkynyl" alone or in combination means straight-chain or branched hydrocarbon residue comprising at least one carbon-carbon triple bond having 2 to 6 carbon atoms. If present at the end of a molecule, examples are $-C\equiv CH$, $-CH_2-C\equiv CH$, $CH_2-CH_2-C\equiv CH$ and $CH_2-C\equiv C-CH_3$. When two moieties of a molecule are linked by the alkynyl group, then an example is: $-C\equiv C-$. Each hydrogen atom of a $C_{2-6}$ alkynyl group may be replaced by a substituent as defined below. Optionally, one or more double bond(s) may occur.

Accordingly, as used herein, the term "$C_{2-20}$ alkynyl" alone or in combination means a straight-chain or branched hydrocarbon residue comprising at least one carbon-carbon triple bond having 2 to 20 carbon atoms and "$C_{2-50}$ alkynyl" alone or in combination means a straight-chain or branched hydrocarbon residue comprising at least one carbon-carbon triple bond having 2 to 50 carbon atoms. If present at the end of a molecule, examples are $-C\equiv CH$, $-CH_2-C\equiv CH$, $CH_2-CH_2-C\equiv CH$ and $CH_2-C\equiv C-CH_3$. When two moieties of a molecule are linked by the alkynyl group, then an example is $-C\equiv C-$. Each hydrogen atom of a $C_{2-20}$ alkynyl or $C_{2-50}$ alkynyl group may be replaced by a substituent as defined below. Optionally, one or more double bond(s) may occur.

As used herein, the terms "$C_{3-8}$ cycloalkyl" or "$C_{3-8}$ cycloalkyl ring" means a cyclic alkyl chain having 3 to 8 carbon atoms, which may be saturated or unsaturated, e.g. cyclopropyl, cyclobutyl, cyclopentyl, cyclohexyl, cyclohexenyl, cycloheptyl, cyclooctyl. Each hydrogen atom of a cycloalkyl carbon may be replaced by a substituent as defined below. The term "$C_{3-8}$ cycloalkyl" or "$C_{3-8}$ cycloalkyl ring" also includes bridged bicycles like norbonane or norbonene. Accordingly, "$C_{3-5}$ cycloalkyl" means a cycloalkyl having 3 to 5 carbon atoms and $C_{3-10}$ cycloalkyl having 3 to 10 carbon atoms.

Accordingly, as used herein, the term "$C_{3-10}$ cycloalkyl" means a carbocyclic ring system having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl, cyclodecyl. The term "$C_{3-10}$ cycloalkyl" also includes at least partially saturated carbomono-and -bicycles.

As used herein, the term "halogen" means fluoro, chloro, bromo or iodo. Particulary preferred is fluoro or chloro.

As used herein, the term "4- to 7-membered heterocyclyl" or "4- to 7-membered heterocycle" means a ring with 4, 5, 6 or 7 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including $-S(O)-$, $-S(O)_2-$), oxygen and nitrogen (including $=N(O)-$) and wherein the ring is linked to the rest of the molecule via a carbon or nitrogen atom. Examples for 4- to 7-membered heterocycles include azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 4- to 7-membered heterocyclyl or 4-to 7-membered heterocyclic group may be replaced by a substituent as defined below.

As used herein, the term "8- to 11-membered heterobicyclyl" or "8- to 11-membered heterobicycle" means a heterocyclic system of two rings with 8 to 11 ring atoms, where at least one ring atom is shared by both rings and that may contain up to the maximum number of double bonds (aromatic or non-aromatic ring which is fully, partially or unsaturated) wherein at least one ring atom up to 6 ring atoms are replaced by a heteroatom selected from the group consisting of sulfur (including -S(O)-, $-S(O)_2-$), oxygen and nitrogen (including =N(O)-) and wherein the ring is linked to the rest of the molecule via a carbon or nitrogen atom. Examples for a 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8-to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

As used herein, the term "interrupted" means that between two carbon atoms or at the end of a carbon chain between the respective carbon atom and the hydrogen atom one or more atom(s) are inserted.

As used herein, the term "prodrug" means a compound that undergoes biotransformation before exhibiting its pharmacological effects. Prodrugs can thus be viewed as biologically active moieties connected to specialized non-toxic protective groups used in a transient manner to alter or to eliminate undesirable properties in the parent molecule. This also includes the enhancement of desirable properties in the drug and the suppression of undesirable properties.

As used herein, the term "carrier-linked prodrug" means a prodrug that comprises a biologically active moiety that is covalently conjugated through a reversible linkage to a carrier moiety and which carrier moiety produces improved physicochemical or pharmacokinetic properties. Upon cleavage of the reversible linkage the biologically active moiety is released as the corresponding drug.

As used herein, the term "hydrogel-linked prodrug" means a carrier-linked prodrug in which the carrier is a hydrogel.

A "reversible linkage" or "biodegradable linkage" is a linkage that is non-enzymatically hydrolytically degradable, i.e. cleavable, under physiological conditions (aqueous buffer at pH 7.4, 37°C) with a half-life ranging from one hour to twelve months.

In contrast, a "permanent linkage" or "stable linkage" is non-enzymatically hydrolytically degradable under physiological conditions (aqueous buffer at pH 7.4, 37°C) with half-lives of more than twelve months.

As used herein, the term "pharmaceutical composition" means one or more active ingredients, and one or more inert ingredients, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the carrier-linked prodrug of the present invention and one or more pharmaceutically acceptable excipient(s).

As used herein, the term "excipient" refers to a diluent, adjuvant, or vehicle with which the therapeutic is administered. Such pharmaceutical excipient can be sterile liquids, such as water and oils, including those of petroleum, animal, vegetable or synthetic origin, including peanut oil, soybean oil, mineral oil and sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water and ethanol. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(*N*-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders and sustained-release formulations. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Examples of suitable pharmaceutical excipients are described in "Remington's Pharmaceutical Sciences" by E.W. Martin. Such compositions will contain a therapeutically effective amount of the drug or biologically active moiety, together with a suitable amount of excipient so as to provide the form for proper administration to the patient. The formulation should suit the

mode of administration.

In general the term "comprise" or "comprising" also encompasses "consist of" or "consisting of".

The present invention relates to a hydrogel-linked IL-1ra prodrug comprising IL-1ra or a pharmaceutically acceptable salt thereof, wherein an IL-1ra moiety is connected through a reversible prodrug linker moiety $L^1$ and a moiety $L^2$ to a hydrogel Z. It is understood that multiple moieties $L^2$-$L^1$-D are conjugated to a hydrogel Z.

The term "IL-1ra" as used in the present invention is described in further detail in the following sections.

As known to the person skilled in the art, it is today routine work to make e.g. minor amino changes in a protein or peptide of interest (here: IL-1ra) without significantly affecting the activity of the protein or peptide.

Preferred IL-1ra drug molecules suitable for use in the hydrogel-linked IL-1ra prodrugs of the present invention can be glycosylated or non-glycosylated. Methods for their production and use are, for example, described in US5075222A; WO91/08285; WO91/17184; AU 9173636; WO92/16221 and WO96/22793.

In particular, all natural variants, such as for example IL-1ra$\alpha$, IL-1ra$\beta$ and IL-1rax, are suitable for the hydrogel-linked IL-1ra prodrugs of the present invention. These variants include IL-1ra variants of human origin, but also those from other mammals.

Different methods for the production of IL-1ra are possible. In a first method, IL-1ra is isolated from human sample material, such as monocytes. A second method for the production of IL-1ra protein may be via chemical synthesis, such as solid-phase synthesis, or a combination of such chemical synthesis and molecular biology methods. In a third method, the gene encoding IL-1ra may be cloned into a suitable vector and subsequently transformed into suitable cell types, from which the protein may then be harvested. Numerous combinations of vectors and cell types are known to the person skilled in the art.

The IL-1ra molecule used for the hydrogel-linked IL-1ra prodrugs of the present invention may also include modified forms of IL-1ra. These include variant polypeptides in which amino acids have been (1) deleted from ("deletion variants"), (2) inserted into ("insertion variants"), and/or (3) substituted for ("substitution variants") residues within the amino acid sequence of IL-1ra.

Further included are variants containing amino acids different from the 20 naturally occurring protein-coding amino acids or variants which comprise chemical modifications at one or more amino acid residues, such as phosphorylation or glycosylation. Also combinations of different variants may be suitable for the hydrogel-linked IL-1ra prodrug of the present invention.

An IL-1ra deletion variant may typically have a deletion ranging from 1 to 30 amino acids, more typically from 1 to 10 amino acids and most typically from 1 to 5 residues. Such deletion variant may contain one continuous deletion, meaning all deleted amino acids are consecutive residues, or the deletion variant may contain more than one deletion wherein the deletions originate from different parts of the protein.

One or more N-terminal, C-terminal and internal intrasequence deletion(s) and combinations thereof may be used. Deletions within the IL-1ra amino acid sequence may be made in regions of low homology with the sequence of other members of the IL-1 family. Deletions within the IL-1ra amino acid sequence may be made in areas of substantial homology with the sequences of other members of the IL-1 family and will be more likely to significantly modify the biological activity.

IL-1ra addition variants may include an amino- and/or carboxyl-terminal fusion ranging in length from one residue to one hundred or more residues, as well as internal intrasequence insertions of single or multiple amino acids residues. Internal additions may range from 1 to 10 amino acid residues, more typically from 1 to 5 amino acid residues and most typically from 1 to 3 amino acid residues.

In a preferred embodiment, the IL-1ra protein is human IL-1ra protein.

In a further preferred embodiment, the IL-1ra protein comprises an additional N-terminal methionine. In a particular preferred embodiment, the IL-1ra protein is anakinra.

Additions at the N-terminus of the IL-1ra protein include the addition of a methionine or an additional amino acid residue or sequence. It may also include the fusion of a signal sequence and/or other pre-pro sequences to facilitate the secretion of the protein from recombinant host cells. Each protein may comprise a signal sequence to be recognized and processed, i.e. cleaved by a signal peptidase, by the host cell.

Variants with additions at their carboxy-terminus include chimeric proteins, wherein each comprises the fusion of IL-1ra with another polypeptide or protein, such as for example all or part of a constant domain of a heavy or light chain of human immunoglobulin, fragments or full-length elastin-like peptide, fragments or full-length of serum albumin (preferably human serum albumin) or fragments or full-length albumin-domain antibodies.

Substitution variants of IL-1ra have at least one amino acid residue exchanged for a different amino acid residue.

Suitable variants also include naturally-occurring allelic variants and variants artificially generated using molecular biology techniques or other forms of manipulation or mutagenesis. Methods for generating substitution variants of proteins are known to the person skilled in the art.

The sequence of IL-1ra may also be modified such that glycosylation sites are added. An asparagine-linked glycosylation recognistion site comprises a tripeptide sequence which is specifically recognized by appropriate cellular

glycosylation enzymes. These tripeptide sequences are either Asn-Xaa-Thr or Asn-Xaa-Ser, where Xaa can be any amino acid other than Pro.

Preferably, the IL-1ra protein used for the hydrogel-linked IL-1ra prodrugs are homologous to the amino acid of mammalian, in particular human IL-1ra, with a degree of homology that is preferably greater than 70%, more preferably greater than 80%, even more preferably greater than 90% and most preferably greater than 95%.

The reference sequence, i.e. the sequence on which the before-mentioned additions, deletions, substitutions, fusions and modification in the form of glycosylation are preferably based, is the sequence of IL-1ra as found in US patent No. 673375, SEQ ID NO:1 therein.

Accordingly, a preferred sequence for the IL-1ra for the hydrogel-linked IL-1ra prodrug of the present invention is SEQ ID NO:1:

Met Arg Pro Ser Gly Arg Lys Ser Ser Lys Met Gln Ala Phe Arg Ile
Trp Asp Val Asn Gln Lys Thr Phe Tyr Leu Arg Asn Asn Gln Leu Val
Ala Gly Tyr Leu Gln Gly Pro Asn Val Asn Leu Glu Glu Lys Ile Asp
Val Val Pro Ile Glu Pro His Ala Leu Phe Leu Gly Ile His Gly Gly
Lys Met Cys Leu Ser Cys Val Lys Ser Gly Asp Glu Thr Arg Leu Gln
Leu Glu Ala Val Asn Ile Thr Asp Leu Ser Glu Asn Arg Lys Gln Asp
Lys Arg Phe Ala Phe Ile Arg Ser Asp Ser Gly Pro Thr Thr Ser Phe
Glu Ser Ala Ala Cys Pro Gly Trp Phe Leu Cys Thr Ala Met Glu Ala
Asp Gln Pro Val Ser Leu Thr Asn Met Pro Asp Glu Gly Val Met Val
Thr Lys Phe Tyr Phe Gln Glu Asp Glu

In one preferred embodiment, the IL-1ra protein according to SEQ ID No.1 is unglycosylated.

In another preferred embodiment, the IL-1ra protein according to SEQ ID No.1 is glycosylated.

In a further preferred embodiment, the IL-1ra protein has the sequence according to SEQ ID No. 1, wherein the N-terminal methionine is missing. Such protein may be unglycosylated or glycosylated.

In one embodiment the term IL-1ra refers to an isolated protein that comprises a chimeric interleukin-1 (IL-1) family cytokine domain wherein at least a first segment of the domain is at least 20 amino acids in length and has at least 80% amino acid identity to a corresponding segment of a first IL-1 family cytokine, and at least a second segment of the domain is at least 20 amino acids in length and has at least 80% amino acid identity to a corresponding segment of a second IL-1 family cytokine, wherein the first and the second IL-1 family cytokines are selected from the group consisting of IL-lbeta, IL-lalpha and IL-IRa, such as disclosed in US20130209396A1, and all specific embodiments as disclosed therein.

$L^1$ may be optionally further substituted. In general, any substituent may be used as far as the cleavage principle is not affected, i.e. the hydrogen marked with the asterisk in formula (I) cannot be replaced and the nitrogen of the moiety

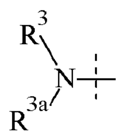

of formula (I) remains part of a primary, secondary or tertiary amine, i.e. $R^3$ and $R^{3a}$ are independently of each other H or are connected to N through an $SP^3$-hybridized carbon atom.

Preferably, the one or more further optional substituent(s) of $L^1$ are independently selected from the group consisting of halogen; -CN; -COOR$^{12}$; -OR$^{12}$; -C(O)R$^{12}$; -C(O)N(R$^{12}$R$^{12a}$); -S(O)$_2$N(R$^{12}$R$^{12a}$); -S(O)N(R$^{12}$R$^{12a}$); -S(O)$_2$R$^{12}$; -S(O)R$^{12}$; -N(R$^{12}$)S(O)$_2$N(R$^{12a}$R$^{12b}$); -SR$^{12}$; -N(R$^{12}$R$^{12a}$); -NO$_2$; -OC(O)R$^{12}$; -N(R$^{12}$)C(O)R$^{12a}$; -N(R$^{12}$)S(O)$_2$R$^{12a}$; -N(R$^{12}$)S(O)R$^{12a}$; -N(R$^{12}$)C(O)OR$^{12a}$; -N(R$^{12}$)C(O)N(R$^{12a}$R$^{12b}$); -OC(O)N(R$^{12}$R$^{12a}$); Q; $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{13}$, which are the same or different and wherein $C_{1-50}$ alkyl; $C_{2-50}$ alkenyl; and $C_{2-50}$ alkynyl are optionally interrupted by one or more groups selected from the group consisting of Q, -C(O)O-; -O-; -C(O)-; -C(O)N(R$^{14}$)-;

-S(O)$_2$N($R^{14}$)-; -S(O)N($R^{14}$)-; -S(O)$_2$-; -S(O)-; -N($R^{14}$)S(O)$_2$N($R^{14a}$)-; -S-; -N($R^{14}$)-; -OC(O)$R^{14}$; -N($R^{14}$)C(O)-; -N($R^{14}$)S(O)$_2$-; -N($R^{14}$)S(O)-; -N($R^{14}$)C(O)O-; -N($R^{14}$)C(O)N($R^{14a}$)-; and -OC(O)N($R^{14}R^{14a}$)-;

$R^{12}$, $R^{12a}$, $R^{12b}$ are independently selected from the group consisting of -H; Q; and $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{13}$, which are the same or different and wherein $C_{1-50}$ alkyl; $C_{2-50}$ alkenyl; and $C_{2-50}$ alkynyl are optionally interrupted by one or more groups selected from the group consisting of Q, -C(O)O-; -O-; -C(O)-; -C(O)N($R^{15}$)-; -S(O)$_2$N($R^{15}$)-; -S(O)N($R^{15}$)-; -S(O)$_2$-; -S(O)-; -N($R^{15}$)S(O)$_2$N($R^{15a}$)-; -S-; -N($R^{15}$)-; -OC(O)$R^{15}$; -N($R^{15}$)C(O)-; -N($R^{15}$)S(O)$_2$-; -N($R^{15}$)S(O)-; -N($R^{15}$)C(O)O-; -N($R^{15}$)C(O)N($R^{15a}$)-; and -OC(O)N($R^{15}R^{15a}$);

Q is selected from the group consisting of phenyl; naphthyl; indenyl; indanyl; tetralinyl; $C_{3-10}$ cycloalkyl; 4- to 7-membered heterocyclyl; and 9- to 11-membered heterobicyclyl, wherein Q is optionally substituted with one or more $R^{13}$, which are the same or different;

$R^{13}$ is halogen; -CN; oxo (=O); -COO$R^{16}$; -O$R^{16}$; -C(O)$R^{16}$; -C(O)N($R^{16}R^{16a}$); -S(O)$_2$N($R^{16}R^{16a}$); -S(O)N($R^{16}R^{16a}$); -S(O)$_2R^{16}$; -S(O)$R^{16}$; -N($R^{16}$)S(O)$_2$N($R^{16a}R^{16b}$); -S$R^{16}$; -N($R^{16}R^{16a}$); -NO$_2$; -OC(O)$R^{16}$; -N($R^{16}$)C(O)$R^{16a}$; -N($R^{16}$)S(O)$_2R^{16a}$; -N($R^{16}$)S(O)$R^{16a}$; -N($R^{16}$)C(O)O$R^{16a}$; -N($R^{16}$)C(O)N($R^{16a}R^{16b}$); -OC(O)N($R^{16}R^{16a}$); and $C_{1-6}$ alkyl, wherein $C_{1-6}$ alkyl is optionally substituted with one or more halogen, which are the same or different;

$R^{14}$, $R^{14a}$, $R^{15}$, $R^{15a}$, $R^{16}$, $R^{16a}$ and $R^{16b}$ are independently selected from the group consisting of -H; and $C_{1-6}$ alkyl, wherein $C_{1-6}$ alkyl is optionally substituted with one or more halogen, which are the same or different.

More preferably, the one or more optional substituent(s) of $L^1$ are independently selected from the group consisting of halogen; -CN; -COO$R^{12}$; -O$R^{12}$; -C(O)$R^{12}$; -C(O)N($R^{12}R^{12a}$); -S(O)$_2$N($R^{12}R^{12a}$); -S(O)N($R^{12}R^{12a}$); -S(O)$_2R^{12}$; -S(O)$R^{12}$; -N($R^{12}$)S(O)$_2$N($R^{12a}R^{12b}$); -S$R^{12}$; -N($R^{12}R^{12a}$); -NO$_2$; -OC(O)$R^{12}$; -N($R^{12}$)C(O)$R^{12a}$; -N($R^{12}$)S(O)$_2R^{12a}$; -N($R^{12}$)S(O)$R^{12a}$; -N($R^{12}$)C(O)O$R^{12a}$; -N($R^{12}$)C(O)N($R^{12a}R^{12b}$); -OC(O)N($R^{12}R^{12a}$); Q; $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{13}$, which are the same or different and wherein $C_{1-50}$ alkyl; $C_{2-50}$ alkenyl; and $C_{2-50}$ alkynyl are optionally interrupted by one or more groups selected from the group consisting of Q, -C(O)O-; -O-; -C(O)-; -C(O)N($R^{14}$)-; -S(O)$_2$N($R^{14}$)-; -S(O)N($R^{14}$)-; -S(O)$_2$-; -S(O)-; -N($R^{14}$)S(O)$_2$N($R^{14a}$)-; -S-; -N($R^{14}$)-; -OC(O)$R^{14}$; -N($R^{14}$)C(O)-; -N($R^{14}$)S(O)$_2$-; -N($R^{14}$)S(O)-; -N($R^{14}$)C(O)O-; -N($R^{14}$)C(O)N($R^{14a}$)-; and -OC(O)N($R^{14}R^{14a}$);

$R^{12}$, $R^{12a}$, $R^{12b}$ are independently selected from the group consisting of H; Q; $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{10}$, which are the same or different and wherein $C_{1-50}$ alkyl; $C_{2-50}$ alkenyl; and $C_{2-50}$ alkynyl are optionally interrupted by one or more groups selected from the group consisting of Q, -C(O)O-; -O-; -C(O)-; -C(O)N($R^{15}$)-; -S(O)$_2$N($R^{15}$)-; -S(O)N($R^{15}$)-; -S(O)$_2$-; -S(O)-; -N($R^{15}$)S(O)$_2$N($R^{15a}$)-; -S-; -N($R^{15}$)-; -OC(O)$R^{15}$; -N($R^{15}$)C(O)-; -N($R^{15}$)S(O)$_2$-; -N($R^{15}$)S(O)-; -N($R^{15}$)C(O)O-; -N($R^{15}$)C(O)N($R^{15a}$)-; and -OC(O)N($R^{15}R^{15a}$);

Q is selected from the group consisting of phenyl; naphthyl; indenyl; indanyl; tetralinyl; $C_{3-10}$ cycloalkyl; 4- to 7-membered heterocyclyl; or 9- to 11-membered heterobicyclyl;

$R^{13}$, $R^{14}$, $R^{14a}$, $R^{15}$ and $R^{15a}$ are independently selected from H, halogen; and $C_{1-6}$ alkyl.

Even more preferably, the one or more optional substituent(s) of $L^1$ are independently selected from the group consisting of halogen; $C_{1-50}$ alkyl; $C_{2-50}$ alkenyl; and $C_{2-50}$ alkynyl, wherein $C_{1-50}$ alkyl; $C_{2-50}$ alkenyl; and $C_{2-50}$ alkynyl are optionally substituted with one or more $R^{13}$;

$R^{13}$ is selected from the group consisting of halogen, $C_{1-6}$ alkyl, $C_{2-6}$ alkenyl and $C_{2-6}$ alkynyl.

Most preferably, the one or more optional substituent(s) of $L^1$ are independently selected from the group consisting of halogen; $C_{1-6}$ alkyl; $C_{2-6}$ alkenyl; and $C_{2-6}$ alkynyl.

Preferably, a maximum of 6 -H atoms of $L^1$ are independently replaced by a substituent, e.g. 5 -H atoms are independently replaced by a substiuent, 4 -H atoms are independently replaced by a substituent, 3 -H atoms are independently replaced by a substituent, 2 -H atoms are independently replaced by a substituent, or 1 -H atom is replaced by a substituent.

In general, $L^2$ can be attached to $L^1$ at any position apart from the replacement of the hydrogen marked with an asterisk in formula (I) and as long as $R^3$ and $R^{3a}$ are independently of each other H or are connected to N through an $SP^3$-hybridized carbon atom.

Preferably, a hydrogen of $R^1$, $R^{1a}$, $R^2$, $R^{2a}$, $R^3$, $R^{3a}$, $R^4$, $R^{4a}$, $R^5$, $R^{5a}$, $R^6$, $R^{7a}$, $R^8$, $R^{8a}$, $R^9$ or $R^{9a}$ of formula (I) directly or as hydrogen of the $C_{1-6}$ alkyl or further groups and rings given by the definition of $R^{1a}$, $R^2$, $R^{2a}$, $R^3$, $R^{3a}$, $R^4$, $R^{4a}$, $R^5$, $R^{5a}$, $R^6$, $R^{7a}$, $R^8$, $R^{8a}$, $R^9$ or $R^{9a}$ of formula (I) is replaced by $L^2$-Z.

Preferably, a hydrogen of $R^3$, $R^{3a}$, $R^4$, $R^{4a}$, $R^5$, $R^{5a}$, $R^6$, $R^{10}$, $R^{10a}$ or $R^{11}$ of formula (I) directly or as hydrogen of the $C_{1-6}$ alkyl or of a further substituent of $R^3$, $R^{3a}$, $R^4$, $R^{4a}$, $R^5$, $R^{5a}$, $R^6$, $R^{10}$, $R^{10a}$ or $R^{11}$ of formula (I) is replaced by $L^2$-Z.

Even more preferably, a hydrogen of $R^3$, $R^{3a}$, $R^{10}$, $R^{10a}$ or $R^{11}$ of formula (I) directly or as hydrogen of the $C_{1-6}$ alkyl or of a further substituent of $R^3$, $R^{3a}$, $R^{10}$, $R^{10a}$ or $R^{11}$ of formula (I) is replaced by $L^2$-Z.

Even more preferably, a hydrogen of $R^{10}$, $R^{10a}$ or $R^{11}$ of formula (I) directly or as hydrogen of the $C_{1-6}$ alkyl or of a further substituent of $R^{10}$, $R^{10a}$ or $R^{11}$ of formula (I) is replaced by $L^2$-Z.

Most preferably, a hydrogen of $R^{11}$ of formula (I) directly or as hydrogen of the $C_{1-6}$ alkyl is replaced by $L^2$-Z.

Preferably, X of formula (I) is $C(R^7R^{7a})$.
Preferably, $X^1$ of formula (I) is C.
In one embodiment, $X^2$ of formula (I) is $C(R^8R^{8a})$.
In another embodiment $X^2$ of formula (I) is $C(R^8R^{8a})$-$C(R^9R^{9a})$.
Preferably, $X^3$ of formula (I) is O.
Preferably, $R^1$ of formula (I) is H.
Preferably, $R^{1a}$ of formula (I) is H.
Preferably, $R^1$ and $R^{1a}$ of formula (I) are both H.
Preferably, $R^2$ of formula (I) is H.
Preferably, $R^{2a}$ of formula (I) is H.
Preferably, $R^2$ and $R^{2a}$ of formula (I) are H.
Preferably, $R^3$ of formula (I) is H or methyl, ethyl or propyl.
Preferably, $R^{3a}$ of formula (I) is H or methyl, ethyl or propyl.
In one preferred embodiment $R^3$ and $R^{3a}$ of formula (I) are both H.
In another preferred embodiment $R^3$ of formula (I) is H and $R^{3a}$ of formula (I) is methyl.
In another preferred embodiment $R^3$ and $R^{3a}$ of formula (I) are both methyl.
In a preferred embodiment $L^1$ is of formula (II)

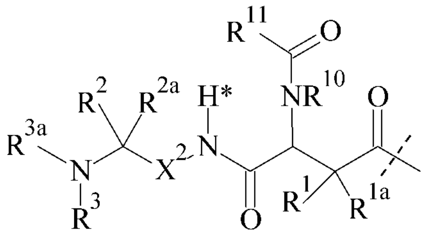

(II),

wherein

the dashed line indicates the attachment to a nitrogen of D by forming an amide bond;
$R^1$, $R^{1a}$, $R^2$, $R^{2a}$, $R^3$, $R^{3a}$, $R^{10}$, $R^{11}$ and $X^2$ are used as defined in formula (I);
and wherein $L^1$ is optionally further substituted, provided that the hydrogen marked with the asterisk in formula (II) is not replaced by a substituent and that $R^3$ and $R^{3a}$ are independently of each other H or are connected to N through an $SP^3$-hybridized carbon atom.

Even more preferably, a hydrogen of $R^3$, $R^{3a}$, $R^{10}$ or $R^{11}$ of formula (II) directly or as hydrogen of the $C_{1-6}$ alkyl or of a further substituent of $R^3$, $R^{3a}$, $R^{10}$ or $R^{11}$ of formula (II) is replaced by $L^2$-Z.
Even more preferably, a hydrogen of $R^{10}$ or $R^{11}$ of formula (II) directly or as hydrogen of the $C_{1-6}$ alkyl or of a further substituent of $R^{10}$ or $R^{11}$ of formula (II) is replaced by $L^2$-Z.
Most preferably, a hydrogen of $R^{11}$ of formula (II) directly or as hydrogen of the $C_{1-6}$ alkyl is replaced by $L^2$-Z.
In one embodiment, $X^2$ of formula (II) is $C(R^8R^{8a})$.
In another embodiment $X^2$ of formula (II) is $C(R^8R^{8a})$-$C(R^9R^{9a})$.
Preferably, $R^1$ of formula (II) is H.
Preferably, $R^{1a}$ of formula (II) is H.
Preferably, $R^1$ and $R^{1a}$ of formula (II) are both H.
Preferably, $R^2$ of formula (II) is H.
Preferably, $R^{2a}$ of formula (II) is H.
Preferably, $R^2$ and $R^{2a}$ of formula (II) are both H.
Preferably, $R^3$ of formula (II) is H or methyl, ethyl or propyl.
Preferably, $R^{3a}$ of formula (II) is H or methyl, ethyl or propyl.
In one preferred embodiment $R^3$ and $R^{3a}$ of formula (II) are both H.
In another preferred embodiment $R^3$ of formula (II) is H and $R^{3a}$ of formula (II) is methyl.
In another preferred embodiment $R^3$ and $R^{3a}$ of formula (II) are both methyl.
In one embodiment, $R^{10}$ of formula (II) is H.
In another preferred embodiment $R^{10}$ of formula (II) is methyl, ethyl, propyl, isopropyl, butyl, isobutyl or tert-butyl. More preferably, $R^{10}$ of formula (II) is methyl, ethyl, propyl or isopropyl. Even more preferably, $R^{10}$ of formula (II) is methyl or ethyl and most preferably, $R^{10}$ of formula (II) is methyl.

Preferably, $R^{11}$ of formula (II) is H.

Even more preferably, $L^1$ is of formula (IIIa) or (IIIb):

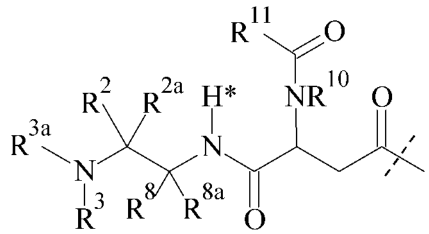

(IIIa),

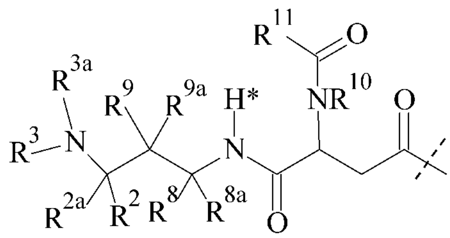

(IIIb),

wherein

the dashed line indicates the attachment to a nitrogen of D by forming an amide bond;
$R^2$, $R^{2a}$, $R^3$, $R^{3a}$, $R^8$, $R^{8a}$, $R^9$, $R^{9a}$, $R^{10}$, and $R^{11}$ are used as defined in formula (I);
and wherein $L^1$ is optionally further substituted, provided that the hydrogen marked with the asterisk in formula (IIIa) or (IIIb) is not replaced by a substituent and that $R^3$ and $R^{3a}$ are independently of each other H or are connected to N through an $SP^3$-hybridized carbon atom.

Even more preferably, a hydrogen of $R^3$, $R^{3a}$, $R^{10}$ or $R^{11}$ of formula (IIIa) or (IIIb) directly or as hydrogen of the $C_{1-6}$ alkyl or of a further substituent of $R^3$, $R^{3a}$, $R^{10}$ or $R^{11}$ of formula (IIIa) or (IIIb) is replaced by $L^2$-Z.

Even more preferably, a hydrogen of $R^{10}$ or $R^{11}$ of formula (IIIa) or (IIIb) directly or as hydrogen of the $C_{1-6}$ alkyl or of a further substituent of $R^{10}$ or $R^{11}$ of formula (IIIa) or (IIIb) is replaced by $L^2$-Z.

Most preferably, a hydrogen of $R^{11}$ of formula (IIIa) or (IIIb) directly or as hydrogen of the $C_{1-6}$ alkyl is replaced by $L^2$-Z.

Preferably, $R^2$ of formula (IIIa) or (IIIb) is H.

Preferably, $R^{2a}$ of formula (IIIa) or (IIIb) is H.

Preferably, $R^2$ and $R^{2a}$ of formula (IIIa) or (IIIb) are H.

Preferably, $R^3$ of formula (IIIa) or (IIIb) is H or methyl, ethyl or propyl.

Preferably, $R^{3a}$ of formula (IIIa) or (IIIb) is H or methyl, ethyl or propyl.

In one preferred embodiment $R^3$ and $R^{3a}$ of formula (IIIa) or (IIIb) are both H.

In another preferred embodiment $R^3$ of formula (IIIa) or (IIIb) is H and $R^{3a}$ of formula (IIIa) or (IIIb) is methyl.

In another preferred embodiment $R^3$ and $R^{3a}$ of formula (IIIa) or (IIIb) are both methyl.

Preferably, $R^8$ of formula (IIIa) or (IIIb) is H.

Preferably, $R^{8a}$ of formula (IIIa) or (IIIb) is H.

Preferably, $R^8$ and $R^{8a}$ of formula (IIIa) or (IIIb) are both H.

Preferably, $R^9$ of formula (IIIb) is H.

Preferably, $R^{9a}$ of formula (IIIb) is H.

Preferably, $R^9$ and $R^{9a}$ of formula (IIIb) are both H.

Preferably, $R^{10}$ of formula (IIIa) is H.

In another preferred embodiment $R^{10}$ of formula (IIIb) is methyl, ethyl, propyl, isopropyl, butyl, isobutyl or tert-butyl. More preferably, $R^{10}$ of formula (IIIb) is methyl, ethyl, propyl or isopropyl. Even more preferably, $R^{10}$ of formula (IIIb) is methyl or ethyl and most preferably, $R^{10}$ of formula (IIIb) is methyl.

Preferably, $R^{11}$ of formula (IIIa) or (IIIb) is H.

Even more preferably, $L^1$ is of formula (IVa) or (IVb):

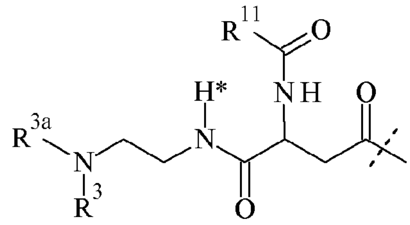

(IVa),

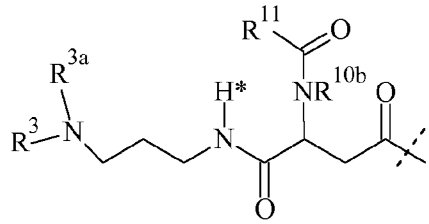

(IVb),

wherein

the dashed line indicates the attachment to a nitrogen of D by forming an amide bond;
$R^3$ and $R^{3a}$ are used as defined in formula (I);
$R^{10b}$ is $C_{1-6}$ alkyl;
and wherein $L^1$ is optionally further substituted, provided that the hydrogel marked with the asterisk in formula (IVa) or (IVb) is not replaced by a substituent and that $R^3$ and $R^{3a}$ are independently of each other H or are connected to N through an $SP^3$-hybridized carbon atom.

Even more preferably, a hydrogen of $R^3$, $R^{3a}$, $R^{10}$ or $R^{11}$ of formula (IVa) or (IVb) directly or as hydrogen of the $C_{1-6}$ alkyl or of a further substituent of $R^3$, $R^{3a}$, $R^{10}$ or $R^{11}$ of formula (IVa) or (IVb) is replaced by $L^2$-Z.
Even more preferably, a hydrogen of $R^{10}$ or $R^{11}$ of formula (IVa) or (IVb) directly or as hydrogen of the $C_{1-6}$ alkyl or of a further substituent of $R^{10}$ or $R^{11}$ of formula (IVa) or (IVb) is replaced by $L^2$-Z.
Most preferably, a hydrogen of $R^{11}$ of formula (IVa) or (IVb) directly or as hydrogen of the $C_{1-6}$ alkyl is replaced by $L^2$-Z.
Preferably, $R^3$ of formula (IVa) or (IVb) is H or methyl, ethyl or propyl.
Preferably, $R^{3a}$ of formula (IVa) or (IVb) is H or methyl, ethyl or propyl.
In one preferred embodiment $R^3$ and $R^{3a}$ of formula (IVa) or (IVb) are both H.
In another preferred embodiment $R^3$ of formula (IVa) or (IVb) is H and $R^{3a}$ of formula (IVa) or (IVb) is methyl.
In another preferred embodiment $R^3$ and $R^{3a}$ of formula (IVa) or (IVb) are both methyl.
In another preferred embodiment $R^{10b}$ of formula (IVb) is methyl, ethyl, propyl, isopropyl, butyl, isobutyl or tert-butyl. More preferably, $R^{10b}$ of formula (IVb) is methyl, ethyl, propyl or isopropyl. Even more preferably, $R^{10b}$ of formula (IVb) is methyl or ethyl and most preferably, $R^{10b}$ of formula (IVb) is methyl.
Preferably, a hydrogen of $R^3$, $R^{3a}$ or $R^{11}$ of formula (Iva) or (IVb) directly or as hydrogen of the $C_{1-6}$ alkyl or of a further substituent of $R^{10}$, $R^{10a}$ or $R^{11}$ is replaced by $L^2$-Z.
Preferably, $R^{11}$ of formula (IVa) or (IVb) is H and is replaced by $L^2$-Z.
$L^2$ is a single chemical bond or a spacer.
When $L^2$ is other than a single chemical bond, $L^2$-Z is preferably $-C(O)N(R^{17})-$; $-S(O)_2N(R^{17})-$; $-S(O)N(R^{17})-$; $-N(R^{17})S(O)_2N(R^{17a})-$; $-N(R^{17})-$; $-OC(O)R^{17}$; $-N(R^{17})C(O)-$; $-N(R^{17})S(O)_2-$; $-N(R^{17})S(O)-$; $-N(R^{17})C(O)O-$; $-N(R^{17})C(O)N(R^{17a})-$; $-OC(O)N(R^{17}R^{17a})-$; Q; $C_{1-50}$ alkyl; $C_{2-50}$ alkenyl; or $C_{2-50}$ alkynyl, wherein Q; $C_{1-50}$ alkyl; $C_{2-50}$ alkenyl; and $C_{2-50}$ alkynyl are optionally substituted with one or more $R^{18}$, which are the same or different and wherein $C_{1-50}$ alkyl; $C_{2-50}$ alkenyl; and $C_{2-50}$ alkynyl are optionally interrupted by one or more groups selected from the group consisting of Q, $-C(O)O-$; $-O-$; $-C(O)-$; $-C(O)N(R^{19})-$; $-S(O)_2N(R^{19})-$; $-S(O)N(R^{19})-$; $-S(O)_2-$; $-S(O)-$; $-N(R^{19})S(O)_2N(R^{19a})-$; $-S-$; $-N(R^{19})-$; $-OC(O)R^{19}$; $-N(R^{19})C(O)-$; $-N(R^{19})S(O)_2-$; $-N(R^{19})S(O)-$; $-N(R^{19})C(O)O-$; $-N(R^{19})C(O)N(R^{19a})-$; and $-OC(O)N(R^{19}R^{19a})$;
$R^{17}$, $R^{17a}$, $R^{17b}$ are independently selected from the group consisting of -H; Z; Q; and $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{17}$, which are the same or different and wherein $C_{1-50}$ alkyl; $C_{2-50}$ alkenyl; and $C_{2-50}$ alkynyl are optionally interrupted by one or more groups selected from the group consisting of Q, $-C(O)O-$; $-O-$; $-C(O)-$; $-C(O)N(R^{20})-$; $-S(O)_2N(R^{20})-$; $-S(O)N(R^{20})-$; $-S(O)_2-$; $-S(O)-$; $-N(R^{20})S(O)_2N(R^{20a})-$; $-S-$; $-N(R^{20})-$; $-OC(O)R^{20}$; $-N(R^{20})C(O)-$; $-N(R^{20})S(O)_2-$;

$-N(R^{20})S(O)-$; $-N(R^{20})C(O)O-$; $-N(R^{20})C(O)N(R^{20a})-$; and $-OC(O)N(R^{20}R^{20a})$;

Q is selected from the group consisting of phenyl; naphthyl; indenyl; indanyl; tetralinyl; $C_{3-10}$ cycloalkyl; 4 to 7 membered heterocyclyl; and 9 to 11 membered heterobicyclyl, wherein Q is optionally substituted with one or more $R^{17}$, which are the same or different;

$R^{18}$ is Z; halogen; -CN; oxo (=O); $-COOR^{21}$; $-OR^{21}$; $-C(O)R^{21}$; $-C(O)N(R^{21}R^{21a})$; $-S(O)_2N(R^{21}R^{21a})$; $-S(O)N(R^{21}R^{21a})$; $-S(O)_2R^{21}$; $-S(O)R^{21}$; $-N(R^{21})S(O)_2N(R^{21a}R^{21b})$; $-SR^{21}$; $-N(R^{21}R^{21a})$; $-NO_2$; $-OC(O)R^{21}$; $-N(R^{21})C(O)R^{21a}$; $-N(R^{21})S(O)_2R^{21a}$; $-N(R^{21})S(O)R^{21a}$; $-N(R^{21})C(O)OR^{21a}$; $-N(R^{21})C(O)N(R^{21a}R^{21b})$; $-OC(O)N(R^{21}R^{21a})$; or $C_{1-6}$ alkyl, wherein $C_{1-6}$ alkyl is optionally substituted with one or more halogen, which are the same or different;

$R^{19}$, $R^{19a}$, $R^{20}$, $R^{20a}$, $R^{21}$, $R^{21a}$ and $R^{21b}$ are independently selected from the group consisting of -H; Z; and $C_{1-6}$ alkyl, wherein $C_{1-6}$ alkyl is optionally substituted with one or more halogen, which are the same or different;

provided that one of $R^{17}$, $R^{17a}$, $R^{17b}$, $R^{18}$, $R^{19}$, $R^{19a}$, $R^{20}$, $R^{20a}$, $R^{21}$, $R^{21a}$ or $R^{21b}$ is Z.

More preferably, $L^2$ is a $C_{1-20}$ alkyl chain, which is optionally interrupted by one or more groups independently selected from -O-; and $-C(O)N(R^{1aa})-$; and which $C_{1-20}$ alkyl chain is optionally substituted with one or more groups independently selected from OH; and $-C(O)N(R^{1aa}R^{1aaa})$; wherein $R^{1aa}$, $R^{1aaa}$ are independently selected from the group consisting of H; and $C_{1-4}$ alkyl.

Preferably, $L^2$ has a molecular weight in the range of from 14 g/mol to 750 g/mol.

Preferably, $L^2$ is attached to Z via a terminal group selected from

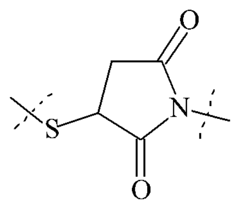

; and

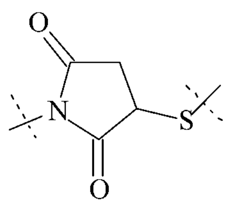

.

In case $L^2$ has such terminal group it is furthermore preferred that $L^2$ has a molecular weight in the range of from 14 g/mol to 500 g/mol calculated without such terminal group.

Preferably, L is represented by formula (Va) or (Vb):

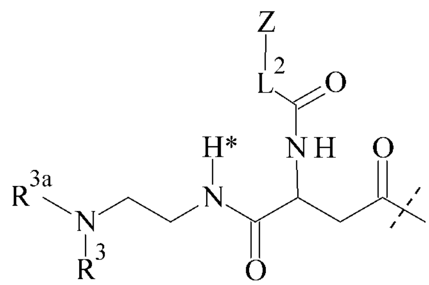

(Va),

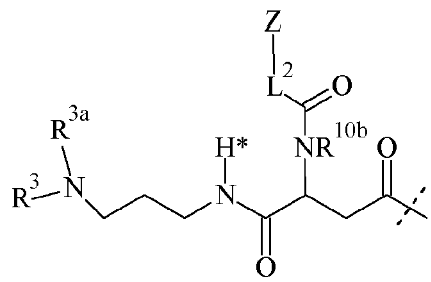

(Vb),

wherein

the dashed line indicates the attachment to a nitrogen of D by forming an amide bond;

$R^3$, R3$^a$, $L^2$ and Z are used as defined in formula (I); and

$R^{10b}$ is used as defined in formula (IVa) and (IVb).

In another preferred embodiment $R^{10b}$ of formula (Vb) is methyl, ethyl, propyl, isopropyl, butyl, isobutyl or tert-butyl. More preferably, $R^{10b}$ of formula (Vb) is methyl, ethyl, propyl or isopropyl. Even more preferably, $R^{10b}$ of formula (Vb) is methyl or ethyl and most preferably, $R^{10b}$ of formula (Vb) is methyl.

Preferably, the hydrogel carrier Z is a shaped article, such as a coating, mesh, stent, nanoparticle or a microparticle. Preferably the hydrogel Z is in the form of a microparticle. More preferably, Z is a microparticulate bead. Even more preferably, such microparticulate bead has a diameter of 1 to 1000 μm, more preferably of 5 to 500 μm, more preferably of 10 to 250 μm, even more preferably of 20 to 200 μm, even more preferably of 30 to 190 μm and most preferably of 50 to 180 μm. The afore-mentioned diameters are measured when the hydrogel microparticles are fully hydrated in water at room temperature.

Preferably, Z is a PEG-based or hyaluronic acid-based hydrogel. Most preferably, Z is a PEG-based hydrogel comprising at least 10 % PEG, more preferably at least 15 % PEG and most preferably at least 20 % PEG.

Suitable hydrogels are known in the art. Preferred hydrogels are those disclosed in WO2006/003014 and WO2011/012715.

Most preferably, the hydrogel Z is a hydrogel obtained from a process for the preparation of a hydrogel comprising the steps of:

(a) providing a mixture comprising

(a-i) at least one backbone reagent, wherein the at least one backbone reagent has a molecular weight ranging from 1 to 100 kDa, and comprises at least three functional groups $A^{x0}$, wherein each $A^{x0}$ is a maleimide, amine ($-NH_2$ or - NH-), hydroxyl (-OH), thiol (-SH), carboxyl (-COOH) or activated carboxyl ($-COY^1$, wherein $Y^1$ is selected from formulas (f-i) to (f-vi):

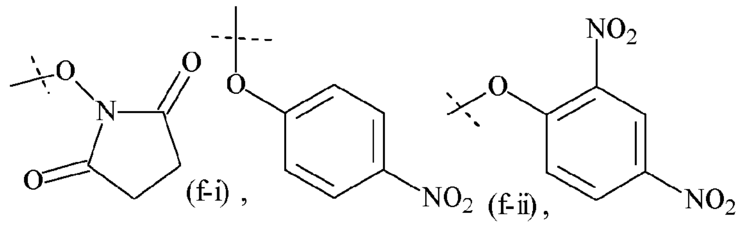
(f-i) , (f-ii), (f-iii),

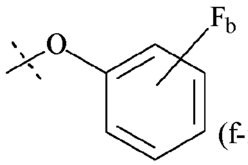
(f-iv),
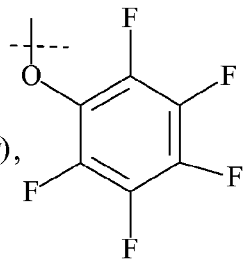
(f-v) and
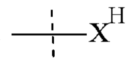
(f-vi)

wherein

the dashed lines indicate attachment to the rest of the molecule,
b is 1, 2, 3 or 4,
$X^H$ is Cl, Br, I, or F);;

(a-ii) at least one crosslinker reagent, wherein the at least one crosslinker reagent has a molecular weight ranging from 0.2 to 40 kDa and comprises at least two functional end groups selected from the group consisting of activated ester groups, activated carbamate groups, activated carbonate groups, activated thiocarbonate groups, amine groups and thiol groups;

in a weight ratio of the at least one backbone reagent to the at least one crosslinker reagent ranging from 1:99 to 99:1 and wherein the molar ratio of $A^{x0}$ to functional end groups is >1;
(b) polymerizing the mixture of step (a) in a suspension polymerization to a hydrogel.

The mixture of step (a) comprises a first solvent and at least a second second solvent. Said first solvent is

preferably selected from the group comprising dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol and water and mixtures thereof.

The at least one backbone reagent and at least one crosslinker reagent are dissolved in the first solvent, i.e. the disperse phase of the suspension polymerization. In one embodiment the backbone reagent and the crosslinker reagent are dissolved separately, i.e. in different containers, using either the same or different solvent and preferably using the same solvent for both reagents. In another embodiment, the backbone reagent and the crosslinker reagent are dissolved together, i.e. in the same container and using the same solvent.

A suitable solvent for the backbone reagent is an organic solvent. Preferably, the solvent is selected from the group consisting of dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol and water and mixtures thereof. More preferably, the backbone reagent is dissolved in a solvent selected from the group comprising acetonitrile, dimethyl sulfoxide, methanol or mixtures thereof. Most preferably, the backbone reagent is dissolved in dimethylsulfoxide.

In one embodiment the backbone reagent is dissolved in the solvent in a concentration ranging from 1 to 300 mg/ml, more preferably from 5 to 60 mg/ml and most preferably from 10 to 40 mg/ml.

A suitable solvent for the crosslinker reagent is an organic solvent. Preferably, the solvent is selected from the group comprising dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol, water or mixtures thereof. More preferably, the crosslinker reagent is dissolved in a solvent selected from the group comprising dimethylformamide, acetonitrile, dimethyl sulfoxide, methanol or mixtures thereof. Most preferably, the crosslinker reagent is dissolved in dimethylsulfoxide.

In one embodiment the crosslinker reagent is dissolved in the solvent in a concentration ranging from 5 to 500 mg/ml, more preferably from 25 to 300 mg/ml and most preferably from 50 to 200 mg/ml.

The at least one backbone reagent and the at least one crosslinker reagent are mixed in a weight ratio ranging from 1:99 to 99:1, e.g. in a ratio ranging from 2:98 to 90:10, in a weight ratio ranging from 3:97 to 88:12, in a weight ratio ranging from 3:96 to 85:15, in a weight ratio ranging from 2:98 to 90:10 and in a weight ratio ranging from 5:95 to 80:20; particularly preferred in a weight ratio from 5:95 to 80:20, wherein the first number refers to the backbone reagent and the second number to the crosslinker reagent.

Preferably, the ratios are selected such that the mixture of step (a) comprises a molar excess of amine groups from the backbone reagent compared to the activated functional end groups of the crosslinker reagent. Consequently, the hydrogel resulting from the process has free amine groups which can be used to couple other moieties to the hydrogel, such as spacers, and/or reversible prodrug linker moieties $L^1$.

The at least one second solvent, i.e. the continuous phase of the suspension polymerization, is preferably an organic solvent, more preferably an organic solvent selected from the group comprising linear, branched or cyclic $C_{5-30}$ alkanes; linear, branched or cyclic $C_{5-30}$ alkenes; linear, branched or cyclic $C_{5-30}$ alkynes; linear or cyclic poly(dimethylsiloxanes); aromatic $C_{6-20}$ hydrocarbons; and mixtures thereof. Even more preferably, the at least second solvent is selected from the group comprising linear, branched or cyclic $C_{5-16}$ alkanes; toluene; xylene; mesitylene; hexamethyldisiloxane; or mixtures thereof. Most preferably, the at least second solvent selected from the group comprising linear $C_{7-11}$ alkanes, such as heptane, octane, nonane, decane and undecane.

Preferably, the mixture of step (a) further comprises a detergent. Preferred detergents are Cithrol DPHS, Hypermer 70A, Hypermer B246, Hypermer 1599A, Hypermer 2296, and Hypermer 1083.

Preferably, the detergent has a concentration of 0.1 g to 100 g per 1 L total mixture, i.e. disperse phase and continous phase together. More preferably, the detergent has a concentration of 0.5 g to 10 g per 1 L total mixture, and most preferably, the detergent has a concentration of 0.5 g to 5 g per 1 L total mixture.

Preferably, the mixture of step (a) is an emulsion.

The polymerization in step (b) is initiated by adding a base. Preferably, the base is a non-nucleophilic base soluble in alkanes, more preferably the base is selected from *N,N,N',N'*-tetramethylethylene diamine (TMEDA), 1,4-dimethylpiperazine, 4-methylmorpholine, 4-ethylmorpholine, 1,4-diazabicyclo[2.2.2]octane, 1,1,4,7,10,10-hexamethyltriethylenetetramine, 1,4,7-trimethyl-1,4,7-triazacyclononane, tris[2-(dimethylamino)ethyl]amine, triethylamine, DIPEA, trimethylamine, N,N-dimethylethylamine, N,N,N',N'-tetramethyl-1,6-hexanediamine, N,N,N',N",N"-pentamethyldiethylenetriamine, 1,8-diazabicyclo[5.4.0]undec-7-ene, 1,5-diazabicyclo[4.3.0]non-5-ene, and hexamethylenetetramine. Even more preferably, the base is selected from TMEDA, 1,4-dimethylpiperazine, 4-methylmorpholine, 4-ethylmorpholine, 1,4-diazabicyclo[2.2.2]octane, 1,1,4,7,10,10-hexamethyltriethylenetetramine, 1,4,7-trimethyl-1,4,7-triazacyclononane, tris[2-(dimethylamino)ethyl]amine, 1,8-diazabicyclo[5.4.0]undec-7-ene, 1,5-diazabicyclo[4.3.0]non-5-ene, and hexamethylenetetramine. Most preferably, the base is TMEDA.

The base is added to the mixture of step (a) in an amount of 1 to 500 equivalents per activated functional end group in the mixture, preferably in an amount of 5 to 50 equivalents, more preferably in an amount of 5 to 25 equivalents and most preferably in an amount of 10 equivalents.

In process step (b), the polymerization of the hydrogel of the present invention is a condensation reaction, which preferably occurs under continuous stirring of the mixture of step (a). Preferably, the tip speed (tip speed = $\pi \times$ stirrer rotational speed $\times$ stirrer diameter) ranges from 0.2 to 10 meter per second (m/s), more preferably from 0.5 to 4 m/s and most preferably from 1 to 2 m/s.

In a preferred embodiment of step (b), the polymerization reaction is carried out in a cylindrical vessel equipped with baffles. The diameter to height ratio of the vessel may range from 4:1 to 1:2, more preferably the diameter to height ratio of the vessel ranges from 2:1 to 1:1.

Preferably, the reaction vessel is equipped with an axial flow stirrer selected from the group comprising pitched blade stirrer, marine type propeller, or Lightnin A-310. More preferably, the stirrer is a pitched blade stirrer.

Step (b) can be performed in a broad temperature range, preferably at a temperature from -10°C to 100 C°, more preferably at a temperature of 0°C to 80°C, even more preferably at a temperature of 10°C to 50 °C and most preferably at ambient temperature. "Ambient temperature" refers to the temperature present in a typical laboratory environment and preferably means a temperature ranging from 17 to 25°C.

Preferably, the hydrogel obtained from the polymerization is a shaped article, such as a coating, mesh, stent, nanoparticle or a microparticle. More preferably, the hydrogel is in the form of microparticular beads having a diameter from 1 to 500 micrometer, more preferably with a diameter from 10 to 300 micrometer, even more preferably with a diameter from 20 and 150 micrometer and most preferably with a diameter from 30 to 130 micrometer. The afore-mentioned diameters are measured when the hydrogel microparticles are fully hydrated in water.

In one embodiment, the process for the preparation of a hydrogel further comprises the step of:
(c) working-up the hydrogel.

Step (c) comprises one or more of the following step(s):

(c1) removing excess liquid from the polymerization reaction,
(c2) washing the hydrogel to remove solvents used during polymerization,
(c3) transferring the hydrogel into a buffer solution,
(c4) size fractionating/sieving of the hydrogel,
(c5) transferring the hydrogel into a container,
(c6) drying the hydrogel,
(c7) transferring the hydrogel into a specific solvent suitable for sterilization, and
(c8) sterilizing the hydrogel, preferably by gamma radiation

Preferably, step (c) comprises all of the following steps

(c1) removing excess liquid from the polymerization reaction,
(c2) washing the hydrogel to remove solvents used during polymerization,
(c3) transferring the hydrogel into a buffer solution,
(c4) size fractionating/sieving of the hydrogel,
(c5) transferring the hydrogel into a container,
(c7) transferring the hydrogel into a specific solvent suitable for sterilization, and
(c8) sterilizing the hydrogel, preferably by gamma radiation.

The at least one backbone reagent has a molecular weight ranging from 1 to 100 kDa, preferably from 2 to 50 kDa, more preferably from 5 and 30 kDa, even more preferably from 5 to 25 kDa and most preferably from 5 to 15 kDa.

Preferably, the backbone reagent is PEG-based comprising at least 10% PEG, more preferably comprising at least 20% PEG, even more preferably comprising at least 30% PEG and most preferably comprising at least 40% PEG.

In one embodiment the backbone reagent of step (a-i) is present in the form of its acidic salt, preferably in the form of an acid addition salt. Suitable acid addition salts are formed from acids which form non-toxic salts. Examples include the acetate, aspartate, benzoate, besylate, bicarbonate, carbonate, bisulphate, sulphate, borate, camsylate, citrate, edisylate, esylate, formate, fumarate, gluceptate, gluconate, glucuronate, hexafluorophosphate, hibenzate, hydrochloride, hydrobromide, hydroiodide, isethionate, lactate, malate, maleate, malonate, mesylate, methylsulphate, naphthylate, nicotinate, nitrate, orotate, oxalate, palmitate, pamoate, phosphate, hydrogen phosphate, dihydrogen phosphate, sacharate, stearate, succinate, tartrate and tosylate. Particularly preferred, the backbone reagent is present in the form of its hydrochloride salt.

In one embodiment, the at least one backbone reagent is selected from the group consisting of
a compound of formula (aI)

$$B(-(A^0)_{x1}-(SP)_{x2}-A^1-P-A^2-Hyp^1)_x \qquad (aI),$$

wherein

| | |
|---|---|
| B | is a branching core, |
| SP | is a spacer moiety selected from the group consisting of $C_{1-6}$ alkyl, $C_{2-6}$ alkenyl and $C_{2-6}$ alkynyl, |
| P | is a PEG-based polymeric chain comprising at least 80% PEG, preferably at least 85% PEG, more preferably at least 90% PEG and most preferably at least 95% PEG, |
| $Hyp^1$ | is a moiety comprising an amine ($-NH_2$ and/or -NH-) or a polyamine comprising at least two amines ($-NH_2$ and/or -NH-), |
| x | is an integer from 3 to 16, |
| x1, x2 | are independently of each other 0 or 1, provided that x1 is 0, if x2 is 0, |
| $A^0$, $A^1$, $A^2$ | are independently of each other selected from the group consisting of |

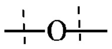, 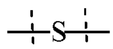, 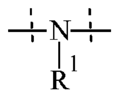, 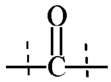, 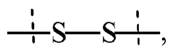, 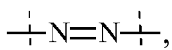,

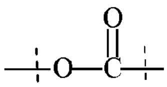, 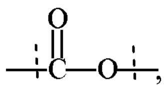, 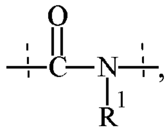, 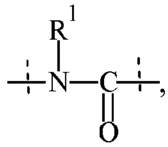, 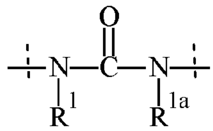,

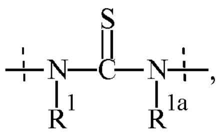, 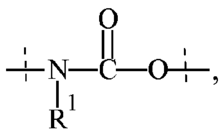, 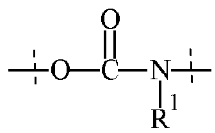,

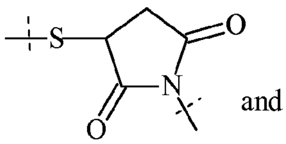 and 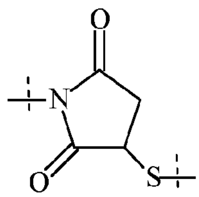,

wherein $R^1$ and $R^{1a}$ are independently of each other selected from H and $C_{1-6}$ alkyl;
a compound of formula (aII)

$$Hyp^2 - A^3 - P - A^4 - Hyp^3 \qquad \text{(aII)},$$

wherein

| | |
|---|---|
| P | is defined as above in the compound of formula (aI), |
| $Hyp^2$, $Hyp^3$ | are independently of each other a polyamine comprising at least two amines ($-NH_2$ and/or -NH-), and |
| $A^3$ and $A^4$ | are independently selected from the group consisting of |

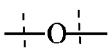, 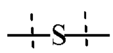, 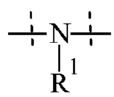, 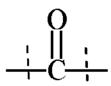, 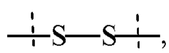, 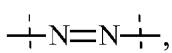,

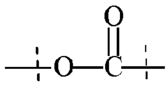, 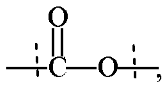, 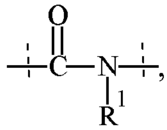, 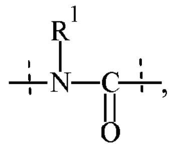, 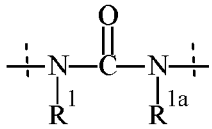,

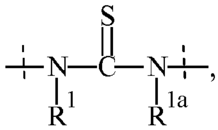, 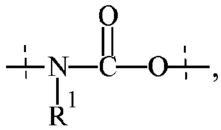, 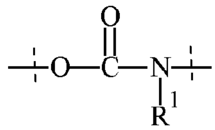,

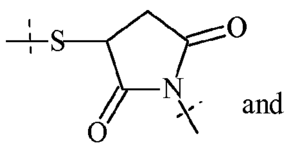 and 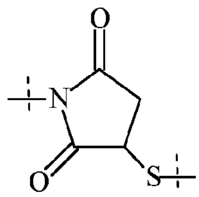,

wherein $R^1$ and $R^{1a}$ are independently of each other selected from H and $C_{1-6}$ alkyl;
a compound of formula (aIII)

$$P^1 - A^5 - Hyp^4 \qquad (aIII),$$

wherein

$P^1$ is a PEG-based polymeric chain comprising at least 80% PEG, preferably at least 85% PEG, more preferably at least 90% PEG and most preferably at least 95% PEG,
$Hyp^4$ is a polyamine comprising at least three amines ($-NH_2$ and/or -NH), and
$A^5$ is selected from the group consisting of

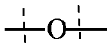, 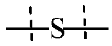, 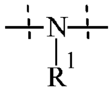, 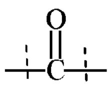, 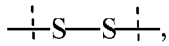, 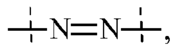,

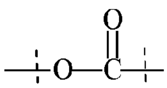, 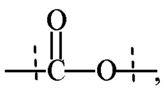, 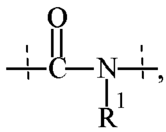, 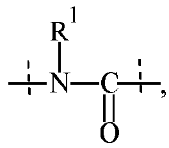, 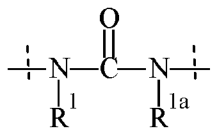,

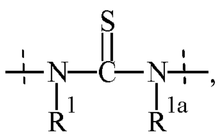, 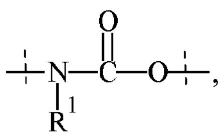, 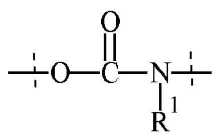,

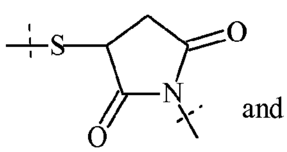 and 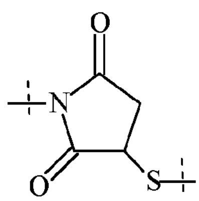,

wherein $R^1$ and $R^{1a}$ are independently of each other selected from H and $C_{1-6}$ alkyl; and

a compound of formula (aIV),

$$T^1 - A^6 - Hyp^5 \qquad \text{(aIV)},$$

wherein

$Hyp^5$ is a polyamine comprising at least three amines ($-NH_2$ and/or -NH), and

$A^6$ is selected from the group consisting of

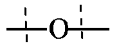 , 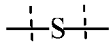 , 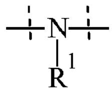 , 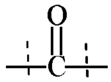 , 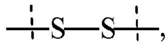 , 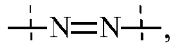 ,

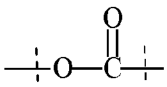 , 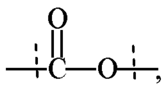 , 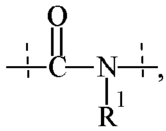 , 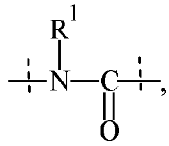 , 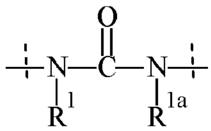 ,

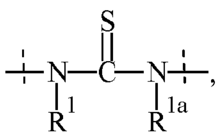 , 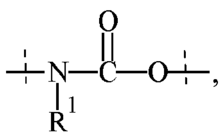 , 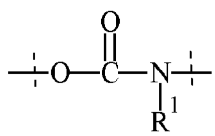 ,

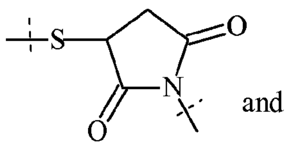 and 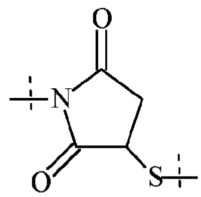 ,

wherein $R^1$ and $R^{1a}$ are independently of each other selected from H and $C_{1-6}$ alkyl; and

$T^1$ is selected from the group consisting of $C_{1-50}$ alkyl, $C_{2-50}$ alkenyl or $C_{2-50}$ alkynyl, which fragment is optionally interrupted by one or more group(s) selected from -NH-, $-N(C_{1-4}$ alkyl)-, -O-, -S-, -C(O)-, -C(O)NH-,$-C(O)N(C_{1-4}$ alkyl)-, -O-C(O)-, -S(O)-, $-S(O)_2-$, 4- to 7-membered heterocyclyl, phenyl or naphthyl.

In the following sections the term "$Hyp^x$" refers to $Hyp^1$, $Hyp^2$, $Hyp^3$, $Hyp^4$ and $Hyp^5$ collectively.

Preferably, the backbone reagent is a compound of formula (aI), (aII) or (aIII), more preferably the backbone reagent is a compound of formula (aI) or (aIII), and most preferably the backbone reagent is a compound of formula (aI).

In a preferred embodiment, in a compound of formula (aI), x is 4, 6 or 8. Preferably, in a compound of formula (aI) x is 4 or 8, most preferably, x is 4.

In a preferred embodiment in the compounds of the formulas (aI) to (aIV), $A^0$, $A^1$, $A^2$, $A^3$, $A^4$, $A^5$ and $A^6$ are selected from the group comprising

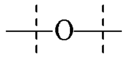 , 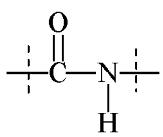 , 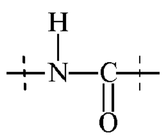 , and 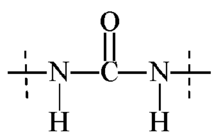 .

Preferably, in a compound of formula (aI), $A^0$ is

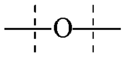 , 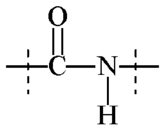 , and 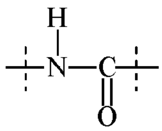 .

Preferably, in a compound of formula (aI), $A^1$ is

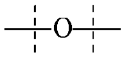 , 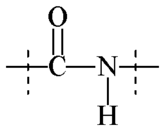 , and 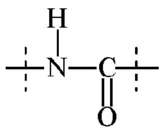 .

Preferably, in a compound of formula (aI), $A^2$ is

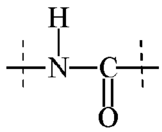 , and 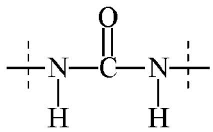 .

Preferably, in a compound of formula (aII), $A^3$

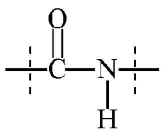 , and 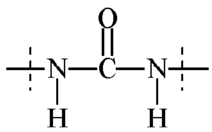

and $A^4$ is

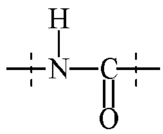 , and 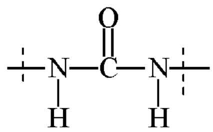 .

Preferably, in a compound of formula (aIII), $A^5$ is

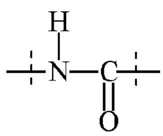 , and 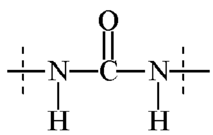 .

Preferably, in a compound of formula (aIV), $A^6$ is

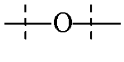 , 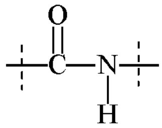 , and 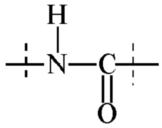 .

Preferably, in a compound of formula (aIV), $T^1$ is selected from H and $C_{1-6}$ alkyl.

In one embodiment, in a compound of formula (aI), the branching core B is selected from the following structures:

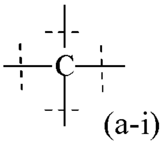 (a-i) 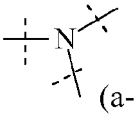 (a-ii) 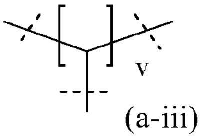 (a-iii) 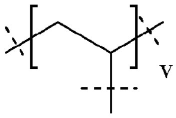 (a-iv)

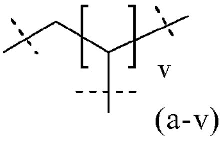 (a-v) 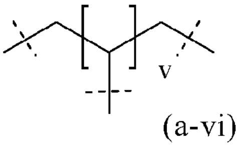 (a-vi) 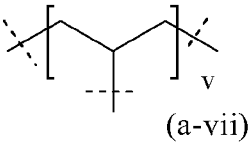 (a-vii)

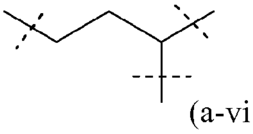 (a-viii) 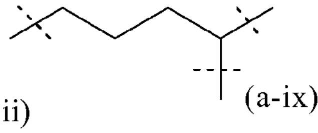 (a-ix) 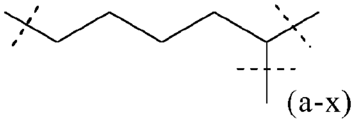 (a-x)

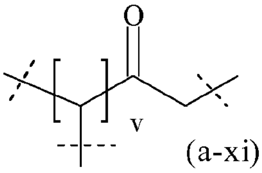 (a-xi) 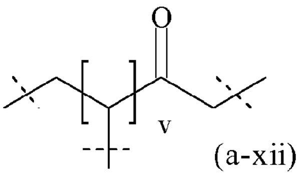 (a-xii) 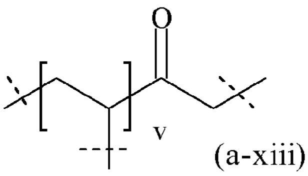 (a-xiii)

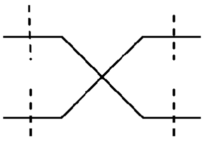 (a-xiv) 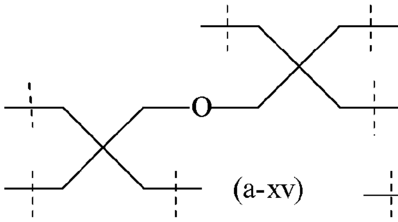 (a-xv) 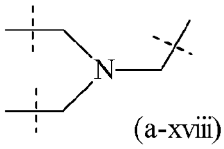 (a-xviii)

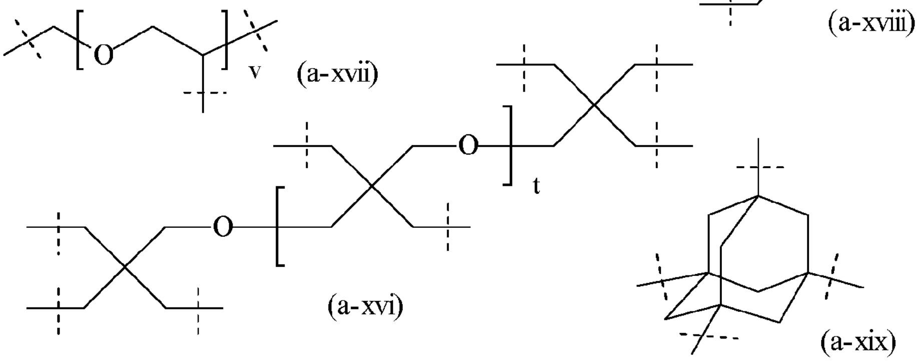 (a-xvii) (a-xvi) (a-xix)

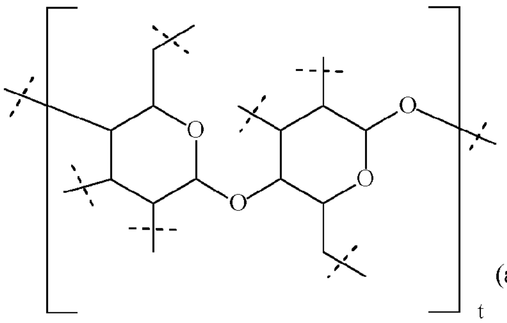

(a-xx)

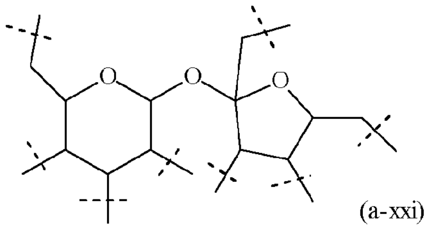

(a-xxi)

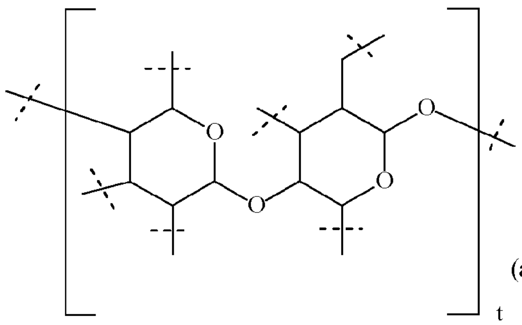

(a-xxii)

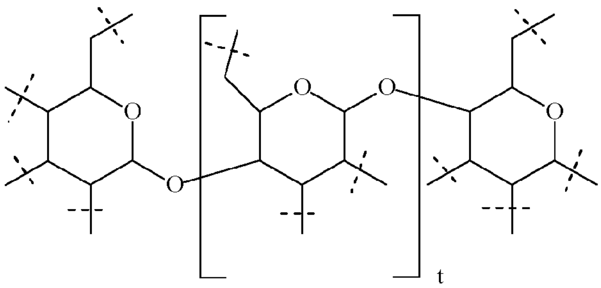

(a-xxiii)

wherein

dashed lines indicate attachment to $A^0$ or, if x1 and x2 are both 0, to $A^1$,
t is 1 or 2; preferably t is 1,
v is 1, 2, 3, 4, 5, ,6 ,7 ,8 , 9, 10, 11, 12, 13 or 14; preferably, v is 2, 3, 4, 5, 6; more preferably, v is 2, 4 or 6; most preferably, v is 2.

In a preferred embodiment, B has a structure of formula (a-i), (a-ii), (a-iii), (a-iv), (a-v), (a-vi), (a-vii), (a-viii), (a-ix), (a-x), (a-xiv), (a-xv) or (a-xvi). More preferably, B has a structure of formula (a-iii), (a-iv), (a-v), (a-vi), (a-vii), (a-viii), (a-ix), (a-x) or (a-iv). Most preferably, B has a structure of formula (a-xiv).

A preferred embodiment is a combination of B and $A^0$, or, if x1 and x2 are both 0 a preferred combination of B and $A^1$, which is selected from the following structures:

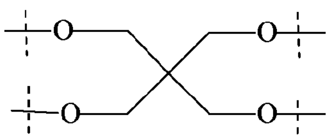

(b-i)

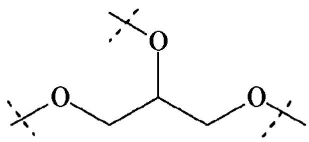

(b-ii)

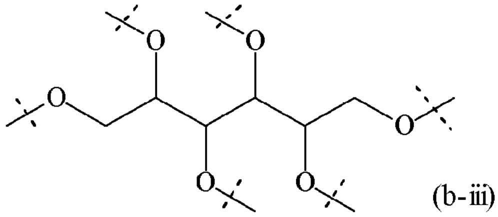

(b-iii)

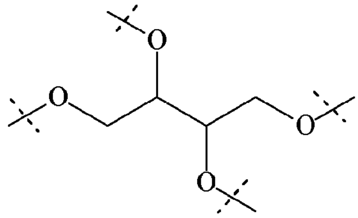

(b-iv)

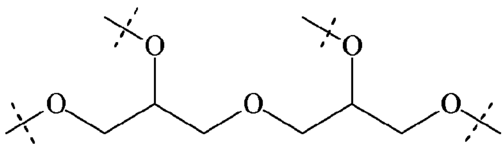

(b-v)

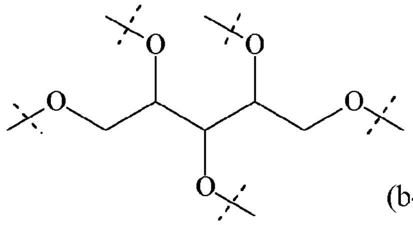

(b-vi)

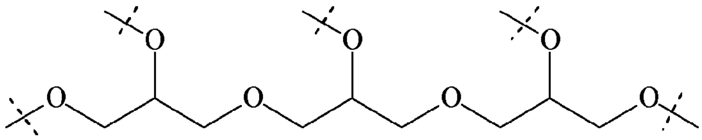

(b-vii)

wherein
dashed lines indicate attachment to SP or, if x1 and x2 are both 0, to P.

More preferably, the combination of B and $A^0$ or, if x1 and x2 are both 0, the combination of B and $A^1$, has a structure of formula of formula (b-i), (b-iv), (b-vi) or (b-viii) and most preferably has a structure of formula of formula (b-i).

In one embodiment, x1 and x2 of formula (aI) are 0.

In one embodiment, the PEG-based polymeric chain P has a molecular weight from 0.3 kDa to 40 kDa; e.g. from 0.4 to 35 kDa, from 0.6 to 38 kDA, from 0.8 to 30 kDa, from 1 to 25 kDa, from 1 to 15 kDa or from 1 to 10 kDa. Most preferably P has a molecular weight from 1 to 10 kDa.

In one embodiment, the PEG-based polymeric chain $P^1$ has a molecular weight from 0.3 kDa to 40 kDa; e.g. from 0.4 to 35 kDa, from 0.6 to 38 kDA, from 0.8 to 30 kDa, from 1 to 25 kDa, from 1 to 15 kDa or from 1 to 10 kDa. Most preferably $P^1$ has a molecular weight from 1 to 10 kDa.

In one embodiment, in the compounds of formulas (aI) or (aII), P has the structure of formula (c-i):

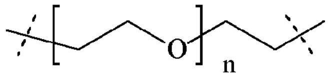

(c-i),

wherein n ranges from 6 to 900, more preferably n ranges from 20 to 700 and most preferably n ranges from 20 to 250.

In one embodiment, in the compounds of formulas (aIII), $P^1$ has the structure of formula (c-ii):

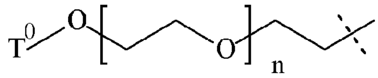

(c-ii),

wherein

n ranges from 6 to 900, more preferably n ranges from 20 to 700 and most preferably n ranges from 20 to 250;

$T^0$ is selected from the group comprising $C_{1-6}$ alkyl, $C_{2-6}$ alkenyl and $C_{2-6}$ alkynyl, which is optionally interrupted by one or more group(s) selected from -NH-,-N($C_{1-4}$ alkyl)-, -O-, -S-, -C(O)-, -C(O)NH-, -C(O)N($C_{1-4}$ alkyl)-, -O-C(O)-,-S(O)- or $-S(O)_2-$.

In one embodiment, in the compounds of formulas (aI) to (aIV), the moiety $Hyp^x$ is a polyamine and preferably comprises in bound form and, where applicable, in R- and/or S-configuration a moiety of the formulas (d-i), (d-ii), (d-iii) and/or (d-vi):

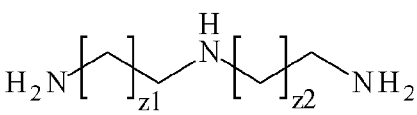

(d-i),

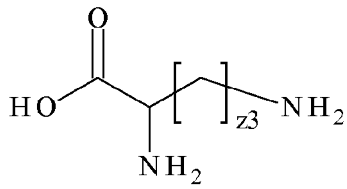

(d-ii),

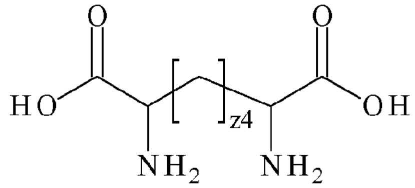

(d-iii),

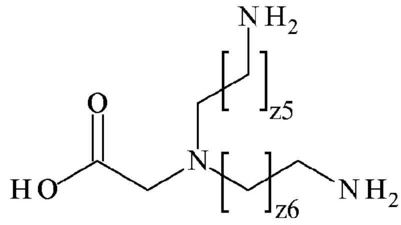

(d-iv),

wherein

z1, z2, z3, z4, z5, z6 are independently of each other 1, 2, 3, 4, 5, 6, 7 or 8.

More preferably, Hyp$^x$ comprises in bound form and in R- and/or S-configuration lysine, ornithine, diaminopropionic acid and/or diaminobutyric acid. Most preferably, Hyp$^x$ comprises in bound form and in R- and/or S-configuation lysine.

Hyp$^x$ has a molecular weight from 40 Da to 30 kDa, preferably from 0.3 kDa to 25 kDa, more preferably from 0.5 kDa to 20 kDa, even more preferably from 1 kDa to 20 kDa and most preferably from 2 kDa to 15 kDa.

Hyp$^x$ is preferably selected from the group consisting of

- a moiety of formula (e-i)

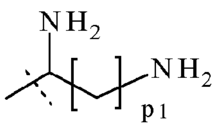

**(e-i)**

wherein

p1 is an integer from 1 to 5, preferably p1 is 4, and

the dashed line indicates attachment to $A^2$ if the backbone reagent has a structure of formula (aI) and to $A^3$ or $A^4$ if the backbone reagent has the structure of formula (aII);

- a moiety of formula (e-ii)

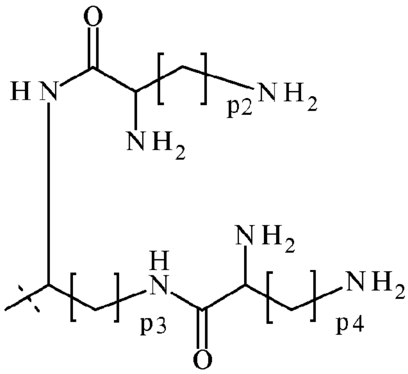

(e-ii)

wherein

p2, p3 and p4 are identical or different and each is independently of the others an integer from 1 to 5, preferably p2, p3 and p4 are 4, and
the dashed line indicates attachment to $A^2$ if the backbone reagent has a structure of formula (aI), to $A^3$ or $A^4$ if the backbone reagent has a structure of formula (aII), to $A^5$ if the backbone reagent has a structure of formula (aIII) and to $A^6$ if the backbone reagent has a structure of formula (aIV);

- a moiety of formula (e-iii)

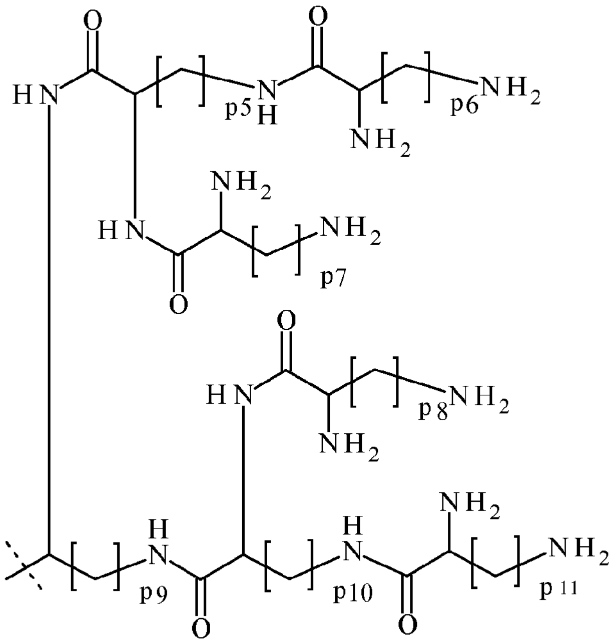

(e-iii)

wherein

p5 to p11 are identical or different and each is independently of the others an integer from 1 to 5, preferably p5 to p11 are 4, and
the dashed line indicates attachment to $A^2$ if the backbone reagent is of formula (aI), to $A^3$ or $A^4$ if the backbone reagent is of formula (aII), to $A^5$ if the backbone reagent is of formula (aIII) and to $A^6$ if the backbone reagent is of formula (aIV);

- a moiety of formula (e-iv)

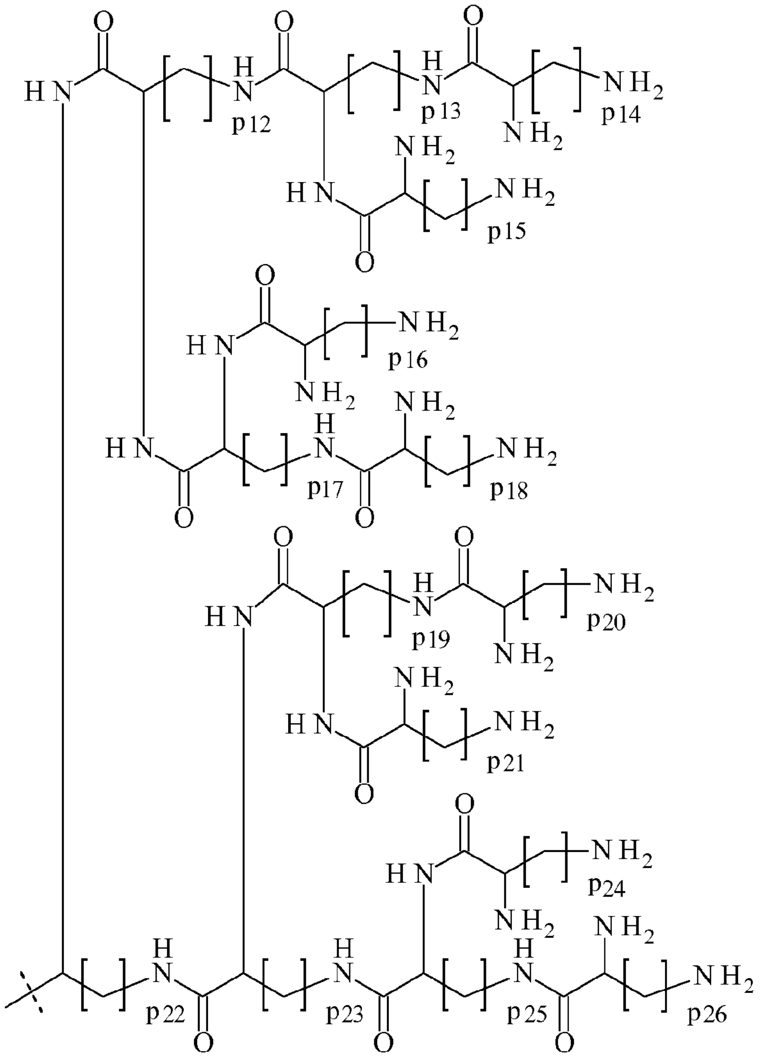

(e-iv)

wherein

p12 to p26 are identical or different and each is independently of the others an integer from 1 to 5, preferably p12 to p26 are 4, and

the dashed line indicates attachment to $A^2$ if the backbone reagent has a structure of formula (aI),to $A^3$ or $A^4$ if the backbone reagent has a structure of formula (aII), to $A^5$ if the backbone reagent has a structure of formula (aIII) and to $A^6$ if the backbone reagent has a structure of formula (aIV);

- a moiety of formula (e-v)

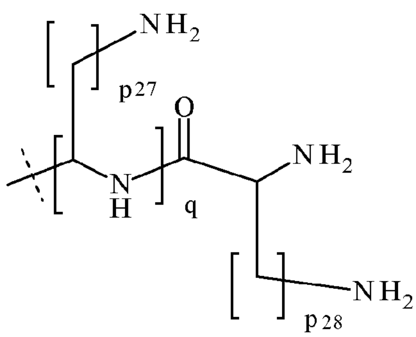

(e-v)

wherein

p27 and p28 are identical or different and each is independently of the other an integer from 1 to 5, preferably p27 and p28 are 4,
q is an integer from 1 to 8, preferably q is 2 or 6 and most preferably 1 is 6, and
the dashed line indicates attachment to $A^2$ if the backbone reagent has a structure of formula (aI), to $A^3$ or $A^4$ if the backbone reagent has a structure of formula (aII), to $A^5$ if the backbone reagent has a structure of formula (aIII) and to $A^6$ if the backbone reagent has a structure of formula (aIV);

- a moiety of formula (e-vi)

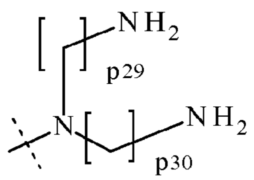

**(e-vi)**

wherein

p29 and p30 are identical or different and each is independently of the other an integer from 2 to 5, preferably p29 and p30 are 3, and
the dashed line indicates attachment to $A^2$ if the backbone reagent has the structure of formula (aI), to $A^3$ or $A^4$ if the backbone reagent has the structure of formula (aII), to $A^5$ if the backbone reagent has the structure of formula (aIII) and to $A^6$ if the backbone reagent has the structure of formula (aIV);

- a moiety of formula (e-vii)

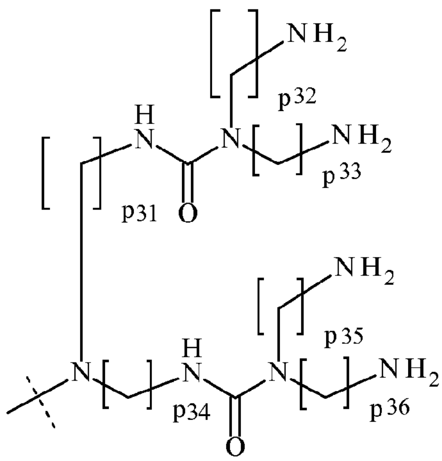

**(e-vii)**

wherein

p31 to p36 are identical or different and each is independently of the others an integer from 2 to 5, preferably p31 to p36 are 3, and
the dashed line indicates attachment to $A^2$ if the backbone reagent has a structure of formula (aI), to $A^3$ or $A^4$ if the backbone reagent has a structure of formula (aII), to $A^5$ if the backbone reagent has a structure of formula (aIII) and to $A^6$ if the backbone reagent has a structure of formula (aIV);

- a moiety of formula (e-viii)

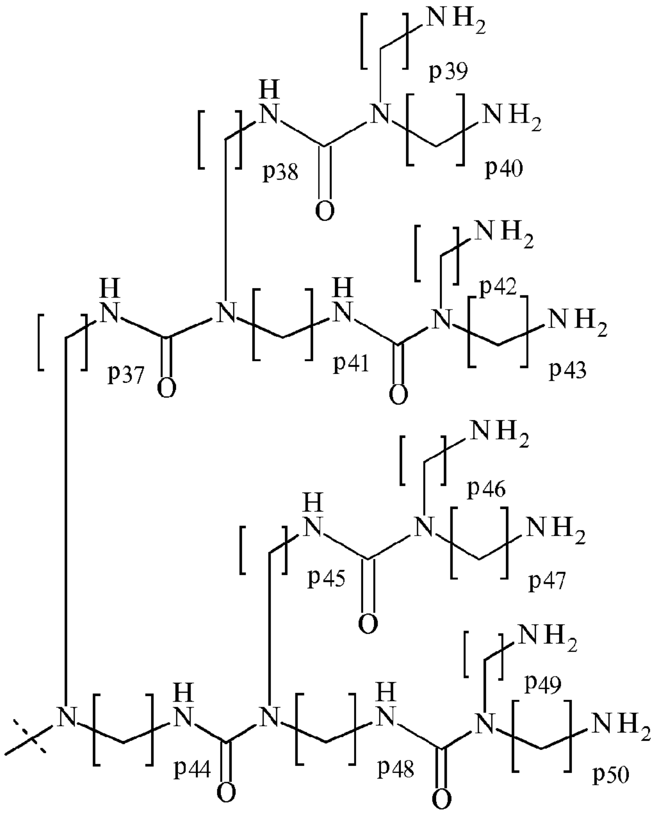

(e-viii)

wherein

p37 to p50 are identical or different and each is independently of the others an integer from 2 to 5, preferably p37 to p50 are 3, and

the dashed line indicates attachment to $A^2$ if the backbone reagent has a structure of formula (aI), to $A^3$ or $A^4$ if the backbone reagent has a structure of formula (aII), to $A^5$ if the backbone reagent has a structure of formula (aIII) and to $A^6$ if the backbone reagent has a structure of formula (aIV); and

- a moiety of formula (e-ix):

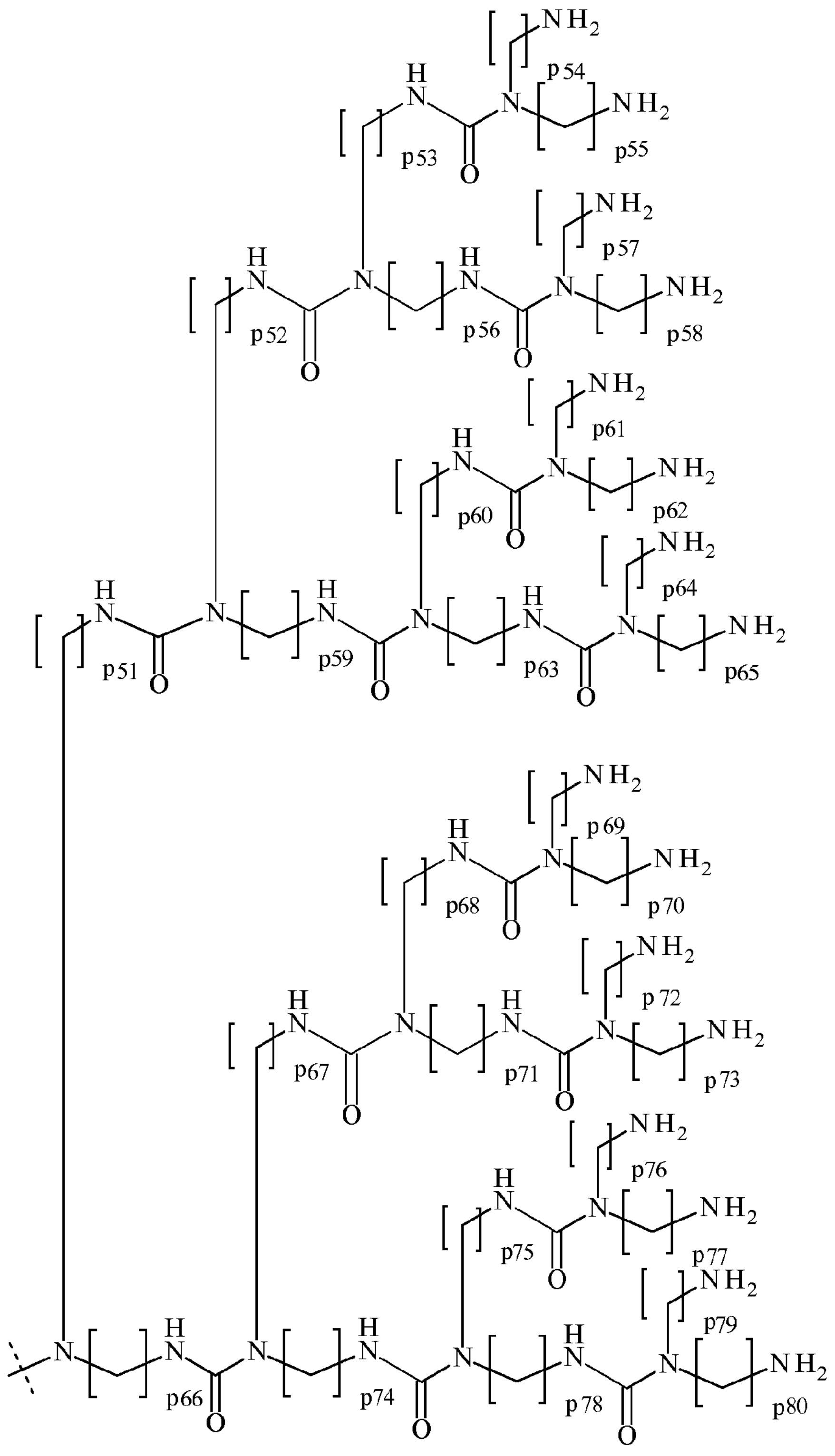

(e-ix)

wherein

p51 to p80 are identical or different and each is independently of the others an integer from 2 to 5, preferably p51 to p80 are 3, and

the dashed line indicates attachment to $A^2$ if the backbone reagent has a structure of formula (aI), to $A^3$ or $A^4$ if the backbone reagent has a structure of formula (aII), to $A^5$ if the backbone reagent has a structure of formula (aIII) and to $A^6$ if the backbone reagent has a structure of formula (aIV); and

wherein the moieties (e-i) to (e-v) may at each chiral center be in either R- or S-configuration, preferably, all chiral centers of a moiety (e-i) to (e-v) are in the same configuration.

Preferably, $Hyp^x$ is has a structure of formulas (e-i), (e-ii), (e-iii), (e-iv), (e-vi), (e-vii), (e-viii) or (e-ix). More preferably, $Hyp^x$ has a structure of formulas (e-ii), (e-iii), (e-iv), (e-vii), (e-viii) or (e-ix), even more preferably $Hyp^x$ has a structure of formulas (e-ii), (e-iii), (e-vii) or (e-viii) and most preferably $Hyp^x$ has the structure of formula (e-iii).

If the backbone reagent has a structure of formula (aI), a preferred moiety - $A^2$ - $Hyp^1$ is a moiety of the formula

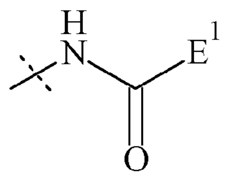

,

wherein

the dashed line indicates attachment to P; and
$E^1$ is selected from formulas (e-i) to (e-ix).

If the backbone reagent has a structure of formula (aII) a preferred moiety $Hyp^2$ - $A^3$ - is a moiety of the formula

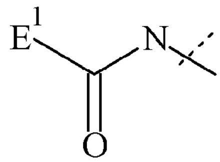

,

wherein

the dashed line indicates attachment to P; and
$E^1$ is selected from formulas (e-i) to (e-ix);
and a preferred moiety - $A^4$ - $Hyp^3$ is a moiety of the formula

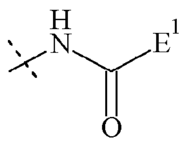

,

wherein

the dashed line indicates attachment to P; and
$E^1$ is selected from formulas (e-i) to (e-ix).

If the backbone reagent has a structure of formula (aIII), a preferred moiety - $A^5$ - $Hyp^4$ is a moiety of the formula

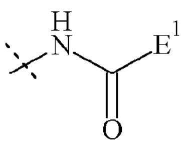

,

wherein

the dashed line indicates attachment to $P^1$; and
$E^1$ is selected from formulas (e-i) to (e-ix).

More preferably, the backbone reagent has a structure of formula (aI) and B is has a structure of formula (a-xiv).
Even more preferably, the backbone reagent has the structure of formula (aI), B has the structure of formula (a-xiv), x1 and x2 are 0, and $A^1$ is -O-.
Even more preferably, the backbone reagent has the structure of formula (aI), B has the structure of formula (a-xiv), $A^1$ is -O-, and P has a structure of formula (c-i).
Even more preferably, the backbone reagent is formula (aI), B is of formula (a-xiv), x1 and x2 are 0, $A^1$ is -O-, P is of formula (c-i), $A^2$ is -NH-(C=O)- and $Hyp^1$ is of formula (e-iii).
Most preferably, the backbone reagent has the following formula:

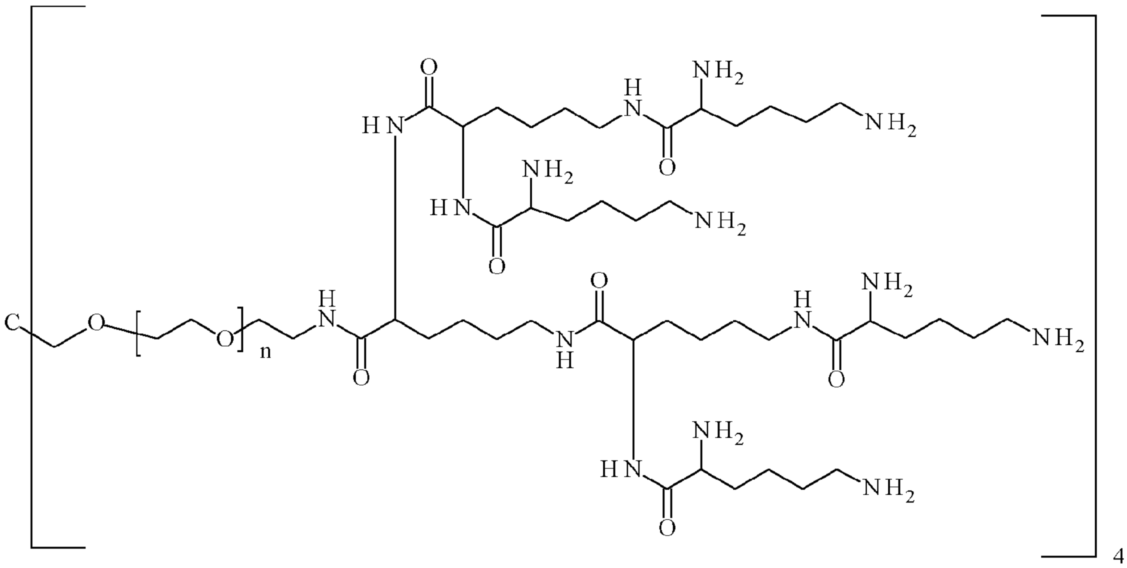

wherein

n ranges from 10 to 40, preferably from 10 to 30, more preferably from 20 to 30 and most preferably n is 28.

SP is a spacer moiety selected from the group comprising $C_{1-6}$ alkyl, $C_{2-6}$ alkenyl and $C_{2-6}$ alkynyl, preferably SP is $-CH_2-$, $-CH_2-CH_2-$, $-CH(CH_3)-$, $-CH_2-CH_2-CH_2-$, $-CH(C_2H_5)-$, $-C(CH_3)_2-$, -CH=CH- or -CH=CH-, most preferably SP is $-CH_2-$, $-CH_2-CH_2-$ or -CH=CH-.
The at least one crosslinker reagent of step (a-ii) comprises at least two carbonyloxy groups (-(C=O)-O- or -O-(C=O)-), which are biodegradable linkages. These biodegradable linkages are necessary to render the hydrogel biodegradable. Additionally, the at least one crosslinker reagent comprises at least two activated functional end groups which during the polymerization of step (b) react with the amines of the at least one backbone reagent.
The crosslinker reagent has a molecular weight ranging from 0.5 to 40 kDa, more preferably ranging from 0.75 to 30 kDa, even more preferably ranging from 1 to 20 kDa, even more preferably ranging from 1 to 10 kDa, even more preferably ranging from 1 to 7.5 kDa and most preferably ranging from 2 kDa to 4 kDa.
The crosslinker reagent comprises at least two activated functional end groups selected from the group comprising activated ester groups, activated carbamate groups, activated carbonate groups and activated thiocarbonate groups, which during polymerization react with the amine groups of the backbone reagents, forming amide bonds.
In one preferred embodiment, the crosslinker reagent is a compound of formula (V-I):

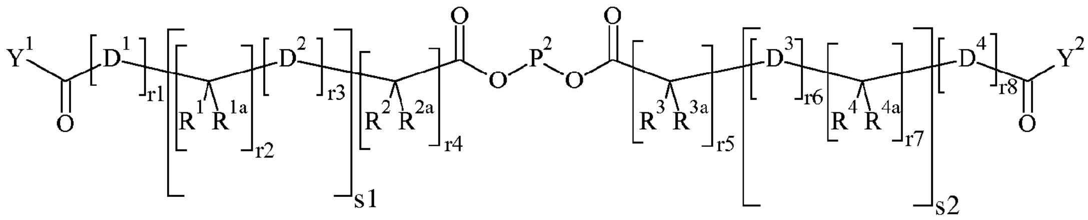

(V-I),

wherein

| | |
|---|---|
| each $D^1$, $D^2$, $D^3$ and $D^4$ | are identical or different and each is independently of the others selected from the group comprising -O-, $-NR^5-$, -S- and $-CR^6R^{6a}-$; |
| each $R^1$, $R^{1a}$, $R^2$, $R^{2a}$, $R^3$, $R^{3a}$, $R^4$, $R^{4a}$, $R^6$ and $R^{6a}$ | are identical or different and each is independently of the others selected from the group comprising -H, $-OR^7$, $-NR^7R^{7a}$, $-SR^7$ and $C_{1-6}$ alkyl; optionally, each of the pair(s) $R^1/R^2$, $R^3/R^4$, $R^{1a}/R^{2a}$, and $R^{3a}/R^{4a}$ may independently form a chemical bond and/or each of the pairs $R^1/R^{1a}$, $R^2/R^{2a}$, $R^3/R^{3a}$, $R^4/R^{4a}$, $R^6/R^{6a}$, $R^1/R^2$, $R^3/R^4$, $R^{1a}/R^{2a}$, and $R^{3a}/R^{4a}$ are independently of each other joined together with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl; |
| each $R^5$ | is independently selected from -H and $C_{1-6}$ alkyl; optionally, each of the pair(s) $R^1/R^5$, $R^2/R^5$, $R^3/R^5$, $R^4/R^5$ and $R^5/R^6$ may independently form a chemical bond and/or are joined together with the atom to which they are attached to form a 4- to 7-membered heterocyclyl or 8- to 11-membered heterobicyclyl; |
| each $R^7$, $R^{7a}$ | is independently selected from H and $C_{1-6}$ alkyl; |
| A | is selected from the group consisting of indenyl, indanyl and tetralinyl; |
| $P^2$ | is |

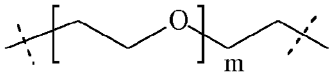

m ranges from 120 to 920, preferably from 120 to 460 and more preferably from 120 to 230;

r1, r2, r7, r8 are independently 0 or 1;

r3, r6 are independently 0, 1, 2, 3, or 4;

r4, r5 are independently 1, 2, 3, 4, 5, 6, 7, 8, 9 or 10;

s1, s2 are independently 1, 2, 3, 4, 5 or 6;

$Y^1$, $Y^2$ are identical or different and each is independently of the other selected from formulas (f-i) to (f-vi):

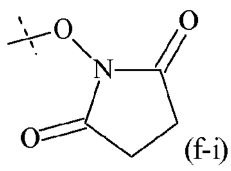
(f-i) ,
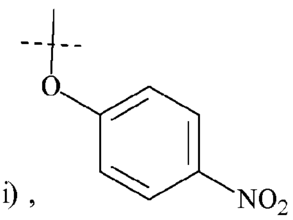
(f-ii) ,
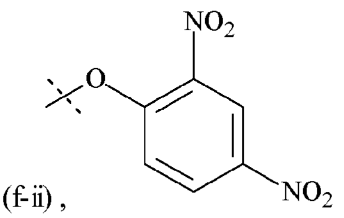
(f-iii),

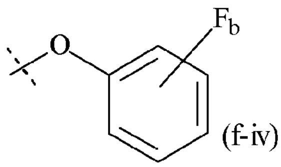 (f-iv), 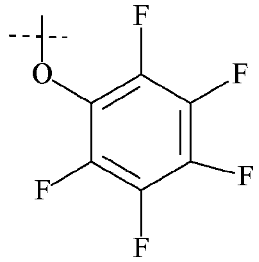 (f-v) or 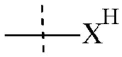 (f-vi)

wherein

the dashed lines indicate attachment to the rest of the molecule,
b is 1, 2, 3 or 4
$X^H$ is Cl, Br, I, or F.

Preferably, the crosslinker reagent is a compound of formula (V-II):

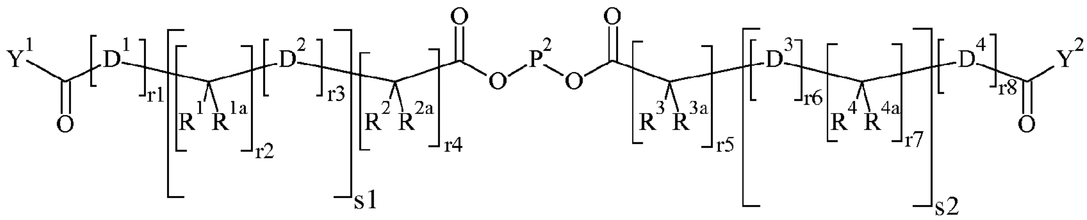

(V-II),

wherein

$D^1$, $D^2$, $D^3$ and $D^4$ are identical or different and each is independently of the others selected from the group comprising O, $NR^5$, S and $CR^5R^{5a}$;

$R^1$, $R^{1a}$, $R^2$, $R^{2a}$, $R^3$, $R^{3a}$, $R^4$, $R^{4a}$, $R^5$ and $R^{5a}$ are identical or different and each is independently of the others selected from the group comprising H and $C_{1-6}$ alkyl; optionally, one or more of the pair(s) $R^1/R^{1a}$, $R^2/R^{2a}$, $R^3/R^{3a}$, $R^4/R^{4a}$, $R^1/R^2$, $R^3/R^4$, $R^{1a}/R^{2a}$, and $R^{3a}/R^{4a}$ form a chemical bond or are joined together with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl;

A is selected from the group consisting of phenyl, naphthyl, indenyl, indanyl and tetralinyl;

$P^2$ is

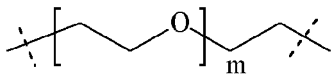

m ranges from 11 to 908, preferably from 17 to 680, even more preferably from 22 to 454, even more preferably from 22 to 227, even more preferably from 22 to 170 and more preferably from 45 to 90;
r1, r2, r7, r8 are independently 0 or 1;
r3, r6 are independently 0, 1, 2, 3, or 4;
r4, r5 are independently 1, 2, 3, 4, 5, 6, 7, 8, 9 or 10;
s1, s2 are independently 1, 2, 3, 4, 5 or 6;
$Y^1$, $Y^2$ are identical or different and each is independently of the other selected from formulas (f-i) to (f-vi):

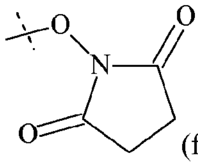 (f-i) , 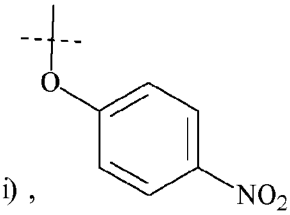 (f-ii) , 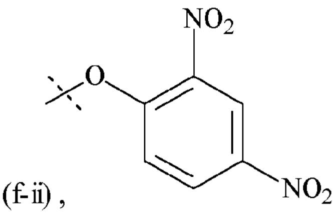 (f-iii),

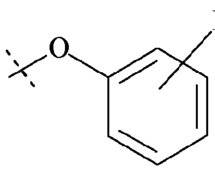 (f-iv), 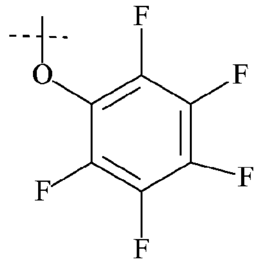 (f-v) or 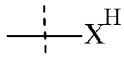 (f-vi)

wherein

the dashed lines indicate attachment to the rest of the molecule,
b is 1, 2, 3 or 4
$X^H$ is Cl, Br, I, or F.

It is understood that the moieties

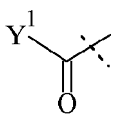 and 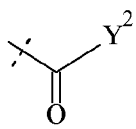

represent the at least two activated functional end groups.

Preferably, $Y^1$ and $Y^2$ of formula (V-I) or (V-II) have a structure of formula (f-i), (f-ii) or (f-v). More preferably, $Y^1$ and $Y^2$ of formula (V-I) or (V-II) have a structure of formula (f-i) or (f-ii) and most preferably, $Y^1$ and $Y^2$ have a structure of formula (f-i).

Preferably, both moieties $Y^1$ and $Y^2$ of formula (V-I) or (V-II) have the same structure. More preferably, both moieties $Y^1$ and $Y^2$ have the structure of formula (f-i).

Preferably, r1 of formula (V-I) or (V-II) is 0.

Preferably, r1 and s1 of formula (V-I) or (V-II) are both 0.

Preferably, one or more of the pair(s) $R^1/R^{1a}$, $R^2/R^{2a}$, $R^3/R^{3a}$, $R^4/R^{4a}$, $R^1/R^2$, $R^3/R^4$, $R^{1a}/R^{2a}$, and $R^{3a}/R^{4a}$ of formula (V-I) or (V-II) form a chemical bond or are joined together with the atom to which they are attached to form a $C_{3-8}$ cycloalkyl or form a ring A.

Preferably, one or more of the pair(s) $R^1/R^2$, $R^{1a}/R^{2a}$, $R^3/R^4$, $R^{3a}/R^{4a}$ of formula (V-I) or (V-II) are joined together with the atom to which they are attached to form a 4- to 7-membered heterocyclyl or 8- to 11-membered heterobicyclyl.

Preferably, the crosslinker reagent of formula (V-I) and (V-II) is symmetric, i.e. the moiety

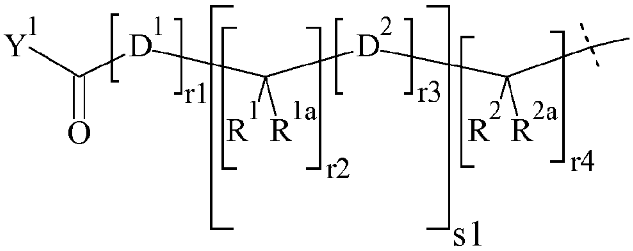

has the same structure as the moiety

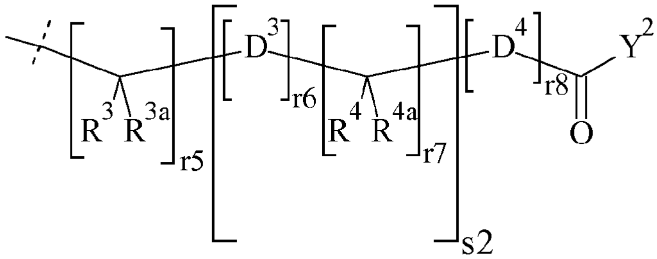

In one preferred embodiment s1, s2, r1 and r8 of formula (V-I) and (V-II) are 0.

In another preferred embodiment s1, s2, r1 and r8 of formula (V-I) and (V-II) are 0 and r4 of formula (V-I) and (V-II) and r5 are 1.

Preferred crosslinker reagents are of formula (V-1) to (V-54):

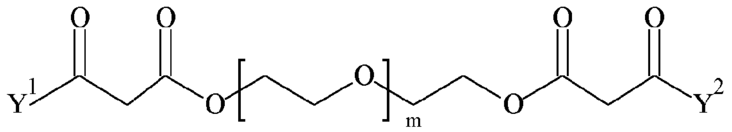

(V-1),

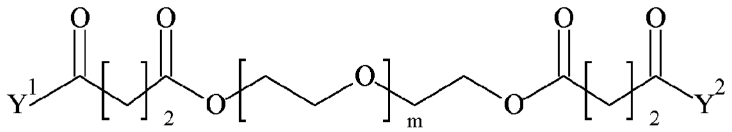

(V-2),

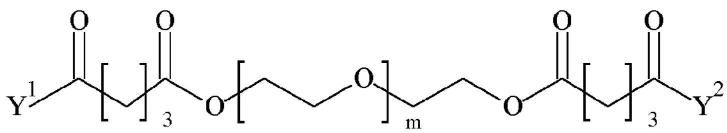

(V-3),

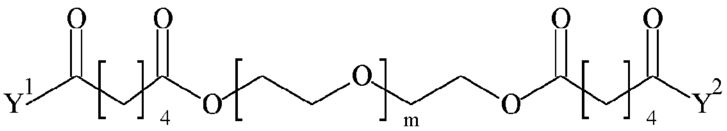

(V-4),

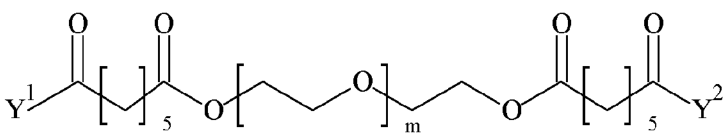

(V-5),

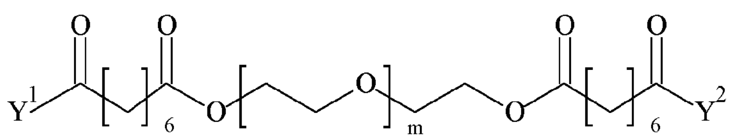

(V-6),

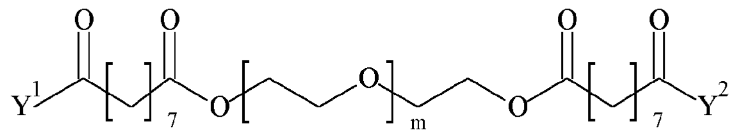

(V-7),

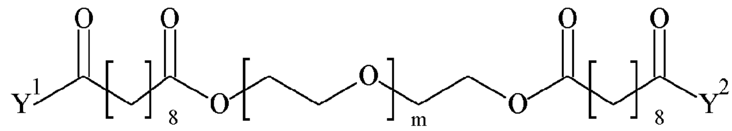

(V-8),

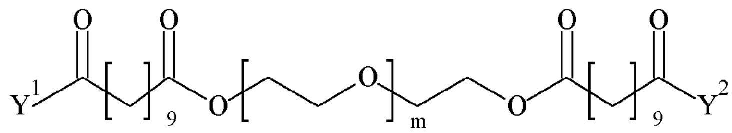

(V-9),

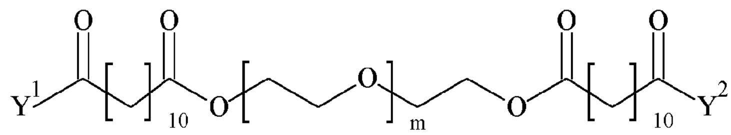

(V-10),

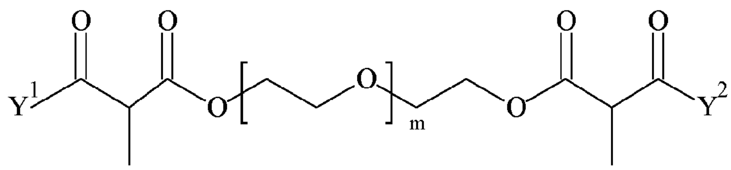

(V-11)
,

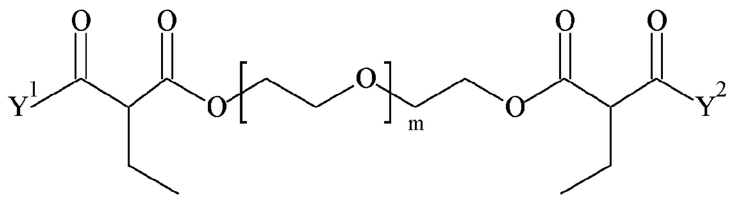

(V-12)
,

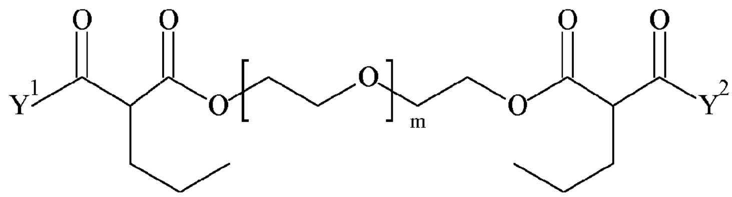

(V-13)
,

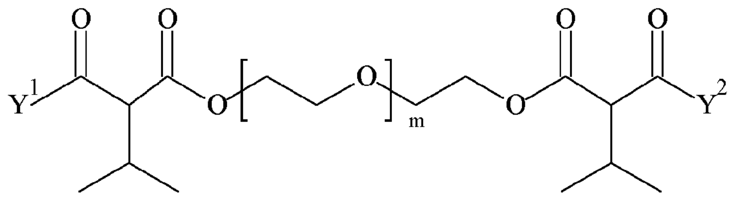

(V-14)
,

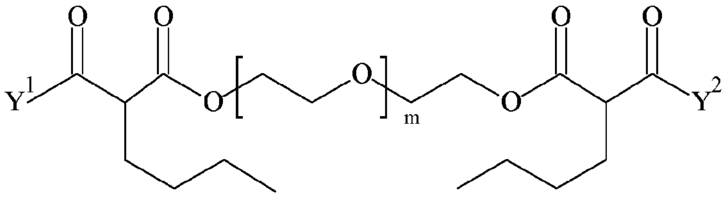

(V-15),

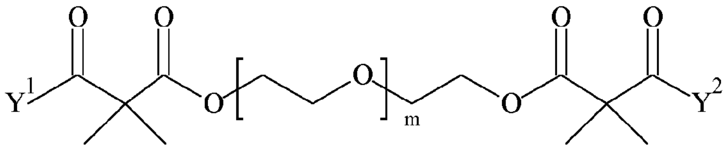

(V-16),

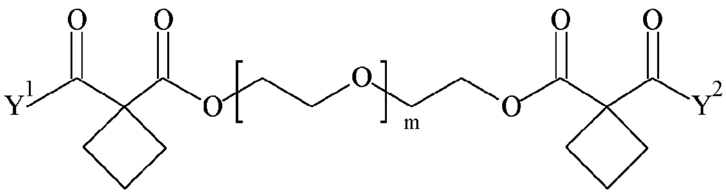

(V-17)
,

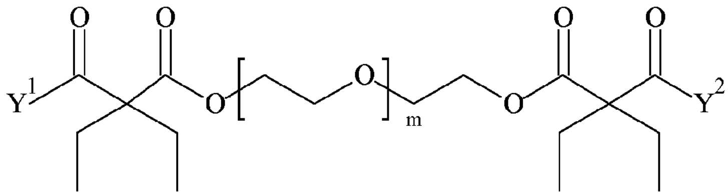

(V-18)
,

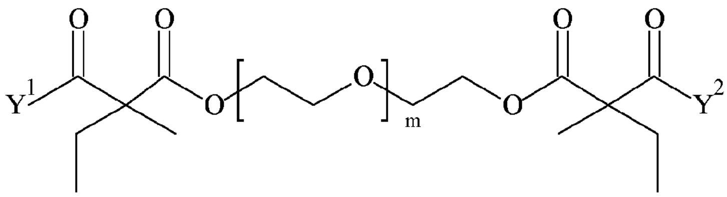

(V-19)

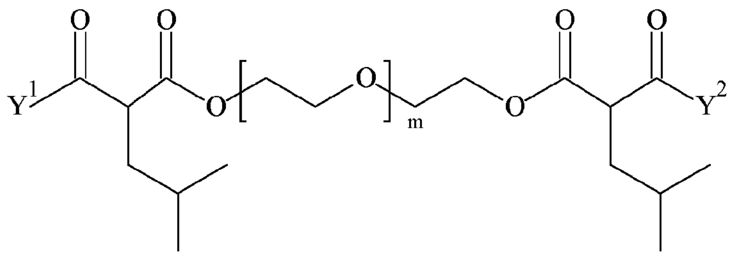

(V-20)

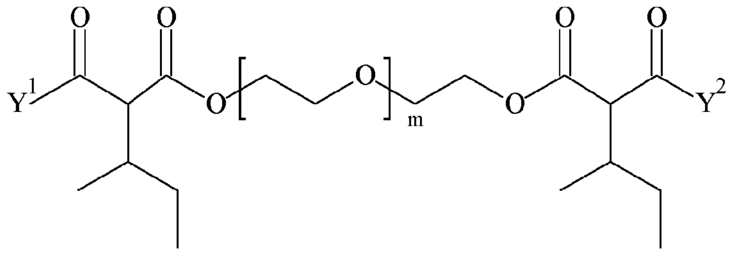

(V-21)

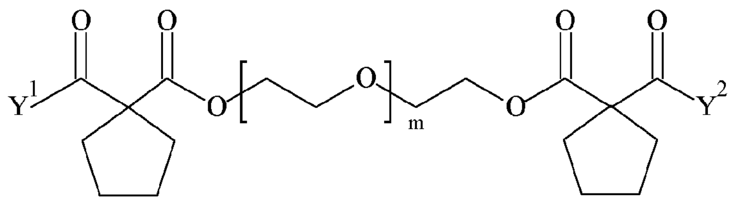

(V-22)

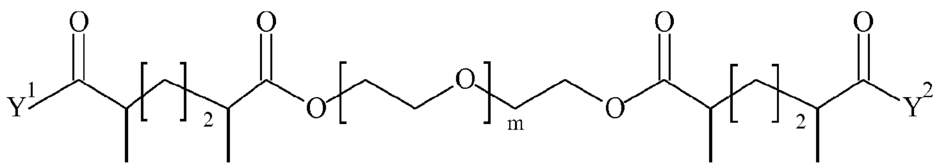

(V-23)

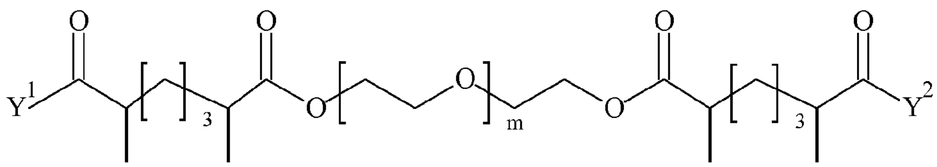

(V-24)

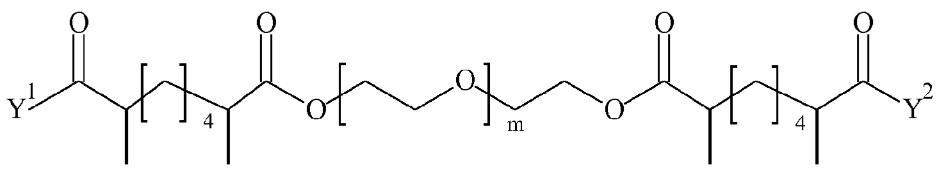

(V-25)

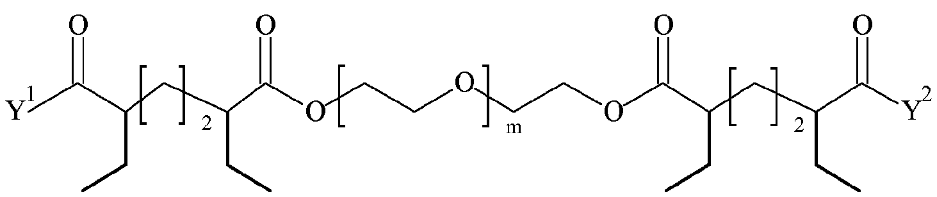

(V-26)

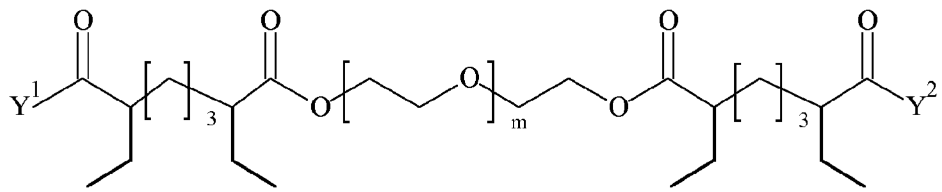

(V-27)

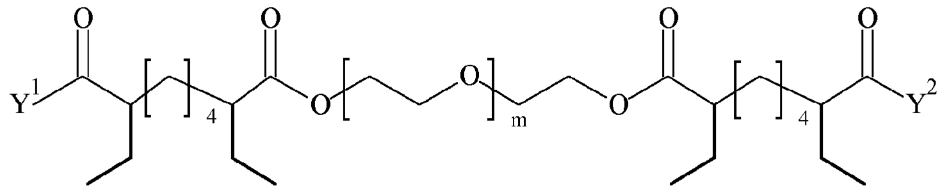

(V-28)

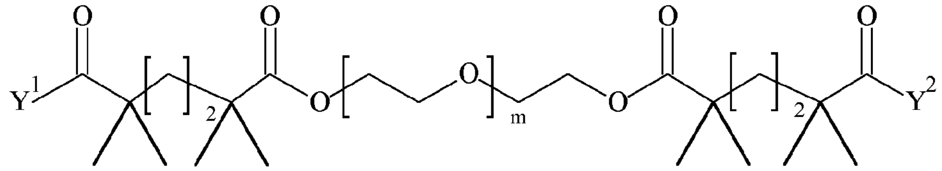

(V-29)

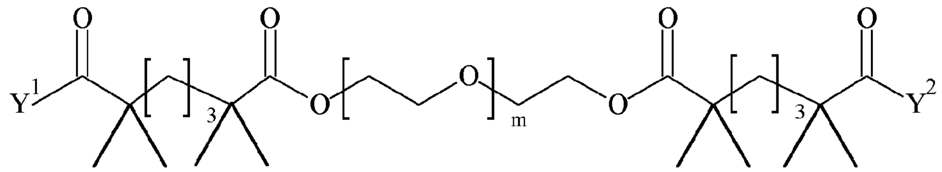

(V-30)

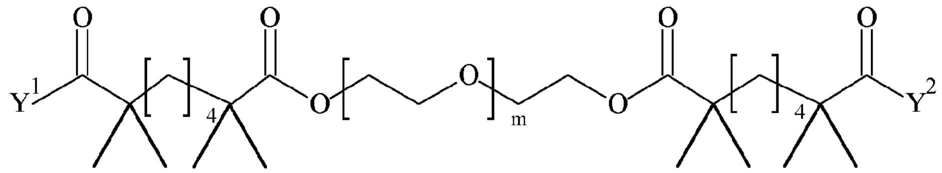

(V-31)

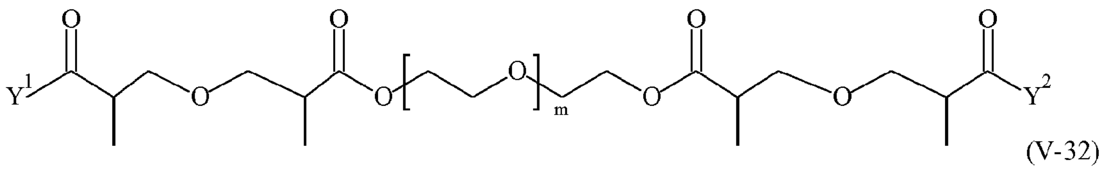

(V-32)

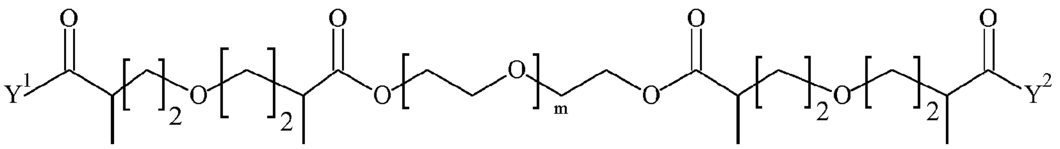

(V-33)

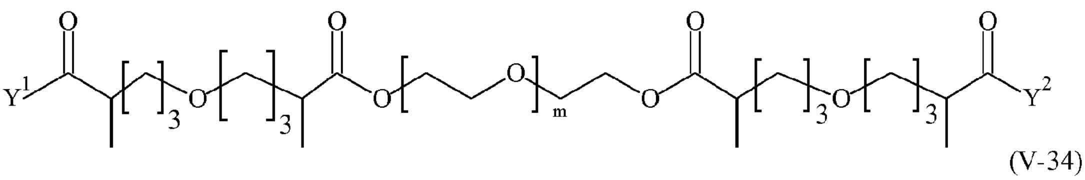

(V-34)

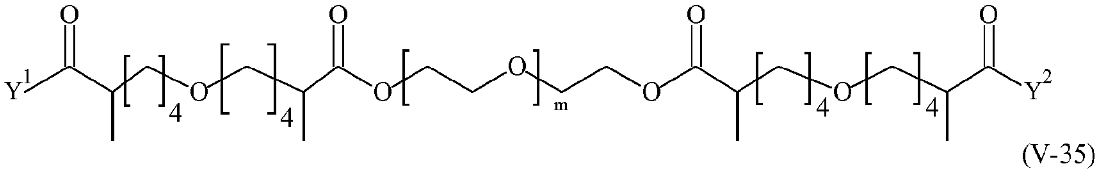

(V-35)

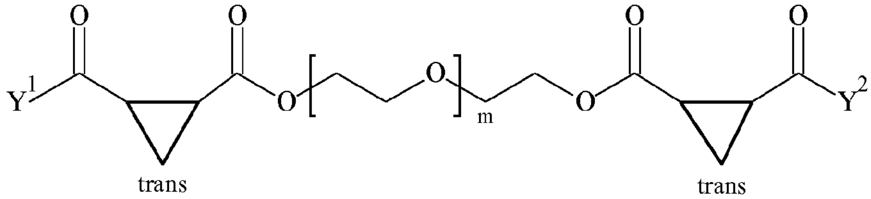

(V-36)

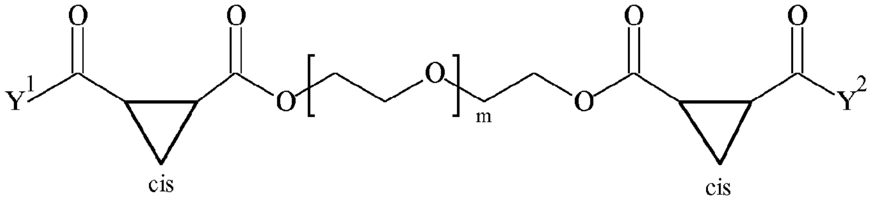

(V-37)

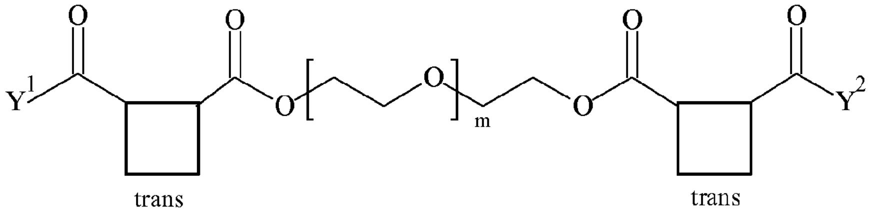

(V-38)

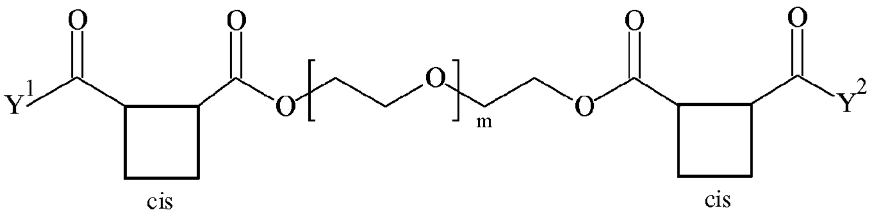

(V-39)

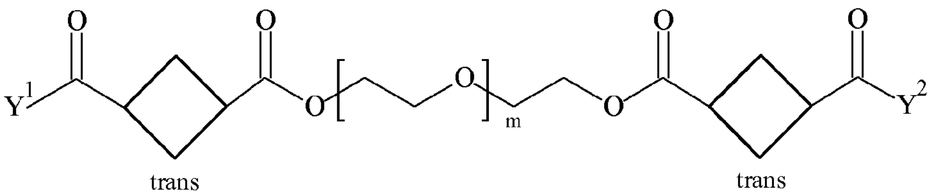

(V-40)

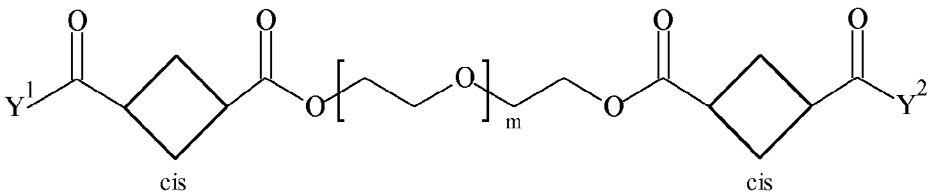

(V-41)

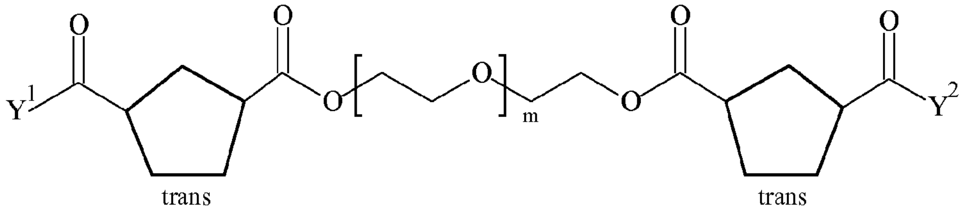

(V-42)

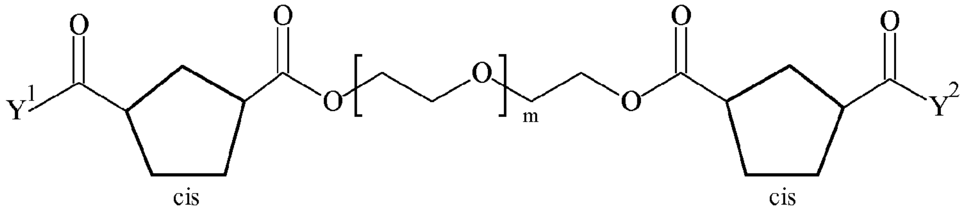

(V-43)

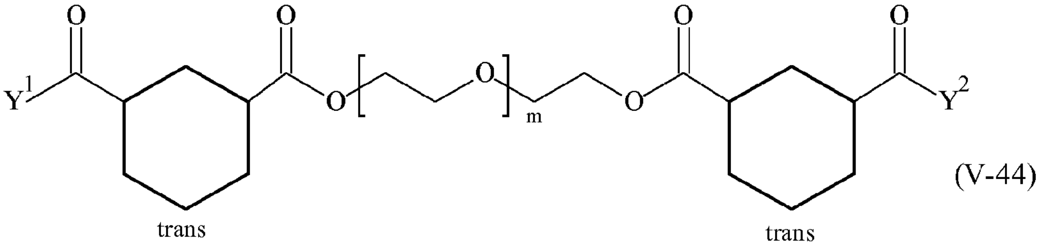

(V-44)

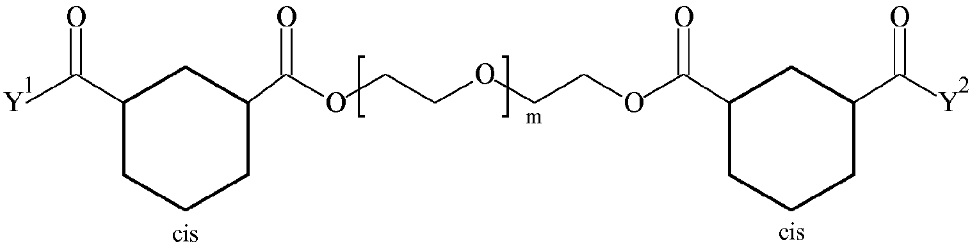

(V-45)

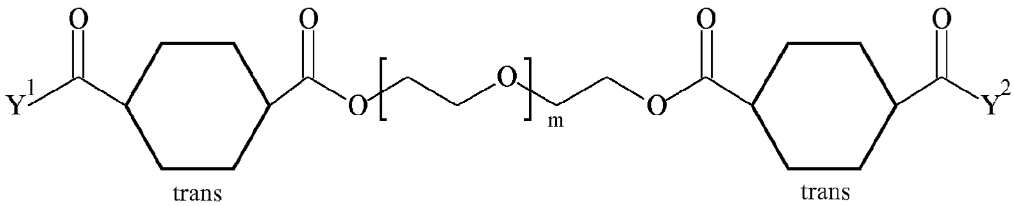

(V-46)

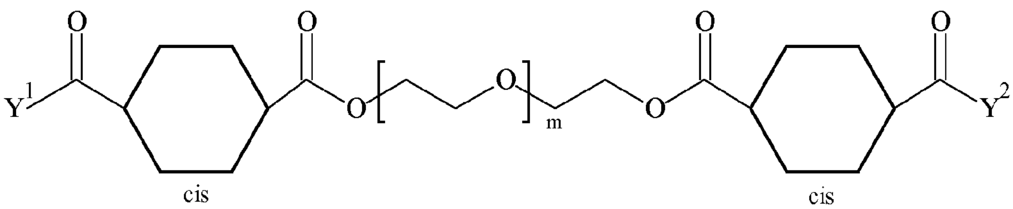

(V-47)

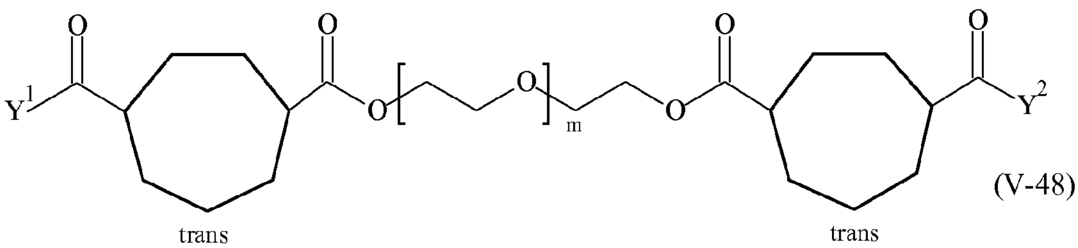

(V-48)

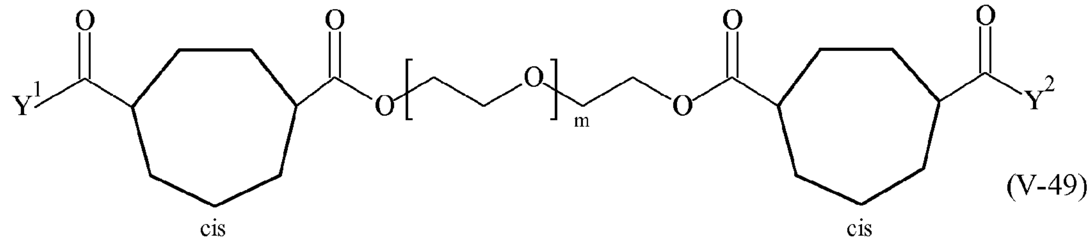

(V-49)

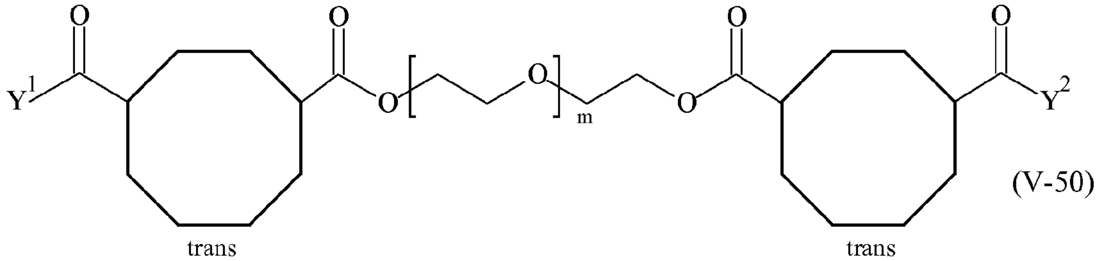

(V-50)

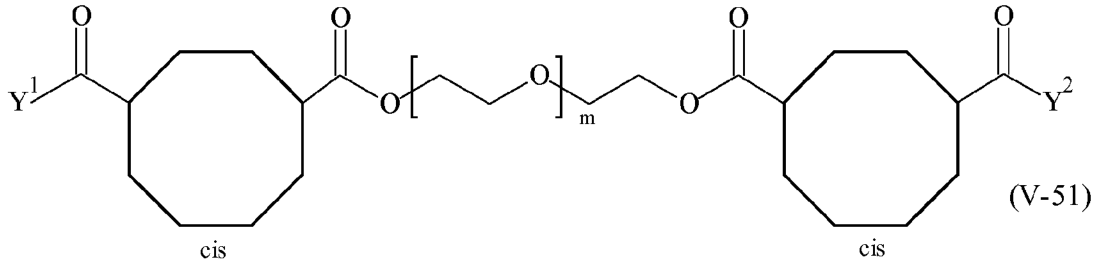

(V-51)

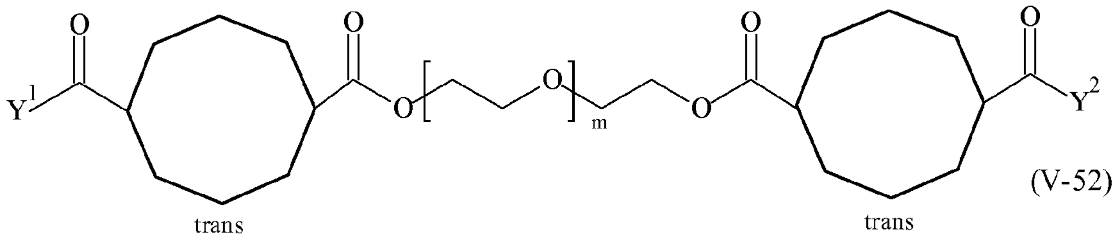

(V-52)

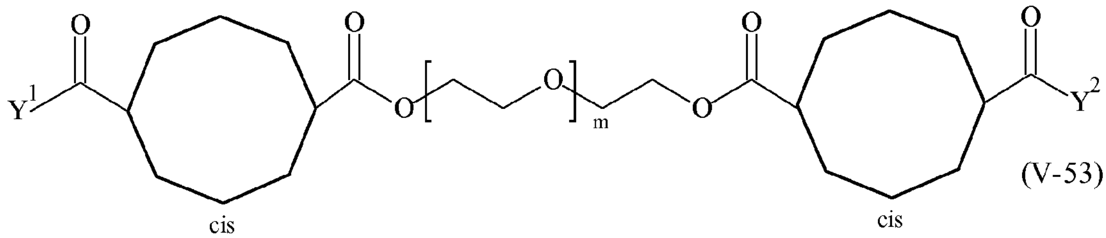

(V-53)

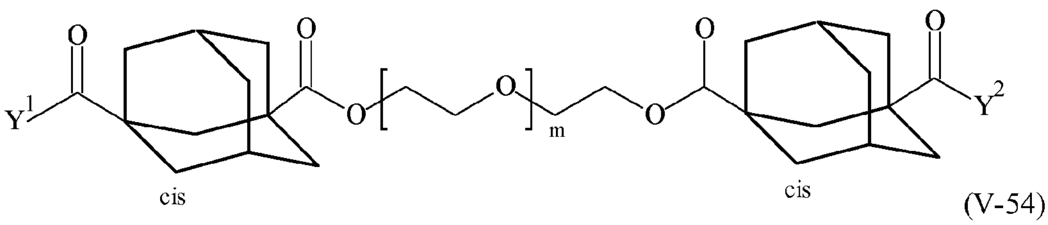

(V-54)

wherein

each crosslinker reagent may be in the form of its racemic mixture, where applicable; and m, $Y^1$ and $Y^2$ are defined as above.

Even more preferred crosslinker reagents are of formula (Va-1) to (Va-54):

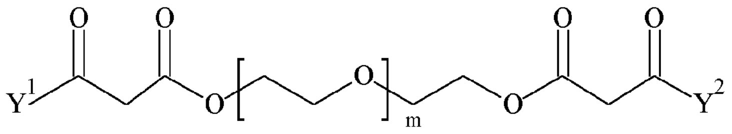

(Va-1)

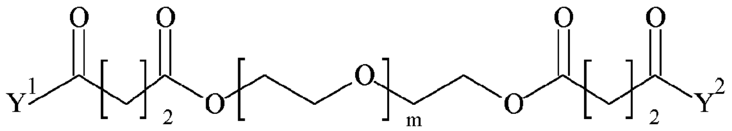

(Va-2)

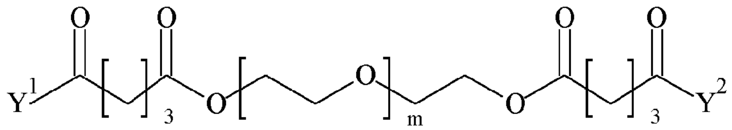

(Va-3)

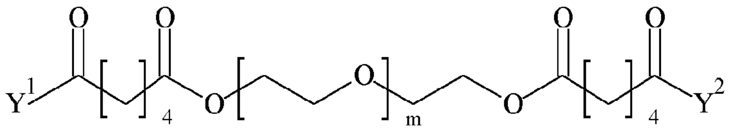

(Va-4)

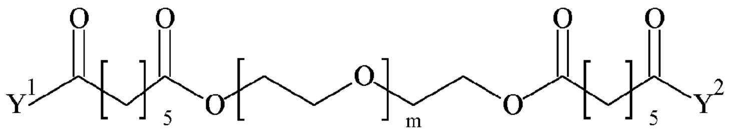

(Va-5)

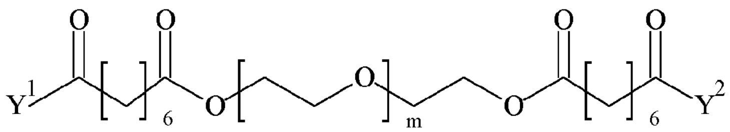

(Va-6)

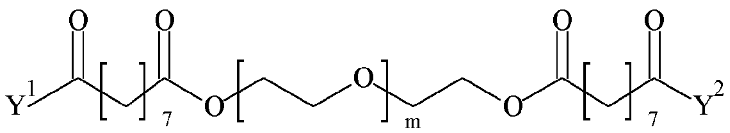

(Va-7)

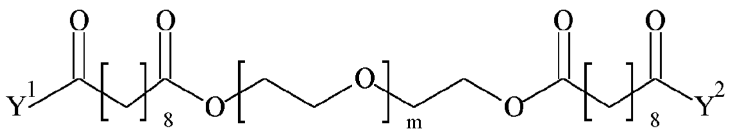

(Va-8)

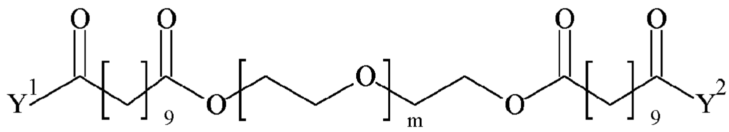

(Va-9)

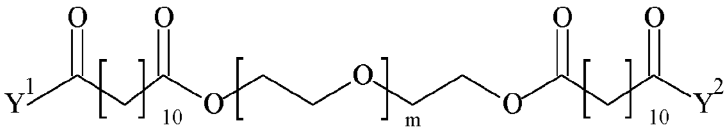

(Va-10)

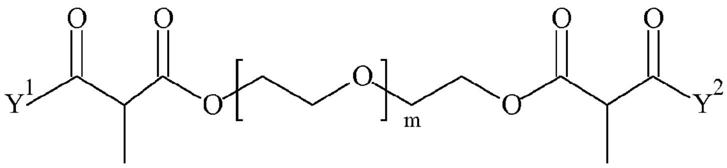

(Va-11)

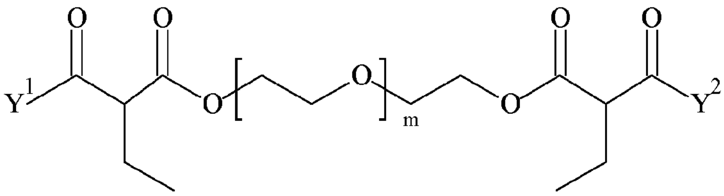

(Va-12)

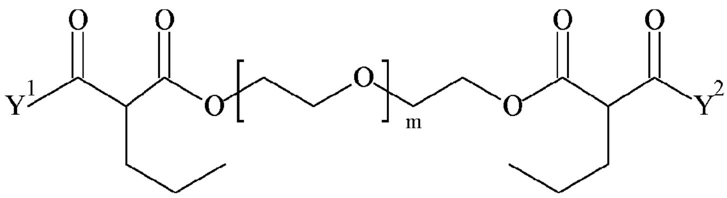

(Va-13)

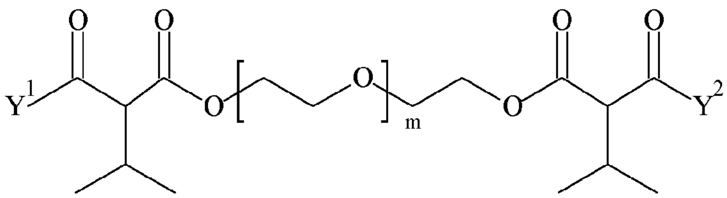

(Va-14)

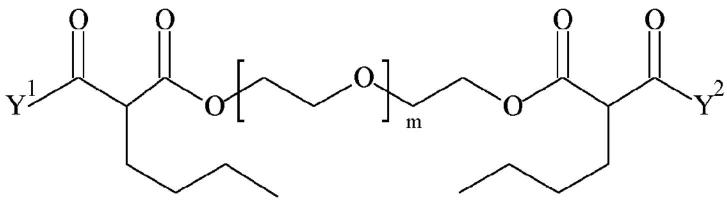

(Va-15)

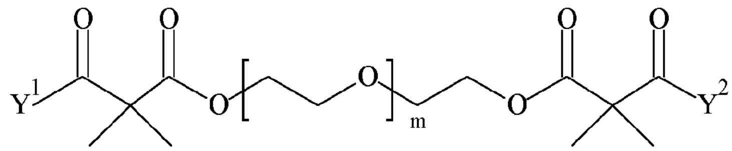

(Va-16)

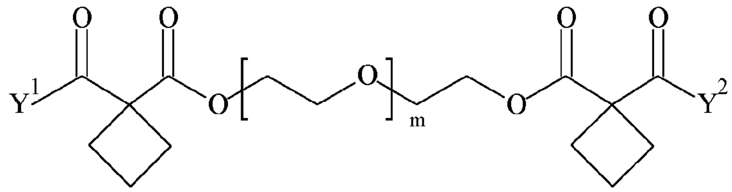

(Va-17)

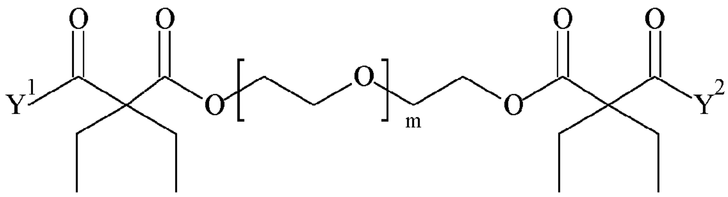

(Va-18)

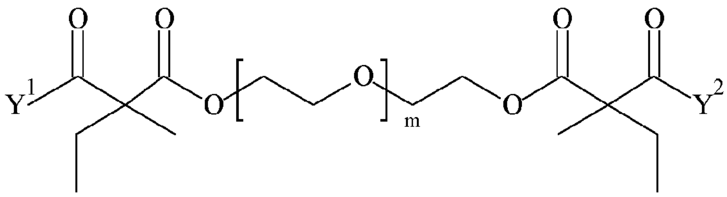

(Va-19)

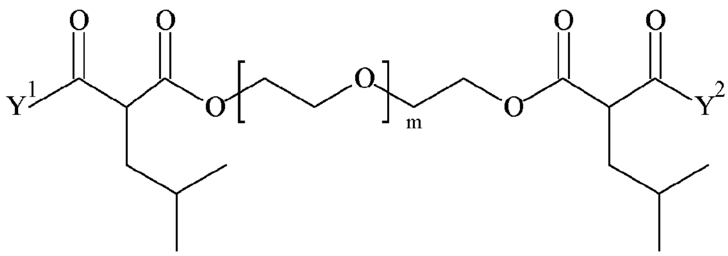

(Va-20)

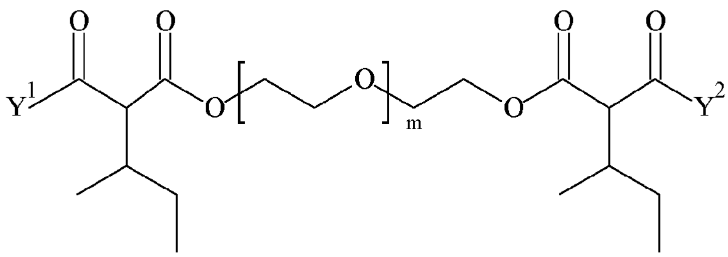

(Va-21)

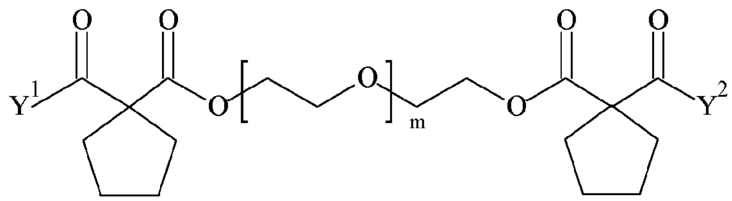
(Va-22)

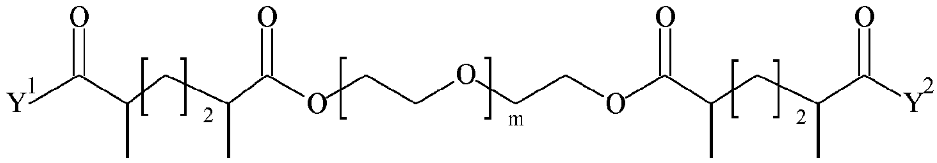
(Va-23)

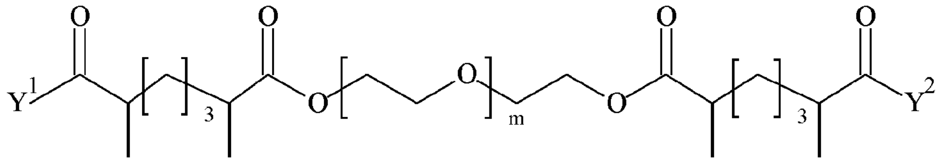
(Va-24)

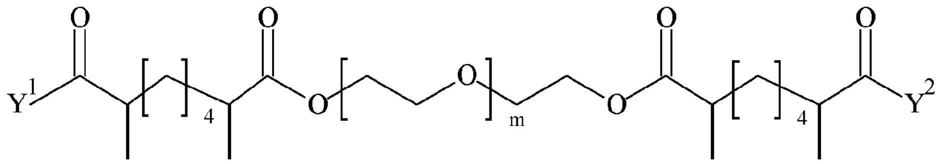
(Va-25)

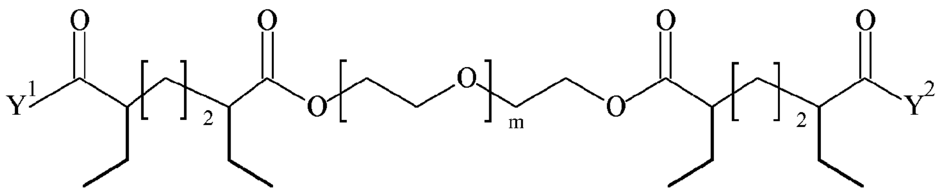
(Va-26)

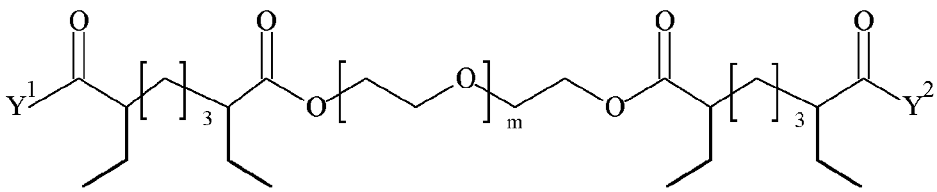
(Va-27)

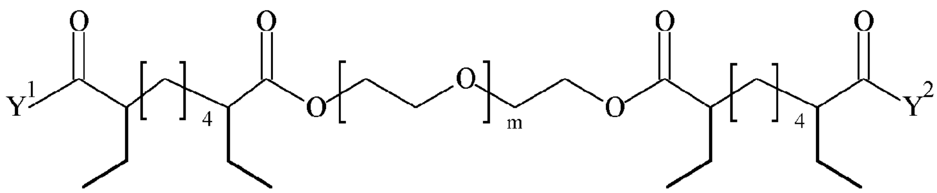
(Va-28)

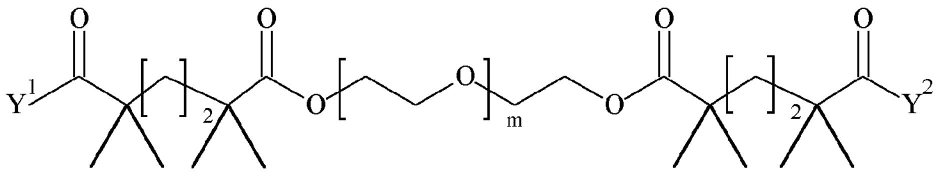

(Va-29)

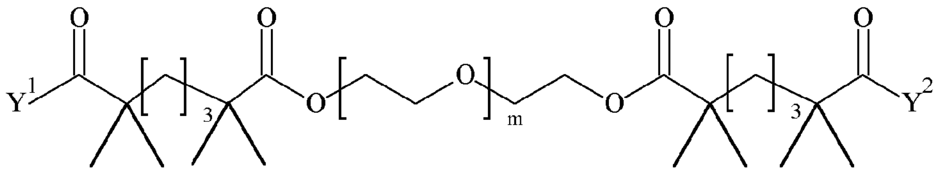

(Va-30)

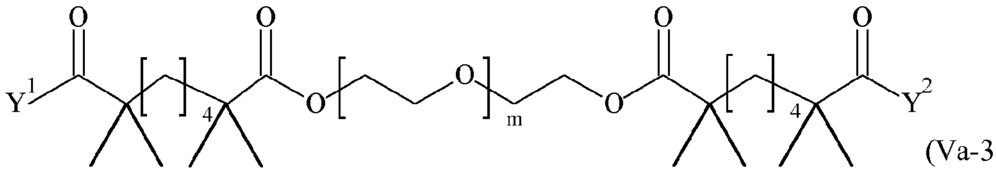

(Va-31)

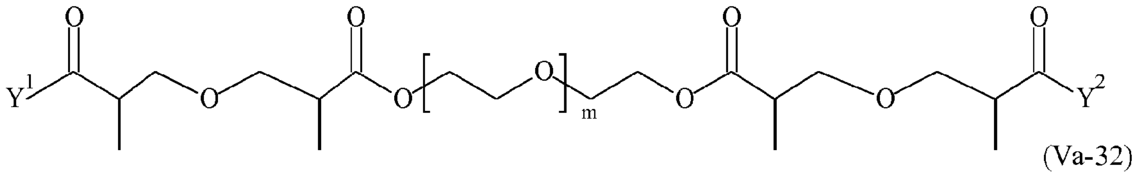

(Va-32)

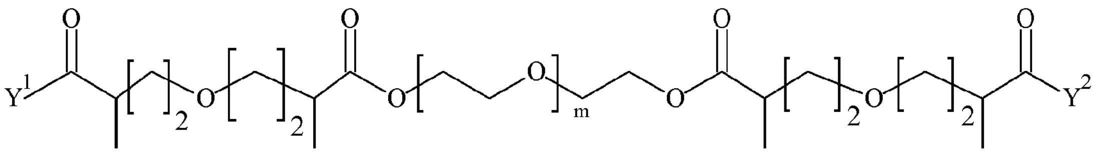

(Va-33)

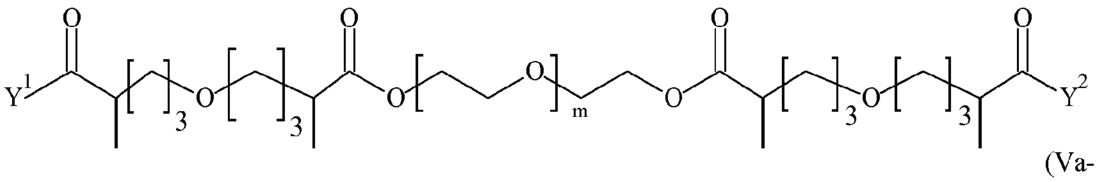

(Va-34)

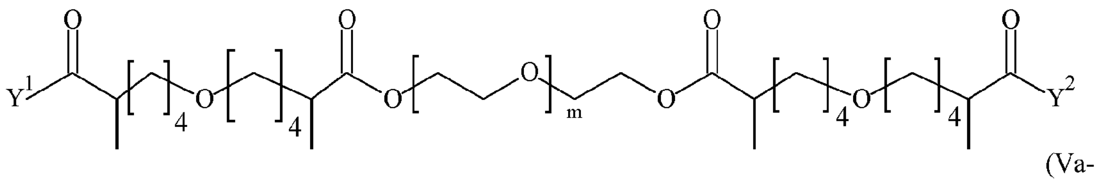

(Va-35)

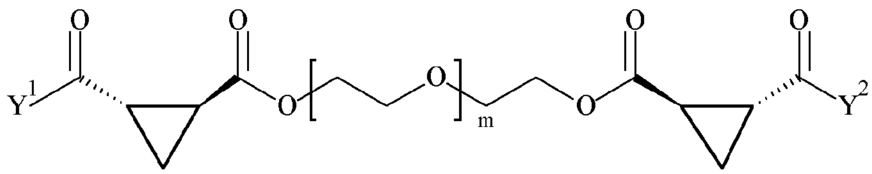

(Va-36)

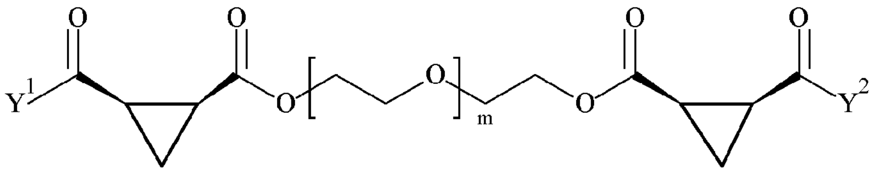

(Va-37)

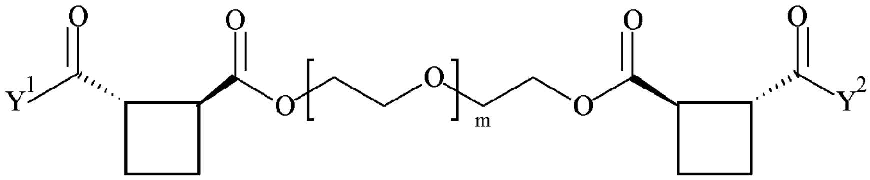

(Va-38)

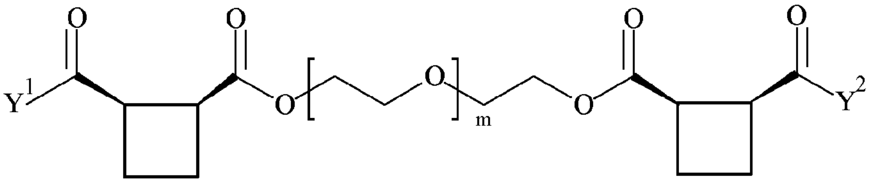

(Va-39)

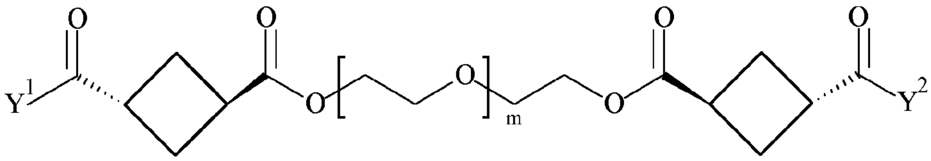

(Va-40)

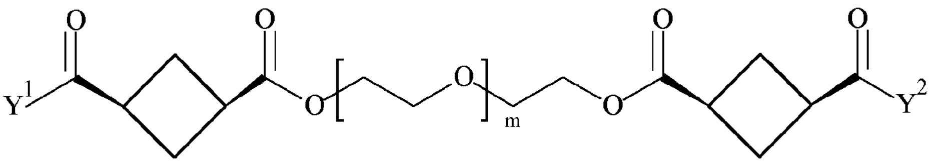

(Va-41)

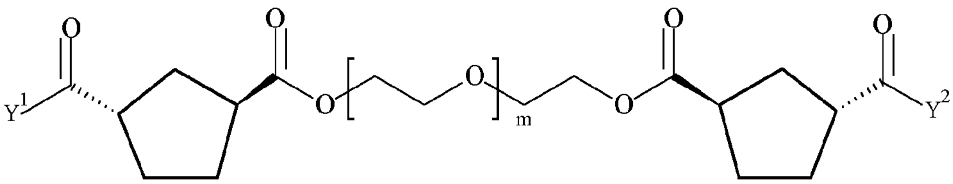

(Va-42)

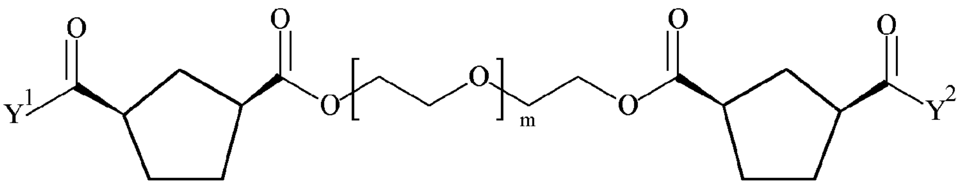

(Va-43)

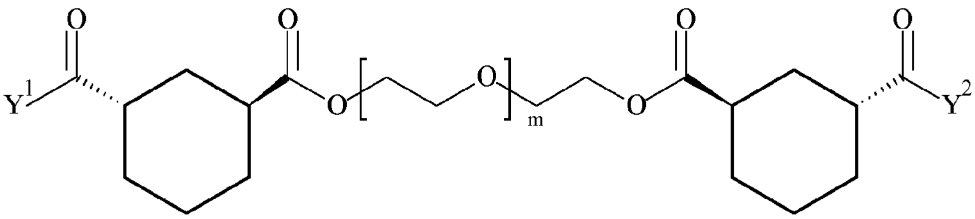

(Va-44)

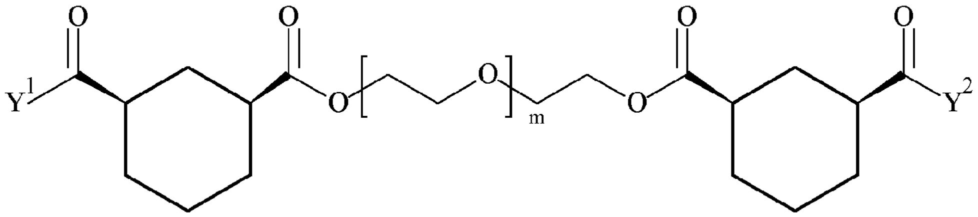

(Va-45)

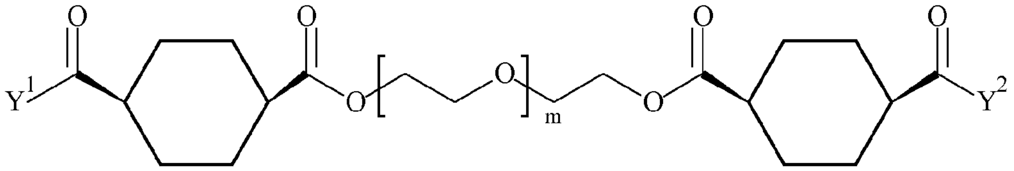

(Va-46)

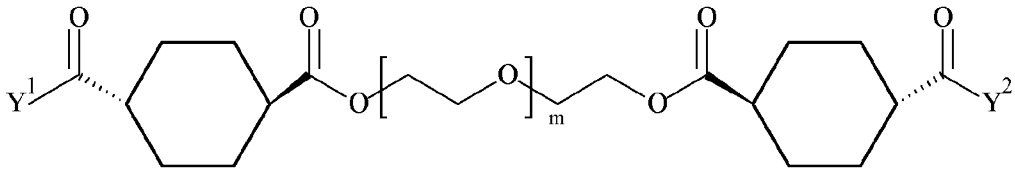

(Va-47)

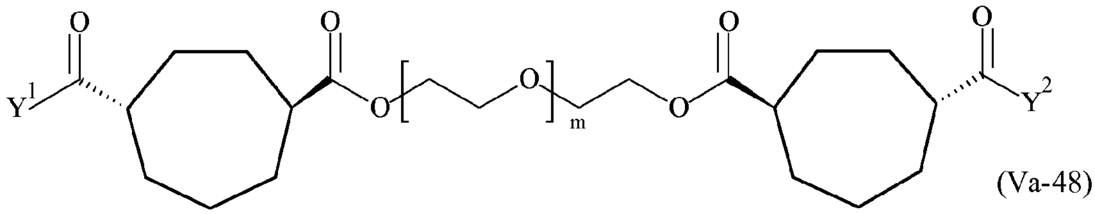

(Va-48)

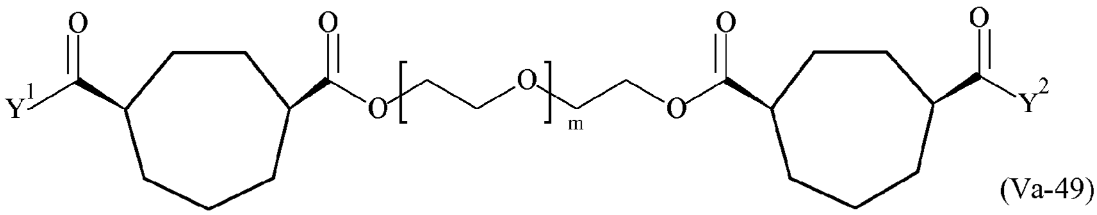

(Va-49)

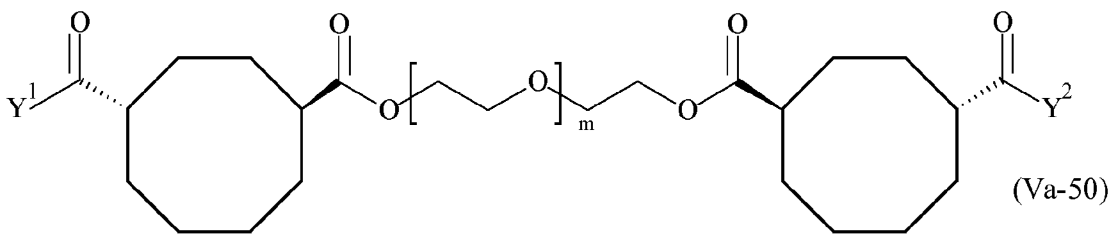

(Va-50)

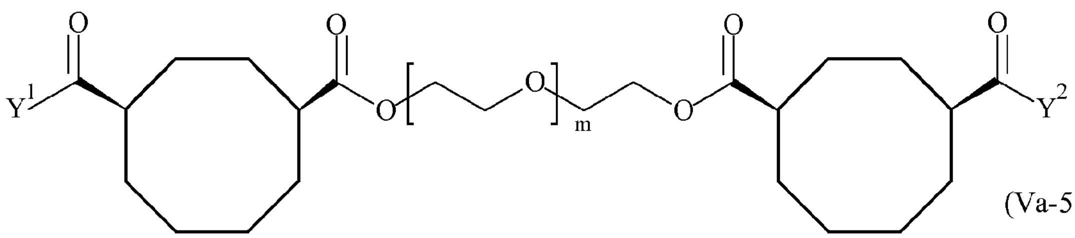

(Va-51)

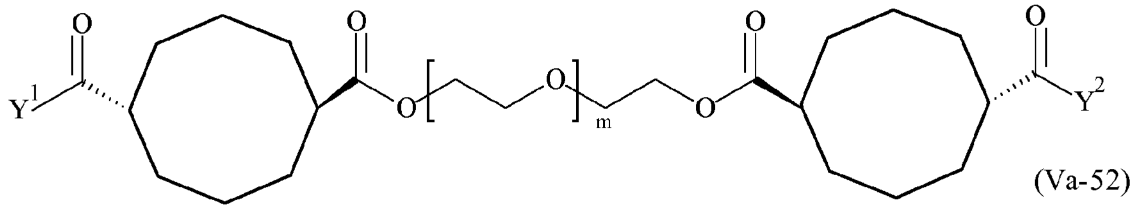

(Va-52)

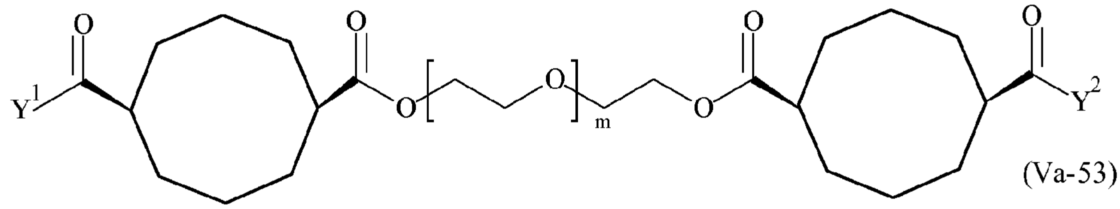

(Va-53)

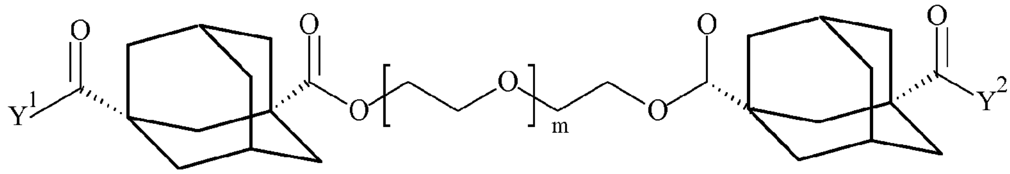

(Va-54)

wherein

each crosslinker reagent may be in the form of its racemic mixture, where applicable; and
m, $Y^1$ and $Y^2$ are defined as above.

It was surprisingly found that the use of crosslinker reagents with branches, i.e. residues other than H, at the alpha carbon of the carbonyloxy group lead to the formation of hydrogels which are more resistant against enzymatic degradation, such as degradation through esterases.

Similarly, it was surprisingly found that the fewer atoms there are between the (C=O) of a carbonyloxy group and the (C=O) of the adjacent activated ester, activated carbamate, activated carbonate or activated thiocarbamate, the more resistant against degradation the resulting hydrogels are, such as more resistant against degradation through esterases.

Accordingly, crosslinker reagents V-11 to V-54, V-1, V-2, Va-11 to Va-54, Va-1 and Va-2 are preferred crosslinker reagents. Crosslinker reagents Va-11 to Va-54, Va-1 and Va-2 are most preferred crosslinker reagents. Most preferred is crosslinker reagent Va-14.

In another embodiment, crosslinker reagents V-1, V-2, V-5, V-6, V-7, V-8, V-9, V-10, V-11, V-12, V-13, V-14, V-15, V-16, V-17, V-18, V-19, V-20, V-21, V-22, V-23, V-24, V-25, V-26, V-27, V-28, V-29, V-30, V-31, V-32, V-33, V-34, V-35, V-36, V-37, V-38, V-39, V-40, V-41, V-42, V-43, V-44, V-45, V-46, V-47, V-48, V-49, V-50, V-51, V-52, V-53 an V-54 are preferred crosslinker reagents. More preferably, the at least one crosslinker reagent is of formula V-5, V-6, V-7, V-8, V-9, V-10, V-14, V-22, V-23, V-43, V-44, V-45 or V-46, and most preferably, the at least one crosslinker reagent is of formula V-5, V-6, V-9 or V-14.

In another embodiment, crosslinker reagents Va-1, Va-2, Va-5, Va-6, Va-7, Va-8, Va-9, Va-10, Va-11, Va-12, Va-13, Va-14, Va-15, Va-16, Va-17, Va-18, Va-19, Va-20, Va-21, Va-22, Va-23, Va-24, Va-25, Va-26, Va-27, Va-28, Va-29, Va-30, Va-31, Va-32, Va-33, Va-34, Va-35, Va-36, Va-37, Va-38, Va-39, Va-40, Va-41, Va-42, Va-43, Va-44, Va-45, Va-46, Va-47, Va-48, Va-49, Va-50, Va-51, Va-52, Va-53 an Va-54 are even more preferred crosslinker reagents. More preferably, the at least one crosslinker reagent is of formula Va-5, Va-6, Va-7, Va-8, Va-9, Va-10, Va-14, Va-22, Va-23, Va-43, Va-44, Va-45 or Va-46, and most preferably, the at least one crosslinker reagent is of formula Va-5, Va-6, Va-9 or Va-14.

The preferred embodiments of the compound of formula (V-I) and (V-II) as mentioned above apply accordingly to the preferred compounds of formulas (V-1) to (V-53).

The hydrogel contains from 0.01 to 1 mmol/g primary amine groups ($-NH_2$), more preferably, from 0.02 to 0.5 mmol/g primary amine groups and most preferably from 0.05 to 0.3 mmol/g primary amine groups. The term "X mmol/g primary amine groups" means that 1 g of dry hydrogel comprises X mmol primary amine groups. Measurement of the

amine content of the hydrogel is carried out according to Gude et al. (Letters in Peptide Science, 2002, 9(4): 203-206, which is described in detail in the Examples section.

Preferably, the term "dry" as used herein means having a residual water content of a maximum of 10%, preferably less than 5% and more preferably less than 2% (determined according to Karl Fischer). The preferred method of drying is lyophilization.

Optionally, the process for the preparation of a hydrogel-spacer conjugate further comprises the step of:
(d) reacting the hydrogel from step (b) or (c) with a spacer reagent of formula (VI)

$$A^{x1}\text{-}S^{0}\text{-}A^{x2} \quad (VI),$$

wherein

- $S^0$ is selected from the group comprising $C_{1-50}$ alkyl, $C_{2-50}$ alkenyl and $C_{2-50}$ alkynyl, which fragment is optionally interrupted by one or more group(s) selected from -NH-, -N($C_{1-4}$ alkyl)-, -O-, -S, -C(O)-, -C(O)NH, -C(O)N($C_{1-4}$ alkyl)-, -O-C(O)-, -S(O)-, -S(O)$_2$-, 4- to 7-membered heterocyclyl, phenyl and naphthyl;
- $A^{x1}$ is a functional group for reaction with $A^{x0}$; and
- $A^{x2}$ is a functional group;

in the presence of a solvent to obtain a hydrogel-spacer conjugate.

Preferably, $A^{x1}$ is selected from the group comprising activated carboxylic acid; Cl-(C=O)-; NHS-(C=O)-, wherein NHS is N-hydroxysuccinimide; $ClSO_2$-; $R^1$(C=O)-; I-; Br-; Cl-; SCN-; and CN-, wherein

- $R^1$ is selected from the group comprising H, $C_{1-6}$ alkyl, alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, and tetralinyl.

Most preferably, $A^{x1}$ is an activated carboxylic acid.

Suitable activating reagents to obtain the activated carboxylic acid are for example N,N'-dicyclohexyl-carbodiimide (DCC), 1-ethyl-3-carbodiimide (EDC), benzotriazol-1-yl-oxytripyrrolidinophosphonium hexafluorophosphate (PyBOP), bromotripyrrolidinophosphonium hexafluorophosphate (PyBrOP), 1-cyano-2-ethoxy-2-oxoethylidenaminooxy)dimethylamino-morpholino-carbenium hexafluorophosphate (COMU), 1-hydroxybenzotriazole (HOBT), 1-hydroxy-7-azabenzotriazole (HOAT), *O*-(6-chlorobenzotriazol-1-yl)-*N,N,N',N'*-tetramethyluronium hexafluorophosphate (HCTU), 1-H-benzotriazolium (HBTU), (*O*-(7-azabenzotriazol-1-yl)-*N,N,N',N'*-tetramethyluronium hexafluorophosphate (HATU), and *O*-(benzotriazol-1-yl)-*N,N,N',N'*-tetramethyluronium tetrafluoroborate (TBTU). These reagents are commercially available and well-known to the skilled person.

Preferably, $A^{x2}$ is selected from the group comprising -maleimide, -SH, -$NH_2$, -SeH, -$N_3$, -C≡CH, -$CR^1$=$CR^{1a}R^{1b}$, -OH, -(CH=$X^0$)-$R^1$, -(C=O)-S-$R^1$, -(C=O)-H, -NH-$NH_2$, -O-$NH_2$, -Ar-$X^0$, -Ar-Sn($R^1$)($R^{1a}$)($R^{1b}$), -Ar-B(OH)(OH),

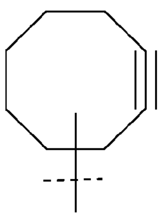
,
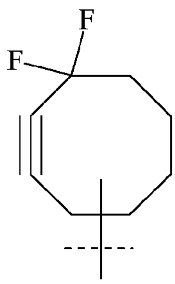
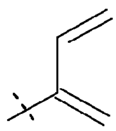
,
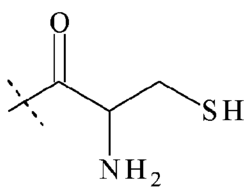

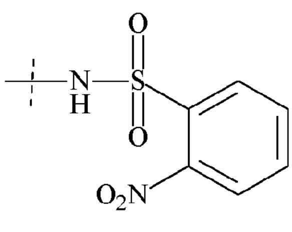
, and
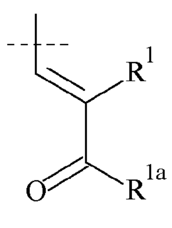
;

with optional protecting groups; wherein

| | |
|---|---|
| $X^0$ | is -OH, $-NR^1R^{1a}$, -SH, and -SeH, |
| Ar | is selected from phenyl, naphthyl, indenyl, indanyl, and tetralinyl, and |
| $R^1$, $R^{1a}$, $R^{1b}$ | are independently of each other selected from the group comprising H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, and tetralinyl. |

More preferably, $A^{x2}$ is selected from $-NH_2$, maleimide and thiol and most preferably $A^{x2}$ is maleimide. Equally preferred is thiol (-SH).

Suitable reaction conditions are described in the Examples sections and are known to the person skilled in the art. Process step (d) may be carried out in the presence of a base. Suitable bases include customary inorganic or organic bases. These preferably include alkaline earth metal or alkali metal hydrides, hydroxides, amides, alkoxides, acetates, carbonates or bicarbonates such as, for example, sodium hydride, sodium amide, sodium methoxide, sodium ethoxide, potassium tert-butoxide, sodium hydroxide, potassium hydroxide, ammonium hydroxide, sodium acetate, potassium acetate, calcium acetate, ammonium acetate, sodium carbonate, potassium carbonate, potassium bicarbonate, sodium bicarbonate or ammonium carbonate, and tertiary amines such as trimethylamine, triethylamine, tributylamine, *N*,*N*-dimethylaniline, *N,N*-dimethylbenzylamine, pyridine, *N*-methylpiperidine, *N*-methylmorpholine, *N,N*-dimethylaminopyridine, diazabicyclooctane (DABCO), diazabicyclononene (DBN), *N,N*-diisopropylethylamine (DIPEA), diazabicycloundecene (DBU) or collidine.

Process step (d) may be carried out in the presence of a solvent. Suitable solvents for carrying out the process step (d) of the invention include organic solvents. These preferably include water and aliphatic, alicyclic or aromatic hydrocarbons such as, for example, petroleum ether, hexane, heptane, cyclohexane, methylcyclohexane, benzene, toluene, xylene or decalin; halogenated hydrocarbons such as, for example, chlorobenzene, dichlorobenzene, dichloromethane, chloroform, carbon tetrachloride, dichloroethane or trichloroethane; alcohols such as methanol, ethanol, n- or i-propanol, n-, i-, sec- or tert-butanol, ethanediol, propane-1,2-diol, ethoxyethanol, methoxyethanol, diethylene glycol monomethyl ether, dimethylether, diethylene glycol; acetonitrile, N-methyl-2-pyrrolidone (NMP), dimethylformamide (DMF), dimethyl sulfoxide (DMSO), N,N-dimethylacetamide, nitromethane, nitrobenzene, hexamethylphosphoramide (HMPT), 1,3-dimethyl-2-imidazolidinone (DMI), 1,3-dimethyl-3,4,5,6-tetrahydro-2(1H)-pyrimidinone (DMPU), ethyl acetate, acetone, butanone; ethers such as diethyl ether, diisopropyl ether, methyl t-butyl ether, methyl t-amyl ether, dioxane, tetrahydrofuran, 1,2-dimethoxyethane, 1,2-diethoxyethane or anisole; or mixtures thereof. Preferably, the solvent is selected from water, acetonitrile or N-methyl-2-pyrrolidone.

In one embodiment the hydrogel of the hydrogel-linked IL-1ra prodrug of the present invention is modified before $L^2$-$L^1$-IL-1ra is conjugated to the hydrogel.

Preferably, the hydrogel is modified by a process comprising the steps of

(A) providing a hydrogel having groups $A^{x0'}$, wherein groups $A^{x0'}$ represent the same or different, preferably same, functional groups;

(B) optionally covalently conjugating a spacer reagent of formula (VI)

$$A^{x1}\text{-}SP^2\text{-}A^{x2} \quad \text{(VI)},$$

wherein

| | |
|---|---|
| $SP^2$ | is $C_{1-50}$ alkyl, $C_{2-50}$ alkenyl or $C_{2-50}$ alkynyl, which $C_{1-50}$ alkyl, $C_{2-50}$ alkenyl and $C_{2-50}$ alkynyl is optionally interrupted by one or more group(s) selected from the group consisting of -NH-, $-N(C_{1-4}$ alkyl)-, -O-, -S, -C(O)-, -C(O)NH, $-C(O)N(C_{1-4}$ alkyl)-, -O-C(O)-, -S(O)-, $-S(O)_2$-, 4- to 7-membered heterocyclyl, phenyl and naphthyl; |
| $A^{x1}$ | is a functional group for reaction with $A^{x0}$ of the hydrogel; and |
| $A^{x2}$ | is a functional group; |

to $A^{x0'}$ of the hydrogel from step (A); and

(C) reacting the hydrogel of step (A) or step (B) with a reagent of formula (VII)

$$A^{x3}\text{-}Z^0 \quad \text{(VII)},$$

wherein

$A^{x3}$ is a functional group; and

$Z^0$ is an inert moiety having a molecular weight ranging from 10 Da to 1000 kDa;

such that at most 99 mol-% of $A^{x0}$ or $A^{x2}$ react with $A^{x3}$.

Preferably, $A^{x0'}$ of step (A) is selected from the group consisting of maleimide, amine ($-NH_2$ or -NH-), hydroxyl (-OH), carboxyl (-COOH) and activated carboxyl ($-COY^1$, wherein $Y^1$ is selected from formulas (f-i) to (f-vi):

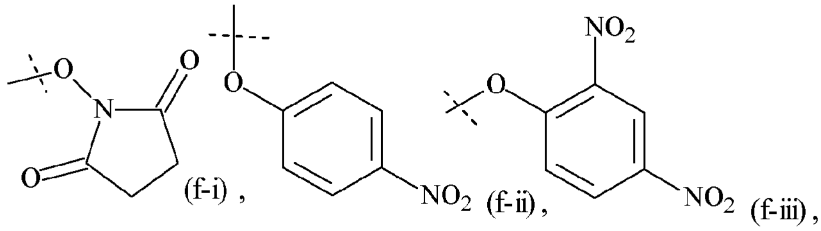
(f-i) , (f-ii) , (f-iii) ,

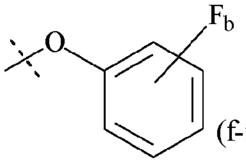
(f-iv),

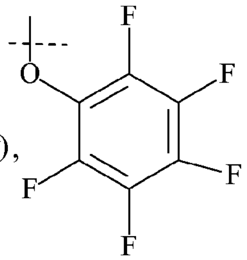
(f-v) and

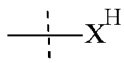
(f-vi)

wherein

the dashed lines indicate attachment to the rest of the molecule,

b is 1, 2, 3 or 4;

$X^H$ is Cl, Br, I, or F).

More preferably, $A^{x0'}$ of step (A) is an amine or maleimide. Most preferably, $A^{x0'}$ of step (A) is an amine.

It is understood that the functional groups $A^{x0'}$ of step (A) correspond to $A^{x0}$ of the at least one backbone reagent, if the hydrogel of the hydrogel-linked IL-1ra prodrug of the present invention is obtained from step (b) or (c) of the process described above, or to $A^{x2}$, if the hydrogel of the hydrogel-linked IL-1ra prodrug of the present invention is obtained from optional step (d).

In a preferred embodiment $A^{x0'}$ of step (A) is an amine and $A^{x1}$ of step (B) is $ClSO_2$-, $R^1(C=O)$-, I-, Br-, Cl-, SCN-, CN-, O=C=N-, $Y^1$-(C=O)-, $Y^1$-(C=O)-NH-, or $Y^1$-(C=O)-O-, wherein

$R^1$ is H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and

$Y^1$ is selected from formulas (f-i) to (f-vi):

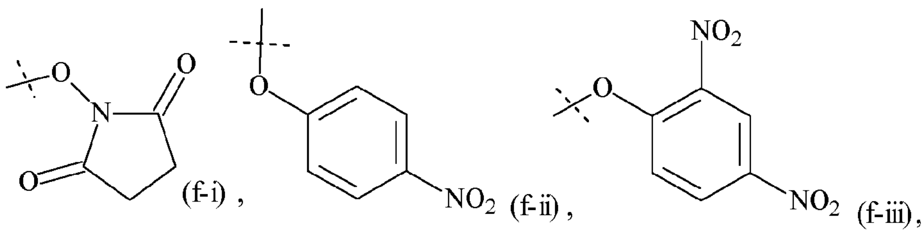
(f-i) , (f-ii) , (f-iii),

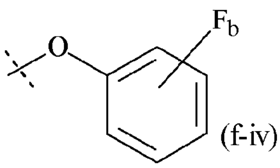 (f-iv), 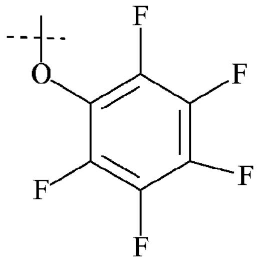 (f-v) and 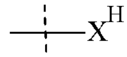 (f-vi)

wherein

the dashed lines indicate attachment to the rest of the molecule,
b is 1, 2, 3 or 4,
$X^H$ is Cl, Br, I, or F.

In another preferred embodiment $A^{x0'}$ of step (A) is a hydroxyl group (-OH) and $A^{x1}$ of step (B) is O=C=N-, I-, Br-, SCN-, or $Y^1$-(C=O)-NH-,
wherein $Y^1$ is selected from formulas (f-i) to (f-vi):

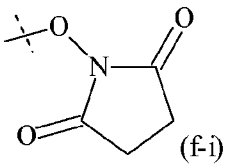 (f-i) , 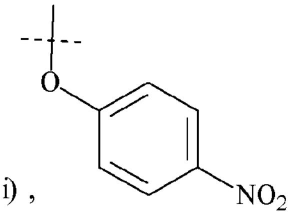 (f-ii) , 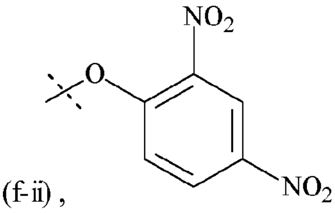 (f-iii),

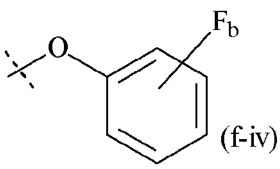 (f-iv), 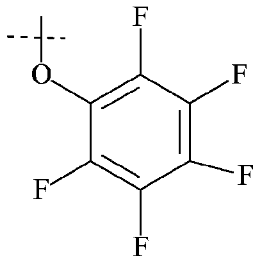 (f-v) and 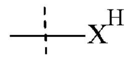 (f-vi)

wherein

the dashed lines indicate attachment to the rest of the molecule,
b is 1, 2, 3 or 4,
$X^H$ is Cl, Br, I, or F.

In another preferred embodiment $A^{x0'}$ of step (A) is a carboxylic acid (-(C=O)OH) and $A^{x1}$ of step (B) is a primary amine or secondary amine.
In another preferred embodiment $A^{x0'}$ of step (A) is a maleimide and $A^{x1}$ of step (B) is a thiol.
More preferably, $A^{x0'}$ of step (A) is an amine and $A^{x1}$ of step (B) is $Y^1$-(C=O)-, $Y^1$-(C=O)-NH-, or $Y^1$-(C=O)-O- and most preferably $A^{x0'}$ of step (A) is an amine and $A^{x1}$ of step (B) is $Y^1$-(C=O)-.
$A^{x1}$ of step (B) may optionally be present in protected form.
Suitable activating reagents to obtain the activated carboxylic acid are for example N,N'-dicyclohexyl-carbodiimide (DCC), 1-ethyl-3-carbodiimide (EDC), benzotriazol-1-yl-oxytripyrrolidinophosphonium hexafluorophosphate (PyBOP), bromotripyrrolidinophosphonium hexafluorophosphate (PyBrOP), 1-cyano-2-ethoxy-2-oxoethylidenaminooxy)dimethylamino-morpholino-carbenium hexafluorophosphate (COMU), 1-hydroxybenzotriazole (HOBT), 1-hydroxy-7-azabenzotriazole (HOAT), 0-(6-chlorobenzotriazol-1-yl)-*N,N,N',N'*-tetramethyluronium hexafluorophosphate (HCTU), 1-H-benzotriazolium (HBTU), (*O*-(7-azabenzotriazol-1-yl)-*N,N,N',N'*-tetramethyluronium hexafluorophosphate (HATU), and *O*-(benzotriazol-1-yl)-*N,N,N',N'*-tetramethyluronium tetrafluoroborate (TBTU). These reagents are commercially available and well-known to the skilled person.

Preferably, $A^{x2}$ of step (B) is selected from the group consisting of -maleimide, -SH, $-NH_2$, -SeH, $-N_3$, -C≡CH, $-CR^1=CR^{1a}R^{1b}$, -OH, $-(CH=X)-R^1$, $-(C=O)-S-R^1$, -(C=O)-H, $-NH-NH_2$, $-O-NH_2$, $-Ar-X^0$, $-Ar-Sn(R^1)(R^{1a})(R^{1b})$, -Ar-B(OH)(OH), Br, I, $Y^1-(C=O)-$, $Y^1-(C=O)-NH-$, $Y^1-(C=O)-O-$,

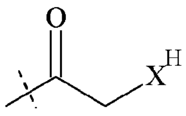 , 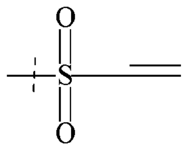 , 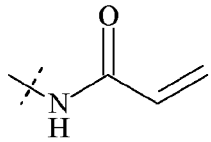 , 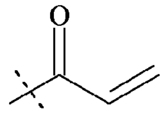 ,

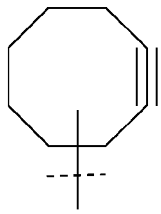 , 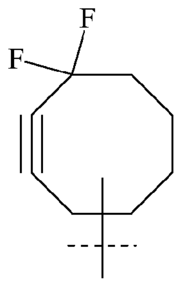 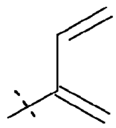 , 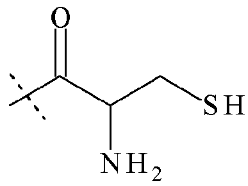 ,

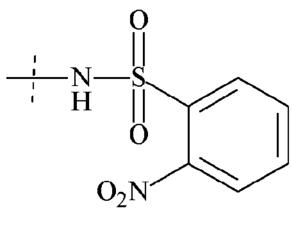 , and 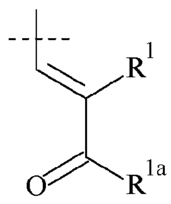 ;

with optional protecting groups; wherein

dashed lines indicate attachment to $SP^2$;
X is O, S, or NH,
$X^0$ is -OH, $-NR^1R^{1a}$, -SH, or -SeH,
$X^H$ is Cl, Br, I or F;
Ar is phenyl, naphthyl, indenyl, indanyl, or tetralinyl;
$R^1$, $R^{1a}$, $R^{1b}$ are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
$Y^1$ is selected from formulas (f-i) to (f-vi):

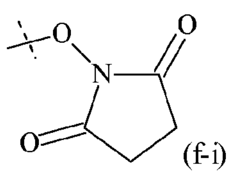 (f-i) , 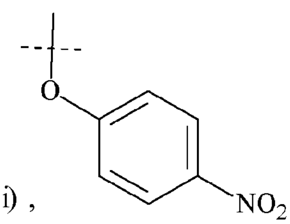 (f-ii) , 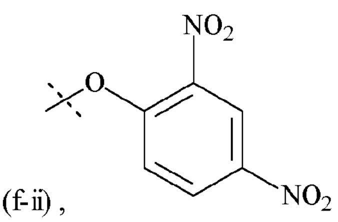 (f-iii),

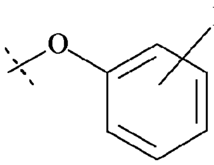 (f-iv), 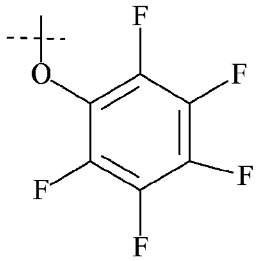 (f-v) and 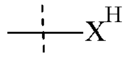 (f-vi)

wherein

the dashed lines indicate attachment to the rest of the molecule,
b is 1, 2, 3 or 4,
$X^H$ is Cl, Br, I, or F.

More preferably, $A^{x2}$ of step (B) is $-NH_2$, maleimide or thiol and most preferably $A^{x2}$ of step (B) is maleimide.
$A^{x2}$ of step (B) may optionally be present in protected form.
If the hydrogel of step (A) is covalently conjugated to a spacer moiety, the resulting hydrogel-spacer moiety conjugate is of formula (VIII):

$$-A^{y1}-SP^2-A^{x2} \quad \text{(VIII)},$$

wherein

the dashed line indicates attachment to the hydrogel of step (A);
$A^{y1}$ is the linkage formed between $A^{x0'}$ and $A^{x1}$; and
$SP^2$ and $A^{x2}$ are used as in formula (VI).

Preferably, $A^{y1}$ of formula (VIII) is a stable linkage.
Preferably, $A^{y1}$ of formula (VIII) is selected from the group consisting of

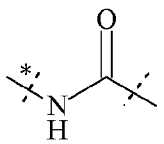 , 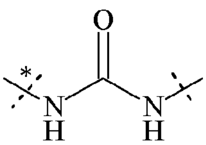 and 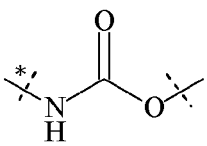 ,

wherein
dashed lines marked with an asterisk indicate attachment to the hydrogel; and unmarked dashed lines indicate attachment to $SP^2$.
Suitable reaction conditions are known to the person skilled in the art.
Process step (B) may be carried out in the presence of a base. Suitable bases include customary inorganic or organic bases. These preferably include alkaline earth metal or alkali metal hydrides, hydroxides, amides, alkoxides, acetates, carbonates or bicarbonates such as, for example, sodium hydride, sodium amide, sodium methoxide, sodium ethoxide, potassium tert-butoxide, sodium hydroxide, potassium hydroxide, ammonium hydroxide, sodium acetate, potassium acetate, calcium acetate, ammonium acetate, sodium carbonate, potassium carbonate, potassium bicarbonate, sodium bicarbonate or ammonium carbonate, and tertiary amines such as trimethylamine, triethylamine, tributylamine, *N,N*-dimethylaniline, *N,N*-dimethylbenzylamine, pyridine, *N*-methylpiperidine, *N*-methylmorpholine, *N,N*-dimethylaminopyridine, diazabicyclooctane (DABCO), diazabicyclononene (DBN), *N,N*-diisopropylethylamine (DIPEA), diazabicycloundecene (DBU) or collidine.
Process step (B) may be carried out in the presence of a solvent. Suitable solvents for carrying out the process step (B) of the invention include organic solvents. These preferably include water and aliphatic, alicyclic or aromatic hydrocarbons such as, for example, petroleum ether, hexane, heptane, cyclohexane, methylcyclohexane, benzene, toluene, xylene or decalin; halogenated hydrocarbons such as, for example, chlorobenzene, dichlorobenzene, dichloromethane, chloroform, carbon tetrachloride, dichloroethane or trichloroethane; alcohols such as methanol, ethanol, n-

or i-propanol, n-, i-, sec- or tert-butanol, ethanediol, propane-1,2-diol, ethoxyethanol, methoxyethanol, diethylene glycol monomethyl ether, dimethylether, diethylene glycol; acetonitrile, N-methyl-2-pyrrolidone (NMP), dimethylformamide (DMF), dimethyl sulfoxide (DMSO), N,N-dimethylacetamide, nitromethane, nitrobenzene, hexamethylphosphoramide (HMPT), 1,3-dimethyl-2-imidazolidinone (DMI), 1,3-dimethyl-3,4,5,6-tetrahydro-2(1H)-pyrimidinone (DMPU), ethyl acetate, acetone, butanone; ethers such as diethyl ether, diisopropyl ether, methyl t-butyl ether, methyl t-amyl ether, dioxane, tetrahydrofuran, 1,2-dimethoxyethane, 1,2-diethoxyethane or anisole; or mixtures thereof. Preferably, the solvent is selected from the group consisting of water, acetonitrile and N-methyl-2-pyrrolidone.

Preferably, $A^{x3}$ of step (C) is selected from the group consisting of -SH, $-NH_2$, -SeH,-maleimide, -C≡CH, $-N_3$, $-CR^1=CR^{1a}R^{1b}$, $-(C=X)-R^1$, -OH, $-(C=O)-S-R^1$, $-NH-NH_2$, $-O-NH_2$, $-Ar-Sn(R^1)(R^{1a})(R^{1b})$, -Ar-B(OH)(OH), $-Ar-X^0$,

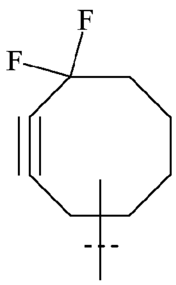 , 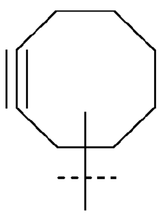 , 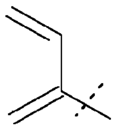 , 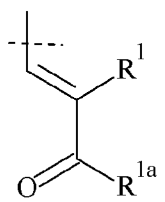 , 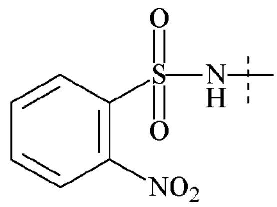 ,

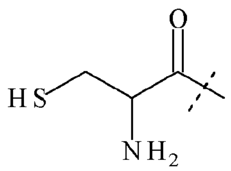 , 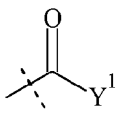 , 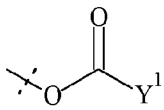 , 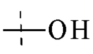 , 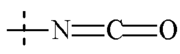 and 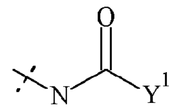 ;

wherein

dashed lines indicate attachment to $Z^0$;
X is O, S, or NH,
$X^0$ is -OH, $-NR^1R^{1a}$, -SH, or -SeH;
$R^1$, $R^{1a}$, $R^{1b}$ are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
Ar is phenyl, naphthyl, indenyl, indanyl, or tetralinyl.
$Y^1$ is an activated carboxylic acid, activated carbonate or activated carbamate, preferably $Y^1$ is selected from formulas (f-i) to (f-vi):

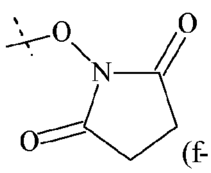 (f-i) , 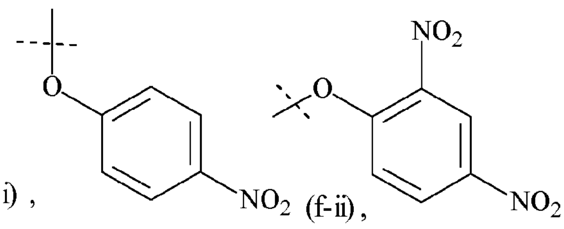 (f-ii), (f-iii),

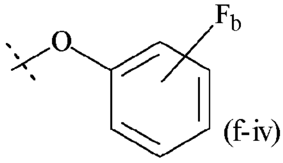 (f-iv), 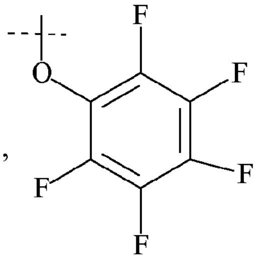 (f-v) and 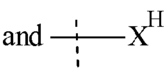 (f-vi)

wherein

the dashed lines indicate attachment to the rest of the molecule,
b is 1, 2, 3 or 4,
$X^H$ is Cl, Br, I, or F

In a preferred embodiment, $Y^1$ is selected from formulas (f-i) to (f-vi):

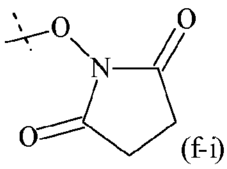
(f-i) ,
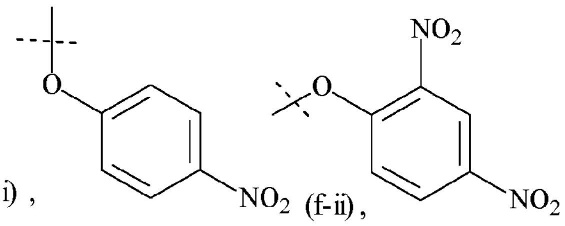
(f-ii), (f-iii),

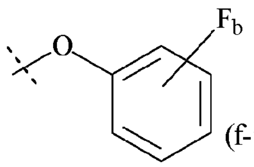
(f-iv),
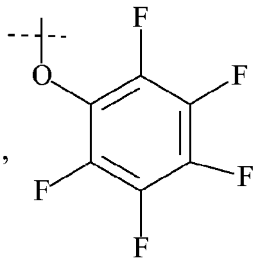
(f-v) and
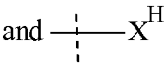
(f-vi)

wherein
the dashed lines, b and $X^H$ are used as above.

More preferably, $A^{x3}$ of step (C) is-SH or -maleimide and most preferably $A^{x3}$ of step (C) is -SH.

In another preferred embodiment $A^{x3}$ is of formula (aI)

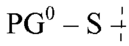
(aI),

wherein

the dashed line indicates attachment to Z of formula (VII);
$PG^0$ is a sulfur-activating moiety; and
S is sulfur;

Preferably, $PG^0$ of formula (aI) is selected from the group consisting of

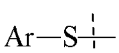
(i),
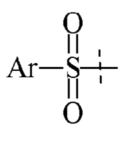
(ii),
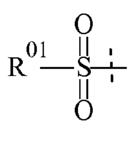
(iii),
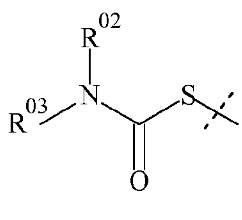
(iv),

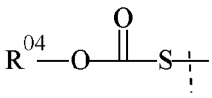 (v), 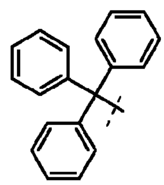 (vi), and 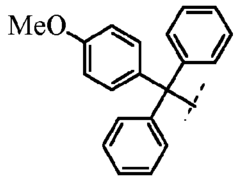 (vii);

wherein

the dashed lines indicate attachment to the sulfur of formula (al);
Ar is an aromatic moiety which is optionally further substituted;
$R^{01}$, $R^{02}$, $R^{03}$, $R^{04}$ are independently of each other -H; $C_{1-50}$ alkyl; $C_{2-50}$ alkenyl; or $C_{2-50}$ alkynyl, wherein $C_{1-50}$ alkyl; $C_{2-50}$ alkenyl; and $C_{2-50}$ alkynyl are optionally substituted with one or more $R^3$, which are the same or different and wherein $C_{1-50}$ alkyl; $C_{2-50}$ alkenyl; and $C_{2-50}$ alkynyl are optionally interrupted by one or more groups selected from the group consisting of -Q-, -C(O)O-; -O-; -C(O)-; -C(O)N($R^4$)-; -S(O)$_2$N($R^4$)-;-S(O)N($R^4$)-; -S(O)$_2$-; -S(O)-; -N($R^4$)S(O)$_2$N($R^{4a}$)-; -S-; -N($R^4$)-;-OC(O)$R^4$; -N($R^4$)C(O)-; -N($R^4$)S(O)$_2$-; -N($R^4$)S(O)-; -N($R^4$)C(O)O-;-N($R^4$)C(O)N($R^{4a}$)-; and -OC(O)N($R^4R^{4a}$);
Q is selected from the group consisting of phenyl; naphthyl; indenyl; indanyl; tetralinyl; $C_{3-10}$ cycloalkyl; 4- to 7-membered heterocyclyl; and 8- to 11- membered heterobicyclyl, wherein T is optionally substituted with one or more $R^3$, which are the same or different;
$R^3$ is halogen; -CN; oxo (=O); -COO$R^5$; -O$R^5$; -C(O)$R^5$; -C(O)N($R^5R^{5a}$); -S(O)$_2$N($R^5R^{5a}$); -S(O)N($R^5R^{5a}$); -S(O)$_2R^5$; -S(O)$R^5$; -N($R^5$)S(O)$_2$N($R^{5a}R^{5b}$); -S$R^5$; -N($R^5R^{5a}$); -NO$_2$; -OC(O)$R^5$; -N($R^5$)C(O)$R^{5a}$; -N($R^5$)S(O)$_2R^{5a}$; -N($R^5$)S(O)$R^{5a}$; -N($R^5$)C(O)O$R^{5a}$; -N($R^5$)C(O)N($R^{5a}R^{5b}$); -OC(O)N($R^5R^{5a}$); or $C_{1-6}$ alkyl, wherein $C_{1-6}$ alkyl is optionally substituted with one or more halogen, which are the same or different; and
$R^4$, $R^{4a}$, $R^5$, $R^{5a}$, $R^{5b}$ are independently selected from the group consisting of -H; or $C_{1-6}$ alkyl, wherein $C_{1-6}$ alkyl is optionally substituted with one or more halogen, which are the same or different.

Preferably, $R^{01}$, $R^{03}$ and $R^{04}$ are independently of each other $C_{1-6}$ alkyl.
Preferably, $R^{02}$ is selected from H and $C_{1-6}$ alkyl.
Preferably, Ar is selected from the group consisting of

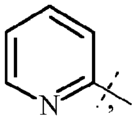, 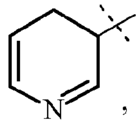, 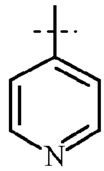, 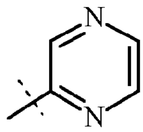, 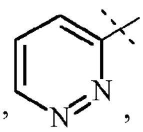,

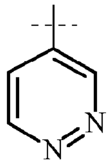, 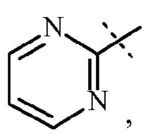, 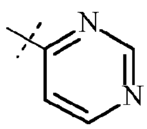, 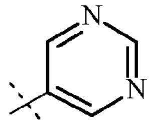, 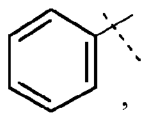,

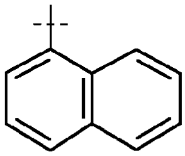, 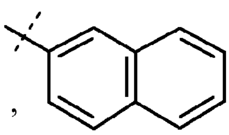,

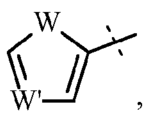, 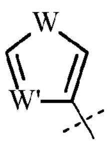, 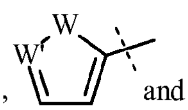 and 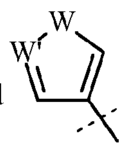.

wherein

dashed lines indicate attachment to the rest of $PG^0$ of formula (aI);
W is independently of each other O, S, or N;
W' is N; and
wherein Ar is optionally substituted with one or more substituent(s) independently selected from the group consisting of $NO_2$, Cl and F.

More preferably, $PG^0$ of formula (aI) is selected from the group consisting of

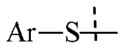 (i), 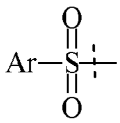 (ii), 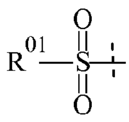 (iii), 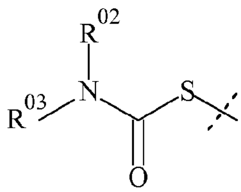 (iv)

and

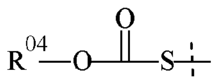 (iv),

wherein
the dashed lines indicate attachment to the sulfur of formula (aI); and Ar, $R^{01}$, $R^{02}$, $R^{03}$ and $R^{04}$ are used as above.
More preferably, $PG^0$ of formula (aI) is

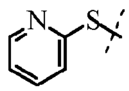,

wherein
the dashed line indicates attachment to the sulfur of formula (aI).
$A^{x3}$ of step (C) may optionally be present in protected form.
Preferred combinations of $A^{x2}$ of step (B) and $A^{x3}$ of step (C) are the following:

| $A^{x2}$ | $A^{x3}$ |
|---|---|
| -maleimide | HS-, $H_2N$-, or HSe- |
| -SH, -$NH_2$, or -SeH | maleimide- |
| -$NH_2$ | $Y^1$-CCO)-, $Y^1$-(C=O)-NH-, or $Y^1$-(C=O)-O- |
| -$N_3$ | HC≡C-, |

(continued)

| $A^{x2}$ | $A^{x3}$ |
| --- | --- |
| | 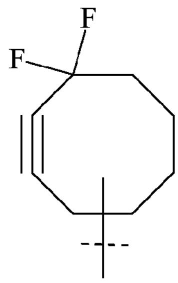 , or |
| | 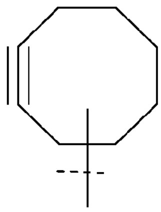 |
| -C≡CH, 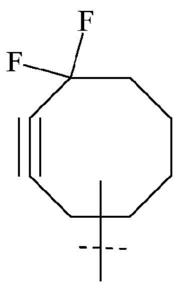 , or 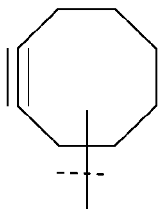 | $N_3$- |
| $-CR^{1a}=CR^{1a}R^{1b}$ | 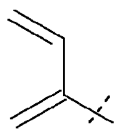 $R^{1b}R^{1a}C=CR^{1}$- or |
| 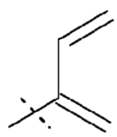 | $R^{1b}R^{1a}C=CR^{1}$- |

(continued)

| $A^{x2}$ | $A^{x3}$ |
|---|---|
| $-(C=X)-R^1$ | 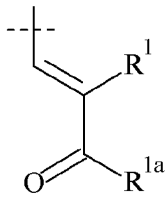 |
| 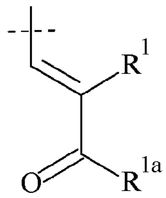 | $R^1-(C=X)-$ |
| -OH | $H_2N$or 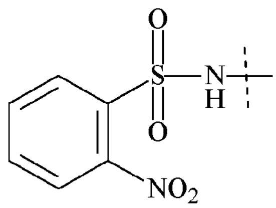 |
| $-NH_2$ or 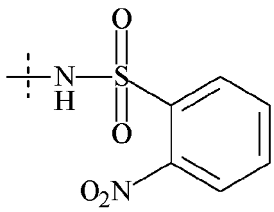 | HO- |
| $-(C=O)-S-R^1$ | 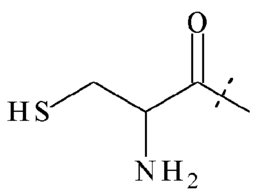 |
| 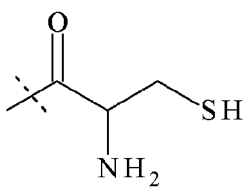 | $R^1-S-(C=O)-$ |
| -(C=O)-H | $H_2N-NH-$ or $H_2N-O-$ |
| $-NH-NH_2$ or $-O-NH_2$ | H-(C=O)- |
| $-Ar-X^0$ | $-Ar-Sn(R^1)(R^{1a})(R^{1b})$ or -Ar-B(OH)(OH) |
| $(R^{1b})(R^{1a})(R^1)Sn-Ar-$ or -Ar-B(OH)(OH) | $X^0-Ar-$ |

wherein

X is O, S, or NH;
$X^0$ is -OH, $-NR^1R^{1a}$, -SH, or -SeH;
$R^1$, $R^{1a}$, $R^{1b}$ are independently of each other selected from the group consisting of H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, and tetralinyl; and
Ar is phenyl, naphthyl, indenyl, indanyl, or tetralinyl.

In another preferred embodiment $A^{x2}$ is -SH and $A^{x3}$ is of formula (aI), wherein $PG^0$ is of formula (i), (ii), (iii), (iv), (v), (vi) or (viii). More preferably, $PG^0$ of formula (aI) is of formula (i), (ii), (iii), (iv) or (v) and even more preferably, $PG^0$ of formula (aI) is of formula (i). Most preferably, $PG^0$ of formula (aI) is of formula

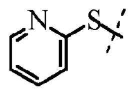
,

wherein
the dashed line indicates attachment to the sulfur of formula (aI).
In one preferred embodiment, $A^{x2}$ of step (B) is an amine and $A^{x3}$ of step (C) is $Y^1$-(C=O)-, $Y^1$-(C=O)-NH-, or $Y^1$-(C=O)-O- and most preferably $A^{x2}$ of step (B) is an amine and $A^{x3}$ of step (C) is $Y^1$-(C=O)-.
In another preferred embodiment $A^{x2}$ of step (B) is maleimide and $A^{x3}$ of step (C) is -SH.
In one embodiment the optional step (B) is omitted, $A^{x0'}$ of step (A) is an amine and $A^{x3}$ of step (C) is $ClSO_2$-, $R^1$(C=O)-, I-, Br-, Cl-, SCN-, CN-, O=C=N-, $Y^1$-(C=O)-, $Y^1$-(C=O)-NH-, or $Y^1$-(C=O)-O-,
wherein

$R^1$ is H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
$Y^1$ is selected from formulas (f-i) to (f-vi):

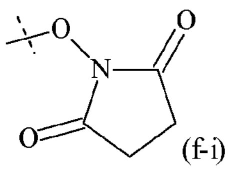 (f-i) , 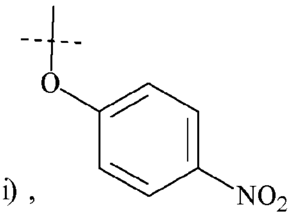 (f-ii) , 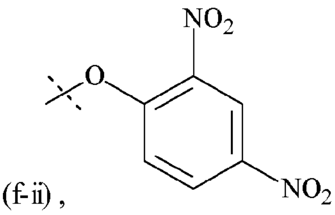 (f-iii),

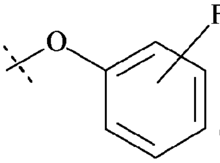 (f-iv), 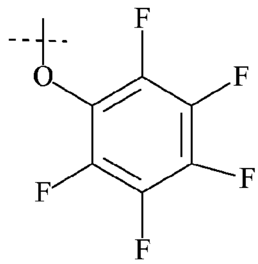 (f-v) and 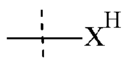 (f-vi)

wherein

the dashed lines indicate attachment to the rest of the molecule,
b is 1, 2, 3 or 4,
$X^H$ is Cl, Br, I, or F.

In another embodiment the optional step (B) is omitted, $A^{x0'}$ of step (A) is a hydroxyl group (-OH) and $A^{x3}$ of step (C) is O=C=N-, I-, Br-, SCN-, or $Y^1$-(C=O)-NH-,
wherein $Y^1$ is selected from formulas (f-i) to (f-vi):

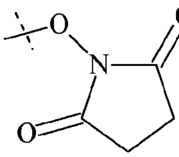 (f-i) , 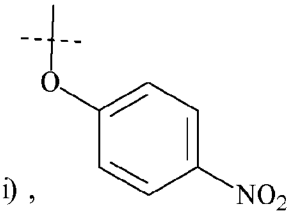 (f-ii) , 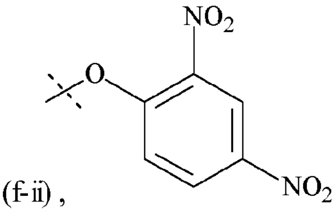 (f-iii),

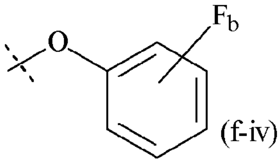 (f-iv), 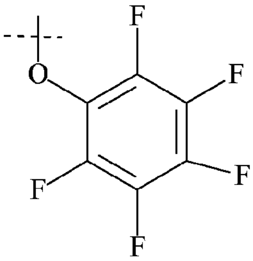 (f-v) and 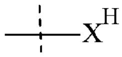 (f-vi)

wherein

the dashed lines indicate attachment to the rest of the molecule,
b is 1, 2, 3 or 4,
$X^H$ is Cl, Br, I, or F.

In another embodiment the optional step (B) is omitted, $A^{x0'}$ of step (A) is a carboxylic acid (-(C=O)OH) and $A^{x3}$ of step (C) is a primary amine or secondary amine.
In another embodiment the optional step (B) is omitted, $A^{x0'}$ of step (A) is an amine and $A^{x3}$ of step (C) is $Y^1$-(C=O)-, $Y^1$-(C=O)-NH-, or $Y^1$-(C=O)-O-.
In another embodiment the optional step (B) is omitted, $A^{x0'}$ of step (A) is a maleimide and $A^{x3}$ of step (C) is thiol.
In a preferred embodiment the optional step (B) is omitted, $A^{x0'}$ of step (A) is an amine and $A^{x3}$ of step (C) is $Y^1$-(C=O)-.
In another preferred embodiment the optional step (b) is omitted, $A^{x0'}$ is -SH and $A^{x3}$ is of formula (aI), wherein $PG^0$ is of formula (i), (ii), (iii), (iv), (v), (vi) or (viii). More preferably, $PG^0$ of formula (aI) is of formula (i), (ii), (iii), (iv) or (v) and even more preferably, $PG^0$ of formula (aI) is of formula (i). Most preferably, $PG^0$ of formula (aI) is of formula

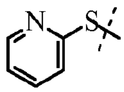
,

wherein
the dashed line indicates attachment to the sulfur of formula (aI).
The hydrogel obtained from step (C) has the structure of formula (IXa) or (IXb):

$$\vdots\, A^{y0} - Z^0 \qquad \text{(IXa)}$$

$$\vdots\, A^{y1} - SP^2 - A^{y2} - Z^0 \qquad \text{(IXb);}$$

wherein

the dashed line indicates attachment to the hydrogel of step (A);
$A^{y0}$ is the linkage formed between $A^{x0'}$ and $A^{x3}$;
$A^{y1}$ is used as in formula (VIII);
$A^{y2}$ is the linkage formed between $A^{x2}$ and $A^{x3}$;

$SP^2$ is used as in formula (VI); and
$Z^0$ is used as in formula (VII).

Preferably, $A^{y0}$ of step (A) and $A^{y2}$ of formula (IXb) are selected from the group consisting of amide, carbamate,

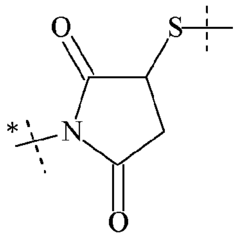
and
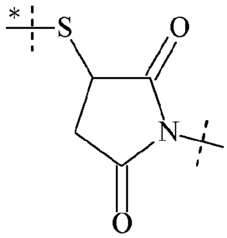
;

wherein

the dashed lines marked with an asterisk indicate attachment to the hydrogel or $SP^2$, respectively; and
the unmarked dashed lines indicate attachment to $Z^0$ of formula (VII).

In one embodiment, $Z^0$ of step (C) is selected from the group consisting of $C_{1-50}$ alkyl, $C_{2-50}$ alkenyl, $C_{2-50}$ alkynyl, $C_{3-10}$ cycloalkyl, 4- to 7-membered heterocyclyl, 8- to 11-membered heterobicyclyl, phenyl; naphthyl; indenyl; indanyl; and tetralinyl; which $C_{1-50}$ alkyl, $C_{2-50}$ alkenyl, $C_{2-50}$ alkynyl, $C_{3-10}$ cycloalkyl, 4- to 7-membered heterocyclyl, 8- to 11-membered heterobicyclyl, phenyl; naphthyl; indenyl; indanyl; and tetralinyl are optionally substituted with one or more $R^{10}$, which are the same or different and wherein $C_{1-50}$ alkyl; $C_{2-50}$ alkenyl; and $C_{2-50}$ alkynyl are optionally interrupted by one or more group(s) selected from the group consisting of T, -C(O)O-; -O-; -C(O)-; -C(O)N($R^9$)-; -S(O)$_2$N($R^9$)-; -S(O)N($R^9$)-; -S(O)$_2$-;-S(O)-; -N($R^9$)S(O)$_2$N($R^{9a}$)-; -S-; -N($R^9$)-; -OC(O)$R^9$; -N($R^9$)C(O)-; -N($R^9$)S(O)$_2$-;-N($R^9$)S(O)-; -N($R^9$)C(O)O-; -N($R^9$)C(O)N($R^{9a}$)-; and -OC(O)N($R^9R^{9a}$); wherein

| | |
|---|---|
| $R^9$, $R^{9a}$ | are independently selected from the group consisting of H; T; $C_{1-50}$ alkyl; $C_{2-50}$ alkenyl; and $C_{2-50}$ alkynyl, which T; $C_{1-50}$ alkyl; $C_{2-50}$ alkenyl; and $C_{2-50}$ alkynyl are optionally substituted with one or more $R^{10}$, which are the same or different and which $C_{1-50}$ alkyl; $C_{2-50}$ alkenyl; and $C_{2-50}$ alkynyl are optionally interrupted by one or more group(s) selected from the group consisting of T, -C(O)O-; -O-; -C(O)-; -C(O)N($R^{11}$)-; -S(O)$_2$N($R^{11}$)-; -S(O)N($R^{11}$)-; -S(O)$_2$-; -S(O)-; -N($R^{11}$)S(O)$_2$N($R^{11a}$)-; -S-; -N($R^{11}$)-; -OC(O)$R^{11}$; -N($R^{11}$)C(O)-; -N($R^{11}$)S(O)$_2$-; -N($R^{11}$)S(O)-; -N($R^{11}$)C(O)O-; -N($R^{11}$)C(O)N($R^{11a}$)-; and -OC(O)N($R^{11}R^{11a}$); |
| T | is selected from the group consisting of phenyl; naphthyl; indenyl; indanyl; tetralinyl; $C_{3-10}$ cycloalkyl; 4- to 7-membered heterocyclyl; and 8- to 11- membered heterobicyclyl, wherein T is optionally substituted with one or more $R^{10}$, which are the same or different; |
| $R^{10}$ | is halogen; CN; oxo (=O); COOR$^{12}$; OR$^{12}$; C(O)R$^{12}$; C(O)N($R^{12}R^{12a}$); S(O)$_2$N($R^{12}R^{12a}$); S(O)N($R^{12}R^{12a}$); S(O)$_2$R$^{12}$; S(O)R$^{12}$; N($R^{12}$)S(O)$_2$N($R^{12a}R^{12b}$); SR$^{12}$; N($R^{12}R^{12a}$); $NO_2$; OC(O)R$^{12}$; N($R^{12}$)C(O)R$^{12a}$; N($R^{12}$)S(O)$_2$R$^{12a}$; N($R^{12}$)S(O)R$^{12a}$; N($R^{12}$)C(O)OR$^{12a}$; N($R^{12}$)C(O)N($R^{12a}R^{12b}$); OC(O)N($R^{12}R^{12a}$); or $C_{1-6}$ alkyl, which $C_{1-6}$ alkyl is optionally substituted with one or more halogen, which are the same or different; |
| $R^{11}$, $R^{11a}$, $R^{12}$, $R^{12a}$, $R^{12b}$ | are independently of each other selected from the group consisting of H; and $C_{1-6}$ alkyl, which $C_{1-6}$ alkyl is optionally substituted with one or more halogen, which are the same or different. |

In another embodiment $Z^0$ of step (C) is an inert polymer having a molecular weight ranging from 0.5 kDa to 1000 kDa, preferably having a molecular weight ranging from 0.5 to 500 kDa, more preferably having a molecular weight ranging from 0.75 to 250 kDa, even more preferably ranging from 1 to 100 kDa, even more preferably ranging from 5 to 60 kDa, even more preferably from 10 to 50 and most preferably Z has a molecular weight of 40 kDa.

Preferably, $Z^0$ of step (C) is an inert polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

In a preferred embodiment $Z^0$ of step (C) is an inert linear or branched PEG-based polymer comprising at least 70% PEG or a hyaluronic acid-based polymer comprising at least 70% hyaluronic acid. More preferably, $Z^0$ of step (C) is an inert linear or branched PEG-based polymer comprising at least 70% PEG, even more preferably comprising at least 80% PEG and most preferably comprising at least 90% PEG.

In another preferred embodiment $Z^0$ of step (C) is a zwitterionic polymer. Preferrably, such zwitterionic polymer comprises poly(amino acids) and/or poly(acrylates).

As used herein, the terms "zwitterion" and "zwitterionic" refer to a neutral molecule or moiety with positive and negative charges at different locations within that molecule or moiety at the same time.

According to Zhang et al. (Nature Biotechnology, 2013, volume 31, number 6, pages 553-557) hydrogels made of zwitterionic polymers resist the foreign body response.

Step (C) comprises reacting the hydrogel of step (A) or step (B) with a reagent of formula (VII) in such manner that no more than 99 mol-% of $A^{x0'}$ or $A^{x2}$ react with $A^{x3}$. This can be achieved, for example, by reacting at most 0.99 chemical equivalents of the reagent of formula (VII) relative to $A^{x0'}$ or $A^{x2}$ with the hydrogel of step (A) or (B).

In order to prevent the reaction of more than 0.99 chemical equivalents, the reagent of formula (VII) can be used in an amount of at most 0.99 chemical equivalents relative to $A^{x0'}$ or $A^{x2}$ or, alternatively, the reaction rate is monitored and the reaction is interrupted when at most 0.99 chemical equivalents relative to $A^{x0'}$ or $A^{x2}$ have reacted, especially when more than 0.99 chemical equivalents are used. It is understood that also due to physical constraints, such as steric hindrance, hydrophobic properties or other characteristics of the inert moiety Z, no more than 0.99 chemical equivalents may be capable of reacting with $A^{x0'}$ or $A^{x2}$, even if more chemical equivalents are added to the reaction.

Preferably, step (C) comprises reacting the hydrogel of step (A) or step (B) with a reagent of formula (VII) in such manner that no more than 80 mol-% of $A^{x0'}$ or $A^{x2}$ react with $A^{x3}$, even more preferably, such that no more than 60 mol-% of $A^{x0'}$ or $A^{x2}$ react with $A^{x3}$, even more preferably, such that no more than 40 mol-% of $A^{x0'}$ or $A^{x2}$ react with $A^{X3}$, even more preferably, such that no more than 20 mol-% of $A^{x0'}$ or $A^{x2}$ react with $A^{x3}$ and most preferably, such that no more than 15 mol-% of $A^{x0'}$ or $A^{x2}$ react with $A^{x3}$.

This can be achieved, for example, by reacting at most 0.8, 0.6, 0.4, 0.2 or 0.15 chemical equivalents of the reagent of formula (VII) relative to $A^{x0'}$ or $A^{x2}$ with the hydrogel of step (A) or (B), respectively.

Methods to prevent the reaction of more chemical equivalents are described above.

Based on the measurements of the amount of substance of $A^{x0'}$ of step (A) and after step (C) the amount of substance of reacted $A^{x0'}$ can be calculated with equation (1):

$$(1)\ \text{Amount of substance of reacted } A^{x0'} \text{ in mmol/g} = (A^{x0'}{}_1 - A^{x0'}{}_2) / (A^{x0'}{}_2 \times MW_Z + 1),$$

wherein

- $A^{x0'}{}_1$ is the amount of substance of functional groups $A^{x0'}$ of the hydrogel of step (A) in mmol/g;
- $A^{x0'}{}_2$ is the amount of substance of functional groups $A^{x0'}$ of the hydrogel after step (C) in mmol/g; and
- $MW_Z$ is the molecular weight of Z in g/mmol.

If the optional spacer reagent was covalently conjugated to the hydrogel of step (A), the calculation of the number of reacted $A^{x2}$ is done accordingly.

The percentage of reacted functional groups $A^{x0'}$ relative to the functional groups $A^{x0'}$ of the hydrogel of step (A) is calculated according to equation (2):

$$(2)\ \text{mol-\% of reacted } A^{x0'} = 100 \times [(A^{x0'}{}_1 - A^{x0'}{}_2) / (A^{x0'}{}_2 \times MW_Z + 1)] / A^{x0'}{}_1,$$

wherein the variables are used as above.

In one embodiment $Z^0$ of step (C) is conjugated to the surface of the hydrogel. This can be achieved by selecting the size and structure of the reagent $A^{x3}$-$Z^0$ such that it is too large to enter the pores or network of the hydrogel. Accordingly, the minimal size of $A^{x3}$-$Z^0$ depends on the properties of the hydrogel. The person skilled in the art however knows methods how to test whether a reagent $A^{x3}$-$Z^0$ is capable of entering into the hydrogel using standard experimentation, for example by using size exclusion chromatography with the hydrogel as stationary phase.

In a preferred embodiment, the hydrogel-linked IL-1ra prodrug or pharmaceutically acceptable salt thereof has a ratio R ranging from 0.1 to 0.8, and wherein R is defined as

$$R = \frac{[\text{total mass of all IL-1ra moieties}]}{[\text{total mass of hydrogel-linked IL-1ra prodrug}]} .$$

More preferably, R ranges from 0.2 to 0.7.

Another non-claimed aspect of the present disclosure is a pharmaceutical composition comprising at least one - preferably, one, two or three; even more preferably one - hydrogel-linked IL-1ra prodrug as described before and optionally one or more excipients.

The pharmaceutical composition of hydrogel-linked IL-1ra prodrug may be provided as a suspension composition or as a dry composition.

The term "suspension composition" relates to a mixture of hydrogel-linked IL-1ra prodrug containing a water-insoluble polymer, i.e. the hydrogel carrier Z, and one or more solvents, such as water. Due to the water-insoluble polymer, the polymeric prodrug cannot dissolve and renders the prodrug in a particulate state.

"Dry composition" means that the prodrug composition is provided in a dry form. Suitable methods for drying are spray-drying and lyophilization, i.e. freeze-drying. Such dry composition of prodrug has a residual water content of a maximum of 10 %, preferably less than 5% and more preferably less than 2%, determined according to Karl Fischer.

In case of dry compositions, suitable methods of drying are, for example, spray-drying and lyophilization, i.e. freeze-drying. Preferably, the pharmaceutical composition comprising hydrogel-linked IL-1ra prodrug is dried by lyophilization.

Preferably, the hydrogel-linked IL-1ra prodrug in either suspension or dry pharmaceutical compositions is sufficiently dosed in the pharmaceutical composition to provide therapeutically effective amount of IL-1ra for at least one week, such as one week, two weeks, three weeks, four weeks, five weeks, six weeks, seven weeks, eight weeks, three months, four months, five months, six months, seven months, eight months, nine months, ten months, eleven months or twelve months.

The pharmaceutical composition of polymeric IL-1ra according to the present invention, whether in dry or suspension form, preferably comprises one or more excipients.

Excipients used in parenteral compositions may be categorized as buffering agents, isotonicity modifiers, preservatives, stabilizers, anti-adsorption agents, oxidation protection agents, viscosifiers/viscosity enhancing agents, or other auxiliary agents. In some cases, these ingredients may have dual or triple functions. The one or more excipients are selected from the groups consisting of:

(i) Buffering agents: physiologically tolerated buffers to maintain pH in a desired range, such as sodium phosphate, bicarbonate, succinate, histidine, citrate and acetate, sulphate, nitrate, chloride, pyruvate. Antacids such as $Mg(OH)_2$ or $ZnCO_3$ may be also used. Buffering capacity may be adjusted to match the conditions most sensitive to pH stability

(ii) Isotonicity modifiers: to minimize pain that can result from cell damage due to osmotic pressure differences at the injection depot. Glycerin and sodium chloride are examples. Effective concentrations can be determined by osmometry using an assumed osmolality of 285-315 mOsmol/kg for serum

(iii) Preservatives and/or antimicrobials: multidose parenteral preparations require the addition of preservatives at a sufficient concentration to minimize risk of patients becoming infected upon injection and corresponding regulatory requirements have been established. Typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride

(iv) Stabilizers: Stabilisation is achieved by strengthening of the protein-stabilising forces, by destabilisation of the denatured stater, or by direct binding of excipients to the protein. Stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as

phenolic derivatives. In addition, oligomers or polymers such as cyclodextrins, dextran, dendrimers, PEG or PVP or protamine or HSA may be used

(v) Anti-adsorption agents: Mainly ionic or non-ionic surfactants or other proteins or soluble polymers are used to coat or adsorb competitively to the inner surface of the composition's container. E.g., poloxamer (Pluronic F-68), PEG dodecyl ether (Brij 35), polysorbate 20 and 80, dextran, polyethylene glycol, PEG-polyhistidine, BSA and HSA and gelatines. Chosen concentration and type of excipient depends on the effect to be avoided but typically a monolayer of surfactant is formed at the interface just above the CMC value

(vi) Lyo- and/or cryoprotectants: During freeze- or spray drying, excipients may counteract the destabilising effects caused by hydrogen bond breaking and water removal. For this purpose sugars and polyols may be used but corresponding positive effects have also been observed for surfactants, amino acids, non-aqueous solvents, and other peptides. Trehalose is particulary efficient at reducing moisture-induced aggregation and also improves thermal stability potentially caused by exposure of protein hydrophobic groups to water. Mannitol and sucrose may also be used, either as sole lyo/cryoprotectant or in combination with each other where higher ratios of mannitol:sucrose are known to enhance physical stability of a lyophilized cake. Mannitol may also be combined with trehalose. Trehalose may also be combined with sorbitol or sorbitol used as the sole protectant. Starch or starch derivatives may also be used

(vii) Oxidation protection agents: antioxidants such as ascorbic acid, ectoine, methionine, glutathione, monothioglycerol, morin, polyethylenimine (PEI), propyl gallate, vitamin E, chelating agents such aus citric acid, EDTA, hexaphosphate, thioglycolic acid

(viii) Viscosifiers or viscosity enhancers: retard settling of the particles in the vial and syringe and are used in order to facilitate mixing and resuspension of the particles and to make the suspension easier to inject (i.e., low force on the syringe plunger). Suitable viscosifiers or viscosity enhancers are, for example, carbomer viscosifiers like Carbopol 940, Carbopol Ultrez 10, cellulose derivatives like hydroxypropylmethylcellulose (hypromellose, HPMC) or diethylaminoethyl cellulose (DEAE or DEAE-C), colloidal magnesium silicate (Veegum) or sodium silicate, hydroxyapatite gel, tricalcium phosphate gel, xanthans, carrageenans like Satia gum UTC 30, aliphatic poly(hydroxy acids), such as poly(D,L- or L-lactic acid) (PLA) and poly(glycolic acid) (PGA) and their copolymers (PLGA), terpolymers of D,L-lactide, glycolide and caprolactone, poloxamers, hydrophilic poly(oxyethylene) blocks and hydrophobic poly(oxypropylene) blocks to make up a triblock of poly(oxyethylene)-poly(oxypropylene)-poly(oxyethylene) (e.g. Pluronic®), polyetherester copolymer, such as a polyethylene glycol terephthalate/polybutylene terephthalate copolymer, sucrose acetate isobutyrate (SAIB), dextran or derivatives thereof, combinations of dextrans and PEG, polydimethylsiloxane, collagen, chitosan, polyvinyl alcohol (PVA) and derivatives, polyalkylimides, poly (acrylamide-co-diallyldimethyl ammonium (DADMA)), polyvinylpyrrolidone (PVP), glycosaminoglycans (GAGs) such as dermatan sulfate, chondroitin sulfate, keratan sulfate, heparin, heparan sulfate, hyaluronan, ABA triblock or AB block copolymers composed of hydrophobic A-blocks, such as polylactide (PLA) or poly(lactide-co-glycolide) (PLGA), and hydrophilic B-blocks, such as polyethylene glycol (PEG) or polyvinyl pyrrolidone. Such block copolymers as well as the above-mentioned poloxamers may exhibit reverse thermal gelation behavior (fluid state at room temperature to facilitate administration and gel state above sol-gel transition temperature at body temperature after injection).

(ix) Spreading or diffusing agent: modifies the permeability of connective tissue through the hydrolysis of components of the extracellular matrix in the intrastitial space such as hyaluronic acid, a polysaccharide found in the intercellular space of connective tissue. A spreading agent such as but not limited to hyaluronidase temporarily decreases the viscosity of the extracellular matrix and promotes diffusion of injected drugs.

(x) Other auxiliary agents: such as wetting agents, viscosity modifiers, antibiotics, hyaluronidase. Acids and bases such as hydrochloric acid and sodium hydroxide are auxiliary agents necessary for pH adjustment during manufacture

In one embodiment the dry composition comprising hydrogel-linked IL-1ra prodrug comprises one or more preservatives and/or antimicrobials.

Another non-claimed aspect of the present disclosure is a container comprising the hydrogel-linked IL-1ra prodrug or the dry or suspension form of the pharmaceutical composition comprising the hydrogel-linked IL-1ra prodrug.

Suitable containers for suspension compositions are, for example, syringes, vials, vials with stopper and seal, ampoules, and cartridges. In particular, a suspension compositions according to the present disclosure may be provided in a syringe.

Suitable containers for dry compositions are, for example, syringes, dual-chamber syringes, vials, vials with

stopper and seal, ampoules, and cartridges. In particular, a dry composition according to the present disclosure may be provided in a first chamber of the dual-chamber syringe and reconstitution solution is provided in a second chamber of the dual-chamber syringe.

In one aspect of the present disclosure, the dry or suspension composition of hydrogel-linked IL-1ra prodrug is provided as a single dose, meaning that the container in which it is supplied contains one pharmaceutical dose.

In another aspect of the present disclosure the dry or suspension composition comprising hydrogel-linked IL-1ra prodrug is provided as a multiple dose composition, meaning that the container in which it is supplied contains more than one pharmaceutical dose. Such multiple dose composition of hydrogel-linked IL-1ra prodrug can either be used for different patients in need thereof or is intended for use in one patient, wherein the remaining doses are stored after the application of the first dose until needed.

Prior to applying a dry composition of hydrogel-linked IL-1ra prodrug to a patient in need thereof, the dry composition is reconstituted.

Reconstitution may take place in the container in which the dry composition of hydrogel-linked IL-1ra prodrug is provided, such as in a vial, vial with stopper and seal, syringe, dual-chamber syringe, ampoule, and cartridge.

Reconstitution is done by adding a predefined amount of reconstitution solution to the dry composition. Reconstitution solutions are sterile liquids, such as water or buffer, which may contain further additives, such as preservatives and/or antimicrobials, such as, for example, benzyl alcohol and cresol. Preferably, the reconstitution solution is sterile water.

A further aspect is a method of preparing a reconstituted composition comprising a therapeutically effective amount of hydrogel-linked IL-1ra prodrug of the present invention, and optionally one or more pharmaceutically acceptable excipients the method comprising the step of

- contacting the dry pharmaceutical composition with a reconstitution solution.

Another aspect is a reconstituted composition comprising a therapeutically effective amount of hydrogel-linked IL-1ra prodrug of the present invention, and optionally one or more pharmaceutically acceptable excipients.

Another non-claimed aspect of the present disclosure is the method of manufacturing a suspension composition of hydrogel-linked IL-1ra prodrug. In one embodiment, such suspension composition is made by

(i) admixing the hydrogel-linked IL-1ra prodrug with one or more excipients,
(ii) transferring amounts equivalent to single or multiple doses into a suitable container, and
(iii) sealing the container.

Suitable containers are syringes, vials, vials with stopper and seal, ampoules, and cartridges.

Another non-claimed aspect of the present disclosure is the method of manufacturing a dry composition of hydrogel-linked IL-1ra prodrug. In one embodiment, such dry composition is made by

(i) admixing the hydrogel-linked IL-1ra prodrug with one or more excipients,
(ii) transferring amounts equivalent to single or multiple doses into a suitable container,
(iii) drying the composition in said container, and
(iv) sealing the container.

Alternatively, the method comprises the steps of

(i) transferring amounts equivalent to single or multiple doses of hydrogel-linked IL-1ra prodrug into a suitable container,
(ii) adding one or more excipients to the container,
(iii) drying the composition in said container, and
(iv) sealing the container.

Suitable containers are syringes, dual-chamber syringes, vials, vials with stopper and seal, ampoules, and cartridges.

"Sealing a container" means that the container is closed in such way that it is airtight, allowing no gas exchange between the outside and the inside and maintaining sterility, if the content of the container is sterile.

Another aspect is a kit of parts for a dry composition according to the present disclosure.

When the administration device is simply a hypodermic syringe then the kit may comprise the syringe, a needle and a container comprising the dry hydrogel-linked IL-1ra prodrug composition for use with the syringe and a second container comprising the reconstitution solution. In more preferred embodiments, the injection device is other than a

simple hypodermic syringe and so the separate container with reconstituted hydrogel-linked IL-1ra prodrug is adapted to engage with the injection device such that in use the suspension composition in the container is in fluid connection with the outlet of the injection device. Examples of administration devices include hypodermic syringes and pen injector devices. Particularly preferred injection devices are syringes suitable for intraarticular injection.

A preferred kit of parts for a dry composition comprises a needle and a container containing the composition according to the present disclosure and optionally further containing a reconstitution solution, the container being adapted for use with the needle. Preferably, the container is a dual-chamber syringe.

Another aspect is a kit of parts for a suspension composition according to the present disclosure.

When the administration device is simply a hypodermic syringe then the kit may comprise a container with the suspension composition and a needle for use with the container.

In another aspect, the disclosure provides a cartridge containing a composition of hydrogel-linked IL-1ra prodrug, whether in dry or suspension form, as hereinbefore described for use with a syringe suitable for intraarticular injection. The cartridge may contain a single dose or a multiplicity of doses of hydrogel-linked IL-1ra prodrug.

Another aspect of the present disclosure is a hydrogel-linked IL-1ra prodrug of the present invention or a pharmaceutically acceptable salt thereof or a pharmaceutical composition comprising such hydrogel-linked IL-1ra prodrugs, for use as a medicament.

In another embodiment, the hydrogel-linked IL-1ra prodrug or a pharmaceutical composition comprising hydrogel-linked IL-1ra prodrug or a pharmaceutically acceptable salt thereof is used in a method of treating IL-1 mediated diseases. According to the invention, the hydrogel-linked IL-1ra prodrug is for use in a method of treating an inflammatory condition of the joint,

more preferably osteoarthritis, via intra-articular administration.

Another aspect of the present disclosure is the use of the hydrogel-linked IL-1ra prodrug or a pharmaceutically acceptable salt thereof or a pharmaceutical composition comprising hydrogel-linked IL-1ra prodrug for the manufacture of a medicament for treating IL-1 mediated diseases, preferably for use in a method of treating an inflammatory condition of the joint, more preferably osteoarthritis. Preferably, such method comprises the step of injecting the hydrogel-linked IL-1ra prodrug or a pharmaceutical salt thereof or a pharmaceutical composition comprising the hydrogel-linked IL-1ra prodrug of the present disclosure intraarticularly.

Thus a further aspect of the present disclosure is a method of treating, controlling, delaying or preventing in a mammalian patient, preferably a human patient, in need of the treatment of one or more IL-1 mediated diseases comprising the step of administering to said patient in need thereof a therapeutically effective amount of hydrogel-linked IL-1ra prodrug or a pharmaceutically acceptable salt thereof or a pharmaceutical composition comprising hydrogel-linked IL-1ra prodrug of the present invention.

A disease or medical condition is considered to be an "interleukin-1 mediated disease" or "IL-1 mediated disease" if the spontaneous or experimental disease or medical condition is associated with elevated levels of IL-1 in bodily fluids or tissue or if cells or tissues taken from the body produce elevated levels of IL-1 in culture. In many cases, such interleukin-1 mediated diseases are also recognized by the following additional two conditions: (1) pathological findings associated with the disease or medical condition can be mimicked experimentally in animals by the administration of IL-1; and (2) the pathology induced in experimental animal models of the disease or medical condition can be inhibited or abolished by treatment with agents which inhibit the action of IL-1. In most interleukin-1 mediated diseases at least two of the three conditions are met, and in many interleukin-1 mediated diseases all three conditions are met.

A non-exclusive list of acute and chronic interleukin-1 (IL-1)-mediated inflammatory diseases includes the following: acute pancreatitis, ALS, Alzheimer's disease, cachexia/anorexia, asthma, atherosclerosis, chronic fatigue syndrome, fever, diabetes (e.g., insulin diabetes), glomerulonephritis, graft versus host rejection, hemohorragic shock, hyperalgesia, inflammatory bowel disease, inflammatory conditions of a joint including osteoarthritis, psoriatic arthritis and rheumatoid arthritis; ischemic injury, including cerebral ischemia (e.g., brain injury as a result of trauma, epilepsy, hemorrhage or stroke, each of which may lead to neurodegeneration); lung diseases (e.g., ARDS), multiple myeloma, multiple sclerosis, myelogenous (e.g., AML and CML) and other leukemias; myopathies (e.g., muscle protein metabolism, esp. in sepsis), osteoporosis, Parkinson's disease, pain, pre-term labor, psoriasis, reperfusion injury, septic shock, side effects from radiation therapy, temporal mandibular joint disease, tumor metastasis, or an inflammatory condition resulting from strain, sprain, cartilage damage, trauma, orthopedic surgery, infection or other disease processes.

According to the invention, the interleukin-1-mediated disease is an inflammatory condition of the joint, more preferably osteoarthritis.

Hydrogel-linked IL-1ra prodrugs and pharmaceutical compositions comprising such prodrugs according to the present disclosure may be administered to a patient in therapeutically effective amounts for the treatment of IL-1 mediated diseases, preferably for the treatment of an inflammatory condition of the joint and most preferably for the treatment of osteoarthritis. The term "patient" is intended to encompass animals (e.g., cats, dogs and horses) as well as humans, preferably humans.

An additional aspect of the present disclosure relates to the way of administration of a hydrogel-linked IL-1ra

prodrug or a reconstituted or suspension pharmaceutical composition of hydrogel-linked IL-1ra prodrug, which can be administered via topical, enteral or parenteral administration and by methods of external application, injection or infusion, including intraarticular, intradermal, subcutaneous, intramuscular, intravenous, intraosseous, and intraperitoneal, intrathecal, intracapsular, intraorbital, intravitreal, intratympanic, intravesical, intracardiac, transtracheal, subcuticular, intraarticular, subcapsular, subarachnoid, intraspinal, intraventricular and intrasternal.

Accordingly another aspect of the present disclosure is a prodrug of the present invention or a pharmaceutical composition of present invention, wherein such prodrug or pharmaceutical composition is suitable to be administered to a patient via topical, enteral or parenteral administration and by methods of external application, injection or infusion, including intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular and intrasternal application. Accordingly another aspect of the present invention is a prodrug of the present invention or a pharmaceutical composition of present invention, wherein such prodrug or pharmaceutical composition is suitable to be administered to a patient via topical, enteral or parenteral administration and by methods of external application, injection or infusion, including intraarticular, intradermal, subcutaneous, intramuscular, intravenous, intraosseous, and intraperitoneal, intrathecal, intracapsular, intraorbital, intravitreal, intratympanic, intravesical, intracardiac, transtracheal, subcuticular, intraarticular, subcapsular, subarachnoid, intraspinal, intraventricular and intrasternal application.

Thus, a further aspect of the present disclosure is a prodrug of the present invention or a pharmaceutical composition of the present invention for use in a method for treating IL-1 mediated diseases by an administration form as mentioned herein. According to the invention, the hydrogel-linked IL-1ra prodrug is administered via intra-articular administration.

In one aspect, the present disclosure relates to a hydrogel-linked IL-1ra prodrug or pharmaceutically acceptable salt thereof or a pharmaceutical composition of the present invention, for use in the treatment of osteoarthritis.

In a preferred embodiment, the present invention relates to a hydrogel-linked IL-1ra prodrug or pharmaceutically acceptable salt thereof or a pharmaceutical composition of the present invention, for use in the treatment of osteoarthritis via intra-articular administration

In a further aspect, the present disclosure relates to a hydrogel-linked IL-1ra prodrug or pharmaceutically acceptable salt thereof or a pharmaceutical composition of the present invention, for use for topical, enteral, or parenteral administration, for external application, injection or infusion, including intra-articular, intradermal, subcutaneous, intramuscular, intravenous, intraosseous, and intraperitoneal, intrathecal, intracapsular, intraorbital, intravitreal, intratympanic, intravesical, intracardiac, transtracheal, subcuticular, intraarticular, subcapsular, subarachnoid, intraspinal, intraventricular and/or for intrasternal application.

The hydrogel-linked IL-1ra prodrug and pharmaceutical compositions comprising such hydrogel-linked IL-1ra prodrug may also be administered via oral administration or be administered through mucus membranes, that is, intranasally, sublingually, buccally or rectally for systemic delivery.

It is preferred that the hydrogel-linked IL-1ra prodrugs, pharmaceutically acceptable salts thereof and pharmaceutical compositions comprising hydrogel-linked IL-1ra prodrugs or pharmaceutically acceptable salts thereof are administered via intraarticular injection.

By way of example, in one specific aspect hydrogel-linked IL-1ra prodrugs, pharmaceutically acceptable salts thereof and pharmaceutical compositions comprising such prodrugs or pharmaceutically acceptable salts thereof may be administered subcutaneously or intramuscularly for the treatment of rheumatoid arthritis.

According to the invention, administration of the hydrogel-linked IL-1ra prodrug, pharmaceutically acceptable salt thereof or pharmaceutical composition comprising hydrogel-linked IL-1ra prodrug or pharmaceutically acceptable salt thereof in the method of treatment of osteoarthritis is via intraarticular administration.

By way of example, in one aspect of the disclosure, hydrogel-linked IL-1ra prodrug, a pharmaceutically acceptable salt thereof or a pharmaceutical composition comprising such prodrug of the pharmaceutically acceptable salt thereof may be administered subcutaneously or intramuscularly in a method of treatment of rheumatoid arthritis, inflammatory bowel disease, multiple sclerosis, multiple myeloma, or myelogenous (e.g., AML and CML) and other leukemias.

By way of example, in another aspect of the disclosure, hydrogel-linked IL-1ra prodrug, a pharmaceutically acceptable salt thereof or a pharmaceutical composition comprising such prodrug of the pharmaceutically acceptable salt thereof may be administered intravenously in a method of treatment of brain injury as a result of trauma, epilepsy, hemorrhage or stroke, or for the treatment of graft-versus-host disease; or administered intraventricularly in a method of treatment of brain injury as a result of trauma.

Regardless of the manner of administration, the treatment of IL-1-mediated disease requires a dose or total dose regimen of the hydrogel-linked IL-1ra prodrug or a pharmaceutically acceptable salt thereof of effective amounts, i.e., effective to prevent, reduce or alleviate symptoms of the disease, such as to counteract progressive cartilage destruction of a joint as caused by degradation of proteoglycans which are a molecular component of articular cartilage.

The specific dose is calculated according to the approximate body weight or surface area of the patient. Other

factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data.

The frequency of dosing depends on the disease and condition of the patient, as well as the pharmacokinetic parameters of the hydrogel-linked IL-1ra prodrug or pharmaceutically acceptable salt thereof used in the formulation, and the route of administration.

The hydrogel-linked IL-1ra prodrug or pharmaceutically acceptable salt thereof may be administered once, or in cases of severe and prolonged disorders, administered daily or in less frequent doses or administered with an initial bolus dose followed by a continuous dose or sustained delivery.

Preferred modes of using polymeric IL-1ra prodrug or a pharmaceutically acceptable salt thereof for treatment of IL-1 mediated diseases, including inflammatory conditions of a joint such as rheumatoid arthritis and psoriatic arthritis, are set forth in AU 9173636. Accordingly, such dosage regiment would require: (1) a single intraarticular injection of hydrogel-linked IL-1ra prodrug or a pharmaceutically acceptable salt thereof given periodically as needed to prevent or remedy the flare-up of arthritis and (2) periodic subcutaneous injections of hydrogel-linked IL-1ra prodrug product or a pharmaceutically acceptable salt thereof.

When administered parenterally, the unit dose may be up to 200 mg, generally up to 150 mg and more generally up to 100 mg. When administered into an articular cavity, the pharmaceutical composition comprising hydrogel-linked IL-1ra prodrug or a pharmaceutically acceptable salt thereof is preferably administered as a single injection from a 0.5 to 10 ml syringe containing a dose up to 200 mg/ml, generally up to 150 mg and more generally up to 100 mg of hydrogel-linked IL-1ra prodrug in isotonic buffered saline, such as isotonic phosphate or citrate buffered saline. The initial single injection of the pharmaceutical composition comprising hydrogel-linked IL-1ra prodrug or a pharmaceutically acceptable salt thereof may be followed by one or more further such injection(s).

The pharmaceutical composition comprising hydrogel-linked IL-1ra prodrug or a pharmaceutically acceptable salt thereof is administered into an articular cavity at a frequency of once every day, once every two days, once every three days, once every four days, once every five days, once every six days, once weekly, once every two weeks, once every three weeks, once every four weeks, once every five weeks, once every six weeks, once every seven weeks, once every eight weeks, once every three months, once every four months, once every five months, once every six months, once every seven months, once every eight months, once every nine months, once every ten months, once every eleven months, once yearly.

The pharmaceutical composition comprising hydrogel-linked IL-1ra prodrug or a pharmaceutically acceptable salt thereof of the present invention may comprise or may be administered with one or more other drug(s) suitable for the indication being treated, i.e. the hydrogel-linked IL-1ra prodrugs or a pharmaceutically acceptable salt thereof may be administered to a patient in need thereof in the form of a combination or concurrent therapy. The pharmaceutical composition comprising hydrogel-linked IL-1ra prodrug or a pharmaceutically acceptable salt thereof may thus additionally comprise one or more drug(s) other than IL-1ra or prodrugs or hydrogel-linked prodrug of such other drug(s), from which the one or more drug(s) other than IL-1ra are released in addition to IL-1ra. The release of said one or more drug(s) other than IL-1ra occurs either before (pretreatment), during (concurrent treatment), or after (post-treatment) the release of IL-1ra or in any combination thereof.

In one preferred embodiment, the present invention relates to a pharmaceutical composition of the present invention which, additionally comprises one or more drugs or prodrugs other than IL-1ra or prodrugs thereof, preferably wherein the one or more drugs is a hydrogel-linked prodrug. Preferably such one or more drug is selected from NSAIDs , SAARDs and biologics or other suitable drugs described below.

In a preferred embodiment, the one or more additional biologically active moieties other than IL-1ra or prodrugs thereof are selected from the group consisting of indomethacin; non-steroidal anti-inflammatory drugs (NSAIDs) such as aspirin, ibuprofen, and other propionic acid derivatives (such as alminoprofen, benoxaprofen, bucloxic acid, carprofen, fenbufen, fenoprofen, fluprofen, flurbiprofen, indoprofen, ketoprofen, miroprofen, naproxen, oxaprozin, pirprofen, pranoprofen, suprofen, tiaprofenic acid, and tioxaprofen); acetic acid derivatives (such as indomethacin, acemetacin, alclofenac, clidanac, diclofenac, fenclofenac, fenclozic acid, fentiazac, fuirofenac, ibufenac, isoxepac, oxpinac, sulindac, tiopinac, tolmetin, zidometacin, and zomepirac); fenamic acid derivatives (such as flufenamic acid, meclofenamic acid, mefenamic acid, niflumic acid and tolfenamic acid); biphenylcarboxylic acid derivatives (such as diflunisal and flufenisal); oxicams (such as isoxicam, piroxicam, sudoxicam and tenoxican); salicylates (such as acetyl salicylic acid, sulfasalazine) and the pyrazolones (such as apazone, bezpiperylon, feprazone, mofebutazone, oxyphenbutazone, phenylbutazone) and opioid analgesics (such as such as fentanyl, morphine, sufentanil, hydromorphone, methadone, oxycodone, buprenorphine); methotrexate, cyclooxygenase-2 (COX-2) inhibitors (such as celecoxib), anti-TNF agents (such as adalimumab, certolizumab pegol, etanercept, golimumab, infliximab); anti-IL-1,-6,-12, -15, -18 and -21 and -23 agents (such as anakinra, Tocilizumab); Nerve growth factor inhibitors, nerve growth factor receptor (NGFR) antagonists, RN64,

REGN475, fasinumab, tanezumab, MEDI578, ABT110, anti-NGF antibodies and antibody derivatives, and anti-NGFR antibodies and antibody derivatives; TrkA antagonists (such as ARRY-470, FX007, ARRY 872) glucocorticoids or steroids (such as cortisone, prednisolone, flurometholone, dexamethasone, medrysone, loteprednol, fluazacort, hydrocortisone, prednisone, betamethasone, clobetasone, prednisone, methylprednisolone, riamcinolone hexacatonide, paramethasone acetate, diflorasone, fluocinonide, fluocinolone, triamcinolone, derivatives thereof, and mixtures thereof); local analgesics (such as lidocaine, bupivacaine, procaine); leflunomide; immunomodulatory agents (such as cyclosporine, tacrolimus, azathioprine, cyclophosphamide, minocycline, rituximab); gold compounds; D-penicillamine; sulfasalazine; chloroquine derivatives (including hydroxychloroquine); CD20 directed antibodies, such as ocrelizumab and ofatumumab; RANKL inhibitors, such as denosumab; TRU-015; INCB018424; VX-V02; bone morphogenetic protein (BMP) (such as BMP-I, BMP-2, BMP-3, BMP-4, BMP-5, BMP-6, BMP-7, BMP-8, BMP-9, BMP-IO, BMP-II, BMP-12, BMP-13, BMP-14, BMP-15, BMP-16, BMP-17, BMP-18, BMP-19, BMP-20, BMP-21); FGF (fibroblast growth factors, such as FGFI FGF2, FGF4, FGF7, FGFIO, FGFI9, FGF21, FGF23); TGF-β (transforming growth factor- β, such as TGF βI); growth hormone; IGF (insulin-like growth factor, such as IGF-I); NELL peptides; VEGF (vascular endothelial growth factor); PDGF (platelet-derived growth factor); PTH (parathyroid hormone)/PTHrp (PTHregulated protein); oxysterols; lipophilic statins, statins (such as atorvastatin, cerivastatin, fluvastatin, lovastatin, mevastatin, pitavastatin, pravastatin, rosuvastatin, simvastatin); growth/differentiation factor 5 (GDF5); LIM mineralization proteins (LMPS); matrix metalloproteinases; aggrecanases (ADAMTSs); cysteine-dependent cathepsins; growth factors; and cell adhesion molecules (CAMs); bisphosphonates(s) (including both N-containing and non-N-containing bisphosphonates(s), selected from the group comprising: pamidronate, neridronate, olpadronate, alendronate, ibandronate, risedronate, and zoledronate. Non-containing bisphosphonates are for example etidronate, clodronate, and tiludronate).

The one or more additional biologically active moieties other than IL-1ra or prodrugs thereof may also be a natural product, isolated or synthesized, and derivatives thereof, including anthraquinones and their prodrugs such as rhein, diacerein, argirein, and aloe-emodin.

The one or more additional biologically active moieties other than IL-1ra or prodrugs thereof may also be a P38 Mitogen activated protein (MAP) kinase inhibitors, such as FX-005, ARRY-797, doramapimod, pamapimod, SB203580, SB202190, LY2228820, VX-702, PH-797804, TAK715, VX-745, SCIO469, ORG48762-0, pyrazolopyridine derivatices, R1503, 5-aminopyrazol-4-yl ketones, and AMG-548; an inhibitor of Matrix metalloproteinase (MMP) activity, such as ALS 1-0635, AC-RCGVPD-NH2 peptide, N-substituted 4-arylsulonylpiperidine-4-hydroxamic acids, 4-aminoprolines, 6-benzyl-5,7-dioxo-6,7-dihydro-5H-thiazolo[3,2-c]pyrimidine-2-carboxylic acid benzyl esters, 4-[1-methyl-2,4-dioxo-6-(3-phenyl-prop-1-ynyl)-1,4-dihydro-2H-quinazolin-3-ylmethyl]-benzoic acids, and galardin; a Tyrosine kinase inhibitor, such as genistein, herbimycin A, 4,5-dianilinophthalimide (DAPH), tyrphostin AG 82, tyrphostin AG 556, anthrapyrazolones, imatinib, gefitinib, erlotinib, sunitinib, polyoxypregane glycoside (PPG), and sorafenib.

The hydrogel-linked IL-1ra prodrug or pharmaceutically acceptable salt thereof and the one or more other prodrug(s) and/or hydrogel-linked prodrug(s) administered to a patient in a combination therapy may exhibit the same or different release kinetics of their corresponding drug(s) as the hydrogel-linked IL-1ra prodrug or pharmaceutically acceptable salt thereof. Hydrogel-linked IL-1ra prodrug or a pharmaceutically acceptable salt thereof and one or more additional anti-inflammatory drug(s) may be administered separately or in combination. Present treatment of IL-1 mediated diseases, as defined above, including acute and chronic inflammation such as inflammatory conditions of a joint (e.g., rheumatoid arthritis) includes first line drugs for control of pain and inflammation, classified as non-steroidal, anti-inflammatory drugs (NSAIDs). Secondary treatments include corticosteroids, slow acting antirheumatic drugs (SAARDs), biologics and/or disease modifying (DM) drugs.

In a specific embodiment, the present disclosure is directed to the use of hydrogel-linked IL-1ra prodrug or pharmaceutically acceptable salt thereof and any of one or more NSAID(s) for the treatment of a IL-1 mediated disease, as defined above, including acute and chronic inflammation such as inflammatory conditions of a joint, e.g., osteoarthritis, psoriatic arthritis and/or rheumatoid arthritis; and graft versus host disease.

NSAIDs owe their anti-inflammatory action, at least in part, to the inhibition of prostaglandin synthesis. NSAIDs can be characterized into nine groups: (1) salicylic acid derivatives; (2) propionic acid derivatives; (3) acetic acid derivatives; (4) fenamic acid derivatives; (5) carboxylic acid derivatives; (6) butyric acid derivatives; (7) oxicams; (8) pyrazoles and (9) pyrazolones.

In one aspect, the present disclosure is directed to the use of the hydrogel-linked IL-1ra prodrug or a pharmaceutically acceptable salt thereof in combination - either as pretreatment, post-treatment or concurrent treatment or combination thereof - with one or more salicylic acid derivative(s) selected from the group comprising: acetaminosalol, aloxiprin, aspirin, benorylate, bromosaligenin, calcium acetylsalicylate, choline magnesium trisalicylate diflusinal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide O-acetic acid, salsalate and sulfasalazine; either in their free form, as prodrug or hydrogel-linked prodrug.

Structurally related salicylic acid derivatives having similar analgesic and anti inflammatory properties are also intended to be encompassed by this group.

In another aspect, the present disclosure is directed to the use of a hydrogel-linked IL-1ra prodrug or pharmaceutically acceptable salt thereof in combination - either as pretreatment, post-treatment or concurrent treatment or combination thereof - with one or more propionic acid derivative(s) selected from the group comprising: alminoprofen, benoxaprofen, bucloxic acid, carprofen, dexindoprofen, fenoprofen, flunoxaprofen, fluprofen, flurbiprofen, furcloprofen, ibuprofen, ibuprofen aluminum, ibuproxam, indoprofen, isoprofen, ketoprofen, loxoprofen, miroprofen, naproxen, oxaprozin, piketoprofen, pimeprofen, pirprofen, pranoprofen, protizinic acid, pyridoxiprofen, suprofen, tiaprofenic acid and tioxaprofen; either in their free form, as prodrug or hydrogel-linked prodrug.

Structurally related propionic acid derivatives having similar analgesic and anti-inflammatory properties are also intended to be encompassed by this group.

In another aspect, the present disclosure is directed to the use of a hydrogel-linked IL-1ra prodrug or pharmaceutically acceptable salt thereof in combination - either as pretreatment, post-treatment or concurrent treatment or combination thereof - with one or more acetic acid derivative(s) selected from the group comprising: acemetacin, alclofenac, amfenac, bufexamac, cinmetacin, clopirac, delmetacin, diclofenac sodium, etodolac, felbinac, fenclofenac, fenclorac, fenclozic acid, fentiazac, furofenac, glucametacin, ibufenac, indomethacin, isofezolac, isoxepac, lonazolac, metiazinic acid, oxametacin, oxpinac, pimetacin, proglumetacin, sulindac, talmetacin, tiaramide, tiopinac, tolmetin, zidometacin and zomepirac; either in their free form, as prodrug or hydrogel-linked prodrug.

Structurally related acetic acid derivatives having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more fenamic acid derivative(s), selected from the group comprising: enfenamic acid, etofenamate, flufenamic acid, isonixin, meclofenamic acid, meclofenamate sodium, medofenamic acid, mefanamic acid, niflumic acid, talniflumate, terofenamate, tolfenamic acid and ufenamate; either in their free form, as prodrug or hydrogel-linked prodrug.

Structurally related fenamic acid derivatives having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more carboxylic acid derivative(s) selected from the group comprising: clidanac, diflunisal, flufenisal, inoridine, ketorolac and tinoridine; either in their free form, as prodrug or hydrogel-linked prodrug.

Structurally related carboxylic acid derivatives having similar and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more butyric acid derivative(s) selected from the group comprising: bumadizon, butibufen, fenbufen and xenbucin; either in their free form, as prodrug or hydrogel-linked prodrug.

Structurally related butyric acid derivatives having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more oxicam(s)selected from the group comprising: droxicam, enolicam, isoxicam, piroxicam, sudoxicam, tenoxicam and 4-hydroxyl-1,2-benzothiazine 1,1-dioxide 4-(N-phenyl)-carboxamide; either in their free form, as prodrug or hydrogel-linked prodrug.

Structurally related oxicams having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more pyrazole(s) selected from the group comprising: difenamizole and epirizole; either in their free form, as prodrug or hydrogel-linked prodrug.

Structurally related pyrazoles having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more pyrazolone(s) selected from the group comprising: apazone, azapropazone, benzpiperylon,

feprazone, mofebutazone, morazone, oxyphenbutazone, phenylbutazone, pipebuzone, propylphenazone, ramifenazone, suxibuzone and thiazolinobutazone; either in their free form, as prodrug or hydrogel-linked prodrug.

Structurally related pyrazalones having similar analgesic and anti-inflammatory properties are also intended to

be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more NSAID(s) selected from the group comrpising: e-acetamidocaproic acid, S-adenosylmethionine, 3-amino-4-hydroxybutyric acid, amixetrine, anitrazafen, antrafenine, bendazac, bendazac lysinate, benzydamine, beprozin, broperamole, bucolome, bufezolac, ciproquazone, cloximate, dazidamine, deboxamet, detomidine, difenpiramide, difenpyramide, difisalamine, ditazol, emorfazone, fanetizole mesylate, fenflumizole, floctafenine, flumizole, flunixin, fluproquazone, fopirtoline, fosfosal, guaimesal, guaiazolene, isonixirn, lefetamine Hel, leflunomide, lofemizole, lotifazole, lysin clonixinate, meseclazone, nabumetone, nictindole, nimesulide, orgotein, orpanoxin, oxaceprolm, oxapadol, paranyline, perisoxal, perisoxal citrate, pifoxime, piproxen, pirazolac, pirfenidone, proquazone, proxazole, thielavin B, tiflamizole, timegadine, tolectin, tolpadol, and tryptamid; either in their free form, as prodrug or hydrogel-linked prodrug.

Structurally related NSAIDs having similar analgesic and anti-inflammatory properties to the above NSAIDs are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more corticosteroid(s) selected from the group comprising: 21-acetoxypregnenolone, alclomerasone, algestone, amcinonide, beclomethasone, betamethasone, betamethasone valerate, budesonide, chloroprednisone, clobetasol, clobetasol propionate, clobetasone, clobetasone butyrate, clocortolone, cloprednol, corticosterone, cortisone, cortivazol, deflazacon, desonide, desoximerasone, dexamethasone, diflorasone, diflucortolone, difluprednate, enoxolone, fluazacort, flucloronide, flumethasone, flumethasone pivalate, flunisolide, flucinolone acetonide, fluocinonide, fluorocinolone acetonide, fluocortin butyl, fluocortolone, fluorocortolone hexanoate, diflucortolone valerate, fluorometholone, fluperolone acetate, fluprednidene acetate, fluprednisolone, flurandenolide, formocortal, halcinonide, halometasone, halopredone acetate, hydrocortamate, hydrocortisone, hydrocortisone acetate, hydrocortisone butyrate, hydrocortisone phosphate, hydrocortisone 21-sodium succinate, hydrocortisone tebutate, mazipredone, medrysone, meprednisone, methylprednicolone, mometasone furoate, paramethasone, prednicarbate, prednisolone, prednisolone 21-diedryaminoacetate, prednisolone sodium phosphate, prednisolone sodium succinate, prednisolone sodium 21-m-sulfobenzoate, prednisolone sodium 21-stearoglycolate, prednisolone tebutate, prednisolone 21-trimethylacetate, prednisone, prednival, prednylidene, prednylidene 21-diethylaminoacetate, tixocortol, triamcinolone, triamcinolone acetonide, triamcinolone benetonide and triamcinolone hexacetonide; either in their free form, as prodrug or hydrogel-linked prodrug.

Structurally related corticosteroids having similar analgesic and anti-inflammatory properties are also intended to be encompassed by this group.

In another aspect, the present disclosure is directed to the use of hydrogel-linked IL-1ra prodrug or pharmaceutically acceptable salt in combination - either as pretreatment, post-treatment or concurrent treatment or combination thereof - with one or more slow-acting antirheumatic drug(s) (SAARD(s)) or disease modifying antirheumatic drug(s) (DMARD(s)) selected from the group comprising: allocupreide sodium, auranofin, aurothioglucose, aurothioglycanide, azathioprine, brequinar sodium, bucillamine, calcium 3-aurothio-2-propanol-1-sulfonate, chlorambucil, chloroquine, clobuzarit, cuproxoline, cyclophosphamide, cyclosporin, dapsone, deoxyspergualin, diacerein, glucosamine, gold salts (e.g., cycloquine gold salt, gold sodium thiomalate, gold sodium thiosulfate), hydroxychloroquine, hydroxyurea, kebuzone, levamisole, lobenzarit, melittin, 6-mercaptopurine, methotrexate, mizoribine, mycophenolate mofetil, myoral, nitrogen mustard, D-penicillamine, pyridinol imidazoles such as SKNF86002 and SB203580, rapamycin, thiols, thymopoietin and vincristine; either in their free form, as prodrug or hydrogel-linked prodrug.

Structurally related SAARDs or DMARDs having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more COX2 inhibitor(s) selected from the group comprising: celecoxib; either in its free form, as prodrug or hydrogel-linked prodrug.

Structurally related COX2 inhibitors having similar analgesic and anti-inflammatory properties are also intended to be encompassed by this group.

In another aspect, the present disclosure is directed to the use of hydrogel-linked IL-1ra prodrug or pharmaceutically acceptable salt thereof in combination - either as pretreatment, post-treatment or concurrent treatment or combination thereof - with one or more antimicrobial(s) selected from the group comprising: ampicillin, amoxycillin, aureomicin, bacitracin, ceftazidime, ceftriaxone, cefotaxime, cephachlor, cephalexin, cephradine, ciprofloxacin, clavulanic acid, cloxacillin, dicloxacillan, erythromycin, flucloxacillan, gentamicin, gramicidin, methicillan, neomycin, oxacillan, penicillin and vancomycin; either in their free form, as prodrug or hydrogel-linked prodrug.

Structurally related antimicrobials having similar analgesic and anti-inflammatory properties are also intended

to be encompassed by this group.

In another aspect, the present disclosure is directed to the use of a hydrogel-linked IL-1ra prodrug or pharmaceutically acceptable salt thereof in combination - either as pretreatment, post-treatment or concurrent treatment or combination thereof - with one or more TNF inhibitor(s) selected from the group comprising: TNF binding proteins (soluble TNF receptors), anti-TNF antibodies, granulocyte colony stimulating factor; thalidomide; BN 50730; tenidap; E 5531; tiapafant PCA 4248; nimesulide; panavir; rolipram; RP 73401; peptide T, MOL 201,449A; (1R,3S)cis-1-[9-(2,6-diaminopurinyl)]-3-hydroxy-4-cyclopentene hydrochloride; (1R,3R)-trans-1-[9-(2,6-diamino)purine]3-acetoxycyclopentane; (1R,3R)-trans-1-[9-adenyl) 3-azidocyclopentane hydrochloride and (1R,3R)-trans-1-[6-hydroxy-purin-9-yl)-3-azidocyclopentane; either in their free form, as prodrug or hydrogel-linked prodrug. TNF binding proteins are known in the art.

In another aspect, the present disclosure is directed to the use of a hydrogel-linked IL-1ra prodrug or pharmaceutically acceptable salt thereof in combination - either as pretreatment, post-treatment or concurrent treatment or combination thereof - with bisphosphonates(s), including both N-containing and non-N-containing bisphosphonates(s), selected from the group comprising: pamidronate, neridronate, olpadronate, alendronate, ibandronate, risedronate, and zoledronate. Non-containing bisphosphonates are for example etidronate, clodronate, and tiludronate; either in their free form, as prodrug or hydrogel-linked prodrug.

## Examples

### Materials and Methods

#### Materials:

Amino 4-arm PEG5000 was obtained from JenKem Technology, Beijing, P. R. China. Cithrol™ DPHS was obtained from Croda International Pic, Cowick Hall, United Kingdom. *cis*-1,4-cyclohexanedicaboxylic acid was obtained from TCI Europe, Zwijndrecht, Belgium. Isopropylmalonic acid was obtained from ABCR GmbH & Co. KG, Karlsruhe, Germany.

*N*-(3-maleimidopropyl)-22-amino-4,7,10,13,16,19-hexaoxa-heneicosanoic acid pentafluorophenyl ester (Mal-PEG6-PFP) was obtained from Biomatrik Inc., Jiaxing, P. R. China.

Oxyma pure and Fmoc-*L*-Asp(OtBu)-OH were purchased from Merck Biosciences GmbH, Schwalbach/Ts, Germany.

(5-methyl-2-oxo-1,3-dioxol-4-yl)-methyl 4-nitrophenyl carbonate was purchased from Chemzon Scientific Inc., Lachine, QC, Canada.

NHS activated carboxy PEG 20kDa (Sunbright 200 HS) was purchased from NOF Europe, Grobbendonk, Belgium.

IL-1ra (Anakinra, Kineret(R), Swedish Orphan Biovitrum AB) ready to use syringes were obtained from a local pharmacy.

Modmoc-Chloride was ordered from Chemzone, Petaling Jaya, Malaysia.

PBS-Tween buffer tablets were obtained from VWR, Bruchsal, Germany.

All other chemicals were from Sigma-ALDRICH Chemie GmbH, Taufkirchen, Germany.

PBSTE buffer was prepared by dissolving a PBS-Tween buffer tablet and 5 mmol EDTA disodium salt in 950 mL water, adjusting the pH to pH 7.40 and filling up with water to 1000 mL. Buffer was filtered sterile through 0.22 μm Nalgene bottle top filter.

Citrate buffer pH 6.5 was prepared by dissolving 7.5 mmol trisodium citrate, 140 mmol NaCl, 0.5 mmol EDTA disodium salt and 1.0 g Polysorbate 80 in 950 mL water, adjusting the pH to pH 6.50 by addition of 1 N HCl and filling up with water to 1000 mL. Buffer was filtered sterile through 0.22 μm Nalgene bottle top filter.

#### Methods:

RP-HPLC was done on a 30 x 150 mm C18 BEH 300 10μm column (Waters) connected to a Waters 600 HPLC System and Waters 2487 Absorbance detector. Linear gradients of solution A (0.1% TFA in $H_2O$) and solution B (0.1% TFA in acetonitrile) were used. HPLC fractions containing product were combined and lyophilized.

Flash chromatography purifications were performed on an Isolera One system from Biotage AB, Sweden, using Biotage KP-Sil silica cartridges and n-heptane, ethyl acetate, and methanol as eluents. Products were detected at 254 nm. For products showing no absorbance above 240 nm fractions were screened by LC/MS.

Analytical ultra-performance LC(UPLC)-ESI-MS was performed on a Waters Acquity system equipped with a Waters BEH300 C18 column (2.1 x 50 mm, 1.7 μm particle size, flow: 0.25 mL/min; solvent A: UP-$H_2O$ + 0.04% TFA, solvent B: UP-Acetonitrile + 0.05% TFA) coupled to a LTQ Orbitrap Discovery mass spectrometer from Thermo Scientific or to a a ZQ 4000 ESI instrument from Waters (positive mode).

MS spectra of PEG products showed a series of $(CH_2CH_2O)_n$ moieties due to polydispersity of PEG staring materials. For easier interpretation only one single representative m/z signal is given in the examples.

Gel filtration (Buffer Exchange) was performed on a GE Healthcare ÄKTA Explorer system using GE Healthcare HiPrep 26/10 Sephadex G-25 column. The flow rate was 5-7 ml/min.

SEC-HPLC was performed on an Agilent 1260 system using a TSK-Gel G2000SWXL column from Tosoh Bioscience. Mobile Phase Buffer: 1.059 mM $KH_2SO_4$, 2.966 mM $Na_2HPO_4$ and 300 mM NaCl dissolved in water to 980 ml, adjusted to pH 7,40 and filled up to 1000 ml followed by addition of 100 ml absolute Ethanol. Flow rate: 0.5 ml/min, runtime 35 min, detector wavelength: 220 nm, reference wavelength: 360 nm, calibrated with IL-1ra standard solutions before each measurement.

IL-1ra concentration in solution was determined photometrically at 280 nm by using an extinction coefficient of 14077 $M^{-1}cm^{-1}$ for IL1RA and 14202 $M^{-1}cm^{-1}$ for oxidized IL1RA (internal disulfide)

Quantitative Amino Acid Analysis (QAAA)

An aliquot of hydrogel suspension in aqueous buffer is weighed into a 10 mL pressure tube. Internal standard solution containing aminobutyric acid and d8-valine is added and the solvents are evaporated. In addition to these samples, several standards are prepared from amino acid stock solutions (mixture of valine, leucine, isoleucine and phenylalanine) and the internal standard. To each tube a hydrolysis mixture (600 μL of 6 M HCl/TFA 2:1) and a stirring bar are added.

The samples are hydrolyzed for 30 min at 190 °C in the microwave. The hydrolysis solution is transferred to a 5 mL volumetric flask. The glass vial is rinsed with cooled 100 mM citrate buffer (pH = 3.0) and the solution is added to the volumetric flask. The solution in the volumetric flask is neutralized with cooled 4 M NaOH and the volumetric flask is filled up to mark with 100 mM citrate buffer (pH = 3.0).

Aliquots from the volumetric flasks are diluted 1:5 with a 1:1 mixture of 100 mM citrate buffer (pH = 3.0) and 50 mM HFBA in water. After vortexing and centrifugation the supernatant is analyzed by LC-MS/MS.

LC-MS/MS is performed on an Agilent Technologies 1290 Infinity LC combined with an Agilent Technologies 6460 Triple Quad using a Waters Accq-Tag Ultra C18, 2.1 x 100 mm, 1.7 μm column (0.36 mL/min, 45 °C). Eluent A: 0.2 % aqueous HFBA Eluent B: 0.2 % HFBA in methanol. A linear 15 min gradient 0.1 - 38 % eluent B is used.

The amount of protein per sample is calculated by the averaged values obtained for valine, leucine, isoleucine and phenylalanine content.

**Example 1**

**Synthesis of backbone reagent Ia and Ig:**

$$\left[\text{PEG1250} — \text{DLys-DLys}_2\text{-DLys}_4(\text{NH}_2)_8\right]_4$$

**1a**

**=**

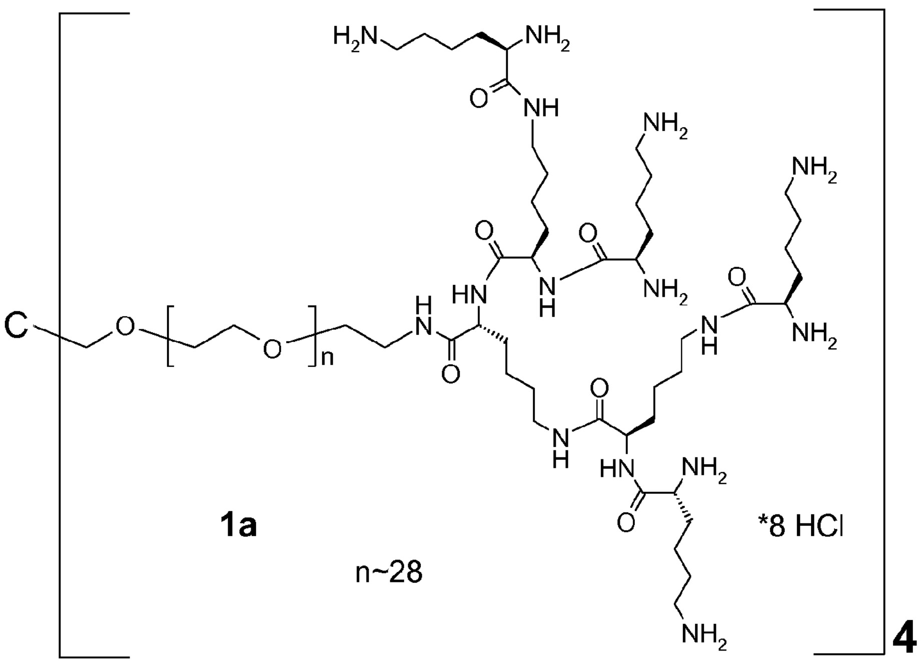

**1a**

n~28

Backbone reagent **1**a was synthesized as described in example 1 of WO 2011/012715 A1 except for the use of Boc-DLys(Boc)-OH instead of Boc-LLys(Boc)-OH.
MS: m/z 888.50 = $[M+10H^+]^{10+}$ (calculated = 888.54)

$$\left[ PEG1250 — TAN\text{-}TAN_2\text{-}TAN_4(NH_2)_8 \right]_4$$

**1g**

**=**

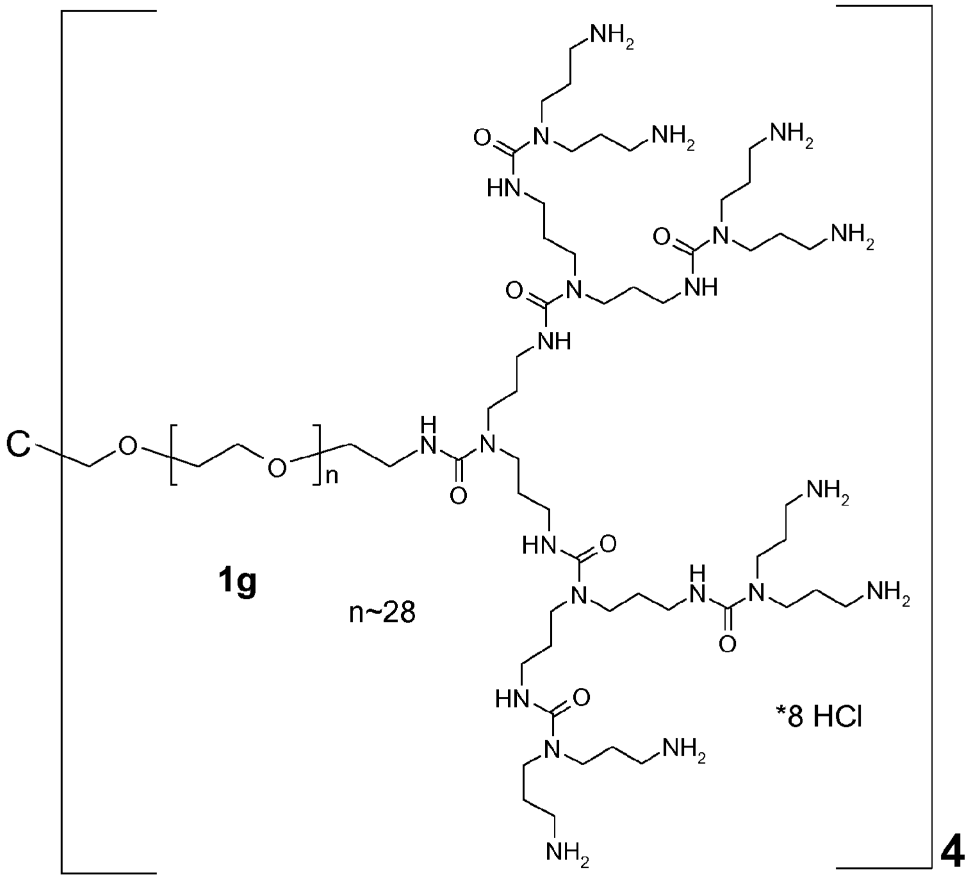

**1g**

n~28

*8 HCl

Backbone reagent **1g** was synthesized from amino 4-arm PEG5000 **1b** according to the following scheme:

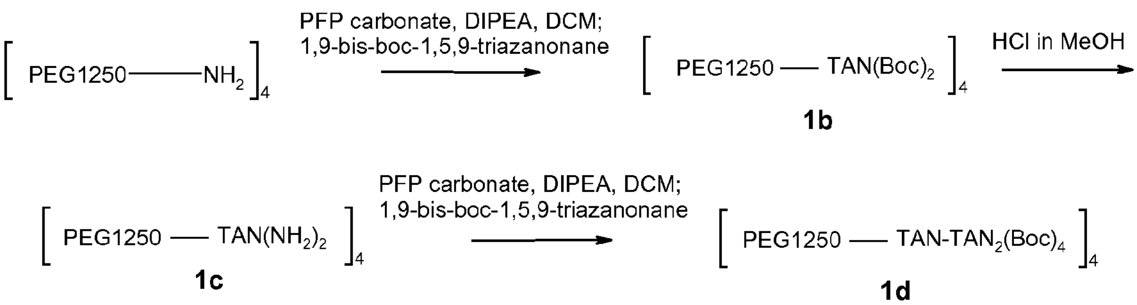


**1b**

**1c**

**1d**

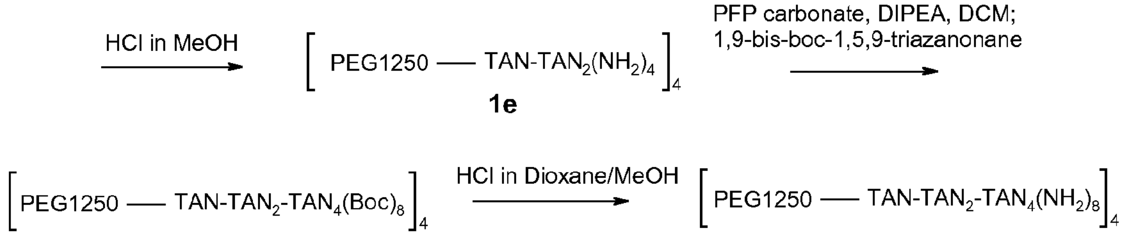


1e

1f

1g

For synthesis of compound **1b**, amino 4-arm PEG5000 (MW ca. 5350 g/mol, 10.7 g, 2.00 mmol, HCl salt) and bis(pentafluorophenyl)carbonate (4.73 g, 12.0 mmol) were dissolved in 43 mL of DCM (anhydrous) and DIPEA (3.10 g, 24.0 mmol, 4.18 mL) was added at room temperature. After 10 min, 1,9-bis-boc-1,5,9-triazanonane (5.30 g, 16.0 mmol) was added and the mixture was stirred for 15 min. Then additional 1,9-bis-boc-1,5,9-triazanonane (0.33 g, 1.0 mmol) was added. After complete dissolution, the reaction mixture was filtered and the solvent was evaporated at room temperature.

The residue was dissolved in 40 mL iPrOH and diluted with 320 mL MTBE. The product was precipitated over night at -20 °C. The precipitate was collected by filtration through a glass filter Por. 3, and washed with 200 mL of cooled MTBE (0 °C). The product was dried *in vacuo* over night.
Yield 11.1 g (83%) white solid **1b**.
MS: m/z 1112.86 = $[M+6H]^{6+}$ (calculated =1113.04).

For synthesis of compound **1c**, the boc-protected compound **1b** (11.1 g, 1.66 mmol) was dissolved in 40 mL of 3 M HCl in MeOH and stirred for 20 min at 45 °C, then for 10 min at 55 °C. For precipitation, 10 mL MeOH and 200 mL of MTBE were added and the mixture was stored for 16 h at -20 °C. The precipitate was collected by filtration through a glass filter Por. 3 and washed with 200 mL of cooled MTBE (0 °C). The product was dried *in vacuo* over night.
Yield 9.14 g (89%) white powder **1c** (HCl salt).
MS: m/z 979.45 = $[M+6H]^{6+}$ (calculated = 979.55).

For synthesis of compound **1d**, compound **1c** (9.06 g, 1.47 mmol, HCl salt) and bis(pentafluorophenyl)carbonate (6.95 g, 17.6 mmol) were dissolved in 50 mL of DCM (anhydrous) and DIPEA (4.56 g, 35.3 mmol, 6.15 mL) was added at room temperature. After 10 min, 1,9-bis-boc-1,5,9-triazanonane (7.80 g, 23.5 mmol) was added and the mixture was stirred for 15 min. Then additional 1,9-bis-boc-1,5,9-triazanonane (0.49 g, 1.5 mmol) was added. After complete dissolution, the solvent was evaporated at room temperature.

The residue was dissolved in 35 mL iPrOH at 40 °C and diluted with 200 mL MTBE. The product was precipitated over night at -20 °C. The precipitate was collected by filtration through a glass filter Por. 3, and washed with 200 mL of cooled MTBE (0 °C). The product was dried *in vacuo* over night to give **1d** as a white solid.
Yield 11.6 g (90%) white solid **1d**.
MS: m/z 1248.08 = $[M+7H]^{7+}$ (calculated = 1248.27).

For synthesis of compound **1e**, the boc-protected compound **1d** (11.4 g, 1.31 mmol) was dissolved in 40 mL of 3 M HCl in MeOH and stirred for 20 min at 45 °C, then for 10 min at 55 °C. For precipitation, 10 mL MeOH and 200 mL of MTBE were added and the mixture was stored for 16 h at -20 °C. The precipitate was collected by filtration through a glass filter Por. 3 and washed with 200 mL of cooled MTBE (0 °C). The product was dried *in vacuo* over night to give white powder **1e**.
Yield 7.60 g (75%) white powder **1e** (HCl salt).
MS: m/z 891.96 = $[M+8H]^{8+}$ (calculated = 892.13).

For synthesis of compound **1f**, compound **1e** (7.56 g, 0.980 mmol, HCl salt) and bis(pentafluorophenyl)carbonate (9.27 g, 23.0 mmol) were dissolved in 250 mL of DCM (anhydrous) and DIPEA (6.08 g, 47.0 mmol, 8.19 mL) was added at 35 °C. After 10 min, 1,9-bis-boc-1,5,9-triazanonane (5.30 g, 16.0 mmol) was added and the mixture was stirred for 15 min. Then additional 1,9-bis-boc-1,5,9-triazanonane (0.33 g, 1.0 mmol) was added. After complete disssolution, the solvent was evaporated at room temperature.

The residue was dissolved in 250 mL iPrOH at 60 °C and diluted with 1350 mL MTBE. The product was precipitated over night at -20 °C. The precipitate was collected by filtration through a glass filter Por. 3, and washed with 400 mL of cooled MTBE (0 °C). The product was dried *in vacuo* over night to give **1f** as a glassy solid.
Yield 11.1 g (83%) glassy solid **1f**.
MS: m/z 1312.01 =$[M+10H]^{10+}$ (calculated = 1312.21).

For synthesis of backbone reagent **1g**, the boc-protected compound **1f** (7.84 g, 0.610 mmol) was dissolved in 16 mL of MeOH at 37 °C and 55 mL of a precooled solution of 4 M HCl (4 °C) in dioxane was added at room temperature. The mixture was stirred without cooling for 20 min. After 20 min 110 mL of 3M HCl in MeOH was added. The solution was partitioned in 24 Falcon tubes (50 mL) and precipitated with by adding 40 mL cold MTBE (-20°C) to each Falcon

tube. After centrifugation at 3214 rcf for 1 min, the supernatant was decanted and the glassy solid was dissolved in 5 mL MeOH per Falcon tube and precipitated by adding 40 mL cold MTBE (-20°C) to each Falcon tube again. The supernatant was discarded and the remaining solid was dried *in vacuo* over night.
Yield 5.74 g (87%) white glassy solid **1g** (HCl salt).
MS: m/z 965.46 = $[M+10H]^{10+}$ (calculated = 965.45).

**Example 2**

**Synthesis of crosslinker reagents 2d, 2g, 2k, and 2o**

Crosslinker reagent **2e** was prepared from azelaic acid monobenzyl ester and PEG10000 according to the following scheme:

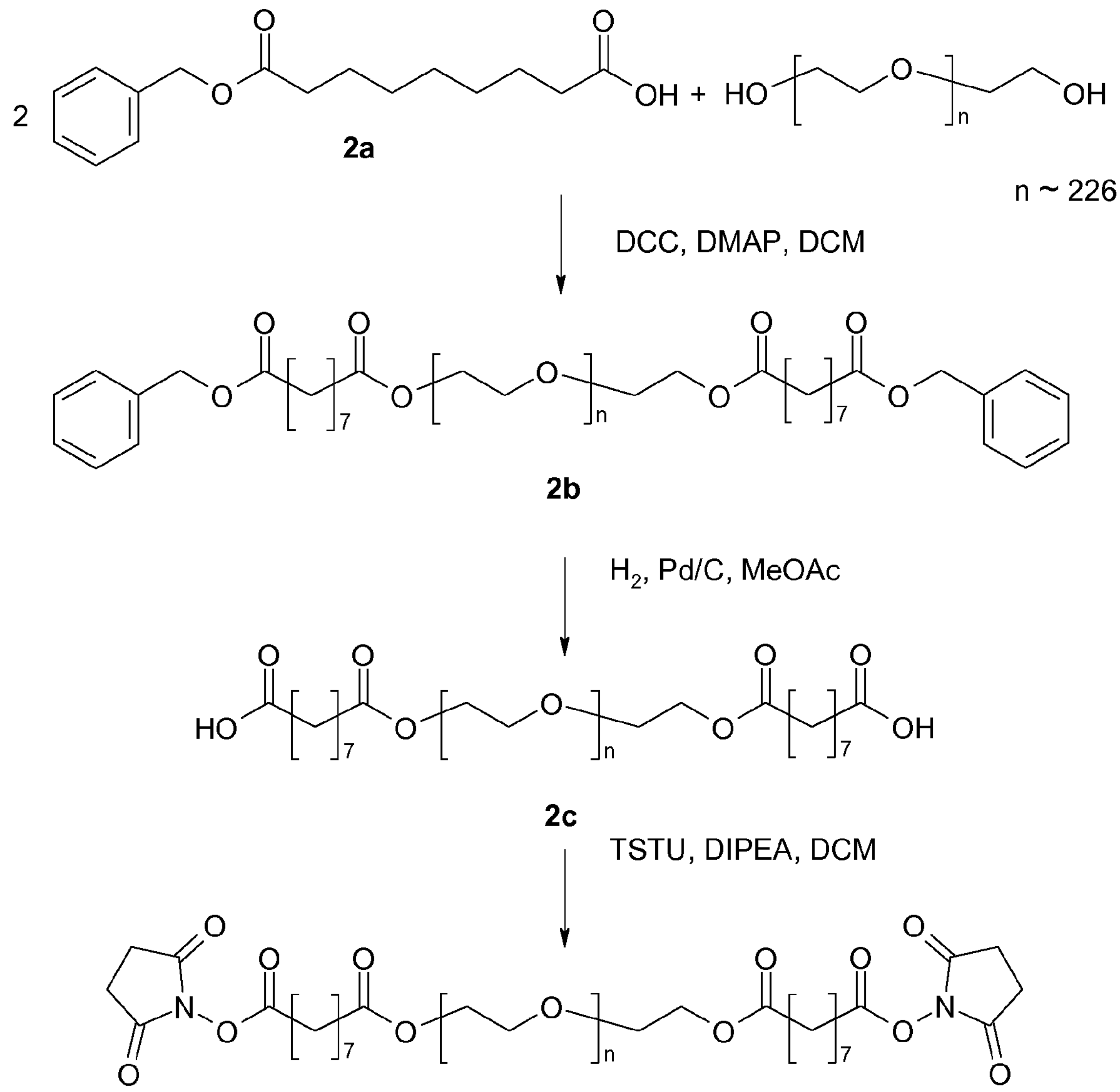


**2a**

**2b**

**2c**

**2d**

For the synthesis of azelaic acid monobenzyl ester **2a**, a mixture of azelaic acid (37.6 g, 200 mmol), benzyl alcohol (21.6 g, 200 mmol), *p*-toluenesulfonic acid (0.80 g, 4.2 mmol), and 240 mL toluene was refluxed for 7 h in a Dean-Stark apparatus. After cooling down, the solvent was evaporated and 300 mL sat. aqueous $NaHCO_3$ solution were added. This mixture was extracted with 3 × 200 mL MTBE. The combined organic phases were dried over $Na_2SO_4$ and the solvent was evaporated. The product was purified on 2 × 340 g silica using ethyl acetate / heptane (10:90 → 25:75) as eluent. The eluent was evaporated and the residue was dried *in vacuo* over night.
Yield 25.8 g (46%) colorless oil **2a.**

MS: m/z 279.16 = $[M+H]^+$ (calculated = 279.16).

For synthesis of compound **2b**, azelaic acid monobenzyl ester **2a** (3.90 g, 14.0 mmol) and PEG 10000 (40.0 g, 4.00 mmol) were dissolved in 64 mL dichloromethane and cooled with an ice bath. A solution of DCC (2.89 g, 14.0 mmol) and DMAP (0.024 g, 0.020 mmol) in 32 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 65 mL dichloromethane and diluted with 308 mL MTBE at room temperature. The mixture was stored over night at -20 °C. The precipitate was collected by filtration through a glass filter Por. 3, and washed with 250 mL of cooled MTBE (-20 °C). The product was dried *in vacuo* over night.

Yield 40.8 g (97%) white powder **2b**.

MS: m/z 835.50 = $[M+14H]^{14+}$ (calculated = 835.56).

For synthesis of compound **2c**, compound **2b** (40.6 g, 3.86 mmol) was dissolved in methyl acetate (250 mL) and 203 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 37.2 g (93%) glassy solid **2c**.

MS: m/z 882.53 = $[M+13H]^{13+}$ (calculated = 882.51).

For synthesis of compound **2d**, compound **2c** (32.0 g, 3.10 mmol) and TSTU (3.73 g, 12.4 mmol) were dissolved in 150 mL dichloromethane at room temperature. Then DIPEA (1.60 g, 12.4 mmol) was added and the mixture was stirred for 1 h. The resulting suspension was filtered and the filtrate was diluted with 170 mL dichloromethane, washed with 140 mL of a solution of 750 g water / 197 g NaCl / 3 g NaOH. The organic phase was dried over $MgSO_4$ and the solvent was evaporated *in vacuo.*

The residue was dissolved in 200 mL toluene, diluted with 180 mL MTBE at room temperature and stored over night at -20 °C. The precipitate was collected by filtration through a glass filter Por. 3, and washed with 100 mL of cooled MTBE (-20 °C). The product was dried *in vacuo* over night.

Yield 28.8 g (88%) white powder **2d**.

MS: m/z 795.47 = $[M+15H]^{15+}$ (calculated = 795.54).

Crosslinker reagent **2g** was prepared from azelaic acid monobenzyl ester and PEG6000 according to the following scheme:

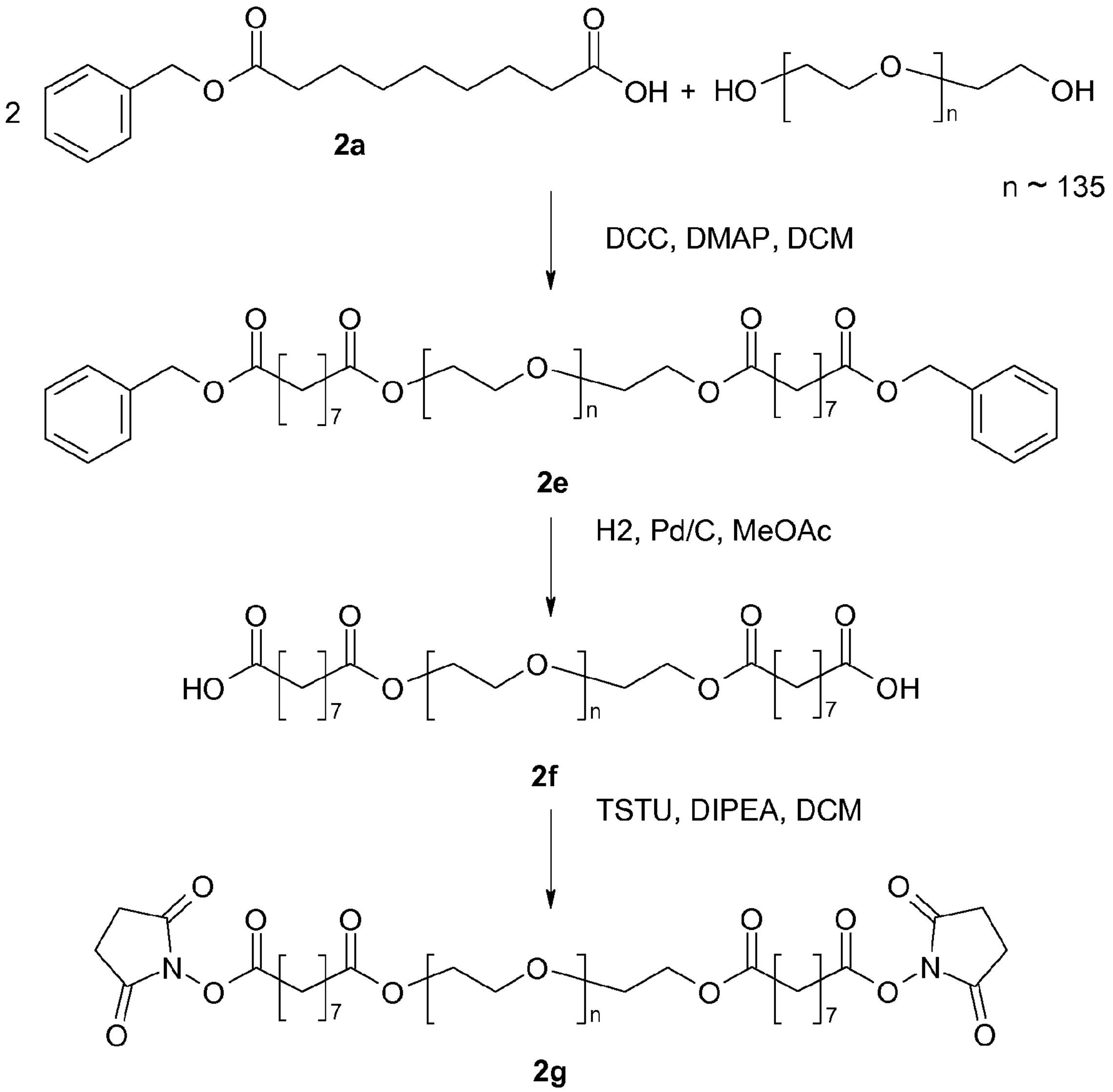


2a

2e

2f

2g

For synthesis of compound **2e**, azelaic acid monobenzyl ester **2a** (6.50 g, 23.3 mmol) and PEG 6000 (40.0 g, 6.67 mmol) were dissolved in 140 mL dichloromethane and cooled with an ice bath. A solution of DCC (4.81 g, 23.3 mmol) and DMAP (0.040 g, 0.33 mmol) in 40 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 70 mL dichloromethane and diluted with 300 mL MTBE at room temperature. The mixture was stored over night at -20 °C. The precipitate was collected by filtration through a glass filter Por. 3, and washed with 500 mL of cooled MTBE (-20 °C). The product was dried *in vacuo* over night.

Yield 41.2 g (95%) white powder **2e**.

MS: m/z 833.75 = $[M+8H]^{8+}$ (calculated = 833.74).

For synthesis of compound **2f**, compound **2e** (41.2 g, 6.32 mmol) was dissolved in methyl acetate (238 mL) and ethanol (40 mL), then 400 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 38.4 g (96%) glassy solid **2f**.

MS: m/z 750.46 = $[M+9H]^{9+}$ (calculated = 750.56).

For synthesis of compound **2g**, compound **2f** (38.2 g, 6.02 mmol) and TSTU (7.25 g, mmol) were dissolved in 130 mL dichloromethane at room temperature. Then DIPEA (3.11 g, 24.1 mmol) was added and the mixture was stirred for 1 h. The resulting suspension was filtered, the filtrate was diluted with 100 mL dichloromethane and washed with 200 mL of a solution of 750 g water / 197 g NaCl / 3 g NaOH. The organic phase was dried over $MgSO_4$ and the solvent was evaporated *in vacuo.*

The residue was dissolved in 210 mL toluene, diluted with 430 mL MTBE at room temperature and stored over night at -20 °C. The precipitate was collected by filtration through a glass filter Por. 3, and washed with 450 mL of cooled MTBE (-20 °C). The product was dried *in vacuo* over night.

Yield 35.8 g (91%) white powder **2g**.

MS: m/z 857.51 = $[M+8H]^{8+}$ (calculated = 857.51).

Crosslinker reagent **2k** was prepared from isopropylmalonic acid monobenzyl ester and PEG3300 according to the following scheme:

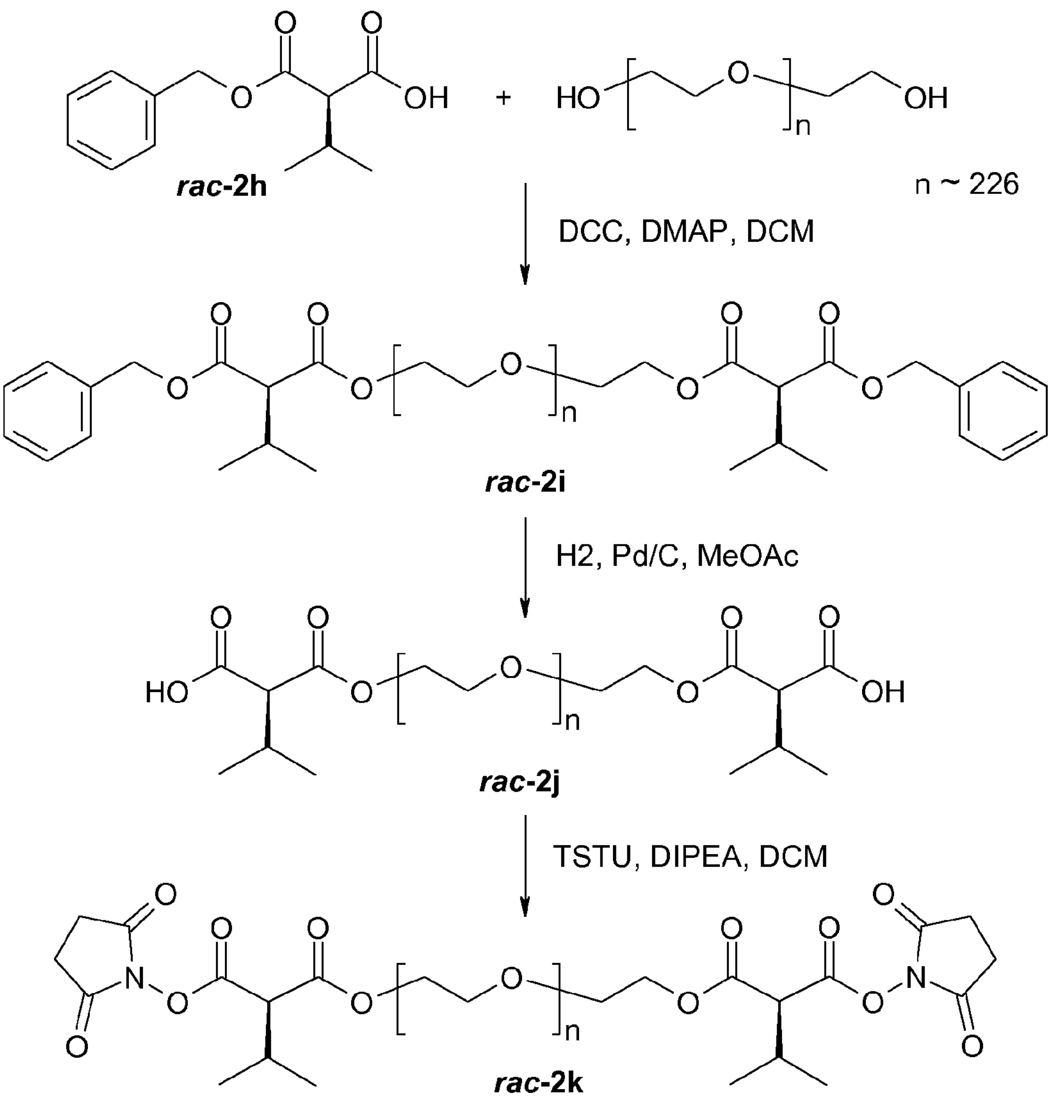


***rac*-2h**, ***rac*-2i**, ***rac*-2j**, ***rac*-2k**

For the synthesis of isopropylmalonic acid monobenzyl ester ***rac*-2h,** isopropylmalonic acid (35.0 g, 239 mmol), benzyl alcohol (23.3 g, 216 mmol) and DMAP (1.46 g, 12.0 mmol) were dissolved in 100 mL acetonitrile. Mixture was cooled to 0 °C with an ice bath. A solution of DCC (49.4 g, 239 mmol) in 150 mL acetonitrile was added within 15 min at 0 °C. The ice bath was removed and the reaction mixture was stirred over night at room temperature, then the solid was filtered off. The filtrate was evaporated at 40 °C *in vacuo* and the residue was dissolved in 300 mL MTBE. This solution was extracted with 2 × 300 mL sat. aqueous $NaHCO_3$ solution, then the combined aqueous phases were acidified to pH = 1-3 using 6 N hydrochloric acid. The resulting emulsion was extracted with 2 × 300 mL MTBE and the solvent was evaporated. The combined organic phases were washed with 200 mL sat. aqueous NaCl and dried over $MgSO_4$. The product was purified on 340 g silica using ethyl acetate / heptane (10:90 → 20:80) as eluent. The eluent was evaporated and the residue was dried *in vacuo* over night.

Yield 9.62 g (17%) colorless oil ***rac*-2h.**

MS: m/z 237.11 = $[M+H]^+$ (calculated = 237.11).

For synthesis of compound ***rac*-2i,** isopropylmalonic acid monobenzyl ester ***rac*-2h** (5.73 g, 24.24 mmol) and PEG3300 (20.0 g, 6.06 mmol) were dissolved in 110 mL dichloromethane and cooled with an ice bath. A solution of

DCC (5.00 g, 24.24 mmol) and DMAP (37 mg, 0.30 mmol) in 20 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 70 mL dichloromethane and diluted with 800 mL MTBE at room temperature. The mixture was stored over night at -20 °C. The precipitate was collected by filtration through a glass filter Por. 3, and washed with 650 mL of cooled MTBE (-20 °C). The product was dried *in vacuo* over night. Precipitation procedure was repeated. The product was dried *in vacuo* over night.
Yield 20.49 g (90%) white powder ***rac*-2i.**
MS: m/z 671.39 =$[M+6H]^{6+}$ (calculated = 671.47).

For synthesis of compound ***rac*-2j**, compound ***rac*-2i** (20.38 g, 5.42 mmol) was dissolved in methyl acetate (130 mL) and 242 mg of palladium on charcoal (10%) was added. Under a hydrogen atmosphere of ambient pressure, the mixture was stirred overnight at room temperature. The reaction mixture was filtered through a pad of celite and the filtrate was evaporated and dried *in vacuo* over night.
Yield 18.24 g (94%) glassy solid ***rac*-2j.**
MS: m/z 641.38 =$[M+6H]^{6+}$ (calculated = 641.43).

For synthesis of compound ***rac*-2k**, compound ***rac*-2j** (11.98 g, 3.35 mmol) and TSTU (4.03 g, 13.39 mmol) were dissolved in 145 mL dichloromethane at room temperature. Then DIPEA (1.73 g, 13.39 mmol) was added and the mixture was stirred for 45 min. The resulting suspension was filtered and the filtrate was washed with 175 mL of a 0.5 M phosphate buffer pH = 6.5. Organic phase was diluted with 350 mL ethyl acetate. The organic phase was dried over $MgSO_4$ and the solvent was evaporated *in vacuo.* The residue was dissolved in 100 mL toluene, diluted with 25 mL MTBE at room temperature and stored over night at -20 °C. The precipitate was collected by filtration through a glass filter Por. 3, and washed with 600 mL of cooled MTBE (-20 °C). The product was dried *in vacuo* over night.
Yield 8.50 g (67%) white powder ***rac*-2k.**
MS: m/z 673.72 =$[M+6H]^{6+}$ (calculated = 673.77).

Crosslinker reagent ***rac*-2o** was prepared from *cis*-1,4-cyclohexanedicarboxylic acid and PEG10000 according to the following scheme:

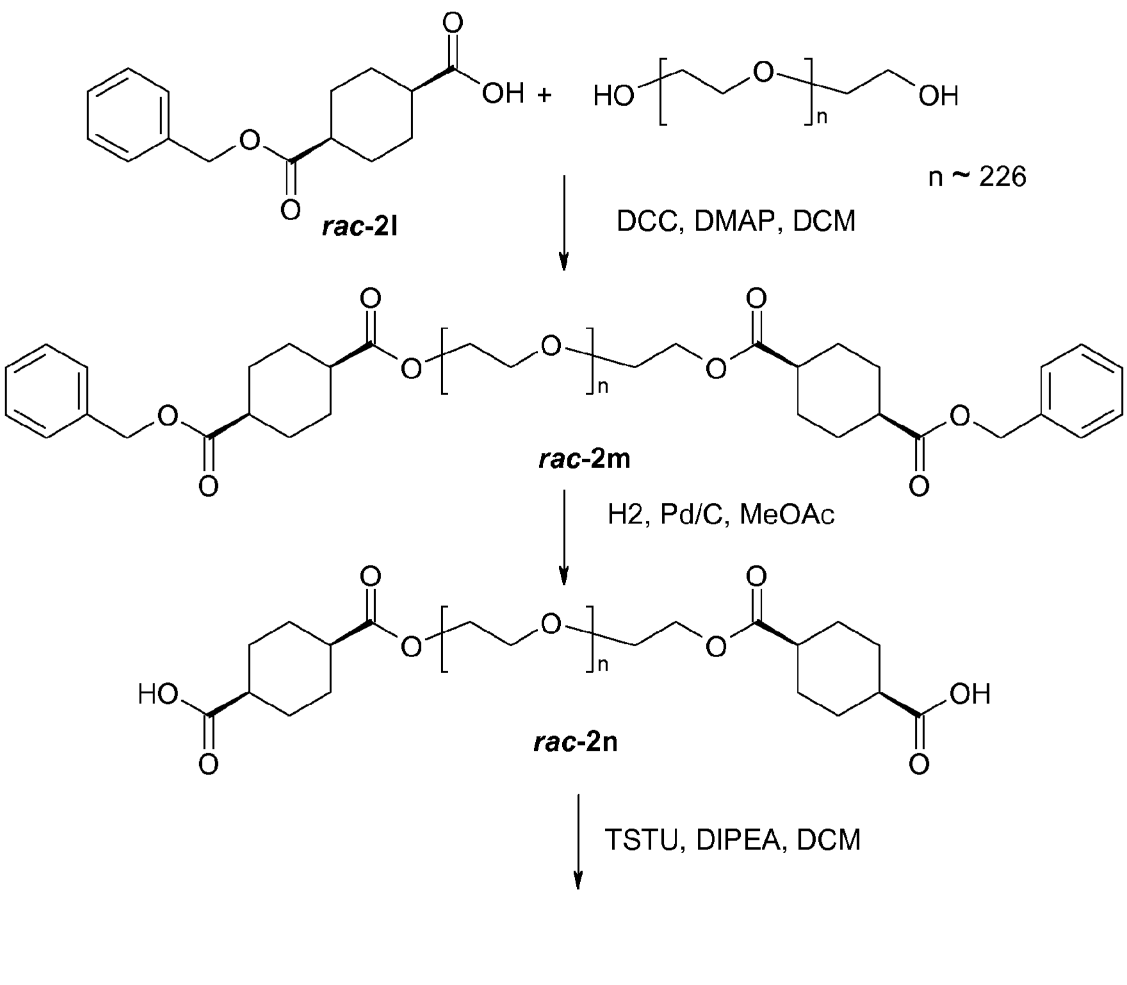

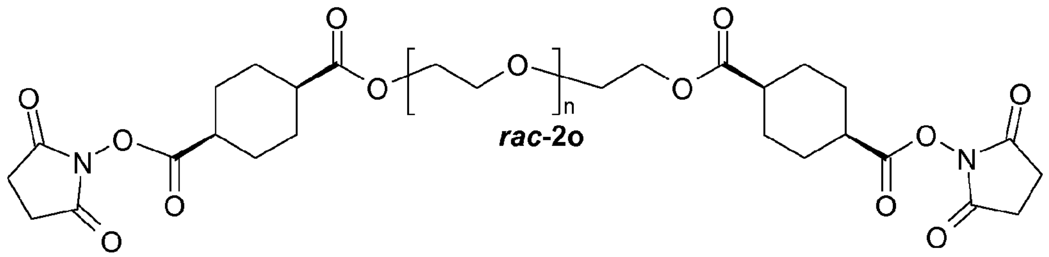

***rac*-2o**

For the synthesis of *cis*-1,4-cyclohexanedicarboxylic acid monobenzyl ester ***rac*-2l**, *cis-1,4*-cyclohexanedicarboxylic acid (20.0 g, 116 mmol), benzyl alcohol (11.3 g, 105 mmol) and DMAP (710 mg, 5.81 mmol) were dissolved in 200 mL THF. Mixture was cooled to 0 °C with an ice bath. A solution of DCC (49.4 g, 239 mmol) in 100 mL THF was added within 15 min at 0 °C. The ice bath was removed and the reaction mixture was stirred over night at room temperature, then the solid was filtered off. The filtrate was evaporated at 40 °C and the residue was dissolved in 300 mL MTBE. This solution was extracted with 2 × 300 mL sat. aqueous $NaHCO_3$ solution, then the combined aqueous phases were acidified to pH = 1-3 using 6 N hydrochloric acid. The resulting emulsion was extracted with 2 × 300 mL MTBE and the solvent was evaporated. The combined organic phases were washed with 200 mL sat. aqueous NaCl and dried over $MgSO_4$. The product was purified on 340 g silica using ethyl acetate / heptane (10:90 → 20:80) as eluent. The eluent was evaporated and the colorless oily residue crystallized during drying *in vacuo* over night.

Yield 4.82 g (16%) colorless crystals ***rac*-2l**.

MS: m/z 263.13 =$[M+H]^+$ (calculated = 263.13).

For synthesis of compound **2m**, *cis*-1,4-cyclohexanedicarboxylic acid monobenzyl ester *rac*-21 (2.10 g, 8.00 mmol) and PEG 10000 (20.0 g, 10.0 mmol) were dissolved in 50 mL dichloromethane and cooled with an ice bath. A solution of DCC (1.65 g, 8.00 mmol) and DMAP (0.012 g, 0.10 mmol) in 25 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 55 mL dichloromethane and diluted with 300 mL MTBE at room temperature. The mixture was stored over night at -20 °C. The precipitate was collected by filtration through a glass filter Por. 3, and washed with 250 mL of cooled MTBE (-20 °C). The product was dried *in vacuo* over night.

Yield 18.2 g (87%) white powder **2m**.

MS: m/z 745.76 =$[M+16H]^{16+}$ (calculated = 745.77).

For synthesis of compound **2n**, compound **2m** (9.00 g, 0.857 mmol) was dissolved in methyl acetate (100 mL) and 157 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 8.83g (100%) glassy solid **2n**.

MS: m/z 734.50 =$[M+16H]]^{6+}$ (calculated =734.50).

For synthesis of compound **2o**, compound **2n** (8.92 g, 0.864 mmol) and TSTU (1.04 g, 3.64 mmol) were dissolved in 35 mL dichloromethane at room temperature. Then DIPEA (0.447 g, 3.46 mmol) was added and the mixture was stirred for 45 min. The resulting suspension was filtered and the filtrate was washed with 2 × 10 mL of a 0.5 M phosphate buffer pH = 6.5. The organic phase was dried over $MgSO_4$ and the solvent was evaporated *in vacuo.*

The residue was dissolved in 50 mL toluene, diluted with 25 mL MTBE at room temperature and stored over night at -20 °C. The precipitate was collected by filtration through a glass filter Por. 3, and washed with 400 mL of cooled MTBE (-20 °C). The product was dried *in vacuo* over night.

Yield 7.62 g (84%) white powder **2o**.

MS: m/z 702.60 = $[M+16H]]^{6+}$ (calculated = 702.59).

### Example 3

**Preparation of hydrogel beads 3a, 3b, 3c, 3d and 3e containing free amino groups.**

In a cylindrical 250 mL reactor with bottom outlet, diameter 60 mm, equipped with baffles, an emulsion of 218 mg Cithrol™ DPHS in 100 mL undecane was stirred with an isojet stirrer, diameter 50 mm at 580 rpm, at ambient temperature. A solution of 250 mg **1a** and 2205 mg **2d** in 22.1 g DMSO was added and stirred for 10 min at RT to form a suspension. 1.1 mL TMEDA were added to effect polymerization. The mixture was stirred for 16 h. 1.7 mL of acetic acid were added and then after 10 min 100 mL of a 15wt% solution of sodium chloride in water was added. After 10 min, the stirrer was stopped and phases were allowed to separate. After 2 h the aqueous phase containing the hydrogel

was drained.

For bead size fractionation, the water-hydrogel suspension was diluted with 40 mL ethanol and wet-sieved on 125, 100, 75, 63, 50, 40, and 32 μm steel sieves using a Retsch AS200 control sieving machine for 15 min. Sieving amplitude was 1.5 mm, water flow 300 mL/min. Bead fractions that were retained on the 63 and 75 μm sieves were pooled and washed 3 times with 0.1% AcOH, 10 times with ethanol and dried for 16 h at 0.1 mbar to give 670 mg of **3a** as a white powder.

Amino group content of the hydrogel was determined to be 0.145 mmol/g by conjugation of a fmoc-amino acid to the free amino groups on the hydrogel and subsequent fmoc-determination.

**3b** was prepared as described for **3a** except for the use of 350 mg **1a,** 2548 mg **2g,** 26.1 g DMSO, 257 mg Cithrol™ DPHS, 1.5 mL TMEDA, and 2.4 mL acetic acid, yielding 550 mg **3b** as a white powder, free amino groups 0.120 mmol/g.

**3c** was prepared as described for **3a** except for the use of 1 L reactor with 100 mm diameter, 400 mL undecane, 1000 mg **1a**, 5698 mg ***rac*-2k**, 60.3 g DMSO, 595 mg Cithrol™ DPHS, 4.5 mL TMEDA, and 6.7 mL acetic acid, yielding 1,24 g (bead fraction on 100 μm sieve) **3c** as a white powder, free amino groups 0.068 mmol/g.

**3d** was prepared as described for **3a** except for the use of 250 mg **1a**, 2258 mg ***rac*-2o**, 22.6 g DMSO, 222 mg Cithrol™ DPHS, 1.1 mL ml TMEDA, and 1.7 mL acetic acid, yielding 186 mg **3d** as a white powder, free amino groups 0.153 mmol/g.

**3e** was prepared as described for **3a** except for the use of 740 mg **1a**, 3362 mg **rac-2k,** 36.9 g DMSO, 365 mg Cithrol™ DPHS, 3.3 mL ml TMEDA, and 5.1 mL acetic acid, yielding 950 mg (bead fraction on 75 μm sieve) 3e as a white powder, free amino groups 0.179 mmol/g.

**Example 4**

**Synthesis of linker reagent 4c**

Linker reagent **4c** was synthesized according to the following scheme:

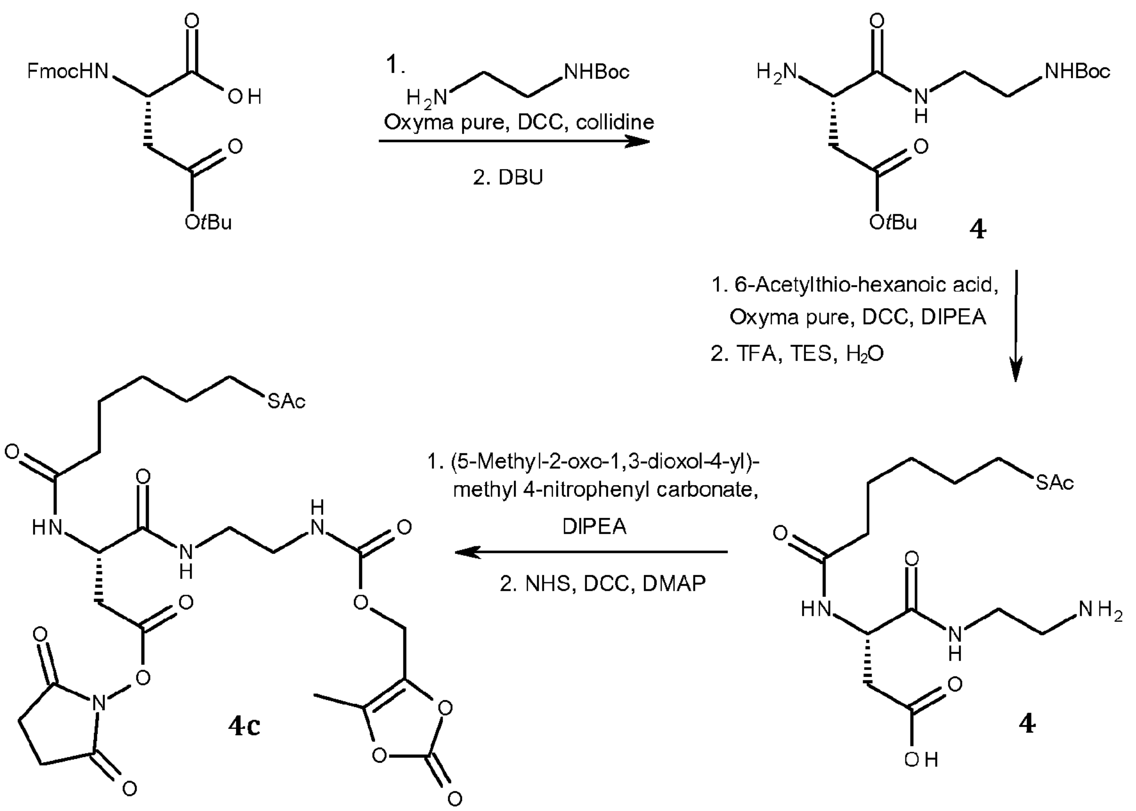


Synthesis of **4a**:

Fmoc-*L*-Asp(O*t*Bu)-OH (1.00 g, 2.43 mmol) was dissolved with DCC (0.70 g, 3.33 mmol) in DCM (25 mL). Oxyma pure (0.51 g, 3.58 mmol) and collidine (0.50 mL, 3.58 mmol) were added in one portion and a solution of *N*-Boc-ethylenediamine (0.41 g, 2.56 mmol) in DCM (15 mL) was added slowly. After stirring the mixture for 90 min at RT the formed precipitate was filtered off and the filtrate washed with aqueous HCl (0.1 M, 50 mL). The aqueous layer was extracted with DCM (2 × 20 mL) and the combined organic fractions were washed with sat. aqueous $NaHCO_3$ (3 × 25 mL) and brine (1 × 50 mL), dried over $Na_2SO_4$, filtered and concentrated *in vacuo.* The crude solid was purified by flash chromatography. The intermediate *N*-boc-*N'*-(*N*-fmoc-4-*tert*.-butyl-*L*-aspartoyl)-ethylenediamine was obtained as white solid (0.98 g, 1.77 mmol, 73%).
MS: m/z 554.29 = $[M+H]^+$, (calculated = 554.29).

*N*-boc-*N'*-(*N*-fmoc-4-*tert*.-butyl-*L*-aspartoyl)-ethylenediamine (0.98 g, 1.77 mmol) was dissolved in THF (15 mL), DBU (0.31 mL) was added and the solution was stirred for 12 min at RT. The reaction was quenched with AcOH (0.5 ml), concentrated *in vacuo* and the residue purified by flash chromatography to give **4a** (0.61 g, 1.77 mmol, 73 % over 2 steps) as white solid.
MS: m/z 332.38 = $[M+H]^+$, (calculated = 332.22).

Synthesis of **4b:**

6-Acetylthiohexanoic acid (0.37 g, 1.95 mmol) was dissolved in DCM (19.5 mL) and Oxyma pure (0.35 g, 2.48 mmol) and DCC (0.40 g, 1.95 mmol) added in one portion. The solution was stirred for 30 min at RT, filtered, and the filtrate added to a solution of **4a** (0.61 g, 1.77 mmol) in DCM (10.5 mL). DIPEA (0.46 mL, 2.66 mmol) was added to the solution and the reaction stirred for 2 h at RT. The solution was washed with aqueous $H_2SO_4$ (0.1 M, 2 × 30 mL), sat. aqueous $NaHCO_3$ (2 × 20 mL) and brine (1 × 20 mL). The organic layer was dried over $Na_2SO_4$, filtered and concentrated *in vacuo.* The crude material was purified by flash chromatography to give *N*-boc-*N'*-(*N*-6-acetylthiohexyl-4-*tert*.-butyl-*L*-aspartoyl)-ethylenediamine (0.65 g, 1.30 mmol, 73% over 2 steps) as white solid.
MS: m/z 504.27 = $[M+H]^+$, (calculated = 504.28).

*N*-boe-*N'*-(*N*-6-Acetylthiohexyl-4-*tert.*-butyl-*L*-aspartoyl)-ethylenediamine (0.60 g, 1.18 mmol) was dissolved in TFA (5 mL) and TES (0.13 mL) and water (0.13 ml) were added. The mixture was stirred for 30 min at RT. TFA was removed in a stream of $N_2$, and crude **4b** dissolved in H2O/ACN 1:1 and purified by RP-HPLC.
Yield: 0.39 g, 0.85 mmol (TFA salt), 72%.
MS: m/z 348.25 = $[M+H]^+$, (calculated = 348.16).

Synthesis of **4c:**

**4b** (TFA salt, 0.38 g, 0.80 mmol) was dissolved in DMF (5 mL) and (5-methyl-2-oxo-1,3-dioxol-4-yl)-methyl 4-nitrophenyl carbonate (0.26 g, 0.88 mmol) and DIPEA (0.28 mL, 1.6 mmol) were added. The resulting suspension was diluted with DCM (5 mL) and stirred for 3 h at RT. More DIPEA (0.28 mL 1.6 mmol) was added and stirring continued for 2 h. DCM was concentrated *in vacuo,* the residue diluted with H2O/ACN 3:1 and purified by RP-HPLC to give N-(5-methyl-2-oxo-1,3-dioxol-4-yl)-methyl-oxocarbonyl-*N'*-(*N*-6-acetylthiohexyl-*L*-aspartyl)-ethylenediamine (0.31 g, 0.62 mmol, 77%) as colorless oil.
MS: m/z 504.16 = $[M+H]^+$, (calculated = 504.17).

*N*-(5-methyl-2-oxo-1,3-dioxo1-4-yl)-methyl oxocarbonyl-*N'*-(*N*-6-acetylthiohexyl-*L*-aspartyl)-ethylene-diamine (150 mg, 0.30 mmol) was dissolved in DCM (17.5 mL) and NHS (41 mg, 0.36 mmol), DCC (74 mg, 0.36 mmol) and DMAP (4 mg, 0.03 mmol) were added in one portion. The reaction was stirred for 1 h at RT and the resulting suspension filtered. The precipitate was washed with a small amount of DCM and the combined filtrates concentrated *in vacuo.* **4c** was purified by RP-HPLC to give a colorless oil (144 mg, 0.24 mmol, 80%).
MS: m/z 601.18 = $[M+H]^+$, (calculated = 601.18).

**Example 5**

**Preparation of gamma sterilized hydrogel beads 5**

A suspension of 523 mg hydrogel beads **3c** in 10 mL 1% n-propylamine in NMP was gamma irradiated ($^{60}Co$) with a dose of 30 kGy at room temperature.

**Example 6**

**Preparation of PEGylated hydrogel beads 6a and 6b**

A suspension of 523 mg of hydrogel beads **5** in 1% n-propylamine in NMP was washed five times with NMP and five times with DMSO. 189 mg NHS activated carboxy PEG 20kDa was dissolved in 3 mL DMSO (37 °C) and added

to the hydrogel beads. 52 µl TMEDA in 1.5 mL DMSO was added and the mixture was shaken for 48 h at room temperature. Resulting PEGylated hydrogel beads **6a** were washed five times each with DMSO and NMP and used in the next reaction without further treatment.

**6b** was prepared as described for **6a** except for the use of 499 mg hydrogel beads **3e**, 390 mg NHS activated carboxy PEG 20 kDa and 134 µl of TMEDA.

**Example 7**

**Preparation of maleimide functionalized hydrogel beads 7a and 7b**

Hydrogel beads **6a** in NMP were washed two times with 2% DIPEA in NMP. 340 mg of Mal-PEG6-PFP was dissolved in 2 mL NMP and added to the washed hydrogel beads **6a**. The hydrogel suspension was incubated for 2 h at room temperature. Resulting maleimide functionalized hydrogel beads **7a** were washed five times each with NMP, water, water/ethanol p.a. 1/1 and ethanol p.a. Hydrogel beads **7a** were dried at 0.1 mbar to constant weight. Maleimide content was determined by quantification of mercaptoethanol consumption (inverse Ellman test) as described in example 4 of WO2011/012718A. A maleimide content of 0.048 mmol/g was obtained.

**7b** was prepared as described for **7a** except for the use of hydrogel beads **6b** and 450 mg of Mal-PEG6-PFP. A maleimide content of 0.104 mmol/g was obtained.

**Example 8**

**Synthesis of deprotected IL-lra-linker 8c**

Deprotected IL-1ra-linker **8c** was synthesized according to the following scheme:

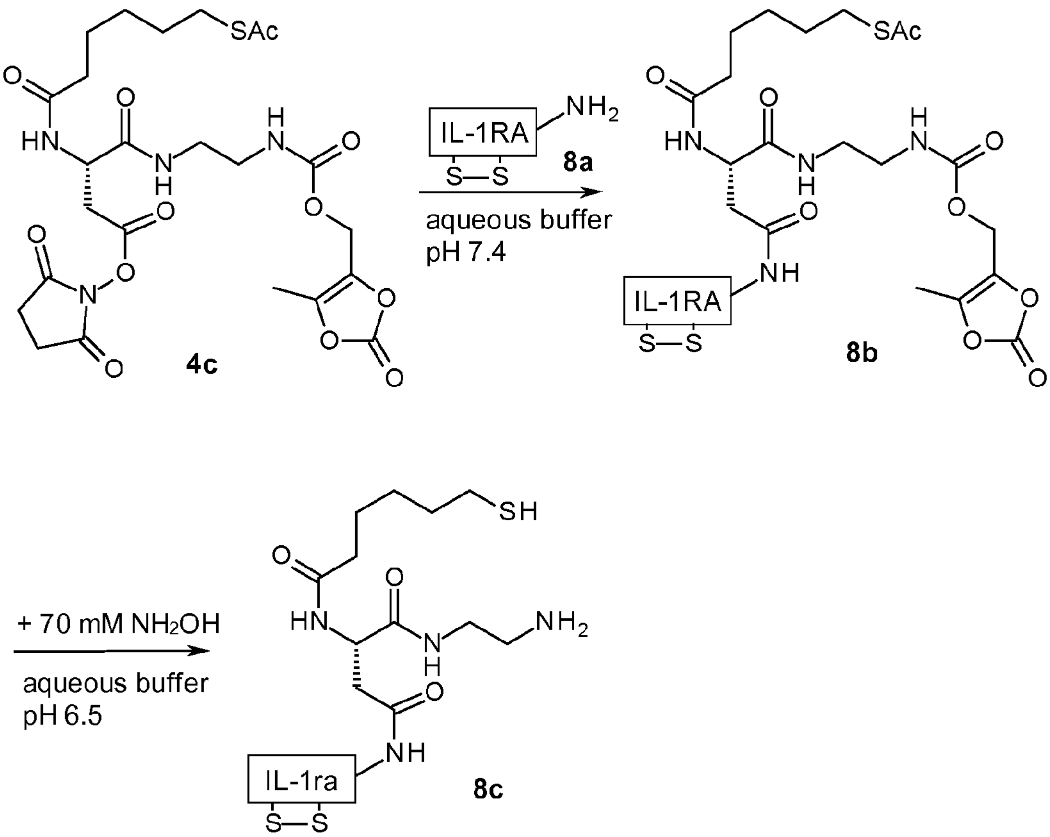


**4c** **8b** **8c**

**Synthesis of oxidized IL-1ra 8a**

1000 mg **IL-1ra** (Kineret®, 10 ready to use syringes, 7450 µL solution) was diluted with 30 mL PBSTE buffer. A solution of 57.4 mg 5,5'-Dithiobis(2-nitrobenzoic acid) in 3.54 mL of 0.5 M phosphate buffer pH 7.4 was added. Mixture was incubated for 1 h at room temperature and oxidized **IL-1ra 8a** (formation of internal disulfide bridge) was buffer

exchanged to PBSTE buffer.
MS: m/z 1726.54 = $[M+10H]^{10+}$, (calculated = 1726.56).

Complete **IL-1ra** oxidation (formation of internal disulfide bridge) can be confirmed in the maleimide reactivity test. Oxidized **IL-1ra 8a** lacks maleimide reactivity due to blocking of a reactive cysteine, while IL-1ra shows complete conversion with a maleimide reagent in a 1/1 ratio.

Maleimide reactivity test: 2 μl IL-1ra solution (23.9 mg/mL) was diluted with 20 μl PBSTE and reacted for 10 min with 1.2 μl of 7.5 mg N-Maleoyl-beta-alanine/mL 0.5 M phosphate buffer pH 7.4). As determined by LCMS, **IL-1ra** showed complete conversion with the maleimide in a 1/1 ratio, while oxidized **IL-1ra 8a** lacked reactivity.
MS (IL-1ra + maleimide reagent): m/z 1743.61 = $[M+10H]^{10}$, (calculated = 1743.67).
MS (oxidized IL-1ra **8a):** m/z 1726.54 = $[M+10H]^{10+}$, (calculated = 1726.56).

**Synthesis of deprotected IL-lra-linker 8c**

6 mg of linker reagent **4c** was dissolved in 100 μL DMSO to yield a concentration of 100 mM. 115 μl (0.5 molar equivalent of linker reagent **4c** relative to the amount of IL-1ra) was added to a solution of **IL-1ra 8a** in PBSTE buffer (17.98 mg/mL, 22.3 mL). The reaction mixture was mixed carefully and incubated for 5 min at room temperature. Subsequently, 2 additional 0.5 molar equivalents of linker reagent **4c** were added and after addition of each equivalent the reaction mixture was incubated for 5 min at room temperature yielding a mixture of IL-1ra **8a** and the protected IL-1ra-linker monoconjugate **8b.** The ratio of IL-1ra **8a** and the protected IL-1ra-linker monoconjugate **8b** is approx. 2/1 as determined by MS $[M+10H]^{10+}$. Buffer was exchanged to pH 6.5 citrate buffer. A final volume of 22 mL was obtained.
MS (protected IL-1ra-linker **8b**): m/z 1775.05 = $[M+10H]^{10+}$, (calculated = 1775.07).

To remove the protecting groups from **8b**, 0.5 M $NH_2OH$ in pH 6.5 citrate buffer ($NH_2OH$ Hydrochloride dissolved in pH 6.5 citrate buffer, adjusted to pH 6.50 by adding 4 N NaOH) was added to a final concentration of 70 mM $NH_2OH$ to the solution of **8b** in 22 mL citrate buffer pH 6.5. The deprotection reaction was incubated at room temperature for 6 h yielding a mixture of deprotected IL-1ra-linker conjugate **8c** and IL-1ra **8a**. The mixture was concentrated (Centrifugal Filter Units, Amicon Ultra 15, MWCO 10 kDa), buffer exchanged to pH 6.5 citrate buffer and filtered sterile through 0.22 μm syringe filter.

A final volume of 15 mL and an overall protein concentration of 22.06 mg/mL of the different IL-1ra species were obtained. The ratio of IL-1ra **8a** and the deprotected IL-1ra-linker conjugate **8c** is approx. 2/1 as determined by MS $[M+10H]^{10+}$.
MS (deprotected IL-1ra-linker **8c):** m/z 1755.29 = $[M+10H]^{10+}$, (calculated = 1755.24).

**Example 9**

**Synthesis of IL-lra-linker-hydrogel prodrug 9a and 9b**

33 mg maleimide functionalized hydrogel beads **7a** were washed five times with pH 6.5 citrate buffer. 3.4 mL of the IL-1ra **8a**/IL-1ra-linker conjugate **8c** mixture in pH 6.5 citrate buffer (22.06 mg overall protein content/mL) were added to the hydrogel and shaken overnight at room temperature. Hydrogel was washed 5 times with pH 6.5 citrate buffer. In order to quench residual maleimide groups, a solution of 2.4 μl mercaptoethanol in 3 mL pH 6.5 citrate buffer was added to the hydrogel and shaken for 1 h. Hydrogel was washed five times with pH 6.5 citrate buffer. Reduction of IL-1ra disulfide was performed by washing the hydrogel three times with DTT solution (0.1 M DTT in 90 % PBSTE and 10 % 0.5 M phosphate buffer pH 7.4, adjusted to pH 7.4). 3 mL DTT solution were drawn to the hydrogel and the suspension was incubated for 1 d at 37 °C. Hydrogel was washed twelve times with pH 6.5 citrate buffer. IL-1ra linker hydrogel prodrug **9a** was transferred in a tared vial and diluted with pH 6.5 citrate buffer in order to obtain a free flowing suspension.

IL-1RA loading of hydrogel was determined by analyzing aliquots of IL-1ra linker hydrogel prodrug **9a** by quantitative amino acid analysis (QAAA).
A loading of 0.67 mg IL1RA/mg hydrogel was obtained.

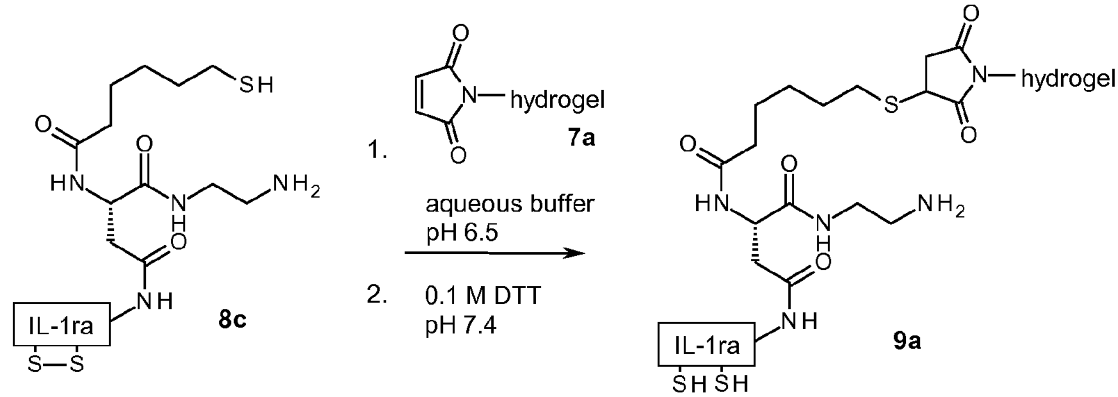


IL-1ra linker hydrogel prodrug **9b** was sythesized accordingly except for the use of 11 mg hydrogel beads **7b,** 3.5 mL of the IL-1ra **8a**/IL-1ra-linker conjugate **8c** mixture in pH 6.5 citrate buffer (22.06 mg/mL) and 0.8 μl mercaptoethanol.

A loading of 1.64 mg IL1RA/mg hydrogel was obtained.

## Example 10

### In vitro release kinetics-determination of in vitro half-life

Aliquots of IL-1ra-linker-hydrogel prodrug **9a** or **9b** (containing approximately 5 mg IL-1ra) were washed five times with PBSTE buffer and incubated in ca. 1 mL PBSTE at 37 C. The buffer was exchanged after different time intervals and released IL-1ra was quantified by SEC-HPLC at 220 nm. Peaks corresponding to liberated IL-1ra were integrated and the total amount of liberated IL-lrawas plotted against total incubation time. Curve fitting software (Graphpad Prism 5.04) was applied to determine first-order cleavage rates. A release half life time of 6 weeks was obtained.

Identity of released IL-1ra was confirmed by SEC-HPLC and MS. Released IL-1ra was reactive in the maleimide test (Example 8), thus confirming successful reduction of disulfide bond of oxidized IL-1ra on hydrogel.

## Example 11

### Chondroprotective effect of intra-articular injection of IL-1ra linker hydrogel prodrug in ACLT-induced arthritic rabbits

Arthritis was induced in 24 weeks old female Hyla NG rabbits (average weight 4.4 kg) by anterior cruciate ligament transection (ACLT) of the right knee. Four days after surgery animals were injected intraarticularly with IL-1ra linker hydrogel prodrug **9a** (7.5 mg IL-1ra content) in 350 μ1 citrate buffer pH 6.5 or with 350 μl citrate buffer alone (8 animals each, right knee). Eight weeks after injection, animals were sacrificed. The severity of macroscopic changes on cartilage of the right knee were graded by India Ink uptake. Intact cartilage shows no ink uptake, while increasing damage of cartilage correlates with increasing uptake of ink. In the IL-1ra group, the cartilage showed much less signs of degradation compared to the control group. Osteophyte formation on the medial condyle was less intense in the IL-1ra group compared to control group. In contrast to the control group, no signs of osteophyte formation were observed in the tibial plateau of the IL-1ra group.

### Abbreviations:

| | |
|---|---|
| Ac | acetyl |
| ACN | acetonitrile |
| AcOH | acetic acid |
| Asp | aspartate |
| Boc | *tert*-butyloxycarbonyl |
| DBU | 1,8-diazabicyclo (5.4.0)undec-7-ene |
| DCC | dicyclohexylcarbodiimide |
| DCM | dichloromethane |

| | |
|---|---|
| DIPEA | diisopropylethylamine |
| DMAP | dimethylaminopyridine |
| DMF | dimethylformamide |
| DMSO | dimethylsulfoxide |
| Fmoc | fluorenylmethyloxycarbonyl |
| HPLC | high performance liquid chromatography |
| iPrOH | isopropanol |
| Mal-PEG6-PFP | *N*-(3-maleimidopropyl)-22-amino-4,7,10,13,16,19-hexaoxa-heneicosanoic acid pentafluoro-phenyl ester |
| MeOAc | methyl acetate |
| MeOH | methanol |
| MS | mass spectrometry |
| MTBE | methyl-*tert*-butyl ether |
| NHS | N-hydroxysuccinimide |
| Oxyma Pure | ethyl 2-cyano-2-(hydroxyimino)acetate |
| PBSTE | PBS buffer containing 0.05 % polysorbate-20 and 5 mM EDTA |
| PEG | polyethyleneglycol |
| RP-HPLC | reversed phase - high performance liquid chromatography |
| RT | room temperature |
| *t*Bu | *tert*.-butyl |
| TAN | 1,5,9-triazanonane |
| TES | triethylsilane |
| TFA | trifluoroacetic acid |
| THF | tetrahydrofurane |
| TMEDA | *N*,*N*,*N'*,*N'*-tetramethylethylene diamine |
| TSTU | *O*-(*N*-succinimidyl)-*N*,*N*,*N'*,*N'*-tetramethyluronium tetrafluoroborate |

**Claims**

1. A hydrogel-linked IL-1ra prodrug or pharmaceutically acceptable salt thereof for use in a method of treating an inflammatory condition of the joint via intra-articular administration, of the formula L - D, wherein

   (i) - D is an IL-1ra moiety;
   and
   (ii) -L comprises a reversible prodrug linker moiety -$L^1$ represented by formula (I),

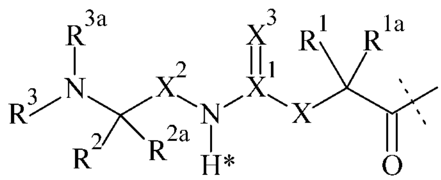

(I)

,

wherein the dashed line indicates the attachment to a nitrogen of D by forming an amide bond;
X is $C(R^4R^{4a})$; $N(R^4)$; O; $C(R^4R^{4a})$-$C(R^5R^{5a})$; $C(R^5R^{5a})$-$C(R^4R^{4a})$; $C(R^4R^{4a})$-$N(R^6)$; $N(R^6)$-$C(R^4R^{4a})$; $C(R^4R^{4a})$-O; O-$C(R^4R^{4a})$; or $C(R^7R^{7a})$;
$X^1$ is C; or S(O);
$X^2$ is $C(R^8R^{8a})$; or $C(R^8R^{8a})$-$C(R^9R^{9a})$;
$X^3$ is O; S; or N-CN;
$R^1$, $R^{1a}$, $R^2$, $R^{2a}$, $R^3$, $R^{3a}$, $R^4$, $R^{4a}$, $R^5$, $R^{5a}$, $R^6$, $R^8$, $R^{8a}$, $R^9$, $R^{9a}$ are independently selected from the group consisting of H; and $C_{1-6}$ alkyl;
$R^7$ is $N(R^{10}R^{10a})$; or $NR^{10}$-(C=O)-$R^{11}$;
$R^{7a}$, $R^{10}$, $R^{10a}$, $R^{11}$ are independently of each other H; or $C_{1-6}$ alkyl;
Optionally, one or more of the pairs $R^{1a}/R^{4a}$ , $R^{1a}/R^{5a}$, $R^{1a}/R^{7a}$, $R^{4a}/R^{5a}$ , $R^{8a}/R^{9a}$ form a chemical bond;
Optionally, one or more of the pairs $R^1/R^{1a}$, $R^2/R^{2a}$, $R^4/R^{4a}$, $R^5/R^{5a}$, $R^8/R^{8a}$, $R^9/R^{9a}$ are joined together with the atom to which they are attached to form a $C_{3-7}$ cycloalkyl; or 4- to 7-membered heterocyclyl;

Optionally, one or more of the pairs $R^1/R^4$, $R^1/R^5$, $R^1/R^6$, $R^1/R^{7a}$, $R^4/R^5$, $R^4/R^6$, $R^8/R^9$, $R^2/R^3$ are joined together with the atoms to which they are attached to form a ring A;
Optionally, $R^3/R^{3a}$ are joined together with the nitrogen atom to which they are attached to form a 4 to 7 membered heterocycle;
A is selected from the group consisting of phenyl; naphthyl; indenyl; indanyl; tetralinyl; $C_{3-10}$ cycloalkyl; 4- to 7-membered heterocyclyl; and 9- to 11-membered heterobicyclyl; and
wherein $L^1$ is substituted with one group $L^2$-Z and wherein $L^1$ is optionally further substituted, provided that the hydrogen marked with the asterisk is not replaced by $L^2$-Z or a substituent and that $R^3$ and $R^{3a}$ are independently of each other H or are connected to N through an $SP^3$-hybridized carbon atom;
wherein

$L^2$ is a single chemical bond or a spacer; and
Z is a hydrogel.

2. The prodrug for use in a method of claim 1, wherein X is $C(R^7R^{7a})$.

3. The prodrug for use in a method of claim 1 or 2, wherein $X^1$ is C.

4. The prodrug for use in a method of any one of claims 1 to 3, wherein $X^3$ is O.

5. The prodrug for use in a method of any one of claims 1 to 4, wherein $L^1$ is of formula (II)

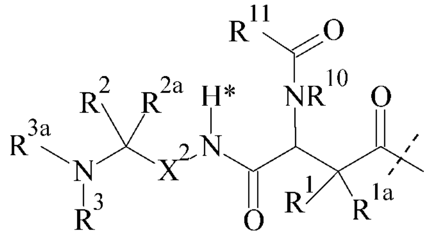

(II),

wherein

the dashed line indicates attachment to D;
$R^1$, $R^{1a}$, $R^2$, $R^{2a}$, $R^3$, $R^{3a}$, $R^{10}$, $R^{11}$ and $X^2$ are used as defined in claim 1;
and wherein $L^1$ is optionally further substituted, provided that the hydrogen marked with the asterisk is not replaced by a substituent and that $R^3$ and $R^{3a}$ are independently of each other H or are connected to N through an $SP^3$-hybridized carbon atom.

6. The prodrug for use in a method of any one of claims 1 to 5, wherein $X^2$ is $C(R^8R^{8a})$.

7. The prodrug for use in a method of any one of claims 1 to 6, wherein $X^2$ is $C(R^8R^{8a})-C(R^9R^{9a})$.

8. The prodrug for use in a method of any one of claims 1 to 7, wherein $L^1$ is of formula (IIIa) or (IIIb):

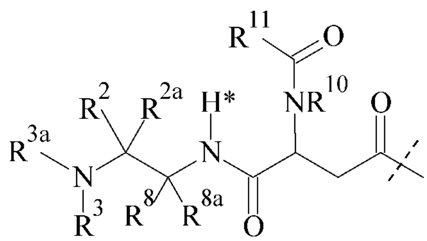

(IIIa),

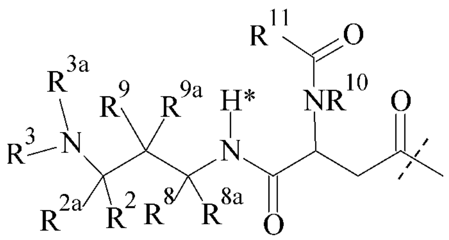

(IIIb),

wherein

the dashed line indicates attachment to D;

$R^2$, $R^{2a}$, $R^3$, $R^{3a}$, $R^8$, $R^{8a}$, $R^9$, $R^{9a}$, $R^{10}$, and $R^{11}$ are used as defined in claim 1;

and wherein $L^1$ is optionally further substituted, provided that the hydrogen marked with the asterisk is not replaced by a substituent and that $R^3$ and $R^{3a}$ are independently of each other H or are connected to N through an $SP^3$-hybridized carbon atom.

**9.** The prodrug for use in a method of any one of claims 1 to 8, wherein a hydrogen of $R^3$, $R^{3a}$, $R^{10}$, $R^{10a}$ or $R^{11}$ directly or as hydrogen of the $C_{1-6}$ alkyl or of a further substituent of $R^3$, $R^{3a}$, $R^{10}$, $R^{10a}$ or $R^{11}$ is replaced by $L^2$-Z.

**10.** The prodrug for use in a method of any one of claims 1 to 9, wherein $L^2$-Z is $-C(O)N(R^{17})-$; $-S(O)_2N(R^{17})-$; $-S(O)N(R^{17})-$; $-N(R^{17})S(O)_2N(R^{17a})-$; $-N(R^{17})-$; $-OC(O)R^{17}$; $-N(R^{17})C(O)-$; $-N(R^{17})S(O)_2-$; $-N(R^{17})S(O)-$; $-N(R^{17})C(O)O-$; $-N(R^{17})C(O)N(R^{17a})-$; $-OC(O)N(R^{17}R^{17a})-$; Q; $C_{1-50}$ alkyl; $C_{2-50}$ alkenyl; or $C_{2-50}$ alkynyl, wherein Q; $C_{1-50}$ alkyl; $C_{2-50}$ alkenyl; and $C_{2-50}$ alkynyl are optionally substituted with one or more $R^{18}$, which are the same or different and wherein $C_{1-50}$ alkyl; $C_{2-50}$ alkenyl; and $C_{2-50}$ alkynyl are optionally interrupted by one or more groups selected from the group consisting of Q, $-C(O)O-$; $-O-$; $-C(O)-$; $-C(O)N(R^{19})-$; $-S(O)_2N(R^{19})-$; $-S(O)N(R^{19})-$; $-S(O)_2-$; $-S(O)-$; $-N(R^{19})S(O)_2N(R^{19a})-$; $-S-$; $-N(R^{19})-$; $-OC(O)R^{19}$; $-N(R^{19})C(O)-$; $-N(R^{19})S(O)_2-$; $-N(R^{19})S(O)-$; $-N(R^{19})C(O)O-$; $-N(R^{19})C(O)N(R^{19a})-$; and $-OC(O)N(R^{19}R^{19a})$;

$R^{17}$, $R^{17a}$, $R^{17b}$ are independently selected from the group consisting of -H; Z; Q; $C_{1-50}$ alkyl; $C_{2-50}$ alkenyl; and $C_{2-50}$ alkynyl, wherein Q; $C_{1-50}$ alkyl; $C_{2-50}$ alkenyl; and $C_{2-50}$ alkynyl are optionally substituted with one or more $R^{17}$, which are the same or different and wherein $C_{1-50}$ alkyl; $C_{2-50}$ alkenyl; and $C_{2-50}$ alkynyl are optionally interrupted by one or more groups selected from the group consisting of Q, $-C(O)O-$; $-O-$; $-C(O)-$; $-C(O)N(R^{20})-$; $-S(O)_2N(R^{20})-$; $-S(O)N(R^{20})-$; $-S(O)_2-$; $-S(O)-$; $-N(R^{20})S(O)_2N(R^{20a})-$; $-S-$; $-N(R^{20})-$; $-OC(O)R^{20}$; $-N(R^{20})C(O)-$; $-N(R^{20})S(O)_2-$; $-N(R^{20})S(O)-$; $-N(R^{20})C(O)O-$; $-N(R^{20})C(O)N(R^{20a})-$; and $-OC(O)N(R^{20}R^{20a})$;

Q is selected from the group consisting of phenyl; naphthyl; indenyl; indanyl; tetralinyl; $C_{3-10}$ cycloalkyl; 4 to 7 membered heterocyclyl; and 9 to 11 membered heterobicyclyl, wherein Q is optionally substituted with one or more $R^{17}$, which are the same or different;

$R^{18}$ is Z; halogen; -CN; oxo; (=O); $-COOR^{21}$; $-OR^{21}$; $-C(O)R^{21}$; $-C(O)N(R^{21}R^{21a})$; $-S(O)_2N(R^{21}R^{21a})$; $-S(O)N(R^{21}R^{21a})$; $-S(O)_2R^{21}$; $-S(O)R^{21}$; $-N(R^{21})S(O)_2N(R^{21a}R^{21b})$; $-SR^{21}$; $-N(R^{21}R^{21a})$; $-NO_2$; $-OC(O)R^{21}$; $-N(R^{21})C(O)R^{21a}$; $-N(R^{21})S(O)_2R^{21a}$; $-N(R^{21})S(O)R^{21a}$; $-N(R^{21})C(O)OR^{21a}$; $-N(R^{21})C(O)N(R^{21a}R^{21b})$; $-OC(O)N(R^{21}R^{21a})$; or $C_{1-6}$ alkyl, wherein $C_{1-6}$ alkyl is optionally substituted with one or more halogen, which are the same or different;

$R^{19}$, $R^{19a}$, $R^{20}$, $R^{20a}$, $R^{21}$, $R^{21a}$ and $R^{21b}$ are independently selected from the group consisting of -H; Z; and $C_{1-6}$ alkyl, wherein $C_{1-6}$ alkyl is optionally substituted with one or more halogen, which are the same or different;

provided that one of $R^{17}$, $R^{17a}$, $R^{17b}$, $R^{18}$, $R^{19}$, $R^{19a}$, $R^{20}$, $R^{20a}$, $R^{21}$, $R^{21a}$ or $R^{21b}$ is Z.

**11.** The prodrug for use in a method of any one of claims 1 to 10, wherein Z is a PEG-based or hyaluronic acid-based hydrogel.

**12.** The prodrug for use in a method of any one of claims 1 to 11, wherein Z is a PEG-based hydrogel comprising at least 10 % PEG.

**13.** The prodrug for use in a method of any one of claims 1 to 12 for use in treating osteoarthritis.

**Patentansprüche**

1. Ein Hydrogel-verknüpftes IL-1ra-Prodrug oder ein pharmazeutisch akzeptables Salz davon zur Verwendung in einem Verfahren zur Behandlung eines Entzündungszustandes im Gelenk mittels intraartikulärer Gabe der Formel L-D, wobei

   (i) -D ein IL-lra-Rest ist;
   und
   (ii) -L einen reversiblen Prodruglinker-Rest $-L^1$ dargestellt durch Formel (I) enthält,

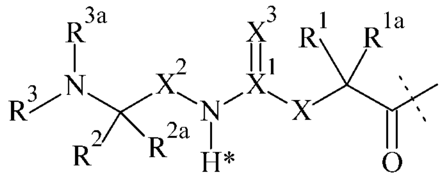

(I)

,

wobei die gestrichelte Linie die Anknüpfung an einen Stickstoff von D durch Bildung einer Amidbindung anzeigt;
X $C(R^4R^{4a})$; $N(R^4)$; O; $C(R^4R^{4a})-C(R^5R^{5a})$; $C(R^5R^{5a})-C(R^4R^{4a})$; $C(R^4R^{4a})-N(R^6)$; $N(R^6)-C(R^4R^{4a})$; $C(R^4R^{4a})-O$; $O-C(R^4R^{4a})$; oder $C(R^7R^{7a})$ ist;
$X^1$ C; oder S(O) ist;
$X^2$ $C(R^8R^{8a})$; oder $C(R^8R^{8a})-C(R^9R^{9a})$ ist;
$X^3$ O; S; oder N-CN ist;
$R^1$, $R^{1a}$, $R^2$, $R^{2a}$, $R^3$, $R^{3a}$, $R^4$, $R^{4a}$, $R^5$, $R^{5a}$, $R^6$, $R^8$, $R^{8a}$, $R^9$, $R^{9a}$ unabhängig ausgewählt sind aus der Gruppe bestehend aus H; und $C_{1-6}$-Alkyl;
$R^7$ $N(R^{10}R^{10a})$; oder $NR^{10}-(C=O)-R^{11}$ ist;
$R^{7a}$, $R^{10}$, $R^{10a}$, $R^{11}$ unabhängig voneinander H; oder $C_{1-6}$Alkyl sind;
optional eines oder mehrere der Paare $R^{1a}/R^{4a}$, $R^{1a}/R^{5a}$, $R^{1a}/R^{7a}$, $R^{4a}/R^{5a}$, $R^{8a}/R^{9a}$ eine chemische Bindung bilden;
optional eines oder mehrere der Paare $R^1/R^{1a}$, $R^2/R^{2a}$, $R^4/R^{4a}$, $R^5/R^{5a}$, $R^8/R^{8a}$, $R^9/R^{9a}$ mit dem Atom, an das sie gebunden sind, verbunden sind und ein $C_{3-7}$-Cycloalkyl; oder ein 4- bis 7-gliedriges Heterocyclyl bilden;
optional eines oder mehrere der Paare $R^1/R^4$, $R^1/R^5$, $R^1/R^6$, $R^1/R^{7a}$, $R^4/R^5$, $R^4/R^6$, $R^8/R^9$, $R^2/R^3$ mit dem Atom, an das sie gebunden sind, verbunden sind und einen Ring A bilden;
optional $R^3/R^{3a}$ verbunden sind mit dem Stickstoff, an den sie gebunden sind, und einen 4- bis 7-gliedrigen Heterocyclus bilden;
A ausgewählt ist aus der Gruppe bestehend aus Phenyl; Naphthyl; Indenyl; Indanyl; Tetralinyl; $C_{3-10}$-Cycloalkyl; 4- bis 7-gliedrigem Heterocyclyl; und 9- bis 11-gliedrigem Heterobicyclyl; und
wobei $L^1$ substituiert ist mit einer Gruppe $L^2$-Z und wobei $L^1$ optional weiter substituiert ist, vorausgesetzt, dass der mit dem Asterisk markierte Wasserstoff nicht ersetzt wird durch $L^2$-Z oder einen Substituenten und dass $R^3$ and $R^{3a}$ unabhängig voneinander H oder zu N über ein $SP^3$ -hybridisiertes Kohlenstoffatom verbunden sind; wobei

   $L^2$ eine einzelne chemische Bindung oder ein Spacer ist; und
   Z ein Hydrogel ist.

2. Das Prodrug zur Verwendung in einem Verfahren nach Anspruch 1, wobei X $C(R^7R^{7a})$ ist.

3. Das Prodrug zur Verwendung in einem Verfahren nach Anspruch 1 oder 2, wobei $X^1$ C ist.

4. Das Prodrug zur Verwendung in einem Verfahren nach einem der Ansprüche 1 bis 3, wobei $X^3$ O ist.

5. Das Prodrug zur Verwendung in einem Verfahren nach einem der Ansprüche 1 bis 4, wobei $L^1$ die Formel (II) hat

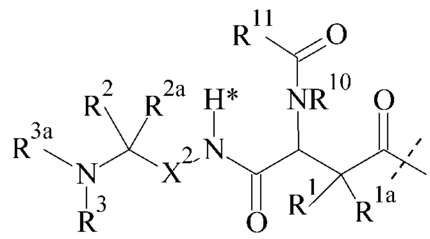

(II),

wobei

die gestrichelte Linie Anknüpfung an D anzeigt;
$R^1$, $R^{1a}$, $R^2$, $R^{2a}$, $R^3$, $R^{3a}$, $R^{10}$, $R^{11}$ und $X^2$ verwendet werden wie in Anspruch 1; und wobei $L^1$ optional weiter substituiert ist, vorausgesetzt, dass der mit dem Asterisk markierte Wasserstoff nicht ersetzt wird durch einen Substituenten und dass $R^3$ and $R^{3a}$ unabhängig voneinander H oder zu N über ein $SP^3$-hybridisiertes Kohlenstoffatom verbunden sind.

6. Das Prodrug zur Verwendung in einem Verfahren nach einem der Ansprüche 1 bis 5, wobei $X^2$ $C(R^8R^{8a})$ ist.

7. Das Prodrug zur Verwendung in einem Verfahren nach einem der Ansprüche 1 bis 6, wobei $X^2$ $C(R^8R^{8a})$-$C(R^9R^{9a})$ ist.

8. Das Prodrug zur Verwendung in einem Verfahren nach einem der Ansprüche 1 bis 7, wobei $L^1$ die Formel (IIIa) oder (IIIb) hat:

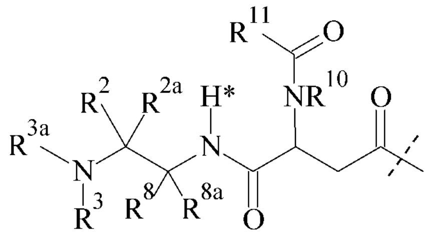

(IIIa),

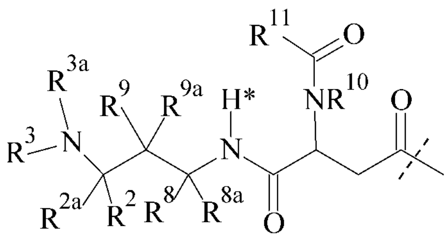

(IIIb),

wobei

die gestrichelte Linie Anknüpfung an D anzeigt;
$R^2$, $R^{2a}$, $R^3$, $R^{3a}$, $R^8$, $R^{8a}$, $R^9$, $R^{9a}$, $R^{10}$, und $R^{11}$ verwendet werden wie in Anspruch 1; und wobei $L^1$ optional weiter substituiert ist, vorausgesetzt, dass der mit dem Asterisk markierte Wasserstoff nicht ersetzt wird durch einen Substituenten und dass $R^3$ and $R^{3a}$ unabhängig voneinander H oder zu N über ein $SP^3$-hybridisiertes Kohlenstoffatom verbunden sind.

9. Das Prodrug zur Verwendung nach einem der Ansprüche 1 bis 8, wobei ein Wasserstoff von $R^{3a}$, $R^{10}$, oder $R^{11}$ direkt oder als Wasserstoff des $C_{1-6}$-Alkyls oder eines weiteren Substituenten von $R^3$, $R^{3a}$, $R^{10}$, oder $R^{11}$ durch $L^2$-Z ersetzt ist.

10. Das Prodrug zur Verwendung nach einem der Ansprüche 1 bis 9, wobei $L^2$-Z $-C(O)N(R^{17})$-; $-S(O)_2N(R^{17})$-;

-S(O)N($R^{17}$)-; -N($R^{17}$)S(O)$_2$N($R^{17a}$)-; -N($R^{17}$)-; -OC(O)$R^{17}$; -N($R^{17}$)C(O)-; -N($R^{17}$)S(O)$_2$-; -N($R^{17}$)S(O)-; -N($R^{17}$)C(O)O-; -N($R^{17}$)C(O)N($R^{17a}$)-; -OC(O)N($R^{17}R^{17a}$)-; Q; $C_{1-50}$-Alkyl; $C_{2-50}$-Alkenyl; oder $C_{2-50}$-Alkinyl ist, wobei Q; $C_{1-50}$-Alkyl; $C_{2-50}$-Alkenyl; und $C_{2-50}$-Alkinyl optional substituiert sind mit einem oder mehreren $R^{18}$, die gleich oder unterschiedlich sind, und wobei $C_{1-50}$-Alkyl; $C_{2-50}$-Alkenyl; und $C_{2-50}$-Alkinyl optional unterbrochen sind durch eine oder mehrere Gruppen ausgewählt aus der Gruppe bestehend aus Q, -C(O)O-; -O-; -C(O)-; -C(O)N($R^{19}$)-; -S(O)$_2$N($R^{19}$)-; -S(O)N($R^{19}$)-; -S(O)$_2$-; -S(O)-; -N($R^{19}$)S(O)$_2$N($R^{19a}$)-; -S-; -N($R^{19}$)-; -OC(O)$R^{19}$; -N($R^{19}$)C(O)-; -N($R^{19}$)S(O)$_2$-; -N($R^{19}$)S(O)-; -N($R^{19}$)C(O)O-; -N($R^{19}$)C(O)N($R^{19a}$)-; und -OC(O)N($R^{19}R^{19a}$);

$R^{17}$, $R^{17a}$, $R^{17b}$ sind unabhängig ausgewählt aus der Gruppe bestehend aus -H; Z; Q; $C_{1-50}$-Alkyl; $C_{2-50}$-Alkenyl; und $C_{2-50}$-Alkinyl, wobei Q; $C_{1-50}$-Alkyl; $C_{2-50}$-Alkenyl; und $C_{2-50}$-Alkinyl optional substituiert sind mit einem oder mehreren $R^{17}$, die gleich oder unterschiedlich sind, und wobei $C_{1-50}$-Alkyl; $C_{2-50}$-Alkenyl, und $C_{2-50}$-Alkinyl optional unterbrochen sind durch eine oder mehrere Gruppen ausgewählt aus der Gruppe bestehend aus Q, -C(O)O-; -O-; -C(O)-; -C(O)N($R^{20}$)-; -S(O)$_2$N($R^{20}$)-; -S(O)N($R^{20}$)-; -S(O)$_2$-; -S(O)-; -N($R^{20}$)S(O)$_2$N($R^{20a}$)-; -S-; -N($R^{20}$)-; -OC(O)$R^{20}$; -N($R^{20}$)C(O)-; -N($R^{20}$)S(O)$_2$-; -N($R^{20}$)S(O)-; -N($R^{20}$)C(O)O-; -N($R^{20}$)C(O)N($R^{20a}$)-; und -OC(O)N($R^{20}R^{20a}$);

Q ist ausgewählt aus der Gruppe bestehend aus Phenyl; Naphthyl; Indenyl; Indanyl; Tetralinyl; $C_{3-10}$-Cycloalkyl; 4- bis 7-gliedrigem Heterocyclyl; und 9- bis 11-gliedrigem Heterobicyclyl, wobei Q optional substituiert ist mit einem oder mehreren $R^{17}$, die gleich oder unterschiedlich sind;

$R^{18}$ ist Z; Halogen; -CN; oxo (=O); -COOR$^{21}$; -OR$^{21}$; -C(O)$R^{21}$; -C(O)N($R^{21}R^{21a}$). -S(O)$_2$N($R^{21}R^{21a}$); -S(O)N($R^{21}R^{21a}$); -S(O)$_2R^{21}$; -S(O)$R^{21}$; -N($R^{21}$)S(O)$_2$N($R^{21a}R^{21b}$); -SR$^{21}$; -N($R^{21}R^{21a}$); -NO$_2$; -OC(O)$R^{21}$; -N($R^{21}$)C(O)$R^{21a}$; -N($R^{21}$)S(O)$_2R^{21a}$; -N($R^{21}$)S(O)$R^{21a}$; -N($R^{21}$)C(O)OR$^{21a}$; -N($R^{21}$)C(O)N($R^{21a}R^{21b}$); -OC(O)N($R^{21}R^{21a}$); oder $C_{1-6}$-Alkyl, wobei $C_{1-6}$-Alkyl optional substituiert ist mit einem oder mehreren Halogen, die gleich oder unterschiedlich sind;

$R^{19}$, $R^{19a}$, $R^{20}$, $R^{20a}$, $R^{21}$, $R^{21a}$ und $R^{21b}$ sind unabhängig ausgewählt aus der Gruppe bestehend aus -H; Z; und $C_{1-6}$-Alkyl, wobei $C_{1-6}$-Alkyl optional substituiert ist mit einem oder mehreren Halogen, die gleich oder unterschiedlich sind;

vorausgesetzt, dass einer von $R^{17}$, $R^{17a}$, $R^{17b}$, $R^{18}$, $R^{19}$, $R^{19a}$, $R^{20}$, $R^{20a}$, $R^{21}$, $R^{21a}$ oder $R^{21b}$ Z ist.

**11.** Das Prodrug zur Verwendung in einem Verfahren nach einem der Ansprüche 1 bis 10, wobei Z ein PEG-basiertes oder ein Hyaluronsäure-basiertes Hydrogel ist.

**12.** Das Prodrug zur Verwendung in einem Verfahren nach einem der Ansprüche 1 bis 11, wobei Z ein PEG-basierte Hydrogel ist, das mindestens 10% PEG enthält.

**13.** Das Prodrug zur Verwendung in einem Verfahren nach einem der Ansprüche 1 bis 12 zur Verwendung bei der Behandlung von Osteoarthritis.

## Revendications

**1.** Promédicament de type IL-Ira (antagoniste du récepteur de l'interleukine 1) lié à un hydrogel ou sel pharmaceutiquement acceptable de celui-ci pour une utilisation dans un procédé de traitement d'une affection inflammatoire de l'articulation par administration intra-articulaire, de formule L-D,

(i) -D étant un groupement de type IL-Ira;
et
(ii) -L comprenant un groupement de liaison de promédicament réversible -$L^1$ représenté par la formule (I),

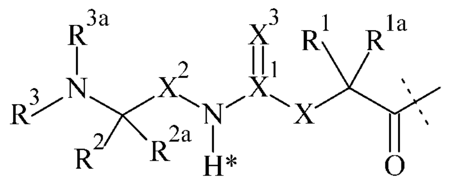 (I)

,

la ligne pointillée indiquant le site d'attache à un azote de D par formation d'une liaison amide;

X représentant C($R^4R^{4a}$); N($R^4$); O; C($R^4R^{4a}$)-C($R^5R^{5a}$); C($R^5R^{5a}$)-C($R^4R^{4a}$); C($R^4R^{4a}$)-N($R^6$); N($R^6$)-C($R^4R^{4a}$); C($R^4R^{4a}$)-O; O-C($R^4R^{4a}$); ou C($R^7R^{7a}$);

$X^1$ représentant C; ou S(O);

$X^2$ représentant $C(R^8R^{8a})$; ou $C(R^8R^{8a})$-$C(R^9R^{9a})$;
$X^3$ représentant O; S; ou N-CN;
$R^1$, $R^{1a}$,$R^2$, $R^{2a}$, $R^3$, $R^{3a}$, $R^4$, $R^{4a}$, $R^5$, $R^{5a}$, $R^6$, $R^8$, $R^{8a}$, $R^9$, $R^{9a}$ étant indépendamment choisis dans le groupe constitué par H; et $C_{1-6}$-alkyle;
$R^7$ représentant $N(R^{10}R^{10a})$; ou $NR^{10}(C=O)$-$R^{11}$;
$R^{7a}$, $R^{10}$, $R^{10a}$, $R^{11}$ étant indépendamment les uns des autres H; ou $C_{1-6}$-alkyle;
éventuellement, une ou plusieurs des paires $R^{1a}/R^{4a}$, $R^{1a}/R^{5a}$, $R^{1a}/R^{7a}$, $R^{4a}/R^{5a}$, $R^{8a}/R^{9a}$ formant une liaison chimique;
éventuellement, une ou plusieurs des paires $R^1/R^{1a}$, $R^2/R^{2a}$, $R^4/R^{4a}$, $R^5/R^{5a}$, $R^8/R^{8a}$, $R^9/R^{9a}$ étant reliées, conjointement avec l'atome auquel elles sont attachées, pour former un groupe $C_{3-7}$-cycloalkyle; ou un groupe hétérocyclyle à 4 jusqu'à 7 chaînons;
éventuellement, une ou plusieurs des paires $R^1/R^4$, $R^1/R^5$, $R^1/R^6$, $R^1/R^{7a}$, $R^4/R^5$, $R^4/R^6$, $R^8/R^9$, $R^2/R^3$ étant reliées, conjointement avec l'atome auquel elles sont attachées, pour former un cycle A;
éventuellement, $R^3/R^{3a}$ étant reliés, conjointement avec l'atome auquel ils sont attachés, pour former un hétérocycle à 4 jusqu'à 7 chaînons;
A étant choisi dans le groupe constitué par phényle; naphtyle; indényle; indanyle; tétralinyle; $C_{3-10}$-cycloalkyle; hétérocyclyle à 4 jusqu'à 7 chaînons; et hétérobicyclyle à 9 jusqu'à 11 chaînons; et
$L^1$ étant substitué par un groupe $L^2$-Z et $L^1$ étant éventuellement substitué davantage, à condition que l'hydrogène signalé par l'astérisque ne soit pas remplacé par $L^2$-Z ou un substituant et que $R^3$ et $R^{3a}$ soient indépendamment l'un de l'autre H ou soient reliés à N par l'intermédiaire d'un atome de carbone à hybridation $sp^3$;

$L^2$ étant une liaison chimique simple ou un espaceur; et
Z étant un hydrogel.

2. Promédicament pour une utilisation dans un procédé selon la revendication 1, X représentant $C(R^7R^{7a})$.

3. Promédicament pour une utilisation dans un procédé selon la revendication 1 ou 2, $X^1$ représentant C.

4. Promédicament pour une utilisation selon l'une quelconque des revendications 1 à 3, $X^3$ étant O.

5. Promédicament pour une utilisation selon l'une quelconque des revendications 1 à 4, $L^1$ étant de formule (II)

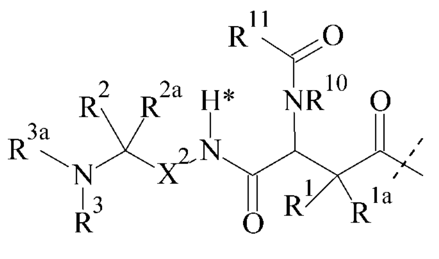

(II),

la ligne pointillée indiquant le site d'attache à D;
$R^1$, $R^{1a}$, $R^2$, $R^{2a}$, $R^3$, $R^{3a}$, $R^{10}$, $R^{11}$ et $X^2$ étant tels que définis selon la revendication 1; et $L^1$ étant éventuellement substitué davantage, à condition que l'hydrogène signalé par l'astérisque ne soit pas remplacé par un substituant et que $R^3$ et $R^{3a}$ soient indépendamment l'un de l'autre H ou soient reliés à N par l'intermédiaire d'un atome de carbone à hybridation $sp^3$.

6. Promédicament pour une utilisation dans un procédé selon l'une quelconque des revendications 1 à 5, $X^2$ représentant $C(R^8R^{8a})$.

7. Promédicament pour une utilisation dans un procédé selon l'une quelconque des revendications 1 à 6, $X^2$ représentant $C(R^8R^{8a})$-$C(R^9R^{9a})$.

8. Promédicament pour une utilisation dans un procédé selon l'une quelconque des revendications 1 à 7, $L^1$ étant de formule (IIIa) ou (IIIb):

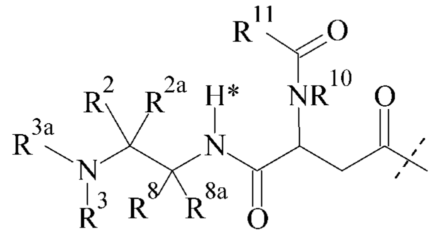

(IIIa),

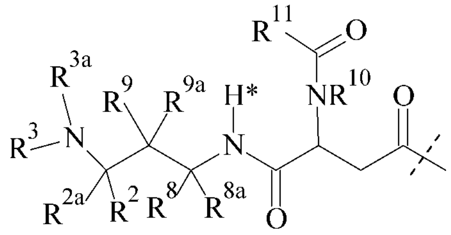

(IIIb),

la ligne pointillée indiquant le site d'attache à D;
$R^2$, $R^{2a}$, $R^3$, $R^{3a}$, $R^8$, $R^{8a}$, $R^9$, $R^{9a}$, $R^{10}$ et $R^{11}$ étant utilisés comme définis selon la revendication 1;
et $L^1$ étant éventuellement substitué davantage, à condition que l'hydrogène signalé par l'astérisque ne soit pas remplacé par un substituant et que $R^3$ et $R^{3a}$ soient indépendamment l'un de l'autre H ou soient reliés à N par l'intermédiaire d'un atome de carbone à hybridation $sp^3$.

**9.** Promédicament pour une utilisation dans un procédé selon l'une quelconque des revendications 1 à 8, un hydrogène de $R^3$, $R^{3a}$, $R^{10}$, ou $R^{11}$, directement, ou bien un hydrogène du groupe $C_{1-6}$-alkyle ou d'un autre substituant de $R^3$, $R^{3a}$, $R^{10}$, $R^{10a}$ ou $R^{11}$ étant remplacé par $L^2$-Z.

**10.** Promédicament pour une utilisation dans un procédé selon l'une quelconque des revendications 1 à 9, L2-Z représentant un groupe -C(O)N(R17)-; -S(O)2N(R17)-; -S(O)N(R17)-; -N(R17)S(O)2N(R17a)-; -N(R17)-; -OC(O)R17-; -N(R17)C(O)-; -N(R17)S(O)2-; -N(R17)S(O)-; -N(R17)C(O)O-; -N(R17)C(O)N(R17a)-; -OC(O)N(R17R17a)-; Q; C1-50-alkyle; C2-50-alcényle; ou C2-50-alcynyle, Q; C1-50-alkyle; C2-50-alcényle; et C2-50-alcynyle étant éventuellement substitués par un ou plusieurs R18 identiques ou différents et C1-50-alkyle; C2-50-alcényle; et C2-50-alcynyle étant éventuellement entrecoupés par un ou plusieurs groupes choisis dans le groupe constitué par Q; -C(O)O-; -O-; -C(O)-; -C(O)N(R19)-; -S(O)2N(R19)-; -S(O)N(R19)-; $-S(O)_2-$; -S(O)-; -N(R19)S(O)2N(R19a)-; -S-; -N(R19)-; -OC(O)R19-; -N(R19)C(O)-; -N(R19)S(O)2-; -N(R19)S(O)-; -N(R19)C(O)O-; -N(R19)C(O)N(R19a)-; et -OC(O)N(R19R19a);
$R^{17}$, $R^{17a}$ $R^{17b}$ étant indépendamment choisis dans le groupe constitué par -H; Z; Q; $C_{1-50}$-alkyle; $C_{2-50}$-alcényle; et $C_{2-50}$-alcynyle, Q, $C_{1-50}$-alkyle; $C_{2-50}$-alcényle; et $C_{2-50}$-alcynyle étant éventuellement substitués par un ou plusieurs $R^{17}$ identiques ou différents, et $C_{1-50}$-alkyle; $C_{2-50}$-alcényle; et $C_{2-50}$-alcynyle étant éventuellement interrompus par un ou plusieurs groupes choisis dans le groupe constitué par Q; -C(O)O-; -O-; -C(O)-; $-C(O)N(R^{20})-$; $-S(O)_2N(R^{20})-$; $-S(O)N(R^{20})-$; $-S(O)_2-$; -S(O)-; $-N(R^{20})S(O)_2N(R^{20a})-$; -S-; $-N(R^{20})-$; $-OC(O)R^{20}-$; $-N(R^{20})C(O)-$; $-N(R^{20})S(O)_2-$; $-N(R^{20})S(O)-$; $-N(R^{20})C(O)O-$; $-N(R^{20})C(O)N(R^{21a})-$; et $-OC(O)N(R^{20}R^{20a})$;
Q étant choisi dans le groupe constitué par phényle; naphtyle; indényle; indanyle; tétralinyle; $C_{3-10}$-cycloalkyle; hétérocyclyle à 4 jusqu'à 7 chaînons; et hétérobicyclyle à 9 jusqu'à 11 chaînons; Q étant éventuellement substitué par un ou plusieurs $R^{17}$ identiques ou différents;
$R^{18}$ représentant Z; halogène; -CN; oxo; (=O); $-COOR^{21}$; $-OR^{21}$; $-C(O)R^{21}$; $-C(O)N(R^{21}R^{21a})$; $-S(O)_2N(R^{21}R^{21a})$; $-S(O)N(R^{21}R^{21a})$; $-S(O)_2R^{21}$; $-S(O)R^{21}$; $-N(R^{21})S(O)_2N(R^{21a}R^{21b})$; $-SR^{21}$; $-N(R^{21}R^{21a})$; $-NO_2$; $-OC(O)R^{21}$; $-N(R^{21})C(O)R^{21a}$; $-N(R^{21})S(O)_2R^{21a}$. $-N(R^{21})S(O)R^{21a}$; $-N(R^{21})C(O)OR^{21a}$; $-N(R^{21})C(O)N(R^{21a}R^{21b})$; $-OC(O)N(R^{21}R^{21a})$. ou $C_{1-6}$-alkyle, $C_{1-6}$-alkyle étant éventuellement substitué par un ou plusieurs halogènes identiques ou différents;
$R^{19}$, $R^{19a}$, $R^{20}$, $R^{20a}$, $R^{21}$, $R^{21a}$ et $R^{21b}$ étant indépendamment choisis dans le groupe constitué par -H; Z; et $C_{1-6}$-alkyle, $C_{1-6}$-alkyle étant éventuellement substitué par un ou plusieurs halogènes identiques ou différents;
à condition que l'un de $R^{17}$, $R^{17a}$, $R^{17b}$, $R^{18}$, $R^{19}$, $R^{19a}$, $R^{20}$, $R^{20a}$, $R^{21}$, $R^{21a}$ ou $R^{21b}$ représente Z.

**11.** Promédicament pour une utilisation dans un procédé selon l'une quelconque des revendications 1 à 10, Z représentant un hydrogel à base de PEG (polyéthylèneglycol) ou à base d'acide hyaluronique.

**12.** Promédicament pour une utilisation dans un procédé selon l'une quelconque des revendications 1 à 11, Z représentant un hydrogel à base de PEG comprenant au moins 10% de PEG.

**13.** Promédicament pour une utilisation dans un procédé selon l'une quelconque des revendications 1 à 12 pour une utilisation dans le traitement de l'arthrose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9822130 A
- WO 9728828 A
- US 2002009454 A
- US 20010007673 A
- EP 0975334 A
- US 2008019940 A
- WO 2005097195 A
- US 5075222 A
- WO 9108285 A
- WO 9117184 A
- AU 9173636
- WO 9216221 A
- WO 9622793 A
- US 673375 A
- US 20130209396 A1
- WO 2006003014 A
- WO 2011012715 A
- WO 2011012715 A1
- WO 2011012718 A


### Non-patent literature cited in the description


- **CHEVALIER et al.** Intraarticular Injection of Anakinra in Osteoarthritis of the Knee: A Multicenter, Randomized, Double-Blind, Placebo-Controlled Study. *Arthritis & Rheumatism,* 2009, vol. 61 (3), 344-352
- **BENDELE A. ; MCABEE T. ; WOODWARD M. ; SCHERRER J. ; COLLINS D. ; FRAZIER J. ; CHLIPALA E. ; MCCABE D.** Effects of interleukin-1 receptor antagonist in a slow-release hylan vehicle on rat type II collagen arthritis. *Pharm Res,* 1998, vol. 15 (10), 1557-61
- **BAR D. ; APTE R. N. ; VORONOV E. ; DINARELLO C. A. ; COHEN S.** A continuous delivery system of IL-1 receptor antagonist reduces angiogenesis and inhibits tumor development. *FASEB J,* 2004, vol. 18 (1), 161-3
- **LAVI G. ; VORONOV E. ; DINARELLO C. A. ; APTE R. N. ; COHEN S.** *J Control Release,* 2007, vol. 123 (2), 123-30
- **GUDE et al.** *Letters in Peptide Science,* 2002, vol. 9 (4), 203-206
- **ZHANG et al.** *Nature Biotechnology,* 2013, vol. 31 (6), 553-557